(19) Europäisches Patentamt
European Patent Office
Office européen des brevets

(11) EP 4 373 506 B1

(12) **EUROPEAN PATENT SPECIFICATION**

(45) Date of publication and mention of the grant of the patent:
**24.06.2026 Bulletin 2026/26**

(21) Application number: **22751398.3**

(22) Date of filing: **20.07.2022**

(51) International Patent Classification (IPC):
***A61K 36/05*** *(2006.01)* ***A61K 9/51*** *(2006.01)*
***C12N 15/88*** *(2006.01)* ***C12N 15/00*** *(2006.01)*
***A61K 48/00*** *(2006.01)*

(52) Cooperative Patent Classification (CPC):
**A61K 8/14; A61K 8/606; A61K 8/9722; A61K 9/5184; A61K 36/05; A61P 1/00; A61Q 19/00; C12N 1/12; C12N 15/111; C12N 15/113; C12N 15/8281; C12N 15/88;**
A61K 31/713; A61K 2800/10; C12N 2310/14;
(Cont.)

(86) International application number:
**PCT/EP2022/070371**

(87) International publication number:
**WO 2023/001894 (26.01.2023 Gazette 2023/04)**

(54) **EXTRACELLULAR VESICLES FROM MICROALGAE, THEIR PREPARATION, AND USES**

EXTRAZELLULÄRE VESIKEL AUS MIKROALGEN, IHRE HERSTELLUNG UND VERWENDUNGEN

VÉSICULES EXTRACELLULAIRES PROVENANT DE MICROALGUES, LEUR PRÉPARATION ET LEURS UTILISATIONS

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **20.07.2021 US 202163223828 P**
**22.07.2021 US 202163224656 P**
**31.01.2022 US 202263305230 P**

(43) Date of publication of application:
**29.05.2024 Bulletin 2024/22**

(60) Divisional application:
**26179596.7**

(73) Proprietors:
- **AGS Therapeutics SAS**
  **75003 Paris (FR)**
- **AGS-M SAS**
  **44000 Nantes (FR)**
- **Nantes Université**
  **44035 Nantes (FR)**

(72) Inventors:
- **DRITTANTI, Lila**
  **75011 PARIS (FR)**
- **VEGA, Juan Pablo**
  **93300 Aubervilliers (FR)**
- **PRUVOST, Jeremy**
  **44250 Saint-Brevin-les-Pins (FR)**
- **VEGA, Manuel**
  **75011 PARIS (FR)**

(74) Representative: **Boult Wade Tennant LLP**
**Salisbury Square House**
**8 Salisbury Square**
**London EC4Y 8AP (GB)**

(56) References cited:
**EP-A1- 3 967 745** **EP-A1- 3 967 746**
**WO-A1-2017/161010**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **PICCIOTTO S. ET AL.: "Isolation of extracellular vesicles from microalgae: towards the production of sustainable and natural nanocarriers of bioactive compounds", BIOMATERIALS SCIENCE, vol. 9, no. 8, 20 April 2021 (2021-04-20), GB, pages 2917 - 2930, XP055979612, ISSN: 2047-4830, Retrieved from the Internet <URL:https://pubs.rsc.org/en/content/articlepdf/2021/bm/d0bm01696a> DOI: 10.1039/D0BM01696A**
- **KURUVINASHETTI K. ET AL.: "Algal Extracellular Vesicles for Therapeutic Applications", 2020 IEEE 20TH INTERNATIONAL CONFERENCE ON NANOTECHNOLOGY (IEEE-NANO), IEEE, 29 July 2020 (2020-07-29), pages 354 - 357, XP033817380, DOI: 10.1109/NANO47656.2020.9183452**


Remarks:

The complete document including Reference Table(s) and the Sequence Listing(s) can be downloaded from the EPO website

(52) Cooperative Patent Classification (CPC): (Cont.)
C12N 2320/32

## Description

### Field

Provided are compositions containing extracellular vesicles from microalgae (MEVs) that are exogenously loaded with bioactive cargo and/or other cargo of interest, such as for therapeutic, industrial, diagnostic, and cosmetic uses. The MEVs have a variety of applications as therapeutics, including as vaccines, as anti-cancer agents, as diagnostics, and other such uses.

### Background

Extracellular vesicles (EVs) are natural particles produced by most cells. EVs include exosomes (about 30-120 nm in size), which are released to the extracellular environment upon fusion of multivesicular endosomes with the plasma membrane, and include microvesicles (about 50-1000 nm), which are produced by the outward budding of membrane vesicles from the cell surface. Exosomes and microvesicles have similar properties, and in general are referred to as EVs.

EVs function in intercellular communication via cell-cell transfer of proteins and nucleic acids, such as micro-RNAs (miRNAs), long noncoding RNAs (lncRNAs), and mRNAs. By virtue of this, EVs derived from mammals and plants have been used as carriers for short interfering RNA (siRNA) delivery, microRNA (miRNA), and small molecule drugs. They can be used as delivery vehicles. There is a need for conveniently produced EVs that are readily delivered to cells and tissues through a variety of routes of administration and that can target or deliver cargo to cells, organs, and tissues of interest. It is an object herein to provide such EVs.

### SUMMARY

The scope of the invention is defined by the appended claims. In particular, the present invention provides a composition comprising isolated microalgae extracellular vesicles (MEVs), wherein:

the microalgae is a species of the family Chlorellaceae;
the composition is formulated for administration to a subject;
the MEVs in the composition comprise heterologous cargo;
the cargo is not RNAi; and
the subject is an animal.

The invention relates to exogenously-loaded microalgae extracellular vesicles. They are loaded with cargo that includes bioactive molecules, including biomolecules and small molecules. The bioactive molecules include, but are not limited to, molecules used as therapeutics, cosmetics, diagnostics, in industry, and other uses. The EVs are from microalgae, which are unicellular green algae, in particular those that belong to the *Chlorellaceae* family, and in particular those that belong to the *Chlorella* genus, such as *Chlorella vulgaris.* Microalgae extracellular vesicles (MEVs) can be manufactured in large scale. MEVs are exogenously loaded (exo-loaded) with the bioactive molecule cargo. The resulting compositions, thus, contain MEVs that have the same exogenously-loaded heterologous cargo. An advantage of exogenously loading (exo-loading) cargo into MEVs is that the amount of cargo/MEV can be controlled, and distribution of the exogenous cargo in the MEVs is predictable, and substantially uniform, such that the average cargo molecule or amount of cargo/MEV can be known. A large variety of bioactive molecules, including biomolecules and small molecules, such as drug and organic compounds, can be loaded into the MEVs. It is shown herein that MEVs can be loaded with cargo, that the resulting MEVs are not toxic, that they can be administered into cells *in vitro,* can be administered *in vivo* and have distribution patterns that depend upon the route of administration. Unlike most extracellular vesicles (EVs) from other sources, such as mammalian EVs, they can be administered orally. Upon oral administration they traffic through the GALT and end up in the spleen; they are not degraded by digestive processes as are EVs from other sources.

The MEVs are loaded with a variety of cargos (also referred to as "payloads"), including, but not limited to, RNA, oligonucleotides, plasmids, peptides, proteins, and small molecules, provided that the cargo is not RNAi. The MEVs can deliver the cargo to organs, tissues, and cells, and can be targeted by the route of delivery, where they can be delivered. It is shown herein that the MEVs, including the *Chlorella* MEVs, have a striking capacity to pass through stringent natural barriers, such as the digestive tract, and olfactory neurons, that are not shared by other extracellular vesicles (EVs) from other sources, including mammalian EVs.

As described herein, the MEVs can be exogenously loaded (exo-loaded) with a diversity and variety of molecules, particularly biologically active molecules, such as siRNA, mRNA, plasmids, anti-sense oligonucleotides (ASOs), peptides, proteins, and small molecules, which allows for a variety of therapeutic and diagnostic and other uses. As shown herein, MEV biodistribution, upon administration, is determined by the route of administration. Thus, MEVs can

deliver their cargo to a variety of tissues and organs, including, for example, the lungs, to the intestine, to the GALT, to the spleen, to the liver, and the brain, depending on whether they are administered intratracheally, orally, intravenously, intranasally, or other route.

As demonstrated and described herein, the MEVs have many uses, including therapeutic uses, such as, but not limited to, delivery of therapeutics for treatment and/or prevention (including reducing the risk or severity) of diseases, disorders, and conditions. These uses include therapeutic uses, including immunomodulation, immuno-oncology, and treatment of genetic disorders, metabolic disorders, neurologic disorders, psychiatric disorders, and respiratory disorders, among others.

Described is a microalgae extracellular vesicle (MEVs), comprising an exogenously-loaded (exo-loaded) bioactive molecule, such as, but not limited to, a protein, polypeptide, peptide, nucleic acid, and small molecule, or a plurality thereof. Compositions comprising the MEVs, and comprising mixtures of MEVs containing different cargo, or comprising MEVs containing mixtures of cargo are provided.

Provided are compositions that contain exogenously cargo-loaded MEVs, particularly those produced by the *Chlorellaceae* family, and in particular the *Chlorella* genus, such as *Chlorella vulgaris.* Methods for loading the MEVs are provided. The cargos are bioactive molecules or combinations thereof, including biomolecules and small molecules. The cargos include, for example, biomolecules, including biopolymers, such as DNA and RNA, proteins, protein complexes, protein-nucleic acid complexes, plasmids, and also include small molecules, such as small molecule drugs, provided that the cargo is not RNAi. The bioactive molecules include therapeutics, such as anti-cancer compounds and biomolecules, such as oligonucleotides, and proteins, and complexes, and diagnostic molecules, such as detectable markers, molecules that are cosmetics, and molecules that act as antiinfectives for humans, animals and plants. The compositions can be formulated for administration to a subject. Compositions for use in methods of treatment of diseases and disorders, including pathogen infections and cancers are provided.

The resulting cargo-loaded MEVs have applications in a variety of fields, including diagnosis, prophylaxis, treatment of human and other animal diseases, industrial uses, cosmetics, veterinary uses, and the crop industry. The MEVs, with appropriate cargo for each application, can be used as vaccines, as gene therapy delivery vectors, for gene silencing, gene editing, transfection for industry and research, analytical methods, cell-based assays, and other uses and applications.

The cargo-loaded MEVs can be used for a variety of uses, including treatment of diseases, disorders, and conditions, industrial, and cosmetic uses. Diseases, disorders, and conditions, include, but not limited to: genetic disorders, disorders of the digestive tract, disorders of the respiratory tract, disorders of the central nervous system (CNS), disorders of the skin, including natural disorders, and disorders induced by trauma, disorders of the urogenital tract, disorders of the naso-buccal cavity, disorders of the cardiovascular system, immune and immunomodulatory disorders, cancers, ocular disorders, disorders of the liver, systemic disorders, diseases, disorders, and conditions caused by or involving a pathogen, such as a bacterium, virus, or parasite.

Target tissues for treatment/delivery include, for example, epithelia and mucosa cells (any kind of either external or internal mucosa: mouth, gut, uterus, trachea, bladder, and others), endothelial cells, sensory cells (visual, auditory), cancer cells, tumor cells, blood cells, blood cell precursors, neural system cells (neurons, glial cells and other CNS and peripheral nervous cells), cells of the immune system (lymphocytes, immuno-regulatory cells, effector cells), germ cells, secretory cells, gland cells, muscle cells, stem cells, including, for example, embryonic or tissue specific stem cells, liver cells, infected cells, such as cells infected with virus, bacteria, fungi, or other pathogens, native cells, and genetically engineered NS cells.

Hence, provided are compositions that contain isolated microalgae extracellular vesicles (MEVs), where the microalgae can be a species of the genus *Chlorella;* and the composition is formulated for administration to a subject. The *Chlorella* extracellular vesicles can contain cargo (or a payload) that is heterologous bioactive cargo molecule that has been introduced into the isolated extracellular vesicles, whereby the vesicles in the composition that contain heterologous bioactive molecule cargo contain substantially the same bioactive molecule cargo, where: the cargo molecule is heterologous to *Chlorella;* and the bioactive cargo includes biomolecules or a small molecule, such as small molecule drugs and reagents.

For all embodiments, the *Chlorella* is any species of *Chlorella,* such as, but are not limited to, *Chlorella* selected from among *Chlorella ellipsoidea, Chlorella pyrenoidosa, Chlorella sorokiniana, Chlorella vulgaris,* and *Chlorella variabilis.* In particular embodiments, the *Chlorella* is *Chlorella vulgaris.*

Provided are compositions that contain isolated microalgae extracellular vesicles (MEVs), where the microalgae is a species of *Chlorella;* the MEVs in the composition contain heterologous bioactive molecule cargo that has been introduced into the isolated MEVs, whereby the vesicles in the composition that contain the heterologous bioactive molecule cargo contain substantially the same loaded cargo. The cargo is heterologous, not endogenous, to *Chlorella;* and the cargo is a biomolecule or a small molecule drug or other such molecule, including detectable reagents, such as a fluorescent protein or a luciferase. Each of the MEVs that contain cargo can comprise a plurality of different heterologous cargos.

The cargo includes any molecules that are intended for delivery to or on an animal. In general, the cargo is bioactive. Bioactive cargo include, for example, any molecules, such as biomolecules, including biopolymers, and small molecules, that can have an effect on an animal when administered. Cargo includes, for example, proteins, peptides, and nucleic acids. The bioactive molecules can be synthetic, naturally-occurring, and modified to alter a property or activity. Included are any molecules that have been used as drugs or therapeutics or diagnostics or cosmetic or in industry. The cargo can be, but are not limited to, a therapeutic for treating or preventing a disease or condition, or treating or preventing a symptom thereof. The cargo can be a nucleic acid molecule, a polypeptide, a protein, a plasmid, an aptamer, or an antisense oligonucleotide.

The cargo in the MEVs in the compositions can comprise a biopolymer. Biopolymers include a naturally-occurring biopolymer, or a synthetic biopolymer, or a modified biopolymer. The biopolymer can be a nucleic acid or protein that includes modifications, where the modifications comprise insertions, deletions, replacements, and transpositions of nucleotides or amino acid residues, and/or, where the biopolymer is a protein, the modifications also can comprise post-translational modifications. Post-translational modifications include, but are not limited to, glycosylation, hyper-glycosylation, PEGylation, sialylation, albumination, other half-life extending moieties, and other modifications that improve or alter pharmacological dynamic or kinetic properties of the protein.

Nucleic acids, such as DNA and RNA, are among the molecules that can be cargo. If the cargo is RNA or protein, it can be provided as the cargo or it can be encoded by nucleic acid that then is expressed in the organism to whom it is administered. Exemplary of RNA is inhibitory RNA (RNAi) and mRNA, including modified mRNA. RNAi, includes, for example, silencing RNA (siRNA) or short-hairpin RNA (shRNA), micro-RNA (miRNA), short activating RNA (saRNA), and long non-coding RNA (lncRNA). In accordance with the claims, the cargo is not RNAi. RNA products also include double-stranded RNA and ribozymes. The cargo also can be an oligonucleotide, such as an anti-sense oligonucleotide or an allele-specific oligonucleotide. The cargo can comprise a gene editing system, such as a CRISPR-CAS system.

Cargo can comprise mRNA or modified mRNA. Modified mRNA, can be synthetic mRNA that comprises a translatable region that contains at least one nucleoside modification in which at least a percentage of the uridine nucleotides in the synthetic mRNA are modified (see, *e.g.,* U.S. Patent No. 9,464,124, which describes exemplary modified mRNA for delivery and translation). For example, at least one nucleoside modification can be pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinomethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thio-uridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoyladenosine, N6-threonylcarbamoyladenosine, 2-methylthio-N6-threonyl carbamoyladenosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. The mRNA can include a) a sequence of linked nucleotides, a 5' UTR, a 3' UTR, and at least one 5' cap structure. The mRNA also can include other regulatory sequences for translation and trafficking in a eukaryotic, such as mammalian, such as a human, host cell.

The MEVs can carry cargos that include reporter genes and proteins and other detectable products, such as, for example, a fluorescent protein, such as, but not limited to an enhanced green fluorescent protein (EGFP; SEQ ID NO:10), a luciferase gene (SEQ ID NO:11), luxA (SEQ ID NO:8), luxB (SEQ ID NO:9), and the Lux operon (luxCDABE and luxABCDE; SEQ ID NO:12).

Other cargos can target genes or products involved in diseases, such as, but not limited to, Peptidyl-prolyl cis-trans isomerase FKBP4 or FKBP52 (SEQ ID NO:1); gamma-aminobutyric acid type B receptor subunit 1 (GABBR1; SEQ ID NO:3); oncogenes such as MYCN or NMYC (SEQ ID NO:38), RAS (H-RAS, N-RAS, and K-RAS; see SEQ ID NOs:39, 40, and 41, respectively), BCL2 (SEQ ID NO:43), and PLK1 (SEQ ID NO:44). Genes involved in diseases, such as oncogenes, and checkpoints, can be modulated by cargo that encodes a product that inhibits or agonizes expression of a

gene, or inhibits or agonizes a gene product. Exemplary of such modulators, are RNAis, such as, for example, siRNAs, miRNAs, shRNAs, peptides and/or tetratricopeptides. For example, siRNAs and ASOs targeting EGFP (SEQ ID NOs:5 and 6), firefly luciferase (SEQ ID NO:7), MYCN (SEQ ID NOs:13-19), RAS (SEQ ID NOs:20-27), BCL2 (SEQ ID NOs:29-31), and PLK1 (SEQ ID NOs:32-35), and microRNA-34A, which targets MYC and BCL2 (SEQ ID NO:28), are exemplified herein. In accordance with the claims, the cargo is not RNAi.

The cargo includes therapeutic or diagnostic or theragnostic proteins or peptides, protein complexes, such complexes that contain two or more proteins or a protein and nucleic acid, or a protein and aptamer, or combinations of proteins, nucleic acids, and other molecules. The cargo can be or can encode a protein that is an antibody or antigen-binding fragment thereof. Antibodies can be of any form, including single chain forms, nanobodies, camelids, and other forms, such as an scFv, a bi-specific antibody, or an antigen-binding fragment thereof. Antibodies and antigen-binding fragment thereof include a checkpoint inhibitor antibody or antigen-binding fragment thereof, or a tumor antigen-specific antibody or antigen-binding fragment thereof, or an anti-oncogene specific antibody or antigen-binding fragment thereof, or is a tumor-specific receptor or signaling molecule antibody or antigen-binding fragment thereof. Exemplary antibodies and antigen-binding fragment thereof specifically binds to and inhibits one or more of CTLA-4, PD-1, PD-L1, PD-L2, the PD-1/PD-L1 pathway, the PD-1/PD-L2 pathway, HER2, EGFR, TIM-3, LAG-3, BTLA-4, HHLA-2, CD28, and other checkpoints or immune suppressors, or tumor antigens.

The cargo in the MEVs in the compositions can include immune stimulating products, or antigens, and can be used as a vaccine to induce an immunoprotective response upon administration. The cargo can be a DNA, RNA, protein, or virus. The cargo can contain nucleic acid or protein or a nucleic acid encoding a protein that is a therapeutic product for treatment of cancer, or an infectious disease, or a neurodegenerative disease or other CNS disorder, or aging, or aging associated disease, or ophthalmic disorders, or immunological disorders. The cargo can be a cosmeceutical or a cosmetic or cosmetically active product. The cargo can comprise a small molecule bioactive molecule, such as a small molecule bioactive molecule, such as a small molecule drug. Exemplary drugs include chemotherapeutic agents or drugs. The drugs include prodrug. The cargo in the MEVs in the compositions can be or comprise a diagnostic marker or detectable product, such as, but not limited to, luciferase or nucleic acid encoding the luciferase, a fluorescent protein or nucleic acid encoding a fluorescent protein, or a luciferase operon.

The cargo can comprise DNA. The DNA can be a plasmid, such as one that encodes a product for expression in the animal or plant to which it is administered. Exemplary products include therapeutic products and diagnostic products. These include protein and RNA product, including the RNA products listed above. Since the MEVs are intended for administration to animals, the plasmids generally encode the product under control of eukaryotic regulatory signals and sequences, including eukaryotic promoters and translation sequences, such as RNA polymerase II and III promoters. Exemplary promoters include RNA polymerase II promoters, such as from promoters from animals, plants, and plant or animal viruses. Exemplary promoters, include, but are not limited to, a cytomegalovirus promoter, a simian virus 40 promoter, a herpes simplex promoter, an Epstein Barr virus promoter, an adenovirus promoter, a synthetic promoter, an actin promoter, and synthetic chimeric promoters. Other eukaryotic transcription sequences and eukaryotic translation sequences, include, but are not limited to, one or more of an enhancer, a poly A sequence, and/or an internal ribosome entry site (IRES) sequence.

The plasmids can encode one or two or more cargo products. For expression of the cargo product the encoding nucleic acid is operably linked to regulatory sequences recognized by a eukaryotic cell.

Methods of preparing the MEVs are provided. The methods include introducing the cargo into isolated MEVs. The cargo includes any molecule for whom delivery into or onto an animal is desired. Generally, the cargo is or contains or provides a bioactive molecule product, including small molecules and biopolymers. The biopolymers are naturally-occurring, or synthetic, or modified, or combinations thereof. The cargo includes a protein, nucleic acid, or small molecule. The cargo can be loaded into the MEVs by any method known to those of skill in the art; these methods include, for example, one or more of electroporation, sonication, extrusion, and use of surfactants. In some embodiments the MEVs are from *Chlorella,* such but not limited to a species of *Chlorella* selected from among *Chlorella ellipsoidea, Chlorella pyrenoidosa, Chlorella sorokiniana, Chlorella vulgaris,* and *Chlorella variabilis.* The MEVs produced by the methods and any of the MEVs provided herein, including the compositions containing the MEVs can be used in one or more of a method of diagnosis, or as vaccine, or therapy for treatment, or diagnosis of a disease, or treatment of a disease or condition, or for cosmetic uses, or for industrial uses, or any use known to those of skill in the art.

The MEVs can be used in any such method, which include methods of treatment of a disease, disorder, or conditions. Exemplary of diseases, disorders, and conditions is cancer, such as a cancer comprises a solid tumor or a hematological malignancy, or metastases thereof. Other diseases, disorders, and conditions include, those of or involving the respiratory system, of or involving the central nervous system or the nervous system, of or involving the skin and exposed epithelia or mucosa, of or involving the digestive tract, of or involving an infectious agent. Infectious agents include, bacteria, viruses, parasites, prions, oomycetes, and fungi.

The cargo can provide therapeutic molecules for treatment, or can induce an immune response to serve as a vaccine. The MEVs can contain a cargo that comprises an immunostimulatory protein or an antigen or encodes an

immunostimulatory protein or antigen, whereby the MEVs, upon administration are immunostimulating and elicit an innate or adaptive immune response, or the MEVs and/or the cargo can elicit an immunoprotective response to prevent or treat a disease or condition. The MEVs can be used to treat a disease, disorder, or condition resulting from trauma. Trauma includes, but is not limited to, trauma from or involving from among wounds, burns, surgery, skin cuts, broken bones, hair loss, dermis exposure, mucosal exposure, fibrosis, lacerations, and ulcerations. The MEVs can be used to elicit an effect to treat a condition resulting from natural aging, or pathogenic or disease or otherwise induced aging. Other diseases, disorders, and conditions that can be treated by the MEVs, are diseases, disorders, and conditions or the skin or the eye. These include dermatitis, wrinkles and/or other age-related changes in the skin, macular degeneration, glaucoma, diabetic retinopathies, cataracts, or conditions resulting from diabetic retinopathy.

The compositions containing the MEVs can be formulated for administration by any route of administration. Routes, include, but are not limited to, local, systemic, topical, parenteral, enteral, mucosal, aerosols for inhalation into the lung or intranasal, parenteral, enteral, vaginal, rectal, aural, oral, and other routes of administration. The MEVs can be formulated in any form, including as a tablet, as a liquid, such as an emulsion, as a powder, as a cream, as gel, for oral administration, for nebulization, for inhalation.

The compositions or MEVs can be used in any of the methods and treatments described herein or known to those of skill in the art. Methods include, for example, any described herein, including, for example, for use for one or more of gene silencing, gene interference, gene therapy, gene/protein overexpression, gene editing, inhibition or stimulation of protein activity, pathway signaling, and diagnostics or detection. The compositions and MEVs can be used for prophylaxis and/or vaccination. They can be used in agro-veterinary applications, such as crop sciences, phyto-pathogens, and animal diseases, for dermatological applications, and for cosmetic applications. They can be used for industrial purposes, for example for manufacturing, characterization, and calibration.

Hence, among the compositions and exogenously-loaded MEVs, provided are compositions, comprising microalgae extracellular vesicles (MEVs) formulated for oral delivery, intravenous delivery, intramuscular delivery, intranasal delivery, subcutaneous delivery, topical delivery, mucosal delivery, intraperitoneal delivery, intratumoral delivery, or inhalation delivery, wherein the MEVs were exogenously loaded. The compositions provided herein can be formulated for a route of delivery, whereby the MEVs containing the exo-loaded cargo are delivered to a target organ or tissue or cell. Exemplary of target organs, cells, or tissues are selected from among lungs, liver, spleen, intestine, brain, spinal cord, peripheral nerves, lymphoid tissues, eyes, mucosal tissues, skin, hematopoietic tissues, pancreas, muscle, bones, heart, endocrine tissues, and kidneys. Others include mucosal tissue that is naso-buccal, ocular, urogenital, vaginal, or rectal.

The compositions can be formulated in any suitable form, such as a suspension or emulsion, such as a nanoemulsion or microemulsion. The compositions can be formulated as tablets, capsules, gel capsule, powders, troches, granules, and liquids or oral administration, oil, or is a suspension or emulsion for nasal administration or oral administration or inhalation or nebulization or intratracheal administration.

As described herein exemplary of microalgae is *Chlorella.* Provided are the compositions where: the MEVs are *Chlorella* extracellular vesicles; and the *Chlorella* extracellular vesicles comprise a heterologous bioactive molecule cargo that was exogenously loaded into the MEVs. In all embodiments herein, the microalgae include *Chlorella.* The *Chlorella* is selected from among *Chlorella ellipsoidea, Chlorella pyrenoidosa, Chlorella sorokiniana, Chlorella vulgaris,* and *Chlorella variabilis.* In exemplary embodiments, the *Chlorella* is *Chlorella vulgaris.*

In the compositions provided herein, the exo-loaded cargo includes therapeutics for treating or preventing a disease, disorder, or condition, or treating or preventing a symptom thereof. For example, the exo-loaded cargo can comprise or encode a protein or a therapeutic that is a prophylactic for preventing or reducing the risk of getting a disease, disorder, or condition; or for reducing the severity of a disease, disorder, or condition. The resulting MEVs can be used as a vaccine, for example. The exo-loaded cargo can comprise a nucleic acid, a protein, a small peptide, a peptide, a polypeptide, and/or a small molecule, such as a small molecule drug, or any other molecule of interest for delivery to a cell, tissue, or organ. Exo-loaded cargo can comprise proteins, polypeptides, peptides, small peptides, nucleic acid, and other molecules, such as small molecule drugs, complexes, aptamers, and any molecule whose delivery to a target is of interest. Exo-loaded MEVs advantageously can be loaded with any molecule of interest for any purpose in which the MEVs can be delivered *in vivo* or *in vitro.* Exemplary of nucleic acids are coding RNA or non-coding RNA, such as heterologous messenger RNA (mRNA), long non coding RNA (lncRNA) comprising a sORF, a long non-coding RNA (lncRNA), a short hairpin RNA (shRNA), a small activating RNA (saRNA), and/or a self-activating RNA. Other examples include cargo that is heterologous RNAi, unmodified mRNA, or modified mRNA, or RNAi that is siRNA, and/or miRNA. Modified RNA includes RNA that is modified to increase stability, such as described above and known in the art (see, *e.g.,* U.S. Patent No. 9,464,124, which describes exemplary modified mRNA for delivery and translation. In accordance with the claims, the cargo is not RNAi.

The exo-loaded cargo can comprise a gene editing system (see, *e.g.,* SEQ ID NOs: 59, 60, 81, and 82). The gene editing system can comprise a CRISPR-Cas system, or a CRISPR-associated or CRISPR-like system, or transcription activator-like effector nucleases (TALENs), zinc-finger nucleases (ZFNs), or homing endonucleases, or meganucleases.

Exo-loaded cargo can comprise the exo-loaded cargo comprising a small peptide, a peptide, a polypeptide, or a protein that has immunomodulatory activity, or, in some instances encoding nucleic acid. For example, the immune modulator can be an immunomodulatory agent to increase or decrease production of one or more cytokines; up- or down-regulate self-antigen presentation; mask MHC antigens; or promote the proliferation, differentiation, migration, or activation state of one or more types of immune cells. The immunomodulatory exo-loaded cargo can increase or decrease production of one or more cytokines, up- or down-regulate self-antigen presentation, mask MHC antigens, or promote the proliferation, differentiation, migration, or activation state of one or more types of immune cells. MEVs that comprise cargo that is immune-modulating can be used to treat a disease is treated by immune modulation.

The exo-loaded cargo can comprise a hormone or a cytokine or a chemokine; or comprises nucleic acid encoding a hormone, or a cytokine, or a chemokine. Exemplary of exo-loaded cargo that comprises a hormone or cytokine or growth factor are those selected from among human growth hormone; N-methionyl human growth hormone; bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factors; fibroblast growth factors; prolactin; placental lactogen; tumor necrosis factor-alpha and -beta; Müllerian-inhibiting substance; gonadotropin-associated peptide; inhibin; activin; vascular endothelial growth factors; integrin; thrombopoietin (TPO); nerve growth factors, transforming growth factors (TGFs); insulin-like growth factor-I and-II; erythropoietin (EPO); osteoinductive factors; interferons such as interferon-alpha, -beta, and -gamma; colony stimulating factors (CSFs) such as macrophage-CSF (M-CSF); granulocyte-macrophage-CSF (GM-CSF); granulocyte-CSF (G-CSF); and an interleukin (IL).

The exo-loaded cargo can comprise nucleic acid for mRNA-mediated gene therapy, gene silencing, gene substitution, gene overexpression, and/or gene editing. It can comprise one or more of peptides, polypeptides, or proteins for gene regulation, gene substitution, gene overexpression, gene editing, regulation of cell metabolism, regulation of cell functions, and protein therapy. The cargo can be for treating an inborn error of metabolism. The exo-loaded MEVs can be a vaccine, such as a prophylactic or immunoprotective vaccine or as a vaccine to treat an existing disease, disorder, or condition. The MEV cargo can comprise a protein or polypeptide or peptide that is a therapeutic product for treatment of cancer, or an infectious disease, or metabolic diseases, or a neurodegenerative disease or other CNS disorder, or aging, or an aging associated disease, or genetic diseases, or ophthalmic disorders, or immunological disorders, or involving internal organs urogenital organs, the cardiovascular system and associated organs and tissues, hematopoietic or lymphoid tissues, sensory organs and tissues, urogenital organs and tissues, muscle tissues, bones, and/or endocrine tissues. In all embodiments, MEVs in the composition comprise two or more cargo products, or there can be mixtures of MEVs that comprise different cargo products.

The compositions provided herein can be formulated for any route of administration, including, but not limited to, oral administration, parenteral administration, topical administration, local administration, intratumoral administration, systemic administration, mucosal administration, intravenous administration, subcutaneous administration, intramuscular administration, intraperitoneal administration, transdermal administration, intranasal administration, inhalation, or intratracheal administration. They can be formulated for oral delivery; or is formulated as an aerosol for intranasal delivery, inhalation, or nebulization. The compositions can be formulated as appropriate, such as formulated for oral administration, intravenous administration, intramuscular administration, subcutaneous administration, intranasal, and/or intratracheal administration or inhalation or nebulization, topical administration, local administration, intratumoral administration, systemic administration, mucosal administration, and/or intraperitoneal administration. The formulation and route(s) of administration is selected based on the target organ(s), tissue(s), and /or cells, since MEVs traffic to different loci depending upon the route of administration. Different diseases, disorders, and conditions can involve or affect different organs, tissues, and cells. Therapeutics, thus, can be targeted to loci involved in the disease, disorder, or condition by appropriately selecting a route of administration and formulation.

The compositions can be for use for or in a method for delivering exo-loaded cargo in an MEV to an organ or tissue, wherein the mode of administration is selected to target the organ or tissue.

Provided are compositions for use in methods of treatment or prevention or reducing the risk of or severity of a disease, disorder, or condition, comprising administering a composition comprising exo-loaded MEVs as describe or provided herein. The disease, disorder, or condition can be selected from among a cancer, a genetic disorder, a liver disease, a disease involving the immune system or immune cell, an infectious disease, a respiratory disease, a lung disease, a neurodegenerative disease, a gastrointestinal disease, and a brain cancer; or involving internal organs, urogenital organs, the cardiovascular system and associated organs and tissues, hematopoietic or lymphoid tissues, sensory organs and tissues, urogenital organs and tissues, muscle tissues, bones, and/or endocrine tissues. Internal organs, include, but are not limited to, the pancreas, liver, and/or spleen.

Diseases, disorders, and conditions include any known to those of skill in the art for which there is a therapeutic that can be exo-loaded into the MEVs. Exemplary of diseases, disorders, and conditions is cancer, cancer metastases, metabolic syndrome, genetic disorders, alpha-anti-trypsin (AAT) deficiency, other inborn errors of metabolism, hemophilia, hypercholesterolemia, liver inflammation, steatohepatitis, and other diseases and disorders that can be treated by

delivery of a therapeutic to the liver. Other diseases, disorders, and conditions include a neurodegenerative disease (such as Parkinson's, or Alzheimer's, or Huntington's, or Creutzfeldt-Jakob disease, or other neurodegenerative disease), or a cognitive disorder (such as dementia, or amnesia, or delirium, or other cognitive disorder), or a brain disorder (such as encephalitis, or seizures, or tumors, or other brain disorder), or a nervous system disorder (such as pain, or seizures, or infections, or other nervous system disorder), or a genetic disease (such as cystic fibrosis, thalassemia, sickle cell anemia, Huntington's Disease, Duchenne's muscular dystrophy, Tay-Sachs disease, or other genetic disease), or a multifactorial disease (such as diabetes, Chronic Obstructive Pulmonary Disease (COPD), or other multifactorial disease), or cancer (such as cancer of the breast, or ovaries, or bowel, or prostate, or skin, or other cancer), or high blood pressure, or high cholesterol, or schizophrenia, or bipolar disorder, or arthritis, or a metabolic disease (such as familial hypercholesterolemia, or Gaucher disease, or Hunter syndrome, or Krabbe disease, or maple syrup urine disease, or metachromatic leukodystrophy, or MELAS (mitochondrial encephalopathy, lactic acidosis, stroke-like episodes), or Niemann-Pick disease, or phenylketonuria (PKU), or porphyria, or Tay-Sachs disease, or Wilson's disease, or other metabolic disease), or a disease of the respiratory system (such as asthma, or Chronic Obstructive Pulmonary Disease (COPD), or chronic bronchitis, or emphysema, or lung cancer, or cystic fibrosis, or bronchiectasis, or pneumonia, or other disease of the respiratory system), or a disease of the digestive tract (such as irritable bowel syndrome (IBS), or inflammatory bowel disease (IBD), or gastroesophageal reflux disease (GERD), or celiac disease, or diverticulitis, or disease of the digestive tract), or a disease of the mucosa (such as Behçet's disease, or burning mouth syndrome, or oral lichen planus, or pemphigus and pemphigoid, or recurrent aphthous stomatitis, or Sjögren's syndrome, or genitourinary infections, or sinusitis, or nasal or sinus polyps, or smell and taste disorders, or allergy, or ocular mucous membrane pemphigoid, or other disease of the mucosa), or a disease of the muscular or neuromuscular tissues (such as amyotrophic lateral sclerosis (ALS), or Charcot-Marie-Tooth disease, or multiple sclerosis, or muscular dystrophy, or myasthenia gravis, or myopathy, or myositis, or peripheral neuropathy, or other muscular or neuromuscular disease), or a disease of the bones (such as cervical spondylosis, or osteoporosis, or metatarsalgia, or polymyalgia rheumatica, or bone cancer, or rheumatoid arthritis, or osteoarthritis, or other), or a disease of the endocrine tissue (such as acromegaly, or adrenal insufficiency, or Addison's disease, or Cushing's syndrome, or Graves' disease, or Hashimoto's disease, or Creutzfeldt-Jakob disease, or hyperthyroidism, or hypothyroidism, or multiple endocrine neoplasia, or polycystic ovary syndrome (PCOS), or primary hyperparathyroidism, or other disease of the endocrine tissue), or a disease of haemopoietic or lymphoid tissues (such as Fanconi anemia, or thrombocytopenia, or Diamond-Blackfan anemia, or Shwachman-Diamond syndrome, or chronic granulomatous disease, or Gaucher's disease, or myeloid malignancies including myeloproliferative neoplasms, myelodysplastic disorders, chronic myelomonocytic leukemia, acute myeloid leukemia (AML), or lymphoma, or Hodgkin's lymphoma, or non-Hodgkin's lymphoma, or lymphadenitis, or lymphangitis, or lymphedema, or lymphocytosis).

The MEVs can be formulated for administration and/or used in treating particular diseases by appropriate selection of a route of administration and a formulation for such route. For example, the MEVS can be formulated for oral administration, such as where the disease involves or the exo-loaded cargo targets the gastrointestinal tract or the immune system or the white spleen for treatment. Methods of treatment and compositions provided herein include those: where the MEVs are formulated for oral administration and the disease involves or the exo-loaded cargo targets any of the organs and tissues accessible through the blood stream; or where the disease is a cancer and/or an immune cell disorder or a disease treated or prevented by a vaccine; or where the disease is an intestinal infection, Crohn's disease, or cancer; or where the method or treatment, cargo, or use of the MEVs comprises microbiota modulation; or where MEVs are formulated for intratracheal administration or inhalation or nebulization, and the disease involves, or the exo-loaded cargo targets the respiratory tract and/or the lungs for treatment or treats respiratory diseases.

Additional diseases, disorders, and conditions treated with compositions, methods and uses provided herein include, for example, a chronic obstructive pulmonary disease (COPD), pulmonary hypertension, asthma, other inflammatory lung disease, cystic fibrosis, alpha-anti-trypsin (AAT) deficiency, an inborn errors of metabolism, lung disease, cancer, and cancer metastases involving the lungs and respiratory system. Other diseases, disorders, and conditions are those of or involving the central nervous system, or the nervous system, or the sensory organs or tissues. The MEVs can be used and formulated to target the brain, such as by intranasal administration.

The compositions, methods and uses can be those where the MEVs are formulated for intranasal administration, or for mucosal administration, or for intraocular administration, such as by topical administration onto the cornea or retina, or by intravitreal administration. The MEVs can deliver and be used for treatment of a disease, disorder, or condition from an infectious agent, such as one or more of a bacterium, a virus, an oomycete, a parasite, and a fungus.

Hence provided are methods of delivering (and uses of compositions for delivering) bioactive agents to a targeted organ, tissue, cell, or system, such as the immune system. This can be effected by orally administering or providing for use a composition comprising MEVs that comprise a bioactive agent as cargo, where the bioactive agent has been exogenously loaded into the MEVs as described herein. As noted, in embodiments, particularly or the compositions, the MEVs are from a microalgae that is a species of *Chlorellaceae,* and particularly a *Chlorella* species selected from among *Chlorella ellipsoidea, Chlorella pyrenoidosa, Chlorella sorokiniana, Chlorella vulgaris,* and *Chlorella variabilis.* Exemplary thereof is *Chlorella* is *Chlorella vulgaris.*

The compositions provided herein can serve as drug delivery systems, such as a drug delivery system, comprising exogenously loaded microalgae extracellular vesicles (MEVs) formulated for oral delivery, intravenous delivery, intratracheal delivery, intramuscular delivery, intranasal delivery, or inhalation delivery into the lungs. Such compositions or drug delivery systems contain the compositions where the microalgae extracellular vesicles (MEVS) are formulated for a route of delivery to deliver a cargo to a target tissue, such as, for example, the lungs, liver, spleen, intestine, brain, naso-buccal mucosa, heart, and kidneys. In some embodiments, the MEVs are *Chlorella* extracellular vesicles; the *Chlorella* extracellular vesicles comprise a heterologous bioactive molecule cargo that has been introduced into the isolated extracellular vesicles, whereby the vesicles in the composition that contain heterologous bioactive molecule cargo contain, on average, the same bioactive molecule cargo; and the cargo molecule(s) is/are heterologous to *Chlorella.* Cargo comprises any molecules, including complexes, biomolecules, nucleic acids, RNA (excluding RNAi), DNA, plasmids, aptamers, small drugs, antibodies, and any molecule of interest for delivery into a cell, tissue, or organ *in vivo* or *in vitro.* Exemplary cargo includes any described herein or known to those of skill in the art include, cargo that comprises DNA, plasmids, a therapeutic product or a diagnostic product, a therapeutic RNA product, an anti-sense oligonucleotide or a ribozyme or a double-stranded RNA, an immune modulator increase or decrease production of one or more cytokines; up- or down-regulate self-antigen presentation; mask MHC antigens; or promote the proliferation, differentiation, migration, or activation state of one or more types of immune cells, a hormone or a cytokine or a chemokine, including but not limited to those listed above, anti-infective, such as an anti-viral agent, an anti-fungal agent, or an antibiotic, such as for treatment of respiratory infections or sinus infections, such as an antibiotic is selected from among aminoglycoside antibiotics (e.g., apramycin, arbekacin, bambermycins, butirosin, dibekacin, gentamicin, kanamycin, neomycin, netilmicin, paromomycin, ribostamycin, sisomicin, and spectinomycin); aminocyclitols (e.g., spectinomycin); amphenicol antibiotics (e.g., azidamfenicol, chloramphenicol, florfenicol, and thiamphenicol); ansamycin antibiotics (e.g., rifamide and rifampin); carbapenems (e.g., imipenem, meropenem, and panipenem); cephalosporins (e.g., cefaclor, cefadroxil, cefamandole, cefatrizine, cefazedone, cefozopran, cefpimizole, cefpiramide, cefpirome, cefprozil, cefuroxime, cefixime, cephalexin, and cephradine); cephamycins (cefbuperazone, cefoxitin, cefminox, cefmetazole, and cefotetan); lincosamides (e.g., clindamycin, and lincomycin); macrolide (e.g., azithromycin, brefeldin A, clarithromycin, erythromycin, roxithromycin, and tobramycin); monobactams (e.g., aztreonam, carumonam, and tigemonam); mupirocin; Oxacephems (e.g., flomoxef, latamoxef, and moxalactam); penicillins (e.g., amdinocillin, amdinocillin pivoxil, amoxicillin, bacampicillin, benzylpenicillin acid, benzylpenicillin sodium, epicillin, fenbenicillin, floxacillin, penamecillin, penethamate hydriodide, penicillin o-benethamine, penicillin O, penicillin V, penicillin V benzoate, penicillin V hydrabamine, penimepicycline, and phenethicillin potassium); polypeptides (e.g., bacitracin, colistin, polymyxin B, teicoplanin, and vancomycin); quinolones (amifloxacin, cinoxacin, ciprofloxacin, enoxacin, enrofloxacin, fleroxacin, flumequine, gatifloxacin, gemifloxacin, grepafloxacin, lomefloxacin, moxifloxacin, nalidixic acid, norfloxacin, ofloxacin, oxolinic acid, pefloxacin, pipemidic acid, rosoxacin, rufloxacin, sparfloxacin, temafloxacin, tosufloxacin, and trovafloxacin); rifampin; streptogramins (e.g., quinupristin, and dalfopristin); sulfonamides (sulfanilamide, and sulfamethoxazole); and tetracyclines (chlortetracycline, demeclocycline hydrochloride, demethylchlortetracycline, doxycycline, Duramycin, minocycline, neomycin, oxytetracycline, streptomycin, tetracycline, and vancomycin). The MEVs can comprise nucleic acid for gene therapy, such as for treating an inborn error of metabolism. The cargo can comprise a product that is a cosmetic or has a cosmetic activity or has industrial use, the cargo can comprise or encode a protein that is an antibody or antigen-binding fragment thereof.

## Description of the Drawings

**Figure 1** provides an exemplary profile of light intensity used in HECTOR PBR cultures.

**Figure 2** depicts an exemplary elution profile for higher purity of MEVs preparations, where MEVs previously concentrated by TFF and purified by ultracentrifugation and formulated in PBS at concentration of $10^{11}$ to $10^{13}$ per mL are seeded in a pre-packed column qEV1 from IZON. The MEVs are eluted using PBS solution. The elution fractions of 0.5 mL are collected. MEVs are recovered in the first fractions as shown in figure below. The most concentrated fractions (4-5) are pooled and stored at 4°C before use.

**Figure 3** provides exemplary images of MEVs obtained using Transmission Electron Microscopy (TEM).

**Figure 4** provides an electropherogram of the small RNA library

**Figure 5** provides representative patterns of biodistribution according to the route of administration, for the Intravenous, Intratracheal and Per os routes.

**Figure 6** depicts the *in vivo* full body imaging of a representative animal after intravenous administration as described in Example 5.

**Figure 7** depicts the *in vivo* full body imaging of a representative animal per os (oral) administration as described in Example 5.

**Figure 8** depicts the *in vivo* full body imaging of a representative animal after intranasal administration as described in

Example 5.

**Figure 9** depicts the *in vivo* full body imaging of a representative animal after intratracheal administration as described in Example 5.

**Figure 10** depicts the kinetics of accumulation in liver, lungs and spleen (average of 6 animals) after intravenous administration, as described in Example 5.

**Figure 11** depicts the kinetics of accumulation in lungs, spleen and intestine (average of 6 animals) per os administration, as described in Example 5.

**Figure 12** depicts the kinetics of accumulation lungs and kidneys (average of 4 animals) after intranasal administration, as described in Example 5.

**Figure 13** depicts the kinetics of accumulation in lungs, spleen and intestine (average of 3 animals) after intratracheal administration, as described in Example 5.

**Figures 14A-D** depict *ex vivo* fluorescence analysis (total radiant efficiency) in organs **[A)** liver; **B)** spleen; **C)** lungs; and **D)** brain] isolated 3 days after intravenous (IV), intranasal (IN), per os (PO), and intratracheal (IT) administration.

**Figures 15A and 15B** depict A) Haematoxyline Eosine staining of intestine (G = GALT tissue) and B) DAPI (nuclei) staining and MEV-PKH26 fluorescence.

**Figure 16** depicts the SPLEEN pulp stained with DAPI (for nuclei) and MEV-PKH26 (red fluorescence), shown as white puncta.

**Figure 17** displays a diagram showing the migration of MEV from the GALT to the spleen.

**Figures 18A-I** show results of assessment of toxicity of MEVs in a mouse model after oral (PO) or intratracheal (IT) administration at different doses in 4 groups of mice for each parameter. MEV toxicity was evaluated by chemistry parameters: ALAT, ASAT, urea and creatine (FIGS. 12A-D, respectively); and 2) by hematology parameters: red blood cells, hemoglobin, hematocrit, MCV and eosinophils (FIGS. 12E-I, respectively). The groups were: Group 1 mice administered 100 μl of PBS (White bars) by PO delivery; Group 2 mice were administered 100 μl of $4*10^{11}$ MEV/mouse by PO delivery (bar with black and white tiles); Group 3 mice were administered 100 μl of $4*10^{12}$ MEV/ mouse by PO delivery (bars with vertical lines); Group 4 mice were administered 100 μl of $4*10^{11}$ MEV/ mouse by IT delivery (squared bars). Data were measured for six mice per group for each parameter. **FIG. 18A** shows ALAT: Alanine Aminotransferase; **FIG. 18B** shows ASAT: Aspartate Aminotransferase (ASAT); **FIG. 18C** shows urea; **FIG. 18D** shows creatine; **FIG. 18E** shows red blood cells; **FIG. 18F** shows hemoglobin; **FIG. 18G** shows hematocrit; **FIG. 18H** shows MCV (Mean Corpuscular Volume); and **FIG. 18I** shows eosinophils. PO designates per os (oral delivery) and IT designates Intratracheal administration.

**Figure 19** shows *in vivo* delivery and expression of mRNA after topical instillation of MEVs into the eyes in rabbits.

**Figures 20A and 20B** depict *in vitro* delivery of GFP protein to human monocytes.

**Figures 21A and 21B** depict *in vitro* delivery of GFP protein to human keratinocytes.

**Figure 22** shows confocal microscopy of Hep-G2 cells including GFP protein expression in Hep-G2 cells after 24h incubation with MEVs loaded with GFP-protein (MEV-GFP) or MEVs loaded with mRNA-eGFP (MEV-mRNA).

**Figure 23** shows confocal microscopy Huh7 cells, including GFP protein expression in Hep-G2 cells.

**Figure 24** shows *in vitro* delivery of GFP protein and GFP-mRNA to human fibroblasts.

**Figures 25A-D** shows results of the flow cytometry analysis in MEV penetration and delivery study using human fibroblasts.

**Figure 26** shows antibacterial activity of *Chlorella* MEVs exogenously loaded with siRNAs directed against *Pto* DC3000 *cfa6* and *hrpL* genes.

**Figure 27** shows delivery of the bioactive flg22 peptides exogenously loaded in Chlorella MEVs.

## DETAILED DESCRIPTION

### Outline

**A. DEFINITIONS**
**B. MICROALGAE AND OVERVIEW**
**C. EXTRACELLULAR VESICLES**

**1. Types of Extracellular Vesicles (EVs)**

**a. Exosomes**
**b. Microvesicles**
**c. Apoptotic Bodies**

**2. Uptake of EVs**
**3. General Methods for Isolating EVs**

**a. Ultracentrifugation**
**b. Size-Based Techniques**
**c. Immunoaffinity Capture-Based Techniques**
**d. Exosome Precipitation**
**e. Microfluidic Based Isolation Techniques**

**4. Microalgae-Derived Extracellular Vesicles (MEVs)**
**5. Green algae - *Chlorella* species**

**a. Life Cycle**
**b. Genomic Analyses of *Chlorella* Species**
**c. Commercial and Biotechnological Uses of *Chlorella***
**d. *Chlorella* MEVs**

**D. EXOGENOUSLY LOADED MICROALGAE EXTRACELLULAR**

**VESICLES (MEVS), CARGO, AND TARGETS**

**1. Isolation of MEVs**
**2. MEV Loading, and Cargos**
**3. Generation of Payload-Loaded MEVs**

**a. Electroporation**
**b. Sonication**
**c. Extrusion**
**d. Surfactants**
**e. Other Methods**

**4. Exemplary Cargo and Exemplary Uses of the Exogenously**

**Loaded MEVs**

**a. Cargo**

**1) RNA Cargo**
**2) Antibody Cargo**

**b. Diseases and Methods of Treatment**
**c. Agro-Veterinary Applications**
**d. Cosmetic and Dermatological Applications**
**e. Other Exemplary Cargo**

**E. PHARMACEUTICAL COMPOSITIONS, FORMULATIONS, KITS, ARTICLES OF MANUFACTURE AND COMBINATIONS**

**1. Pharmaceutical Compositions and Formulations**
**2. Articles of Manufacture/Kits and Combinations**
**3. Combination Therapies**

**F. ADMINISTRATION OF EXOGENOUSLY LOADED MEVS AND ROUTES OF ADMINISTRATION**

**1. Routes of administration**
**2. Compositions Containing MEVs as Drug Delivery Systems**

**G. BIODISTRIBUTION OF MEVs FOLLOWING ADMINISTRATION VIA VARIOUS ROUTES**

1. **Biodistribution of mammalian EVs**
2. **Microalgae EVs Biodistribution**

   a. **Components of the Lymphatic System**
   b. **Targeting GALT**

3. **Exemplary Diseases and conditions treated by MEVs**

**H. EXAMPLES**

## A. Definitions

Unless defined otherwise, all technical and scientific terms used herein have the same meaning as is commonly understood by one of skill in the art to which the invention(s) belong. In the event that there are a plurality of definitions for terms herein, those in this section prevail. Where reference is made to a URL or other such identifier or address, it is understood that such identifiers can change and particular information on the internet can come and go, but equivalent information can be found by searching the internet. Reference thereto evidences the availability and public dissemination of such information.

As used herein, cargo refers to any heterologous or exogenous molecules, such as bioactive molecules, including biomolecules, and small molecules, that are loaded into the microalgae extracellular vesicles (MEVs) provided herein after the MEVs have been isolated. Cargo can be used interchangeably with "payload."

As used herein, exogenous cargo refers to cargo loaded after the MEVs are produced and isolated or partially isolated sufficiently for introduction of exogenous cargo.

As used herein, a bioactive molecule refers to any molecule that can have a biological activity or that can act *in vivo* on a subject. Bioactive molecules include biomolecules, such as DNA, RNA, proteins, other biopolymers, and small molecules, such as small molecule drugs and pharmaceuticals, and any molecules that would be delivered to a subject, such as a human or other animal or a plant or a microorganism (bacteria or other), in connection with a therapy, a diagnostic, such as a detectable molecule, and other such uses, such as cosmetic uses, and industrial uses. The biomolecule can function as or have an activity as, for example, a therapeutic, an immunogen, a diagnostic, a detectable marker, or a cosmetic. The bioactive molecules for use herein are any that can be loaded into a microalgae extracellular vesicle (MEV) after they isolated or partially isolated from the microalgae.

As used herein, a biomolecule refers to any biologically active biopolymer or molecule that occurs, or can occur, in a living organism or virus or that is a modified form of such biopolymer or molecule. Biomolecules, thus, include modified naturally-occurring biomolecules, such as, for example proteins that include a modified primary sequence, such as by deletions, insertions, and/or replacements of amino acids to alter the primary sequence, and or by modification, such as post-translational modifications of the protein.

As used herein, when it is stated that MEVs have the same or substantially the same loaded cargo or amount thereof, it is understood that this refers to an average among the population of MEVs in a composition. It is understood, that when MEVs are loaded exogenously the ratio of cargo/MEV can be selected so that each MEV has on the average a pre-determined amount of cargo. As a simple example, to load an average of one molecule of cargo/MEV, the skilled person could calculate the amount of cargo to load into a composition of MEVs, and understands that in the composition of MEVs, some would have more than one molecule of cargo/MEV, and others would have none. On the average, the MEVs would have one molecule of cargo/MEV. The skilled person understands, that, in general, the amount of cargo/MEV will be more than the one molecule/MEV, and that the amount of cargo depends upon a variety of parameters, including the cargo, the target tissues and/or cells, the disease, disorder, or condition treated, and the subject treated. Generally, more than one molecule of cargo per MEV, on the average, such as at least 10 or about 10 molecules/MEV are loaded. Substantially more cargo also can be loaded. As noted, the amount depends upon the target, disease, disorder, condition, and/or the subject and the cargo. It is within the skill in the art to select the amount.

As used herein, a subject is any organism, generally an animal or plant, into which or on which the composition containing the MEV is introduced. Subjects include, but are not limited to, humans, plants, particularly crop plants, and animals, including farm animals and pets, such as dogs and cats, and zoo animals.

As used herein, disease or disorder or condition refers to a pathological or undesirable or undesired condition in an organism resulting from a cause or condition including, but not limited to, infections, acquired conditions, and genetic conditions, and that is characterized by identifiable symptoms.

As used herein, treating a subject with a disease or condition means that the subject's symptoms or manifestations of the disease or conditions are partially or totally alleviated, or remain static following treatment.

As used herein, treatment refers to any effects that ameliorate symptoms of a disease or disorder. Treatment encompasses prophylaxis, therapy and/or cure. Treatment also encompasses any pharmaceutical use of any MEV or

composition provided herein. Treatment refers to any effects that ameliorate or prevent or other reduce or eliminate any symptom or manifestation of a disease or disorder. Treatment also encompasses any pharmaceutical use of any MEV or composition provided herein.

As used herein, prophylaxis refers to prevention of a potential disease and/or a prevention of worsening of symptoms or progression of a disease. Prevention or prophylaxis, and grammatically equivalent forms thereof, refer to methods in which the risk or probability of developing a disease or condition is reduced or eliminated and products that reduce or eliminate the risk or probability of developing a disease or condition.

As used herein, a modification with reference to modification of a sequence of amino acids of a polypeptide or a sequence of nucleotides in a nucleic acid molecule refers to and includes deletions, insertions, and replacements of amino acids or nucleotides, respectively. These include modifications of the primary sequence of a polypeptide or protein. Methods of modifying a polypeptide and nucleic acid molecule are routine to those of skill in the art, such as by using recombinant DNA methodologies. Modifications, when referring to polypeptide or protein, not to a sequence, refer to post-translational or post-purification changes, such as conjugation or linkage of moieties that alter properties of polypeptide or protein, such as half-life extending moieties, glycosylation, purification tags, detectable reporters, and other such moieties.

As used herein, a modification of a genome or a plasmid or gene includes deletions, replacements, insertions, and translocations of nucleic acid. These include any changes to the native or naturally-occurring nucleic acid sequence.

As used herein, RNA interference (RNAi) is a biological process in which RNA molecules inhibit gene expression or translation, by neutralizing targeted mRNA molecules to inhibit translation and thereby expression of a targeted gene.

As used herein, RNA molecules that act via RNAi are referred to as inhibitory by virtue of their silencing of expression of a targeted gene. Silencing expression means that expression of the targeted gene is reduced or suppressed or inhibited.

As used herein, gene silencing via RNAi is said to inhibit, suppress, disrupt or silence expression of a targeted gene. A targeted gene contains sequences of nucleotides that correspond to the sequences in the inhibitory RNA, whereby the inhibitory RNA silences expression of mRNA. Small interfering RNAs (siRNAs) are small pieces of double-stranded (ds) RNA, usually about 21 nucleotides long, with 3' overhangs (2 nucleotides) at each end that can be used to interfere with the translation of proteins by binding to and promoting the degradation of messenger RNA (mRNA) at specific sequences. In doing so, siRNAs prevent the production of specific proteins based on the nucleotide sequences of their corresponding mRNAs. The process is called RNA interference (RNAi), and also is referred to as siRNA silencing or siRNA knockdown. A short-hairpin RNA or small-hairpin RNA (shRNA) is an artificial RNA molecule with a tight hairpin turn that can be used to silence target gene expression via RNA interference (RNAi). Expression of shRNA in cells is typically accomplished by delivery of plasmids or through viral or bacterial vectors.

As used herein, non-coding RNAs are RNAs that do not encode a protein. Classes of non-coding RNA, include, but are not limited to, small interfering RNAs (siRNAs) and microRNAs (miRNAs). Also included long noncoding RNAs (lncRNAs).

As used herein, inhibiting, suppressing, disrupting or silencing a targeted gene refers to processes that alter expression, such as translation, of the targeted gene, whereby activity or expression of the product encoded by the targeted gene is reduced. Reduction includes a complete knock-out or a partial knockout, whereby, with reference to the MEVs provided herein and administration herein, treatment is effected.

As used herein, small activating RNA (saRNA) is a small double-stranded RNA that targets a gene promoter to activate a gene. They are generally small, generally 21 nucleotides long. They are structurally similar to siRNA, but the activity of the saRNA is to activate expression; whereas siRNA inhibits expression of its target.

As used herein, self-amplifying RNA refers to RNA that encodes a viral replicase. Upon entry into a host cell, the replicase is translated, which makes a complementary negative copy of the mRNA, which then is used by the replicase as a template to synthesize more mRNA.

As used herein, an open reading frame (ORF) is sequence of nucleotides that encodes a start codon followed by a downstream in-frame stop codon. Eukaryotic mRNAs predominantly have a single primary ORF and comprises the protein coding sequence (CDS). Shorter ORFs also can occur in the transcript. These are called small ORFs (sORFs).

As used herein, small ORF-encoded peptides (sORFs) occur in non-coding RNA, including in long non-coding RNA (lncRNA), circular RNA, and ribosomal RNA. sORFs encode proteins, small peptides and peptides that are generally less than 100 amino acids. sORFs can include an AUG canonical start codon, but also can include near-cognate codons, such CUG, GUG, UUG, and ACG, that differ from AUG by one nucleotide; sORFs generally terminate with a stop codon (UAA, UGA, and UAG). Many of the products encoded by sORFs have regulatory functions, such as modulating ribosomal.

As used herein, long RNA is an RNA molecule that can be enzymatically processed into shorter RNAs, such as short-hairpin RNA (shRNA), micro-RNA, and other RNAs that inhibit or silence expression of mRNA or is long noncoding RNA (lncRNA). For example, small interfering RNAs (siRNAs) are small pieces of double-stranded (ds) RNA, usually about 18 - 23 nucleotides long, with 3' overhangs (2 nucleotides) at each end that can interfere with the translation of

proteins by binding to and promoting the degradation of messenger RNA (mRNA) at specific sequences. In doing so, siRNAs prevent the production of specific proteins based on the nucleotide sequences of their corresponding mRNAs. The process is called RNA interference (RNAi), and also is referred to as siRNA silencing or siRNA knockdown. A short-hairpin RNA or small-hairpin RNA (shRNA) is an artificial RNA molecule with a tight hairpin turn that can be used to silence target gene expression via RNA interference (RNAi). Expression of shRNA in cells can be accomplished by delivery of plasmids or through viral or bacterial vectors.

As used herein, inhibiting, suppressing, disrupting or silencing a targeted gene refers to processes that alter expression, such as translation, of the targeted gene, whereby activity or expression of the product encoded by the targeted gene is reduced. Reduction includes a complete knock-out or a partial knockout, whereby, with reference to the MEVs provided herein and administration herein, treatment is effected.

As used herein, coding RNA is RNA that encodes a protein, polypeptide, peptide or small Open Reading Frame (sORFs) or a small peptide, generally 10 or fewer amino acids, or peptides (less than hundred amino acids). Classes of coding RNA, include, but are not limited to, mRNA or messenger RNAs that code for proteins and peptides, and long noncoding RNAs (lncRNAs) that encode a sORF encoding a small peptides or peptides.

As used herein, a tumor microenvironment (TME) is the cellular environment in which the tumor exists, including surrounding blood vessels, immune cells, fibroblasts, bone marrow-derived inflammatory cells, lymphocytes, signaling molecules and the extracellular matrix (ECM). Conditions that exist include, but are not limited to, increased vascularization, hypoxia, low pH, increased lactate concentration, increased pyruvate concentration, increased interstitial fluid pressure and altered metabolites or metabolism, such as higher levels of adenosine, indicative of a tumor.

As used herein, recitation that a nucleic acid or encoded RNA targets a gene means that it inhibits or suppresses or silences expression of the gene by any mechanism. Generally, such nucleic acid includes at least a portion complementary to the targeted gene, where the portion is sufficient to form a hybrid with the complementary portion.

As used herein, deletion, when referring to a nucleic acid or polypeptide sequence, refers to the deletion of one or more nucleotides or amino acids compared to a sequence, such as a target polynucleotide or polypeptide or a native or wild-type sequence.

As used herein, insertion, when referring to a nucleic acid or amino acid sequence, describes the inclusion of one or more additional nucleotides or amino acids, within a target, native, wild-type or other related sequence. Thus, a nucleic acid molecule that contains one or more insertions compared to a wild-type sequence, contains one or more additional nucleotides within the linear length of the sequence.

As used herein, additions to nucleic acid and amino acid sequences describe addition of nucleotides or amino acids onto either termini compared to another sequence.

As used herein, substitution or replacement refers to the replacing of one or more nucleotides or amino acids in a native, target, wild-type or other nucleic acid or polypeptide sequence with an alternative nucleotide or amino acid, without changing the length (as described in numbers of residues) of the molecule. Thus, one or more substitutions in a molecule does not change the number of amino acid residues or nucleotides of the molecule. Amino acid replacements compared to a particular polypeptide can be expressed in terms of the number of the amino acid residue along the length of the polypeptide sequence.

As used herein, at a position corresponding to, or a recitation that nucleotides or amino acid positions correspond to nucleotides or amino acid positions in a disclosed sequence, such as set forth in the Sequence Listing, refers to nucleotides or amino acid positions identified upon alignment with the disclosed sequence to maximize identity using a standard alignment algorithm, such as the GAP algorithm. By aligning the sequences, one skilled in the art can identify corresponding residues, for example, using conserved and identical amino acid residues as guides. In general, to identify corresponding positions, the sequences of amino acids are aligned so that the highest order match is obtained (see, *e.g.,* Computational Molecular Biology, Lesk, A.M., ed., Oxford University Press, New York, 1988; Biocomputing: Informatics and Genome Projects, Smith, D. W., ed., Academic Press, New York, 1993; Computer Analysis of Sequence Data, Part I, Griffin, A.M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M Stockton Press, New York, 1991; and Carrillo et al. (1988) SIAM J Applied Math 48:1073).

As used herein, alignment of a sequence refers to the use of homology to align two or more sequences of nucleotides or amino acids. Typically, two or more sequences that are related by 50% or more identity are aligned. An aligned set of sequences refers to 2 or more sequences that are aligned at corresponding positions and can include aligning sequences derived from RNAs, such as ESTs and other cDNAs, aligned with genomic DNA sequence. Related or variant polypeptides or nucleic acid molecules can be aligned by any method known to those of skill in the art. Such methods typically maximize matches, and include methods, such as using manual alignments and by using the numerous alignment programs available (*e.g.*, BLASTP) and others known to those of skill in the art. By aligning the sequences of polypeptides or nucleic acids, one skilled in the art can identify analogous portions or positions, using conserved and identical amino acid residues as guides. Further, one skilled in the art also can employ conserved amino acid or nucleotide residues as guides to find corresponding amino acid or nucleotide residues between and among human and non-human

sequences. Corresponding positions also can be based on structural alignments, for example by using computer simulated alignments of protein structure. In other instances, corresponding regions can be identified. One skilled in the art also can employ conserved amino acid residues as guides to find corresponding amino acid residues between and among human and non-human sequences.

As used herein, a property of a polypeptide, such as an antibody, refers to any property exhibited by a polypeptide, including, but not limited to, binding specificity, structural configuration or conformation, protein stability, resistance to proteolysis, conformational stability, thermal tolerance, and tolerance to pH conditions. Changes in properties can alter an activity of the polypeptide. For example, a change in the binding specificity of the antibody polypeptide can alter the ability to bind an antigen, and/or various binding activities, such as affinity or avidity, or *in vivo* activities of the polypeptide.

As used herein, an activity or a functional activity of a polypeptide, such as an antibody, refers to any activity exhibited by the polypeptide. Such activities can be empirically determined. Exemplary activities include, but are not limited to, ability to interact with a biomolecule, for example, through antigen-binding, DNA binding, ligand binding, dimerization, or enzymatic activity, for example, kinase activity or proteolytic activity. For an antibody (including antibody fragments), activities include, but are not limited to, the ability to specifically bind a particular antigen, affinity of antigen-binding (*e.g.*, high or low affinity), avidity of antigen-binding (*e.g.*, high or low avidity), on-rate, off-rate, effector functions, such as the ability to promote antigen neutralization or clearance, virus neutralization, and *in vivo* activities, such as the ability to prevent infection or invasion of a pathogen, or to promote clearance, or to penetrate a particular tissue or fluid or cell in the body. Activity can be assessed *in vitro* or *in vivo* using recognized assays, such as ELISA, flow cytometry, surface plasmon resonance or equivalent assays to measure on- or off-rate, immunohistochemistry and immunofluorescence histology and microscopy, cell-based assays, flow cytometry and binding assays (*e.g.*, panning assays).

As used herein, bind, bound, and grammatical variations thereof refers to the participation of a molecule in any interaction with another molecule or among molecules, resulting in a stable association in which the molecules are in close proximity to one another. Binding includes, but is not limited to, non-covalent bonds, covalent bonds (such as reversible and irreversible covalent bonds), and includes interactions between molecules such as, but not limited to, proteins, nucleic acids, carbohydrates, lipids, and small molecules, such as chemical compounds including drugs.

As used herein, antibody refers to immunoglobulins and immunoglobulin fragments, whether natural or partially or wholly synthetically, such as recombinantly produced, including any fragment thereof containing at least a portion of the variable heavy chain and light region of the immunoglobulin molecule that is sufficient to form an antigen binding site and, when assembled, to specifically bind an antigen. Hence, an antibody includes any protein having a binding domain that is homologous or substantially homologous to an immunoglobulin antigen-binding domain (antibody combining site). For example, an antibody refers to an antibody that contains two heavy chains (which can be denoted H and H') and two light chains (which can be denoted L and L'), where each heavy chain can be a full-length immunoglobulin heavy chain or a portion thereof sufficient to form an antigen binding site (*e.g.*, heavy chains include, but are not limited to, VH chains, VH-CH1 chains and VH-CH1-CH2-CH3 chains), and each light chain can be a full-length light chain or a portion thereof sufficient to form an antigen binding site (*e.g.,* light chains include, but are not limited to, VL chains and VL-CL chains). Each heavy chain (H and H') pairs with one light chain (L and L', respectively). Typically, antibodies minimally include all or at least a portion of the variable heavy (VH) chain and/or the variable light (VL) chain. The antibody also can include all or a portion of the constant region.

For purposes herein, the term antibody includes full-length antibodies and portions thereof including antibody fragments, such as anti-tumor antibody or anti-pathogen or gene silencing fragments. Antibody fragments, include, but are not limited to, Fab fragments, Fab' fragments, $F(ab')_2$ fragments, Fv fragments, disulfide-linked Fvs (dsFv), Fd fragments, Fd' fragments, single-chain Fvs (scFv), single-chain Fabs (scFab), diabodies, anti-idiotypic (anti-Id) antibodies, or antigen-binding fragments of any of the above. Antibody also includes synthetic antibodies, recombinantly produced antibodies, multispecific antibodies (*e.g.*, bispecific antibodies), human antibodies, non-human antibodies, humanized antibodies, chimeric antibodies, and intrabodies. Antibodies provided herein include members of any immunoglobulin class (*e.g.,* IgG, IgM, IgD, IgE, IgA and IgY), any subclass (*e.g.*, IgG1, IgG2, IgG3, IgG4, IgA1 and IgA2) or sub-subclass (*e.g.*, IgG2a and IgG2b).

As used herein, nucleic acid refers to at least two linked nucleotides or nucleotide derivatives, including a deoxyribonucleic acid (DNA) and a ribonucleic acid (RNA), joined together, typically by phosphodiester linkages. Also included in the term nucleic acid are analogs of nucleic acids such as peptide nucleic acid (PNA), phosphorothioate DNA, and other such analogs and derivatives or combinations thereof. Nucleic acids also include DNA and RNA derivatives containing, for example, a nucleotide analog or a backbone bond other than a phosphodiester bond, for example, a phosphotriester bond, a phosphoramidate bond, a phosphorothioate bond, a thioester bond, or a peptide bond (peptide nucleic acid). The term also includes, as equivalents, derivatives, variants and analogs of either RNA or DNA made from nucleotide analogs, single (sense or antisense) and double-stranded nucleic acids. Deoxyribonucleotides include deoxyadenosine, deoxycytidine, deoxyguanosine and deoxythymidine. For RNA, the uracil base is uridine.

As used herein, an isolated nucleic acid molecule is one which is separated from other nucleic acid molecules

which are present in the natural source of the nucleic acid molecule. An isolated nucleic acid molecule, such as a cDNA molecule, can be substantially free of other cellular material, or culture medium when produced by recombinant techniques, or substantially free of chemical precursors or other chemicals when chemically synthesized. Exemplary isolated nucleic acid molecules provided herein include isolated nucleic acid molecules encoding RNAi or a therapeutic protein.

As used herein, operably linked with reference to nucleic acid sequences, regions, elements or domains means that the nucleic acid regions are functionally related to each other. For example, a nucleic acid encoding a leader peptide can be operably linked to a nucleic acid encoding a polypeptide, whereby the nucleic acids can be transcribed and translated to express a functional fusion protein, wherein the leader peptide effects secretion of the fusion polypeptide. In some instances, the nucleic acid encoding a first polypeptide (*e.g.*, a leader peptide) is operably linked to a nucleic acid encoding a second polypeptide and the nucleic acids are transcribed as a single mRNA transcript, but translation of the mRNA transcript can result in one of two polypeptides being expressed. For example, an amber stop codon can be located between the nucleic acid encoding the first polypeptide and the nucleic acid encoding the second polypeptide, such that, when introduced into a partial amber suppressor cell, the resulting single mRNA transcript can be translated to produce either a fusion protein containing the first and second polypeptides, or can be translated to produce only the first polypeptide. In another example, a promoter can be operably linked to nucleic acid encoding a polypeptide, whereby the promoter regulates or mediates the transcription of the nucleic acid.

As used herein, synthetic, with reference to, for example, a synthetic nucleic acid molecule or a synthetic gene or a synthetic peptide refers to a nucleic acid molecule or polypeptide molecule that is produced by recombinant methods and/or by chemical synthesis methods.

As used herein, the residues of naturally occurring $\alpha$-amino acids are the residues of those 20 $\alpha$-amino acids found in nature which are incorporated into protein by the specific recognition of the charged tRNA molecule with its cognate mRNA codon in humans.

As used herein, polypeptide refers to two or more amino acids covalently joined. The terms polypeptide and protein generally are used interchangeably. Polypeptide, peptide, and protein refer to polymers of amino acids of any length. Where not used interchangeably other characteristics, such as size, and structure, are contemplated, as defined below. The polymer can be linear or branched, it can contain or include modified amino acids, and it can be interrupted by non-amino acids. Also included are amino acid polymers that include sequence modifications including, replacements, insertions, deletions, and transpositions. Also included are amino acid polymers that contain post-translational modifications, such as disulfide bonds, glycosylation, sialylation, conjugation to other proteins, peptides, and polypeptides, such, but not limited to, conjugation to a detectable marker, or reporter.

As used herein, a peptide contains at least 2 amino acids, and generally fewer than 100 amino acids, such as 2 to 40. A small peptide is a peptide that contains fewer than 10 amino acids, typically 2 to 6 amino acids.

As used herein, a polypeptide is an amino acid chain that contains a plurality of peptides, and is generally 100 amino acids or longer.

As used herein, a protein is a polypeptide that has a three dimensional structure and can include bonds in addition to peptide bonds, such as disulfide bonds and other interactions, that participate in forming the two- and three-dimensional structure.

As used herein, an amino acid is an organic compound containing an amino group and a carboxylic acid group. A polypeptide contains two or more amino acids. For purposes herein, amino acids contained in the antibodies provided include the twenty naturally-occurring amino acids (see Table below), non-natural amino acids, and amino acid analogs (*e. g.*, amino acids wherein the $\alpha$-carbon has a side chain). As used herein, the amino acids, which occur in the various amino acid sequences of polypeptides appearing herein, are identified according to their well-known, three-letter or one-letter abbreviations (see Table below). The nucleotides, which occur in the various nucleic acid molecules and fragments, are designated with the standard single-letter designations used routinely in the art.

As used herein, amino acid residue refers to an amino acid formed upon chemical digestion (hydrolysis) of a polypeptide at its peptide linkages. The amino acid residues described herein are generally in the L isomeric form. Residues in the D isomeric form can be substituted for any L-amino acid residue, as long as the desired functional property is retained by the polypeptide. $NH_2$ refers to the free amino group present at the amino terminus of a polypeptide. COOH refers to the free carboxy group present at the carboxyl terminus of a polypeptide. In keeping with standard polypeptide nomenclature described in J. Biol. Chem., 243:3557-59 (1968) and adopted at 37 C.F.R. §§ 1.821 - 1.822, abbreviations for amino acid residues are shown in the following Table:

**Table of Correspondence**

| SYMBOL | | |
|---|---|---|
| **1-Letter** | **3-Letter** | **AMINO ACID** |
| Y | Tyr | Tyrosine |

(continued)

| SYMBOL | | |
|---|---|---|
| 1-Letter | 3-Letter | AMINO ACID |
| G | Gly | Glycine |
| F | Phe | Phenylalanine |
| M | Met | Methionine |
| A | Ala | Alanine |
| S | Ser | Serine |
| I | Ile | Isoleucine |
| L | Leu | Leucine |
| T | Thr | Threonine |
| V | Val | Valine |
| P | Pro | Proline |
| K | Lys | Lysine |
| H | His | Histidine |
| Q | Gln | Glutamine |
| E | Glu | Glutamic acid |
| Z | Glx | Glutamic Acid and/or Glutamine |
| W | Trp | Tryptophan |
| R | Arg | Arginine |
| D | Asp | Aspartic acid |
| N | Asn | Asparagine |
| B | Asx | Aspartic Acid and/or Asparagine |
| C | Cys | Cysteine |
| X | Xaa | Unknown or other |

All sequences of amino acid residues represented herein by a formula have a left to right orientation in the conventional direction of amino-terminus to carboxyl-terminus. The phrase amino acid residue is defined to include the amino acids listed in the above Table of Correspondence, modified, non-natural and unusual amino acids. A dash at the beginning or end of an amino acid residue sequence indicates a peptide bond to a further sequence of one or more amino acid residues or to an amino-terminal group such as $NH_2$ or to a carboxyl-terminal group such as COOH.

In a peptide or protein, suitable conservative substitutions of amino acids are known to those of skill in the art and generally can be made without altering a biological activity of a resulting molecule. Those of skill in the art recognize that, in general, single amino acid substitutions in non-essential regions of a polypeptide do not substantially alter biological activity (see, *e.g.,* Watson et al., Molecular Biology of the Gene, 4th Edition, 1987, The Benjamin/Cummings Pub. Co., p. 224).

Such substitutions can be made in accordance with the exemplary substitutions set forth in the following Table:

**Exemplary conservative amino acid substitutions**

| Original residue | Exemplary Conservative substitution(s) |
|---|---|
| Ala (A) | Gly; Ser |
| Arg (R) | Lys |
| Asn (N) | Gln; His |

(continued)

| Original residue | Exemplary Conservative substitution(s) |
|---|---|
| Cys (C) | Ser |
| Gln (Q) | Asn |
| Glu (E) | Asp |
| Gly (G) | Ala; Pro |
| His (H) | Asn; Gln |
| Ile (I) | Leu; Val |
| Leu (L) | Ile; Val |
| Lys (K) | Arg; Gln; Glu |
| Met (M) | Leu; Tyr; Ile |
| Phe (F) | Met; Leu; Tyr |
| Ser (S) | Thr |
| Thr (T) | Ser |
| Trp (W) | Tyr |
| Tyr (Y) | Trp; Phe |
| Val (V) | Ile; Leu |

Other substitutions also are permissible and can be determined empirically or in accord with other known conservative or non-conservative substitutions.

As used herein, naturally occurring amino acids refer to the 20 L-amino acids that occur in polypeptides.

As used herein, the term non-natural amino acid refers to an organic compound that has a structure similar to a natural amino acid but has been modified structurally to mimic the structure and reactivity of a natural amino acid. Non-naturally occurring amino acids thus include, for example, amino acids or analogs of amino acids other than the 20 naturally occurring amino acids and include, but are not limited to, the D-stereoisomers of amino acids. Exemplary non-natural amino acids are known to those of skill in the art, and include, but are not limited to, 2-Aminoadipic acid (Aad), 3-Aminoadipic acid (bAad), β-alanine/β-Amino-propionic acid (Bala), 2-Aminobutyric acid (Abu), 4-Aminobutyric acid/piperidinic acid (4Abu), 6-Aminocaproic acid (Acp), 2-Aminoheptanoic acid (Ahe), 2-Aminoisobutyric acid (Aib), 3-Aminoisobutyric acid (Baib), 2-Aminopimelic acid (Apm), 2,4-Diaminobutyric acid (Dbu), Desmosine (Des), 2,2'-Diaminopimelic acid (Dpm), 2,3-Diaminopropionic acid (Dpr), N-Ethylglycine (EtGly), N-Ethylasparagine (EtAsn), Hydroxylysine (Hyl), allo-Hydroxylysine (Ahyl), 3-Hydroxyproline (3Hyp), 4-Hydroxyproline (4Hyp), Isodesmosine (Ide), allo-Isoleucine (Aile), N-Methylglycine, sarcosine (MeGly), N-Methylisoleucine (MeIle), 6-N-Methyllysine (MeLys), N-Methylvaline (MeVal), Norvaline (Nva), Norleucine (Nle), and Ornithine (Orn).

As used herein, a DNA construct is a single or double stranded, linear or circular DNA molecule that contains segments of DNA combined and juxtaposed in a manner not found in nature. DNA constructs exist as a result of human manipulation, and include clones and other copies of manipulated molecules.

As used herein, a DNA segment is a portion of a larger DNA molecule having specified attributes. For example, a DNA segment encoding a specified polypeptide is a portion of a longer DNA molecule, such as a plasmid or plasmid fragment, which, when read from the 5' to 3' direction, encodes the sequence of amino acids of the specified polypeptide.

As used herein, the term polynucleotide means a single- or double-stranded polymer of deoxyribonucleotides or ribonucleotide bases read from the 5' to the 3' end. Polynucleotides include RNA and DNA, and can be isolated from natural sources, synthesized *in vitro,* or prepared from a combination of natural and synthetic molecules. The length of a polynucleotide molecule is given herein in terms of nucleotides (abbreviated nt) or base pairs (abbreviated bp). The term "nucleotides" is used for single- and double-stranded molecules where the context permits. When the term is applied to double-stranded molecules it is used to denote overall length and will be understood to be equivalent to the term base pairs. It will be recognized by those skilled in the art that the two strands of a double-stranded polynucleotide can differ slightly in length and that the ends thereof can be staggered; thus, all nucleotides within a double-stranded polynucleotide molecule cannot be paired. Such unpaired ends will, in general, not exceed 20 nucleotides in length.

As used herein, production by recombinant methods refers to or means the use of the well-known methods of molecular biology for expressing proteins encoded by cloned DNA.

As used herein, heterologous nucleic acid is nucleic acid that encodes products (*i.e.*, RNA and/or proteins) that

are not normally produced *in vivo* by the cell in which it is expressed, or nucleic acid that is in a locus in which it does not normally occur, or that mediates or encodes mediators that alter expression of endogenous nucleic acid, such as DNA, by affecting transcription, translation, or other regulatable biochemical processes. Heterologous nucleic acid, such as DNA, also is referred to as foreign nucleic acid. Any nucleic acid, such as DNA, that one of skill in the art would recognize or consider as heterologous or foreign to the cell in which it is expressed, is herein encompassed by heterologous nucleic acid; heterologous nucleic acid includes exogenously added nucleic acid that is also expressed endogenously. Heterologous nucleic acid is generally not endogenous to the cell into which it is introduced, but has been obtained from another cell or prepared synthetically or is introduced into a genomic locus in which it does not occur naturally, or its expression is under the control of regulatory sequences or a sequence that differs from the natural regulatory sequence or sequences.

Examples of heterologous nucleic acid herein include, but are not limited to, a DNA molecule, an RNA molecule, a plasmid, and an antisense oligonucleotide. In the MEV, the heterologous nucleic acid can be encoded on a plasmid. Heterologous nucleic acid, such as DNA, includes nucleic acid that can, in some manner, mediate expression of DNA that encodes a therapeutic product, or it can encode a product, such as a peptide or RNA, that in some manner mediates, directly or indirectly, expression of a therapeutic product.

As used herein, cell therapy involves the delivery of MEVs to a subject to treat a disease or condition. These are exogenously loaded with cargo, so that they deliver or express products when introduced to a subject.

As used herein, genetic therapy involves the transfer of heterologous nucleic acid, such as DNA, into certain cells, such as target cells, of a mammal, particularly a human, with a disorder or condition for which such therapy is sought. The nucleic acid, such as DNA, is introduced into the selected target cells in a manner such that the heterologous nucleic acid, such as DNA, is expressed and a therapeutic product(s) encoded thereby is produced. Genetic therapy can also be used to deliver nucleic acid encoding a gene product that replaces a defective gene or supplements a gene product produced by the mammal or the cell in which it is introduced. The introduced nucleic acid can encode a therapeutic compound, such as a growth factor or inhibitor thereof, or a tumor necrosis factor or inhibitor thereof, such as a receptor thereof, that is not normally produced in the mammalian host or that is not produced in therapeutically effective amounts or at a therapeutically useful time. The heterologous nucleic acid, such as DNA, encoding the therapeutic product, can be modified prior to introduction into the cells of the afflicted host in order to enhance or otherwise alter the product or expression thereof. Genetic therapy can also involve delivery of an inhibitor or repressor or other modulator of gene expression.

As used herein, expression refers to the process by which polypeptides are produced by transcription and translation of polynucleotides. The level of expression of a polypeptide can be assessed using any method known in art, including, for example, methods of determining the amount of the polypeptide produced from the host cell. Such methods can include, but are not limited to, quantitation of the polypeptide in the cell lysate by ELISA, Coomassie blue staining following gel electrophoresis, Lowry protein assay and Bradford protein assay.

As used herein, a host cell is a cell that is used to receive, maintain, reproduce and/or amplify a vector. A host cell also can be used to express the polypeptide encoded by the vector. The nucleic acid contained in the vector is replicated when the host cell divides, thereby amplifying the nucleic acids.

As used herein, a vector is a replicable nucleic acid from which one or more heterologous proteins, can be expressed when the vector is transformed into an appropriate host cell. Reference to a vector includes those vectors into which a nucleic acid encoding a polypeptide or fragment thereof can be introduced, typically by restriction enzyme digestion and ligation. Reference to a vector also includes those vectors that contain nucleic acid encoding a polypeptide or RNA. The vector is used to introduce the nucleic acid encoding the polypeptide into the host cell for amplification of the nucleic acid or for expression/display of the polypeptide encoded by the nucleic acid. The vectors typically remain episomal, but can be designed to effect integration of a gene or portion thereof into a chromosome of the genome. Also contemplated are vectors that are artificial chromosomes, such as yeast artificial chromosomes and mammalian artificial chromosomes. Selection and use of such vehicles are well-known to those of skill in the art. A vector also includes virus vectors or viral vectors. Viral vectors are engineered viruses that are operatively linked to exogenous genes to transfer (as vehicles or shuttles) the exogenous genes into cells.

As used herein, an expression vector includes vectors capable of expressing DNA that is operatively linked with regulatory sequences, such as promoter regions, that are capable of effecting expression of such DNA fragments. Such additional segments can include promoter and terminator sequences, and optionally can include one or more origins of replication, one or more selectable markers, an enhancer, a polyadenylation signal, and the like. Expression vectors are generally derived from plasmid or viral DNA, or can contain elements of both. Thus, an expression vector refers to a recombinant DNA or RNA construct, such as a plasmid, a phage, recombinant virus or other vector that, upon introduction into an appropriate host cell, results in expression of the cloned DNA. Appropriate expression vectors are well-known to those of skill in the art and include those that are replicable in eukaryotic cells and/or prokaryotic cells and those that remain episomal or those which integrate into the host cell genome.

As used herein, primary sequence refers to the sequence of amino acid residues in a polypeptide or the sequence of nucleotides in a nucleic acid molecule.

As used herein, sequence identity refers to the number of identical or similar amino acids or nucleotide bases in a

comparison between a test and a reference polypeptide or polynucleotide. Sequence identity can be determined by sequence alignment of nucleic acid or protein sequences to identify regions of similarity or identity. For purposes herein, sequence identity is generally determined by alignment to identify identical residues. The alignment can be local or global. Matches, mismatches and gaps can be identified between compared sequences. Gaps are null amino acids or nucleotides inserted between the residues of aligned sequences so that identical or similar characters are aligned. Generally, there can be internal and terminal gaps. When using gap penalties, sequence identity can be determined with no penalty for end gaps (*e.g.*, terminal gaps are not penalized). Alternatively, sequence identity can be determined without taking into account gaps as the number of identical positions/length of the total aligned sequence x 100.

As used herein, a global alignment is an alignment that aligns two sequences from beginning to end, aligning each letter in each sequence only once. An alignment is produced, regardless of whether or not there is similarity or identity between the sequences. For example, 50% sequence identity based on global alignment means that in an alignment of the full sequence of two compared sequences each of 100 nucleotides in length, 50% of the residues are the same. It is understood that global alignment also can be used in determining sequence identity even when the length of the aligned sequences is not the same. The differences in the terminal ends of the sequences will be taken into account in determining sequence identity, unless the "no penalty" for end gaps is selected. Generally, a global alignment is used on sequences that share significant similarity over most of their length. Exemplary algorithms for performing global alignment include the Needleman-Wunsch algorithm (Needleman et al. (1970) J. Mol. Biol. 48: 443). Exemplary programs for performing global alignment are publicly available and include the Global Sequence Alignment Tool available at the National Center for Biotechnology Information (NCBI) website (ncbi.nlm.nih.gov/), and the program available at deepc2.psi.iastate.edu/aat/align/align.html.

As used herein, a local alignment is an alignment that aligns two sequences, but only aligns those portions of the sequences that share similarity or identity. Hence, a local alignment determines if sub-segments of one sequence are present in another sequence. If there is no similarity, no alignment will be returned. Local alignment algorithms include BLASTor Smith-Waterman algorithm (Adv. Appl. Math. 2: 482 (1981)). For example, 50% sequence identity based on local alignment means that in an alignment of the full sequence of two compared sequences of any length, a region of similarity or identity of 100 nucleotides in length has 50% of the residues that are the same in the region of similarity or identity.

For purposes herein, sequence identity can be determined by standard alignment algorithm programs used with default gap penalties established by each supplier. Default parameters for the GAP program can include: (1) a unary comparison matrix (containing a value of 1 for identities and 0 for non-identities) and the weighted comparison matrix of Gribskov et al. (1986) Nucl. Acids Res. 14:6745, as described by Schwartz and Dayhoff, eds., Atlas of Protein Sequence and Structure, National Biomedical Research Foundation, pp. 353-358 (1979); (2) a penalty of 3.0 for each gap and an additional 0.10 penalty for each symbol in each gap; and (3) no penalty for end gaps. Whether any two nucleic acid molecules have nucleotide sequences or any two polypeptides have amino acid sequences that are at least 80%, 85%, 90%, 95%, 96%, 97%, 98% or 99% identical, or other similar variations reciting a percent identity, can be determined using known computer algorithms based on local or global alignment (*see e.g.,* wikipedia.org/wiki/Sequence_alignment_software, providing links to dozens of known and publicly available alignment databases and programs). Generally, for purposes herein sequence identity is determined using computer algorithms based on global alignment, such as the Needleman-Wunsch Global Sequence Alignment tool available from NCBI/BLAST (blast.ncbi.nlm.nih.gov/Blast.cgi?CMD=Web&Page_TYPE=BlastHome); LAlign (William Pearson implementing the Huang and Miller algorithm (Adv. Appl. Math. (1991) 12:337-357)); and program from Xiaoqui Huang available at deepc2.psi.iastate.edu/aat/align/align.html. Typically, the full-length sequence of each of the compared polypeptides or nucleotides is aligned across the full-length of each sequence in a global alignment. Local alignment also can be used when the sequences being compared are substantially the same length.

Therefore, as used herein, the term identity represents a comparison or alignment between a test and a reference polypeptide or polynucleotide. In one nonlimiting example, at least 90% identical to refers to percent identities from 90 to 100% relative to the reference polypeptide or polynucleotide. Identity at a level of 90% or more is indicative of the fact that, assuming for exemplification purposes a test and reference polypeptide or polynucleotide length of 100 amino acids or nucleotides are compared, no more than 10% (*i.e.*, 10 out of 100) of amino acids or nucleotides in the test polypeptide or polynucleotide differ from those of the reference polypeptide. Similar comparisons can be made between a test and reference polynucleotides. Such differences can be represented as point mutations randomly distributed over the entire length of an amino acid sequence or they can be clustered in one or more locations of varying length up to the maximum allowable, *e.g.*, 10/100 amino acid difference (approximately 90% identity). Differences also can be due to deletions or truncations of amino acid residues. Differences are defined as nucleic acid or amino acid substitutions, insertions or deletions. Depending on the length of the compared sequences, at the level of homologies or identities above about 85-90%, the result can be independent of the program and gap parameters set; such high levels of identity can be assessed readily, often without relying on software.

As used herein, a pharmaceutically effective agent includes any therapeutic agent or bioactive agents, including, but not limited to, for example, anesthetics, vasoconstrictors, dispersing agents, and conventional therapeutic drugs,

including small molecule drugs and therapeutic proteins.

As used herein, a therapeutic effect means an effect resulting from treatment of a subject that alters, typically improves or ameliorates, the symptoms of a disease or condition or that cures a disease or condition.

As used herein, a therapeutically effective amount or a therapeutically effective dose refers to the quantity of an agent, compound, material, or composition containing a compound that is at least sufficient to produce a therapeutic effect following administration to a subject. Hence, it is the quantity necessary for preventing, curing, ameliorating, arresting or partially arresting a symptom of a disease or disorder.

As used herein, therapeutic efficacy refers to the ability of an agent, compound, material, or composition containing a compound to produce a therapeutic effect in a subject to whom the agent, compound, material, or composition containing a compound has been administered.

As used herein, a prophylactically effective amount or a prophylactically effective dose refers to the quantity of an agent, compound, material, or composition containing a compound that when administered to a subject, will have the intended prophylactic effect, *e.g.*, preventing or delaying the onset, or reoccurrence, of disease or symptoms, reducing the likelihood of the onset, or reoccurrence, of disease or symptoms, or reducing the incidence of viral infection. The full prophylactic effect does not necessarily occur by administration of one dose, and can occur only after administration of a series of doses. Thus, a prophylactically effective amount can be administered in one or more administrations.

As used herein, amelioration of the symptoms of a particular disease or disorder by a treatment, such as by administration of a pharmaceutical composition or other therapeutic, refers to any lessening, whether permanent or temporary, lasting or transient, of the symptoms that can be attributed to or associated with administration of the composition or therapeutic.

As used herein, an anti-cancer agent refers to any agent that is destructive or toxic to malignant cells and tissues. For example, anti-cancer agents include agents that kill cancer cells or otherwise inhibit or impair the growth of tumors or cancer cells. Exemplary anti-cancer agents are chemotherapeutic agents.

As used herein, gene editing systems or technology include clustered regularly interspaced short palindromic repeats (CRISPR)-CRISPR-associated protein 9 (Cas9), and also other systems and technologies that are used to edit genomes. These include, for example: transcription activator-like effector nucleases (TALENs), zinc-finger nucleases (ZFNs), and homing endonucleases or meganucleases, and other "CRISPR-like" and CRISPR-associated systems, such as the systems developed by Metagenomi.

As used herein therapeutic activity refers to the *in vivo* activity of a therapeutic polypeptide. Generally, the therapeutic activity is the activity that is associated with treatment of a disease or condition.

As used herein, a vaccine refers to compositions comprising the MEVs, such as the MEVs exo-loaded with an antigen (such as a small peptide, peptide, polypeptide, and protein) or antigen-producer molecule (such as a mRNA). The vaccine can be used to treat a disease or prevent (reduce the risk of getting a disease), such as for prophylaxis. Vaccines can elicit a humoral immune response, or a cellular immune response, or a combination thereof, such as against pathogens, cancer cells, immune checkpoints, and other therapeutic targets for intervention.

As used herein, the term subject refers to an animal, including a mammal, such as a human being.

As used herein, a patient refers to a human subject.

As used herein, animal includes any animal, such as, but not limited to, primates including humans, gorillas and monkeys; rodents, such as mice and rats; fowl, such as chickens; ruminants, such as goats, cows, deer, and sheep; and pigs and other animals. Non-human animals exclude humans as the contemplated animal.

As used herein, a composition refers to any mixture. It can be a solution, suspension, liquid, powder, paste, aqueous, non-aqueous, or any combination thereof.

As used herein, a combination refers to any association between or among two or more items. The combination can be two or more separate items, such as two compositions or two collections, a mixture thereof, such as a single mixture of the two or more items, or any variation thereof. The elements of a combination are generally functionally associated or related.

As used herein, combination therapy refers to administration of two or more different therapeutics. The different therapeutic agents can be provided and administered separately, sequentially, intermittently, or can be provided in a single composition.

As used herein, a kit is a packaged combination that optionally includes other elements, such as additional reagents and instructions for use of the combination or elements thereof, for a purpose including, but not limited to, activation, administration, diagnosis, and assessment of a biological activity or property.

As used herein, a unit dose form refers to physically discrete units suitable for human and animal subjects and packaged individually as is known in the art.

As used herein, a single dosage formulation refers to a formulation for direct administration.

As used herein, a multi-dose formulation refers to a formulation that contains multiple doses of a therapeutic agent and that can be directly administered to provide several single doses of the therapeutic agent. The doses can be administered over the course of minutes, hours, weeks, days or months. Multi-dose formulations can allow dose

adjustment, dose-pooling and/or dose-splitting. Because multi-dose formulations are used over time, they generally contain one or more preservatives to prevent microbial growth.

As used herein, an article of manufacture is a product that is made and sold. As used throughout this application, the term is intended to encompass any of the compositions provided herein contained in articles of packaging.

As used herein, a fluid refers to any composition that can flow. Fluids thus encompass compositions that are in the form of semi-solids, pastes, solutions, aqueous mixtures, gels, lotions, creams and other such compositions.

As used herein, an isolated or purified polypeptide or protein (*e.g.*, an isolated antibody or antigen-binding fragment thereof) or biologically-active portion thereof (*e.g.*, an isolated antigen-binding fragment) is substantially free of cellular material or other contaminating proteins from the cell or tissue from which the protein is derived, or substantially free from chemical precursors or other chemicals when chemically synthesized. Preparations can be determined to be substantially free if they appear free of readily detectable impurities as determined by standard methods of analysis, such as thin layer chromatography (TLC), gel electrophoresis and high performance liquid chromatography (HPLC), used by those of skill in the art to assess such purity, or sufficiently pure such that further purification does not detectably alter the physical and chemical properties, such as enzymatic and biological activities, of the substance. Methods for purification of the compounds to produce substantially chemically pure compounds are known to those of skill in the art. A substantially chemically pure compound, however, can be a mixture of stereoisomers. In such instances, further purification might increase the specific activity of the compound. As used herein, a cellular extract or lysate refers to a preparation or fraction which is made from a lysed or disrupted cell.

As used herein, a control refers to a sample that is substantially identical to the test sample, except that it is not treated with a test parameter, or, if it is a plasma sample, it can be from a normal volunteer not affected with the condition of interest. A control also can be an internal control.

As used herein, the singular forms "a," "an," and "the" include plural referents unless the context clearly dictates otherwise. Thus, for example, reference to a polypeptide, comprising an immunoglobulin domain includes polypeptides with one or a plurality of immunoglobulin domains.

As used herein, the term "or" is used to mean "and/or" unless explicitly indicated to refer to alternatives only or the alternatives are mutually exclusive.

As used herein, ranges and amounts can be expressed as about a particular value or range. "About" also includes the exact amount. Hence about 5 amino acids means about 5 amino acids and also 5 amino acids.

As used herein, optional or optionally means that the subsequently described event or circumstance does or does not occur and that the description includes instances where said event or circumstance occurs and instances where it does not. For example, an optionally variant portion means that the portion is variant or non-variant.

As used herein, the abbreviations for any protective groups, amino acids and other compounds, are, unless indicated otherwise, in accord with their common usage, recognized abbreviations, or the IUPAC-IUB Commission on Biochemical Nomenclature (see, Biochem. (1972) 11(9):1726-1732).

For clarity of disclosure, and not by way of limitation, the detailed description is divided into the subsections that follow.

## B. MICROALGAE AND OVERVIEW

Algae are a complex, polyphyletic collection of predominantly photosynthetic organisms. These organisms include micro- and macroscopic forms. Macroalgae (seaweed) are multicellular, large-size algae, visible with the naked eye. Microalgae are microscopic single cells and include prokaryotes (*e.g.*, cyanobacteria), and eukaryotes, such as green algae.

Compared to photosynthetic crops, microalgae have a higher growth rate and can be cultivated on non-arable land, and also in bioreactors. Many species of microalgae can be grown year round in industrial scale photobioreactors under controlled cultivation conditions (Adamo et al. (2021) Journal of Extracellular Vesicles 10:e12081).

Algae generally are classified into eleven major phyla: Cyanophyta, Chlorophyta, Rhodophyta, Glaucophyta, Euglenophyta, Chlorarachniophyta, Charophyta, Cryptophyta, Haptophyta, Heterokontophyta, and Dinophyta (Barkia et al. (2019) Mar. Drugs 17(5):304). Different pigments occur in each algae group. Cyanobacteria (or Cyanophyta) contain chlorophyll-a, -d, and -f, in addition to the phycobiliproteins (proteins that capture light energy), phycocyanin, allophycocyanin, and phycoerythrin. Glaucophytes contain chlorophyll-a and harvest light via phycobiliproteins. Chlorophytes have chlorophyll-a and -b, as well as carotenoids, including β-carotene and various xanthophylls (*e.g.*, astaxanthin, canthaxanthin, lutein, and zeaxanthin). The primary pigments of Rhodophyta (red algae) are phycoerythrin and phycocyanin, which can mask chlorophyll-a; red algae also produce a broad spectrum of carotenes and xanthophyll light-harvesting pigments (Barkia et al. (2019) Mar. Drugs 17(5):304).

Provided herein are extracellular vesicles produced by algae, particularly unicellular green algae, such as species of *Chlorella,* for use for delivery of exogenously loaded cargo to animals and plants. The algae are unicellular eukaryotes that typically are haploid, but can have a diploid stage of the life cycle. The algae can be cultured in bioreactors

and the extracellular vesicles isolated therefrom. The resulting extracellular vesicles can be loaded by methods such as electroporation, with cargo, generally a cargo of heterologous bioactive molecules to produce compositions that contain the extracellular vesicles for administration to animals and also to plants. The compositions can be formulated for any desired route of administration, including topical, local, systemic, parenteral, and oral. These routes include oral, intravenous, subcutaneous, inhalation, mucosal, rectal, vaginal, and other suitable routes. The cargo includes biomolecules, such as DNA, RNA, proteins, protein complexes, protein-nucleic acid complexes, plasmids, and also includes small molecules, such as small molecule drugs. The extracellular vesicles can be formulated as liquids, powders, including lyophilized powders, tablets, capsules, emulsions, particles, sprays, gels, ointments, creams, and other formulations. They can be used for therapeutic, diagnostic, theragnostic, cosmetic, and other uses. The extracellular vesicles can be used to treat diseases and conditions, that include cancers, inflammatory diseases and conditions in which the immune system plays a role in the etiology or symptoms, nervous system disorders, and pathogen infections, including viral and bacterial and other pathogens. They can be used to treat dermatological diseases and conditions, lung diseases and conditions, and gastric diseases and conditions. The extracellular vesicles can be targeted to specific organs or tissues or can be locally administered.

As with extracellular vesicles (EVs) from other sources, such as mammalian EVs, microalgae EVs (MEVs) have evolved to efficiently pass genetic material and other kinds of molecules from cell to cell. They orchestrate intercellular and cross-kingdom communication between cells via exchange of biologically active molecules. MEV are natural nanoparticles. They are cell-derived, so, absent synthetic cargo, and genetic modifications, there are no synthetic components; they are safe, for example, there is no risk of endogenous viruses that are potentially dangerous to humans.

The MEVs provided herein include *Chlorella,* MEVs, particularly *Chlorella vulgaris,* a freshwater microalgae. *Chlorella* is a unicellular haploid alga that is a natural and efficient producer of extracellular vesicles. *Chlorella vulgaris* has been consumed worldwide as a food supplement for decades; it is non-toxic and non-immunogenic, and can be cultured at large industrial scale at low cost. The MEVs provided herein can be directly used to protect, convey, and deliver a broad spectrum of innovative therapeutic molecules into target cells relevant to specific diseases.

As shown and described herein, the MEVs have a number of advantageous features including, for example, biodistribution patterns by route of administration, low toxicity, good pharmacokinetic profiles *in vivo.* They can be administered by a variety of routes including oral administration, administration to the respiratory tract, intranasally, intravenously, among other routes. They traffic to specific organs, according to the route of administration, such as the intestine, the GALT, the spleen, the lungs, the liver, and the brain. Based on data herein and comparison with data for other EVs and other drug delivery systems, the MEVs can have longer clearance rates so that they last longer *in vivo* in targeted organs, tissues, and cells than reported for other delivery systems, including mammalian EVs. As shown herein, the MEVs overcome natural body barriers (such as oral delivery, or specific lymphoid tissues delivery, or nose-to-brain delivery) that have not been attained with liquid nanoparticles and EVs of mammalian origin.

The MEVs provided herein address unmet needs. These include the ability to convey and reliably deliver therapeutic molecules specifically to the site of treatment, while avoiding premature degradation or inactivation of the therapeutic agent by the immune system or by enzymes; for treatment of diseases for which a therapeutic agent already exists but cannot be properly delivered.

As shown herein, the purified or partially-purified MEVs can be loaded by physical methods (exogenous loading; exo-loading). Exo-loading is scalable and industrializable. The MEVs can be exo-loaded with a variety of molecules, varying in size, hydrophobicity, and nature, such as, but not limited to mRNA, peptides, proteins, plasmids, oligonucleotides, and small molecules. The biological activity and/or properties of the exo-loaded cargo is preserved, while at the same time it is protected from degradation by enzymes and other agents present *in vivo.* The MEVs can deliver their cargo to recipient cells of myriad origins, such as microalgae, bacteria, higher plant, mammal, and human. MEVs can also deliver the cargo to the proper cell compartments, ensuring the proper expression and biological activity of cargo molecules, including those having complex biological pathways such as mRNA, receptor-binding peptides, and among others.

### C. EXTRACELLULAR VESICLES

Extracellular vesicles (EVs) are biomolecular structures released from plant and animal cells that play a role in cell-to-cell communication. Structurally, EVs are negatively charged lipid bilayer vesicles with a density of 1.13 to 1.19 g/mL. EVs are able to cross barriers such as the plasma (or cytoplasmic) membrane and the blood/brain barrier, and enable the horizontal transfer of their functional contents (*i.e.,* proteins, lipids, RNA molecules, and circulating DNA) from a donor to a recipient cell (Kuruvinashetti et al. (2020) 20th International Conference on Nanotechnology 354-357). EVs also are naturally stable in various biological fluids, immunologically inert, and can exhibit organ-specific targeting abilities (Picciotto et al. (2021) Biomater. Sci. doi:10.1039/d0bm01696a).

EVs contain endogenous lipids, nucleic acids, and proteins. Although results differ due to variations in isolation techniques and methods of analyzing the data, EVs generally contain proteins associated with the plasma membrane, cytosol, and those involved in lipid metabolism (see, *e.g.,* Doyle and Wang (2019) Cells 8(7):727). Proteins involved in the

biogenesis of EVs (*e.g.*, components of the ESCRTs), EV formation and release *(e.g.,* RAB27A, RAB11B, and ARF6), signal transduction, and antigen presentation, as well as tetraspanins commonly occur in EVs (Abels and Breakefield (2016) Mol. Neurobiol. 36(3):301-312). EVs are enriched for cholesterol, sphingomyelin, glycosphingolipids, and phosphatidylserine (Kuruvinashetti et al. (2020) 20th International Conference on Nanotechnology 354-357). Although studies have identified genomic and mitochondrial DNA in EVs, EVs, as they occur in microalgae, primarily are enriched with endogenous (to the microalgae) small RNAs. Studies have identified mRNAs, miRNAs, rRNAs, long and short non-coding RNA, tRNA fragments, piwi-interacting RNA, vault RNA, and Y RNA in EVs. Most of the RNA that naturally occurs in EVs is ~200 nucleotides long (with a small portion up to 4 kb) and thus it is fragmented, although circular RNAs also have been shown to be enriched and stable in EVs. RNA in EVs is protected from RNase digestion in the extracellular environment by the lipid bilayer (Abels and Breakefield (2016) Mol. Neurobiol. 36(3):301-312). The Exocarta, Vesiclepedia, and EVpedia databases are publicly available and provide data on the protein, nucleic acid, and lipid content of EVs (generally EVs from mammalian origin, such as human origin), as well as the isolation and purification procedures used, from EV studies (Abels and Breakefield (2016) Mol. Neurobiol. 36(3):301-312).

EVs are used by cells to mediate several physiological processes or affect various pathological conditions associated with the activation of an immune response or the spread of disease or infection, and also constitute cross-species communication and are in all kingdoms of life. Sources of EVs include mammalian cells, bacteria, bovine milk and plants (Adamo et al. (2021) J. Extracell. Vesicles 10:e12081). Although plants and algae possess a cell wall outside their plasma membrane, which could be a physical barrier for the release of EVs, plants and algae release EVs (Picciotto et al. (2021) Biomater. Sci. doi:10.1039/d0bm01696a).

### 1. Types of Extracellular Vesicles (EVs)

#### a. Exosomes

There are three primary subtypes of EVs; they are classified based on their biogenesis, mode of release, size, content, and function: microvesicles (MVs), exosomes, and apoptotic bodies (Doyle and Wang (2019) Cells 8(7):727). Exosomes, or intraluminal vesicles (ILVs), are 30-150 nm in diameter and are released through multivesicular bodies (MVBs) in the endosomal pathway. In the endosomal pathway, early endosomes form by inward budding of the plasma membrane and can transform into late endosomes, which accumulate ILVs by inward budding of the endosomal membrane. Late endosomes which contain a number of small vesicles are called MVBs. MVBs either fuse with the lysosome and are degraded, or the plasma membrane which releases the ILVs as exosomes into the extracellular space. The endosomal sorting complexes required for transport (ESCRT) pathway regulates MVB transportation and exosome formation and is reported to be the primary driver of exosome biogenesis, although other mechanisms of exosome biogenesis exist, including those mediated by the sphingolipid ceramide, which can facilitate membrane invagination, or proteins in the tetraspanin family. The ESCRT accessory proteins Alix, TSG101, HSC70 and HSP90β are often referred to as exosomal marker proteins (Doyle and Wang (2019) Cells 8(7):727).

Exosomes are released into the extracellular space by the fusion of the MVB limiting membrane with the plasma membrane. A number of proteins are involved in the release of exosomes, including Rab GTPases, diacylglycerol kinase α, and SNARE proteins (Abels and Breakefield (2016) Cell Mol. Neurobiol. 36(3):301-312).

Exosomes are candidates for drug delivery systems: they have a long circulating half-life; exosomes are tolerated by the human body and can penetrate cell membranes and target specific cell types; and they can be loaded with genetic material, a protein, or a small molecule (Doyle and Wang (2019) Cells 8(7):727).

#### b. Microvesicles

Microvesicles (MVs, or ectosomes) form by outward budding, or pinching, of the cell's plasma membrane, and have a diameter of 100 nm to 1 μm. The formation of MVs involves cytoskeleton components, such as actin and microtubules, molecular motors such as kinesins and myosins, and fusion machinery such as SNAREs and tethering factors. The physiological state and microenvironment of the donor cell effects the number of MVs produced, and the physiological state and microenvironment of the recipient cell effects the number of MVs consumed. MVs also have a number of marker proteins, including cytosolic and plasma membrane associated proteins, as well as cytoskeletal proteins, heat shock proteins, integrins, and proteins containing post-translational modifications, although there are no known specific markers to distinguish MVs from exosomes. Like exosomes, MVs can be loaded with cargo (such as proteins, nucleic acids, and lipids) for delivery to another cell, thereby altering the recipient cell's functions (Doyle and Wang (2019) Cells 8(7):727).

### c. Apoptotic Bodies

Apoptotic bodies are released by dying cells into the extracellular space, and have a diameter from 50 nm to 5000 nm. Apoptotic bodies are formed when the cell's plasma membrane separates from the cytoskeleton due to increased hydrostatic pressure after the cell contracts. Unlike exosomes and MVs, apoptotic bodies contain intact organelles, chromatin, and small amounts of glycosylated proteins (Doyle and Wang (2019) Cells 8(7):727).

## 2. Uptake of EVs

Cells internalize EVs by fusion with the plasma membrane, or more commonly by endocytosis (Abels and Breakefield (2016) Cell Mol. Neurobiol. 36(3):301-312). Uptake via endocytosis can be through several types of endocytotic processes, and different processes have been described in different cell types: clathrin-dependent endocytosis and phagocytosis have been described in neurons, macropinocytosis in microglia, phagocytosis and receptor-mediated endocytosis in dendritic cells, caveolin-mediated endocytosis in epithelial cells, and cholesterol- and lipid raft-dependent endocytosis in tumor cells. Blocking heparin sulfate proteoglycans (HSPGs) on the plasma membrane with heparin reduces the uptake of EVs in cell culture, as does blocking the scavenger receptor type B-1 (SR-B1) with a synthetic nanoparticle mimic of HDL, which suggests a role for HSPGs and SR-B1 in EV uptake (Abels and Breakefield (2016) Cell Mol. Neurobiol. 36(3):301-312). Fusion of EVs with the plasma membrane also is a method of uptake, and requires low pH conditions; treatment of EVs with the combination of a pH-sensitive fusogenic peptide with cationic lipids resulted in increased cellular uptake of exosomes and the cytosolic release of cargo within the exosomes (Nakase and Futaki (2015) Sci. Rep. 5:10112). Low pH conditions occur in tumors (Abels and Breakefield (2016) Cell Mol. Neurobiol. 36(3):301-312), so that EVs for delivering therapeutic payloads to tumor cells can enter cells through fusion with the plasma membrane.

Like cells, EVs have extracellular receptors and ligands on the outside and cytoplasmic proteins and nucleic acid on the inside, and thus communicate with cells in different ways. EVs bind to the cell surface, undergo endocytosis, and/or fuse with the plasma membrane, and release their cargos in the extracellular space. If entering by endocytosis, the EV cargo must escape the degradative pathway; late endosomes can fuse with lysosomes or the plasma membrane, so cargo must exit before it is degraded in a lysosome or re-released through the fusion of MVBs with the plasma membrane. EVs containing cargo, including mRNAs and non-coding RNAs, can be transferred to recipient cells in culture and *in vivo* (Abels and Breakefield (2016) Cell Mol. Neurobiol. 36(3):301-312; Maas et al. (2017) Trends Cell Biol. 27(3):172-188).

## 3. General Methods for Isolating EVs

### a. Ultracentrifugation

Ultracentrifugation methods are used to isolate exosomes; alternative methods also have been developed. Due to the complex nature of the biological fluids from which exosomes are isolated, the overlap in physiochemical and biochemical properties between exosomes and other types of EVs, and the heterogeneity among exosomes, isolation methods can result in complex mixtures of EVs and other components of the extracellular space. Differential ultracentrifugation depends on the initial sedimentation of larger and denser particles from the extracellular matrix, and results in an enrichment of exosomes, but not a complete separation of exosomes from other components in the extracellular space. Density gradient centrifugation is another ultracentrifugation method and is based on separation by size and density in the presence of a density gradient (typically made of sucrose or iodoxinol) in the centrifuge tube. Density gradient centrifugation effectively separates EVs from protein aggregates and non-membranous particles but has low exosome recovery, although purity can be improved by coupling differential ultracentrifugation with types of density gradient centrifugation, such as rate-zonal centrifugation or isopycnic centrifugation (Doyle and Wang (2019) Cells 8(7):727).

### b. Size-Based Techniques

There are a number of size-based techniques for isolating exosomes (Doyle and Wang (2019) Cells 8(7):727). Ultrafiltration separates particles based on the size and molecular weight cut off of the membrane, whereby particles larger than the molecular weight cut off of the membrane are retained, and particles smaller than the molecular weight cut off of the membrane are passed through into the filtrate; low isolation efficiency can occur however if the filter becomes clogged and vesicles become trapped. The ExoMir™ Kit (Bioo Scientific; Austin, TX) is a commercially available kit in which two membranes (200 nm and 20 nm) are placed into a syringe and a sample (typically pre-treated with centrifugation and proteinase K) is passed through the syringe; the larger vesicles remain above the first 200 nm filter, the smallest vesicles are passed through the syringe and discarded, and the vesicles between 20 and 200 nm remain between the two filters in the syringe. Sequential filtration also relies on a series of filtration steps to isolate exosomes (Doyle and Wang (2019) Cells

8(7):727).

Size Exclusion Chromatography (SEC), often used in parallel with ultracentrifugation methods (in which the exosome pellet obtained from ultracentrifugation is resuspended and further purified using SEC), of exosomes is similar to using SEC to separate proteins. In SEC, a column is packed with a porous stationary phase in which small particles can penetrate and thus elute after larger particles. Typically SEC methods require several hours of run time; however, the qEV Exosome Isolation Kit (iZON Science, New Zealand) allows for rapid and precise exosome isolation by SEC within 15 minutes (Doyle and Wang (2019) Cells 8(7):727).

In Flow Field-Flow Fractionation (FFFF), a sample injected into a chamber is subjected to parabolic flow as it is pushed down the chamber, in addition to a flow perpendicular to the parabolic flow, a crossflow, to separate particles in the sample. Larger particles are more affected by the crossflow and are pushed toward the walls of the chamber, which have a slower parabolic flow, and smaller particles remain in the center. Smaller particles elute earlier, and larger particles later, in FFFF (Doyle and Wang (2019) Cells 8(7):727).

In Hydrostatic Filtration Dialysis (HFD), hydrostatic pressure forces a sample through a dialysis tube with a membrane having a molecular weight cut off of 1000 kDa. The result is that small solutes are able to pass through the tube, but larger particles, including exosomes and EVs, remain in the tube and can then be further separated using, for example, ultracentrifugation (Doyle and Wang (2019) Cells 8(7):727).

**c. Immunoaffinity Capture-Based Techniques**

Immunoaffinity capture-based techniques can isolate exosomes based on expression of an antigen on the surface of the exosome, and allow for the isolation of exosomes derived from a particular source. In these methods, an antibody specific for a target antigen can be attached to a plate *(e.g.,* in Enzyme-Linked Immunosorbent Assay, ELISA), magnetic beads *(e.g.,* in magneto-immunoprecipitation), resins and microfluidic devices; these surfaces are then exposed to the exosome sample, resulting in the immobilization of the exosomes expressing the antigen. This assay requires that the protein/antigen for isolating the exosomes be expressed on the surface of the exosomes, and its specificity is limited by the specificity of the antibody that is used, often resulting in a lower yield but higher purity of isolated exosomes. These methods also can be used to separate exosomes within mixed populations of EVs. Immunoaffinity capture-based techniques often are used after ultracentrifugation or ultrafiltration (Doyle and Wang (2019) Cells 8(7):727).

**d. Exosome Precipitation**

Methods for precipitation of exosomes include precipitation by polyethylene glycol (PEG) and lectin. In PEG precipitation, the PEG polymer ties-up the water molecules, allowing the other particles, including exosomes, to precipitate out of solution. PEG precipitation is quick and is not limited to the starting volume of solution, but lacks selectivity, as other EVs, extracellular proteins, and protein aggregates are precipitated with EVs. Sample pretreatment using filtration and/or ultracentrifugation can improve exosome yield. Commercially available kits for isolating exosomes using precipitation include, for example, ExoQuick® kit (System Biosciences, Palo Alto, CA) and Total Exosome Isolation Kit (Thermo Fisher Scientific, Waltham, MA). Alternatively, lectin precipitation can be used, typically after ultracentrifugation, whereby lectins bind to carbohydrates on the surface of exosomes, altering their solubility and leading to their precipitation out of solution (Doyle and Wang (2019) Cells 8(7):727).

**e. Microfluidic Based Isolation Techniques**

Microfluidic based techniques isolate exosomes based on their physical and biochemical properties simultaneously, and are rapid, efficient, and require small starting volumes. In acoustic nanofilter, a matrix containing EVs and other cellular components is injected into a chamber and exposed to ultrasound waves. The particles respond differently to the radiation forces exerted by the waves, depending on their size and density; large particles experience stronger forces and migrate faster toward the pressure nodes. The immuno-based microfluidic isolation technique is similar to that of an ELISA, although, unlike ELISAs, it does not require prior ultrafiltration or ultracentrifugation of exosomes (Doyle and Wang (2019) Cells 8(7):727). The ExoChip (Kanwar et al. (2014) Lab Chip. 14(11):1891-1900) and ExoSearch Chip (Zhao et al. (2016) Lab Chip. 16(3):489-496) have been developed to isolate exosomes using microfluidic technology.

**4. Microalgae-Derived Extracellular Vesicles (MEVs)**

Microalgae are bioresources for the production of EVs for use in nanomedicine and other fields. The mechanism of secretion of EVs from microalgae is known in relation to primary and motile cilia/flagella (Picciotto et al. (2021) Biomater. Sci. doi:10.1039/d0bm01696a). *Chlamydomonas* flagella are devoid of MVBs, thus, ciliary EVs shed from *Chlamydomonas* are classified as ectosomes. Studies have shown the shedding of ectosomes from flagellar and ciliary tips of the

chlorophyte *Chlamydomonas reinhardtii.* EVs also have been observed along the length of the cilium in *Chlamydomonas.* Membrane budding and ciliary EV formation are mediated by components of the endosomal sorting complex required for transport (ESCRT), which are found in isolated ciliary transition zones, ciliary membranes, and ciliary EVs in *Chlamydomonas* and can act as sensors of membrane curvature. The formation of ciliary EVs also can occur when ciliary membrane trafficking is disrupted or during ciliary resorption (Wang and Barr (2018) Essays Biochem. 62(2):205-213). Ciliary ectosomes from *Chlamydomonas* contain a lytic enzyme that digests the mother cell wall and is required for the release of daughter cells. Ift88-null mutants that do not have flagella were unable to be released from the mother cell, and the addition of ciliary ectosomes from wild-type cells rescued the phenotype, suggesting a role for the flagella and intraflagellar transport (IFT) machinery in EV production (Wang and Barr (2016) Cell Mol. Neurobiol. 36(3):449-457).

EVs have been extracted from algal cells using ultra-centrifugation (Kuruvinashetti et al. (2020) 20th International Conference on Nanotechnology 354-357). This method is well established for the isolation of EVs. Briefly, the algal cells are cultured; the cultured algal cells are collected and centrifuged; the supernatant is collected (and further centrifuged); a sucrose solution is added to the supernatant; and the algal supernatant with the sucrose solution is ultra-centrifuged; because of the sucrose solution, the high-density EVs settle at the bottom of the ultra-centrifugation tube and can be collected using a pipette. Extracted algal EVs can be characterized in size and concentration using Nanoparticle Tracking Analysis (NTA). Studies using this method have isolated green algal EVs that range in size from 25-200 nm, with a concentration of 0.89E8 to 0.94E8 particles/mL (Kuruvinashetti et al. (2020) 20th International Conference on Nanotechnology 354-357).

An ultra-centrifugation protocol also can be used to isolate EVs from marine microalgae grown under various conditions; NTA showed that the nano-particles have a size distribution between 100 and 200 nm, and western blotting of proteins confirmed the presence of EV markers (VES4US, Extracellular vesicles from a natural source for tailor-made nanomaterials, 2020). Subsequent studies have identified microalgal small EVs (sEVs) isolated from the marine photosynthetic microalgal chlorophyte *Tetraselmis chuii,* termed nanoalgosomes. The production of nanoalgosomes is an evolutionarily conserved trait within microalgal strains as similar results were obtained using sEVs isolated from batch cultures of two other microalgae species, the chlorophyte *Dunaliella tertiolecta,* and the dinoflagellate *Amphidinium sp.* The nanoalgosomes were isolated using differential ultracentrifugation (dUC) and tangential flow filtration (TFF), as well as gradient ultracentrifugation, which was used to further purify samples enriched for small EVs by TFF or dUC. The isolated nanoalgosomes were shown to share characteristics of EVs from other sources. The EV yield (measured by sEV protein content and sEV number) from dUC and TFF was consistent with reported numbers of isolated EVs, around $10^9$ EV particles/$\mu$g EV proteins. Biophysical analysis of particle size using multi-angle dynamic light scattering (DLS), nanoparticle tracking analysis (NTA), fluorescence nanoparticle tracking analysis (F-NTA), and fluorescence correlation spectroscopy (FCS) yielded consistent size distributions, with the size that appeared the most frequently from DLS (DLS mode) around 70 nm. Compared to exosomes derived from mammalian cells, which have a density of 1.15-1.19 g/mol, nanoalgosomes had a slightly lower density of 1.13 g/mol. Electron microscopy revealed that the nanoalgosomes are spherical, heterogeneous in size and shape, and possess a lipid-bilayer structure. Compared to the microvesicles (or large EVs, lEVs) and lysates, the sEVs were enriched for three of the four target protein biomarkers (Alix, enolase, HSP70 and $\beta$-actin). DLS measurements indicated that the nanoalgosomes were resistant to changes in pH and stable in human blood plasma. The tumorigenic MDA-MB 231 breast cancer cell line, the non-tumorigenic 1-7 HB2 cell line, and the human hepatocarcinoma Hep G2 cell line did not show cytotoxic or genotoxic effects after nanoalgosome treatment. Furthermore, the nanoalgosome were successfully taken up by the MDA-MB 231 and 1-7 HB2 cell lines (Adamo et al. (2021) J. Extracell. Vesicles 10:e12081).

EVs can be isolated from different microalgae strains, and the microalgae-isolated EVs exhibit the key features of EVs. Eighteen microalgae strains *(Ankistrodesmus sp., Brachiomonas sp., Chlamydomonas reinhardtii, Dunaliella tertiolecta, Tetraselmis chuii, Chloromonas sp., Rhodella violacea, Kirchneriella sp., Pediastrum sp., Nannochloropsis sp., Cyanophora paradoxa, Cryptomonas pyrenoidifera, Phaeodactylum tricomutum, Phaeothamnion sp., Diacronema sp., Isochrysis galbana, Stauroneis sp.,* and *Amphidinium sp.*) from the main microalgal lineages were studied, and included strains with a variety of features such as saltwater and freshwater inhabitants, small and large sized cells, colonial and single cells, and species with sequenced genomes. EVs were isolated using a differential ultracentrifugation protocol and characterized following the International Society for Extracellular Vesicles (ISEV) guidelines, and all strains tested showed the presence of EVs in the culture medium. EV-producing microalgae strains were established based on the EV protein content, the expression of EV protein markers (e.g., Alix, Hsp70, enolase, and $\beta$-actin), the total scattering signal (measured by dynamic light scattering, DLS) or total particle number (measured by NTA), and the sEV average size and size range. These promising EV-producing strains include *Cyanophora paradoxa, Tetraselmis chuii, Amphidinium sp., Rhodella violacea, Diacronema sp., Dunaliella tertiolecta, Phaeodactylum tricornutum, Pediastrum sp.,* and *Phaeothamnion sp.* Specifically, the data for *Cyanophora paradoxa* showed ~$2\times10^9$ sEV particles per mL of microalgal-conditioned media, with strong positive signals for EV markers, and a size distribution with a mode of 130 $\pm$ 5 nm, in agreement with data from plant-derived vesicles. Cytotoxicity and genotoxicity studies showed that sEVs isolated from *Cyanophora paradoxa,* a freshwater Glaucophyte, did not show toxicity on the tumorigenic MDA-MB 231 breast cancer or C2C12

myoblast cell lines, neither over time nor at different concentrations, nor did MDA MB 231 cells treated with the sEVs show morphological nuclear changes associated with apoptotic events (Picciotto et al. (2021) Biomater. Sci. doi:10.1039/d0bm01696a). Such studies have not considered *Chlorella* species.

EVs also have been isolated from *Synechocystis sp.* PCC6803 (a cyanobacterium), *Chlamydomonas reinhardtii* (a green microalgae), *Euglena gracilis* (a euglenophyte), and *Haematococcus pluvialis* (a chlorophyte) in work done by Zhao *et al.,* who also performed RNomic and proteomic analyses in EVs isolated from C. *reinhardtii* at different stages of cell growth and under different types of abiotic stress (Zhao *et al.* (2020) doi:10.21203/rs.3.rs-38027/v1). EVs were isolated using differential ultracentrifugation and filtration, and the resuspension was shown to contain membrane structures with small clumps of particles 110-120 nm in diameter, in line with the reported diameter of exosomes and small MVs, although there were differences in diameters between the species of microalgae. Specifically, EVs from C. *reinhardtii* had diameters between 37-710 nm, with an average particle diameter of 120.1 nm. *Synechocystis*-derived EVs had diameters between 24-450 nm, with an average particle size of 94.68 nm. Despite the presence of a cell wall, *Chlamydomonas* cells were able to uptake EVs, as shown by the presence of EVs labeled with a fluorescent lipophilic dye inside microalgal cells. Thus, microalgal EVs can be absorbed by recipient cells. Non-coding RNAs were detected in microalgal EVs at different growth stages and treatment (biotic stress, nitrogen depletion, and nitrogen recovery), and proteomic analyses identified many flagellar-associated membrane proteins in microalgal EVs (Zhao *et al.* (2020) doi:10.21203/rs.3.rs-38027/v1).

These studies show that microalgae produce EVs that can be isolated using traditional methods; microalgal-derived EVs are similar in size and concentration, and exhibit similar markers compared to EVs isolated from other species; EVs isolated from microalgae do not show cytotoxic or genotoxic effects *in vitro*; and that microalgal-derived EVs can be taken up by cells.

It has been shown that EVs from mammalian origin can deliver cargo to a target cell and thus have therapeutic use for delivery of a variety of cargos for use in treating a number of diseases or conditions. For example, small molecules such as hydrophobic and hydrophilic drugs can be injected into exosomes, or macromolecular proteins and nucleic acids can be embedded into the exosomes. The nucleic acids can include those encoding a gene of interest. Specific targeting ligands, imaging probes, and covalent linkage could be attached to the exosome surface and tracked using NTA, fluorescence, or by bioluminescence. Besides a mention in a publication that microalgae EVs possibly can be used to deliver a drug of interest to a targeted cell, tissue, or organ (Kuruvinashetti et al. (2020) 20th International Conference on Nanotechnology 354-357), there is no published evidence nor technical descriptions that the uses described for mammalian EVs can be applied to or developed for microalgae-derived extracellular vesicles.

As described and shown herein, however, algal EVs have a number of advantages over the use of existing drug delivery systems, such as, exosomes derived from mesenchymal stem cells, gold nanoparticles, liposomes and other plant- and animal-derived EVs. Mesenchymal stem cells are a commonly used source of exosomes, and exosomes derived from mesenchymal stem cells are used in drug delivery, for example anti-cancer vaccines, because they have enhanced passive targeting (a method of preparing a drug carrier system so that it remains circulating in the blood stream), as a result of their small size, indigenous nature, and ability to cross biological barriers. Mesenchymal stem cells, however, have limited secretion of exosomes, and scaling up production of exosomes is difficult due to the need to optimize purification, increase the homogeneity of exosomes, and establish efficient transfection strategies. Nanoparticles can lead to toxicity and current techniques for synthesizing nanoparticles are limited in their ability to scale for manufacturing purposes. Nanoparticle and liposome-based drug delivery methods also can lead to the formation of a teratoma (a tumor comprised of several different types of tissue). Liposome-based drug delivery methods have been further shown to be less efficient for internalization into a specific cell, tissue or organ, compared to exosomes. Plant-derived EVs, such as those from curcumin, ginger, grapefruit, and lemon, have been used for drug delivery, but their extraction process and use in treatment has not yet been optimized. The production of EVs from agricultural products, such as fruits and milk, is economically impractical and need 3-4 months to grow, compared to algal EVs, which can be grown anywhere and within a few days. Algal EVs avoid phagocytosis or degradation by macrophages and circulate for prolonged times *in vivo,* and have low immunogenicity. Algal EVs also have a lower risk of teratoma formation. Algae, thus, provide a source from which pure, well-characterized EVs of high quality can be obtained (Kuruvinashetti et al. (2020) 20th International Conference on Nanotechnology 354-357). Kuruvinashetti *et al.* does not describe the use of *Chlorella* species as a source of EVs, nor its advantages as a source.

### 5. Green algae - *Chlorella* species

Most of the previous studies and consideration of EVs have not focused on nor assessed *Chlorella* species as sources of EVs. *Chlorella* and the resulting EVs have advantages for growth, manipulation, and administration of drugs that other species and EVs do not provide. Green algae belong to phylum Chlorophyta, and encompass a diverse group of photosynthetic eukaryotes. Green algae include unicellular and multicellular organisms. Algae originally included in the genus *Chlorella* are among the most widely distributed and frequently encountered algae in freshwater. These algae exist

in aqueous environments and on land. They are typically small (~2 to 10 μm in diameter), unicellular, spherical in shape, non-motile, and contain a single chloroplast, and some have a rigid cell wall (Blanc et al. (2010) Plant Cell 22(9):2943-2955).

Molecular analyses have separated *Chlorella* species into two classes of chlorophytes: the Trebouxiophyceae, which contains the true *Chlorella;* and the Chlorophyceae. For use herein, *Chlorella* species include any that can be or that are used as food complement or that can be consumed by humans or other animals, such as livestock. Exemplary species include, but are not limited to, the species: *Chlorella ellipsoidea, Chlorella pyrenoidosa, Chlorella sorokiniana, Chlorella vulgaris,* and *Chlorella variabilis.*

True *Chlorella* species are characterized by glucosamine as a major component of their rigid cell walls. Although most *Chlorella* species are naturally free-living, the Trebouxiophyceae include most of the known green algal endosymbionts, living in lichens, unicellular eukaryotes, plants, and animals (for example mussels and hydra). For example, *Chlorella variabilis* NC64A is a hereditary photosynthetic endosymbiont (or photobiont) of *Paramecium bursaria,* a unicellular protozoan, and NC64A also is a host for a family of large double-stranded DNA viruses that are occur in freshwater (Blanc et al. (2010) Plant Cell 22(9):2943-2955).

### a. Life Cycle

In unicellular organisms, such as microalgae, life cycle is the same as the cell cycle. *Chlorella* is a haploid organism that reproduces asexually by autosporulation. The cell cycle and proliferation of *Chlorella vulgaris* has been investigated using flow cytometric analysis of 5(6)-carboxyfluorescein diacetate succinimidyl ester (CFSE)-stained algal cells by Rioboo *et al.* Their results indicate that, as generally described for microalgae, the growth of C. *vulgaris* mother cells takes place during light periods, whereas cytoplasmic division and liberation of daughter cells takes place during dark periods. *C. vulgaris* also shows a distinct light/dark cycle, marked by an increase in cell size, cell complexity, and autofluorescence during periods of light, measured over a 96-hour period. A monoparametric histogram of CFSE-stained C. *vulgaris* cells showing only one peak of daughter cells indicates that each mother cell undergoes only one division cycle in 96 hours; the cytoplasmic division was further shown to take place during periods of darkness. Thus, the strain of C. *vulgaris* used exhibits three life cycle phases: 1) growth of mother cells, 2) cell division, and 3) liberation of daughter cells. *C. vulgaris* cells grew during 2 light periods and began to divide during following dark period; cell division occurs once the mother cells are double the size of daughter cells. Furthermore, C. *vulgaris* cells exposed to the herbicide terbutryn need a longer growth period in order to reach a large enough cell size to divide. This suggests there is a critical threshold size needed for *C. vulgaris* to complete the growth phase and begin the division phase, and that this critical threshold can control the progression of the G1 phase of the *C. vulgaris* cell cycle. Finally, this study demonstrates that the intensity of the peak of CFSE-fluorescence of mother cells is four times greater than that of the daughter cells, indicating that 4 daughter cells are produced from each mother cell. Thus, *C. vulgaris* cells undergo a first mitosis followed by cytoplasmic division, and then two other simultaneous mitoses, which result in the liberation of 4 daughter cells (Rioboo et al. (2009) Aquat Toxicol 94(3):229-37).

### b. Genomic Analyses of *Chlorella* Species

Although species of *Chlorella* are reported to be non-motile and lack a sexual cycle, genomic analyses of *Chlorella variabilis* NC64A (NC64A) and *Chlorella vulgaris* 211/11P (211/11P) reveal the presence of genes involved in sexual reproduction and motility (Blanc et al. (2010) Plant Cell 22(9):2943-2955; Cecchin et al. (2019) Plant J. 100(6):1289-1305). The NC64A nuclear genome (GenBank Accession No. ADIC00000000.1) is 46.2 Mb, and composed of 12 chromosomes. The meiosis-specific proteins dosage suppressor of MCk1 DMC1, homologous-pairing proteins HOP1 and HOP2, meiotic recombination protein MER3, meiotic nuclear division protein MND1, and mutS homolog protein MSH4 are encoded in NC64A; these genes also occur in most of the other sequenced chlorophyte algal species. Nineteen homologs of the *Chlamydomonas* gametolysin proteins, which promote disassembly of the gametic cell walls and allow gamete fusion, also were identified in NC64A. Additionally, an ortholog of the *Chlamydomonas* GCS1 protein, which is essential for cell fusion, occurs in NC64A (Blanc et al. (2010) Plant Cell 22(9):2943-2955). The primary genes involved in meiosis also occur in the *Chlorella vulgaris* 211/11P 40 Mb genome (GenBank Accession No. SIDB00000000), in addition to the gene encoding gametolysin (g3347), and a gene encoding a protein that contains a domain with a putative GCS1/HAP2 function (Cecchin et al. (2019) Plant J. 100(6):1289-1305). Thus, although *Chlorella* species have been observed only in the haploid phase, the presence of meiosis genes indicates that the life cycle of *Chlorella* could include a diploid phase.

Similarly, while flagella have not been observed in NC64A, orthologs of the *Chlamydomonas* flagellar proteins were identified in the NC64A genome, including orthologs to the intraflagellar transport (IFT) proteins IFT52, IFT57, and IFT88, kinesin-2 motor protein FLA8, the kinesin-associated protein KAP, and proteins involved in the axonemal outer dynein arm (Blanc et al. (2010) Plant Cell 22(9):2943-2955).

Sequencing of three *Chlorella sorokiniana* strains, strain 1228, UTEX 1230, and DOE1412, reveals the presence of sex- and flagella-related genes (Hovde et al. (2018) Algal Research 35:449-461). The genome of several other *Chlorella* species has been sequenced: *Chlorella protothecoides* sp. 0710 (Gao et al. (2014) BMC Genomics 15(1):582; GenBank Accession No. APJO00000000); *Chlorella sorokiniana* UTEX 1602 (GenBank Accession No. LHPG00000000) and *Chlorella* sp. strain SAG 241.80 (*Micractinium conductrix;* GenBank Accession No. LHPF00000000) (Arriola et al. (2018) Plant J. 93(3):566-586); and the *Chlorella vulgaris* strains UTEX 395 (Guarnieri et al. (2018) Front. Bioeng. Biotechnol. 6:37; GenBank Accession No. LDKB00000000), UMT-M1 (Teh et al. (2019) Data Brief 27:104680; GenBank Accession No. VJNP00000000), UTEX 259 (GenBank Accession No. VATW00000000) and NJ-7 (Wang et al. (2020) Mol. Biol. Evol. 37(3):849-863; GenBank Accession No. VATV00000000).

### c. Commercial and Biotechnological Uses of *Chlorella*

The commercial cultivation of microalgae for food purposes began with the production of *Chlorella vulgaris* in Japan and Taiwan in the 1960s. Dried biomass products from *Arthrospira* and *Chlorella* are included in dietary supplements due to reports of high protein content, nutritive value, and health benefits. For example, *Chlorella* extracts have been shown to lower cholesterol and have antioxidant, antibacterial, and antitumor activities. Production of high yields of *Chlorella* is routine, and, as detailed herein, MEVs can be isolated from the cell culture medium. For its use as a pharmaceutical, it is known that ingestion of *Chlorella* is non-toxic and non-immunogenic in humans.

*Chlorella* has been used in a variety of biotechnology applications, including biofuels, sequestering $CO_2$, producing molecules of high economic value, or removing heavy metals from wastewaters (Blanc et al. (2010) Plant Cell 22(9):2943-2955*). Chlorella* species show metabolic flexibility in response to environmental perturbations, and are capable of using nutrients, such as organic carbon and minerals, directly from wastewater for growth. Among microalgae, *Chlorella* species have higher photosynthetic efficiency over other photosynthetic organisms. Additionally, *Chlorella vulgaris* is able to grow either in autotrophic, heterotrophic or mixotrophic conditions (Zuñiga et al. (2016) Plant Physiol. 172(1):589-602).

*Chlorella* species also can be genetically modified by *Agrobacterium*-mediated transformation. A study by Cha *et al.* developed a method to genetically transform *Chlorella vulgaris* using the *Agrobacterium tumefaciens* strain LBA4404, and the presence of gene fragments in 30% of the transgenic lines, compared to the wild-type non-infected *Chlorella,* indicates the T-DNA was integrated into the *Chlorella* genome (Cha et al. (2012) World J. Microbiol. Biotechnol. 28:1771-1779).

### d. *Chlorella* MEVs

As described herein, *Chlorella* species, such as C. *vulgaris,* are advantageous species for the production of EVs, referred to herein as MEVs, for use for delivery of biomolecules and small molecules for many applications, including therapeutic, diagnostic, and cosmetic uses. Of particular interest herein are MEVs produced by *Chlorella* species. *Chlorella* EVs have not been exploited as sources of MEVs for exogenous loading of biomolecular products or small molecule drugs or diagnostic agents. *Chlorella,* as a source of EVs for such applications, provides numerous advantages. *Chlorella* is a haploid organism, which means that specific and targeted variants can be produced by genetic engineering; it readily can be genetically modified or loaded to produce or contain biologically active molecules and small molecules. Stable cell lines can be produced, including stable producers of encoded products. They are defined products, and, when exogenously loaded, the resulting compositions contain EVs that contain the same cargo.

Detailed genetic maps can be obtained, and correlations between genotype and phenotype can be established. *Chlorella* genomes have been fully sequenced, so the structure and function of various genes can be known. Phylogenetically, *Chlorella* is at the very crossroads between higher plants and microalgae. As such, *Chlorella* shares with higher plants a significant (and useful) number of molecular biological and metabolic features, but still is a unicellular haploid microalgae. Exemplary of molecular biological features shared with eukaryotes is the intracellular machinery that involves the dicer enzyme system for processing exogenous RNA into siRNA. *Chlorella* is autotrophic; unlike mammalian and other animal cells, it can therefore be cultured and reproduced without the need for nutrients or factors of animal origin.

With respect to use of its EVs as therapeutics, *Chlorella* species are not toxic. For example, tablets made from *Chlorella* vulgaris biomass (*i.e.,* compressed whole *Chlorella* cells) have been consumed regularly for years by the public worldwide as a dietary supplement, without constraints related to toxicity or immunogenicity. Japan is the world leader in the consumption of *Chlorella* biomass. It also is used, for example, in Japan, for medical treatments because it has shown to have immunomodulatory properties and purported anti-cancer activities, for use for anti-aging applications, such as for cardiovascular diseases, hypertension and cataracts; it reduces the risk of atherosclerosis and stimulates the synthesis of collagen for the skin.

*Chlorella* cells naturally produce extracellular vesicles (EVs) that respond to the 'standard specifications' of better known EVs (such as mammalian EVs). EVs from plant origin bear a number of features that make them more

promising/convenient than synthetic nanoparticles or semisynthetic EVs, for use as a drug delivery system in humans. These include, for example, higher stability, lower toxicity, and lower immunogenicity. Being as close as plants as it is, *Chlorella* provides a source of EVs with similar characteristics to plant EVs. At the same time, mass production of *Chlorella* in large scale is easier and cheaper than for higher plants. The glycosylation pattern of membrane proteins in *Chlorella* is similar/identical to the glycosylation pattern present in higher plants.

### D. EXOGENOUSLY LOADED MICROALGAE EXTRACELLULAR

### VESICLES (MEVS), CARGO, AND TARGETS

Targets and cargo (see discussions below) include any known to those of skill in the art.

#### 1. Isolation of MEVs

Methods for isolation are discussed in the sections above and detailed in the Examples.

#### 2. MEV Loading, and Cargos

In accordance with the claims, the MEVs can be loaded with any desired cargo (also referred to as a payload), including, but not limited to, nucleic acid molecules, including, for example plasmids, anti-sense nucleic acids, nucleic acids encoding the anti-sense nucleic acid, detectable marker proteins and tags, small molecule drugs, gene editing systems, and others, and combinations thereof, provided that the cargo is not RNAi. The MEVs can deliver therapeutic molecules, can serve as vaccines, and can be used in human and other animal health, agricultural applications, gene therapy applications, including delivery genes, modification of genes with gene editing systems, and gene silencing nucleic acids, cosmetic applications, dermatological applications, diagnostic applications, industrial uses, and others. The MEVs can deliver nutrients, or regulators of gene pathways to produce a beneficial product, and can be used to deliver gene editing systems, such as CRISPR/Cas and to effect gene editing. The MEVs can be used to deliver gene therapy vectors, such as, but not limited to, adeno-associated (AAV) virus vectors, adenovirus vectors, vaccinia virus-derived vectors, and others, and products.

Diseases and conditions that can be treated include any known to those of skill in the art, including but not limited to, cardiovascular diseases, metabolic diseases, infections, including respiratory infections, bladder infections and other urinary tract infections, infectious diseases, including viral disease, such as hepatitis, HIV, corona viruses, including SARS-Cov-2, CNS diseases, ocular diseases, and liver diseases. As discussed, delivered cargo includes protein products, such as antibodies and antigen-binding forms thereof, RNA products, such as, mRNA, nucleic acid encoding the products, such as plasmids, nucleic acid products such as DNA encoding anti-sense oligonucleotides and also the anti-sense oligonucleotides, and small molecule drugs.

The MEVs can carry cargos that include reporter genes and proteins and other detectable products, such as, for example, a fluorescent protein, such as, but not limited to an enhanced green fluorescent protein (EGFP; SEQ ID NO:10), a luciferase gene (SEQ ID NO:11), luxA (SEQ ID NO:8), luxB (SEQ ID NO:9), and the Lux operon (luxCDABE and luxABCDE; SEQ ID NO: 12).

Other cargos can target genes or products involved in diseases, such as, but not limited to, Peptidyl-prolyl cis-trans isomerase FKBP4 or FKBP52 (SEQ ID NO:1); gamma-aminobutyric acid type B receptor subunit 1 (GABBR1; SEQ ID NO:3); oncogenes such as MYCN or NMYC (SEQ ID NO:38), RAS (H-RAS, N-RAS, and K-RAS; see SEQ ID NOs:39, 40, and 41, respectively), BCL2 (SEQ ID NO:43), and PLK1 (SEQ ID NO:44). Genes involved in diseases, such as oncogenes, and checkpoints, can be modulated by cargo that encodes a product that inhibits or agonizes expression of a gene, or inhibits or agonizes a gene product. Exemplary of such modulators, are RNAis, such as for example, siRNAs, miRNAs, shRNAs, anti-sense oligonucleotides (ASOs), peptides and/or tetratricopeptides. For example, siRNAs and ASOs targeting EGFP (SEQ ID NOs:5 and 6), firefly luciferase (SEQ ID NO:7), MYCN (SEQ ID NOs:13-19), RAS (SEQ ID NOs:20-27), BCL2 (SEQ ID NOs:29-31), and PLK1 (SEQ ID NOs:32-35), and microRNA-34A, which targets MYC and BCL2 (SEQ ID NO:28), are exemplified herein. In accordance with the claims, the cargo is not RNAi.

Gene silencing using RNA interference, including siRNAs and microRNAs, can be used to silence developmental genes, such as, for example, adhesion molecules, cyclin kinase inhibitors, Wnt family members, Pax family members, Winged helix family members, Hox family members, cytokines/lymphokines and their receptors, growth/differentiation factors and their receptors, and neurotransmitters and their receptors; oncogenes; tumor suppressor genes; enzymes; genes associated with a pathological condition; genes associated with autoimmune diseases; anti-angiogenic genes; angiogenic genes; immunomodulator genes; genes associated with alcohol metabolism and liver function; genes associated with neurological disease; genes associated with tumorigenesis or cell transformation; and genes associated with metabolic diseases and disorders (see, *e.g.,* WO 2009/082606, JP 2014-240428A, WO 2011/072292A2, WO

2010/141724, and WO 2020/097540). These types of products can be delivered in or encoded in MEVs to activate genes or pathways or to provide therapeutic effects. Certain cytokines can be used to treat diseases/disorders, such as certain cancers, in which immune suppression plays a role.

Extracellular vesicles and exosomes also can be used to transfer therapeutic agents such as nucleic acids, such as microRNA, mRNA, tRNA, rRNA, siRNA, regulatory RNA, non-coding and encoding RNA, DNA fragments, and DNA plasmids (see, *e.g.*, CN105821081A and CN110699382A); nucleotides or amino acids comprising a detectable moiety or a toxin or that disrupts transcription or translation, respectively; polypeptides (e.g., enzymes); lipids; carbohydrates; and small molecules *(e.g.,* small molecule drugs and toxins) (see, U.S. Patent No. 10,195,290). Non-limiting examples of proteins that may be encoded for by the nucleic acid cargo molecule include, but are not limited to: antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, cytokines, cytokine receptors, caspases and any combination and/or derivatives thereof (see, *e.g.*, AU2018365299).

As summarized in the table below, although not forming part of the present invention, is a cocktail of three siRNA oligonucleotides targeting human MYCN with two thymidine residues (dTdT) at the 3'-end of the sequence (purchased from B-Bridge International Inc. (Sunnyvale, CA)). The anti-MYCN siRNA (siMYCN) and negative control siRNA (nontarget control pool) (siNeg) (both ON-TARGETplus siRNA, Dharmacon, Cambridge, UK) were used (see Ref. 1 (Nara et al. (2007) Int. J. Oncol. 30(5):1189-1196)). Exemplary target oncogenes and exemplary sequences of siRNA are provided in the table below.

| Target oncogene | Type of sequence | Sequence(s) | SEQ ID NO. |
|---|---|---|---|
| [1]MYCN | siRNA cocktail | siMYCN-1: sense 5'-CGGAGATGCTGCTTGAGAA-3'<br>siMYCN-2: sense 5'-CGGAGTTGGTAAAGAATGA-3'<br>siMYCN-3: sense 5'-CAGCAGTTGCTAAAGAAAA-3' | 90<br>91<br>92 |
| [2]MYCN | siRNA | sense 5'-CAGCAGUUGCUAAAGAAAAUU-3'<br>antisense 5'-UUUUCUUUAGCAACUGCUGUU-3' | 93<br>94 |
| [3]MYCN | siRNA | 5'-UGAUCUGCAAGAACCCAGA-3' | 95 |
| [4]MYCN | ASO | 5'-AAC GTT GAG GGG CAT-3' | 96 |
| [5]RAS | Synthetic miR-NA | Syn-miR-143s<br>#1: Wild type<br>S 3'-GGUCUCUACGUCGUGACGUGGAGU-5'<br>AS 5'-UGAGAUGAAGCACUGUAGCUCAGG-3'<br>#12: F/Ome type<br>S 3'- GGUCUCUACGUCGUGACGUGGAGU-5'<br>AS 5'-_U_^**G**^_A_**G**_A_**U**_G_**A**_A_**G**_C_**A**_C_**U**_G_**U**_A_**G**_C_**U**_C_**A**^_dT_^_dT_-3'<br>Key: *N* (italicized): 2'-F RNA<br>**N** (bold): 2'-Ome RNA<br>N (normal font):RNA<br>^:PS<br>N (underlined): mismatch | <br><br>97<br>98<br><br>97<br>99 |
| [6]RAS | siRNA | siR-KRAS: 5'-AAUGCAUGA CAACACUGGAUGACCG-3'<br>siR-HRAS: 5'-CCAUCCAGCUGAUCCAGAACCAUUU-3' | 100<br>101 |
| [7]RAS | siRNA | GGACUCUGAAGAUGUACCUAGGUACAUCUUCAGAGUCC | 102 |
| [8]mutated K-RAS | ASO | 5'-CTACGCCACAAGCTCCA-3' | 103 |
| [9]MYC, BCL2 | miRNA | Homo sapiens microRNA 34a (MIR34A), microRNA | 28 |
| [10]BCL2 | 3p-siRNA | sense GCAUGCGGCCUCUGUUUGA<br>anti-sense CGUACGCCGGAGACAAACU | 104<br>105 |

(continued)

| Target oncogene | Type of sequence | Sequence(s) | SEQ ID NO. |
|---|---|---|---|
| [11]BCL2 | ASO | TCT CCC AGC GTG CGC CAT | 106 |
| [12]PLK1 | Modified siRNA | sense: 5'-AGA **U**CA CCC **U**CC U**U**A AA**U** AUU-3'<br>antisense: 5'-UAU UUA A**G**G AGG GUG A**U**C UUU-3'<br>note: 2'-O-methylated modified nucleotides in bold | 107<br>108 |
| [13]PLK1 | siRNA | 5'-AAGGGCGGCUUUGCCAAGUGC-3' | 109 |
| [14]PLK1 | ASO | P12: ACC AGT CCG GAG GGG AGG GC | 110 |

1 Nara et al. (2007) Int. J. Oncol. 30(5):1189-1196; Silencing of MYCN by RNA interference induces growth inhibition, apoptotic activity and cell differentiation in a neuroblastoma cell line with MYCN amplification
2 Maeshima et al. (2020) Nucleic Acid Ther. 30(4):237-248; MYCN Silencing by RNAi Induces Neurogenesis and Suppresses Proliferation in Models of Neuroblastoma with Resistance to Retinoic Acid
3 Veas-Perez de Tudela et al. (2010) J. Neurochem. 113(4):819-825; Human neuroblastoma cells with MYCN amplification are selectively resistant to oxidative stress by transcriptionally up-regulating glutamate cysteine ligase
4 Watson et al. (1991) Cancer Res. 51(15):3996-4000; Inhibition of c-myc expression by phosphorothioate antisense oligonucleotide identifies a critical role for c-myc in the growth of human breast cancer
5 Yoshikawa et al. (2019) Mol. Ther. Methods Clin. Dev. 13:290-302; Anti-cancer Effects of a Chemically Modified miR-143 on Bladder Cancer by Either Systemic or Intravesical Treatment
6 Tsujino et al. (2019) Cancer Sci. 110(7):2189-2199; MicroRNA-143/Musashi-2/KRAS cascade contributes positively to carcinogenesis in human bladder cancer
7 Tirella et al. (2019) Int. J. Pharm. 561:114-123; CD44 targeted delivery of siRNA by using HA-decorated nanotechnologies for KRAS silencing in cancer treatment
8 Nakada et al. (2001) Pancreatology 1(4):314-319; Antisense oligonucleotides specific to mutated K-ras genes inhibit invasiveness of human pancreatic cancer cell lines
9 Adams et al. (2015) Expert Opin. Ther. Targets 20(6):737-753; The Tumor-Suppressive and Potential Therapeutic Functions of miR-34a in Epithelial Carcinomas
10 Poeck et al. (2008) Nat. Med. 14(11):1256-1263; 5'-Triphosphate-siRNA: turning gene silencing and Rig-I activation against melanoma
11 Szegedi et al. (2008) Pathol. Oncol. Res. 14(3):275-279; Bcl-2 Antisense Oligonucleotide Inhibits the Proliferation of Childhood Leukemia/lymphoma Cells of the B-cell Lineage
12 Ripoll et al. (2018) RSC Adv. 8:20758-20763; Co-delivery of anti-PLK-1 siRNA and camptothecin by nanometric polydiacetylenic micelles results in a synergistic cell killing
13 Liu et al. (2012) BMC Cancer 12:519; MicroRNA-100 is a potential molecular marker of non-small cell lung cancer and functions as a tumor suppressor by targeting polo-like kinase 1
14 Spänkuch et al. (2008) Neoplasia 10(3):223-234; Downregulation of Plk1 expression by receptor-mediated uptake of antisense oligonucleotide-loaded nanoparticles

Cargo bioactive molecules can target central nervous system diseases, such as neurodegenerative diseases, such as Alzheimer's Disease. Exemplary of such is FKBP52 and the tetratricopeptide derivative therefrom. The full sequence of human peptidyl-prolyl cis-trans isomerase FKBP4 is (SEQ ID NO:1):

```
MTAEEMKATESGAQSAPLPMEGVDISPKQDEGVLKVIKREGTGTEMPMIGDRVFVHYTGW
LLDGTKFDSSLDRKDKFSFDLGKGEVIKAWDIAIATMKVGEVCHITCKPEYAYGSAGSPP
KIPPNATLVFEVELFEFKGEDLTEEEDGGIIRRIQTRGEGYAKPNEGAIVEVALEGYYKD
KLFDQRELRFEIGEGENLDLPYGLERAIQRMEKGEHSIVYLKPSYAFGSVGKEKFQIPPN
AELKYELHLKSFEKAKESWEMNSEEKLEQSTIVKERGTVYFKEGKYKQALLQYKKIVSWL
EYESSFSNEEAQKAQALRLASHLNLAMCHLKLQAFSAAIESCNKALELDSNNEKGLFRRG

EAHLAVNDFELARADFQKVLQLYPNNKAAKTQLAVCQQRIRRQLAREKKLYANMFERLAE
EENKAKAEASSGDHPTDTEMKEEQKSNTAGSQSQVETEA
```

The tetratricopeptide repeat (TPR) domain 260-400 is (SEQ ID NO:2):

MNSEEKLEQSTIVKERGTVYFKEGKYKQALLQYKKIVSWLEYESSFSNEEAQKAQALRLA
SHLNLAMCHLKLQAFSAAIESCNKALELDSNNEKGLFRRGEAHLAVNDFELARADFQKVL
QLYPNNKAAKTQLAVCQQRI

A relatively simple *in vitro* model was developed by a group from Institut National de la Santé et de la Recherche Médicale, Université Paris XI and they described their studies (see, Chambraud et al. (2007) FASEB J. 21(11):2787-97; and Chabraud et al. (2010) Proc. Natl. Acad. Sci. U.S.A. 107(6):2658-63). The effect of depletion of FKBP52 in PC12 cultured cells was examined by introducing two different small, interfering RNA (siRNA) duplexes specific for rat FKBP52, named RNAi 1 and RNAi 2. The sense sequence of siRNAs and an oligonucleotide duplex with a scrambled sequence corresponding to RNAi 1 were used as negative control. In these experiments, the level of FKBP52 analyzed by Western blot was substantially reduced after 48 h and remained low 72 h post-transfection. Tubulin and FKBP52 staining was performed 72 h post transfections. In cells transfected with RNAi 1 or 2, FKBP52 staining was significantly lower than that observed in control cells, and tubulin staining revealed a change in the PC12 cell phenotype-in particular, the loss of FKBP52 in PC12 cells results in these cells forming extensions. Therefore, these cells acquired a differentiated phenotype that could be compared with PC12 cells treated with NGF. No significant modification could be observed in cells transfected with controls. In another study, Chambraud et al. (Proc. Natl. Acad. Sci. U.S.A., cited above) reports that FKBP52 prevents Tau Accumulation and Neurite Outgrowth in PC12 Cells. The FKBP52-inducible expression system based on a tetracycline-responsive element was used. The system allows the generation of a stably transformed PC12 cell line to determine a cellular role for FKBP52. Among clones that were positively tested, one clone, so-called H7C2, was selected and used to study the effects of FKBP52 overexpression on PC12 cells and to further investigate the relationship between FKBP52 and Tau. Under basal conditions, H7C2 cells expressed endogenous FKBP52, and treatment with doxycycline (Dox) resulted in a marked increase of recombinant FKBP52 protein expression. FKBP52 induction in H7C2 cells was about four-fold after 5 days of Dox treatment. Next, the effect of FKBP52 on the accumulation of Tau was examined. The amount of Tau protein was determined by Western blotting of extracts from cultures of either PC12 cells or H7C2 cells, treated or not with nerve growth factor (NGF) (50 nM) for 5 days with or without Dox. In PC12 cells, FKBP52 expression was unchanged after treatment with NGF. As expected, in both PC12 and H7C2 cells, an increase in Tau was observed after NGF treatment. When H7C2 cells were exposed to Dox in addition to NGF, so that they overexpress FKBP52, no additional accumulation of Tau protein occurred. An increase in Tau protein was still observed in PC12 cells treated with NGF and Dox, ruling out the possibility that Dox was responsible for the lack of decrease in Tau. The report concludes that FKBP52 prevents the accumulation of Tau induced by NGF in PC12 cells.

Because one role of Tau is to stimulate neurite outgrowth, the consequence of FKBP52 overexpression on neurite length in PC12 and H7C2 cells also was investigated. In the absence of NGF, no neurite outgrowth was observed in H7C2 cells, whether or not they were treated with Dox for a week. In H7C2 cells treated with 50 nM NGF and Dox, a 40% (±7) decrease in neurite length, compared to control (H7C2 not treated with Dox) was observed. The same effect of Dox on neurite length was observed in H7C2 cells treated with 10 or 20 nM NGF. Dox by itself was not involved in the process of neurite outgrowth because there was no difference in neurite length between Dox-treated and untreated PC12 cells observed. The inhibition of neurite outgrowth resulting from FKBP52 overexpression is in agreement with the previous report from Chambraud *et al.* showing that the loss of FKBP52 in PC12 cells results in the formation of neurite extensions. The FKBP52 effect on neurite length could be explained by the binding of Tau to FKBP52, removing Tau from microtubules. The prevention of Tau accumulation by overexpression of FKBP52 is consistent with the decrease of neurite length and suggests a potential role of this immunophilin in Tau function. Hence, the above target and sequences can be delivered or encoded in MEVs for treatment of Alzheimer's Disease by preventing accumulation of Tau.

**Reporter genes, reporter proteins, and/or modulators thereof can be delivered in the MEVs.**

**Reporter proteins**

Target sequences, in the form of anti-sense oligonucleotides (ASOs), peptides and/or tetratricopeptides, to modulate (inhibition or stimulation) of each of the marker genes, such as a GFP protein, a eukaryotic luciferase, or a prokaryotic Luciferase, such as: Lux operon (luxCDABE) and lux operon (luxABCDE), can be used, for example for diagnostics and gene expression assessments (SEQ ID NOs:5-6, 7, and 62-65, respectively):

| Target gene | Type of sequence | Sequence(s) | SEQ ID NO. |
|---|---|---|---|
| **EGFP** | siRNA | sense: 5'-GCAAGCUGACCCUGAAGUUCAUUU-3'<br>antisense:5'-AUGAACUUCAGGGUCAGCUUGCCG-3' | 5<br>6 |
| **firefly luciferase** | shRNA | 5'-CTGACGCGGAATACTTCGA-3' | 7 |

(continued)

| Target gene | Type of sequence | Sequence(s) | SEQ ID NO. |
|---|---|---|---|
| luxA (Lux operon) | siRNA | sense: 5'-CAAACAGAGGUAAUGAAAUGGUUG-3'<br>antisense:3'-CAACCAUUUCAUUACCUCUGUUUG-5' | 62<br>63 |
| luxB (Lux operon) | siRNA | sense: 5'-AUGUUAAGUUGAAUAAGUUCUGCA-3'<br>antisense:3'-UGCUCUUGAAUAAGUUGAAUUGAU-5' | 64<br>65 |

Other exemplary cargo can include chemotherapeutic agents, which include but are not limited to alkylating agents such as thiotepa and cyclophosphamide (available under the trademark CYTOXAN®); alkyl sulfonates such as busulfan, improsulfan and piposulfan; androgens such as calusterone, dromostanolone propionate, epitiostanol, mepitiostane, and testolactone; anti-adrenals such as aminoglutethimide, mitotane, and trilostane; anti-androgens such as flutamide, nilutamide, bicalutamide, leuprolide, and goserelin; antibiotics such as aclacinomycin, actinomycin, anthramycin, azaserine, bleomycin, cactinomycin, calicheamicin, carubicin, carminomycin, carzinophilin, chromomycin, dactinomycin, daunorubicin, detorubicin, 6-diazo-5-oxo-L-norleucine, doxorubicin, epirubicin, esorubicin, idarubicin, marcellomycin, mitomycin, mycophenolic acid, nogalamycin, olivomycin, peplomycin, porfiromycin, puromycin, quelamycin, rodorubicin, streptonigrin, streptozocin, tubercidin, ubenimex, zinostatin, and zorubicin; anti-estrogens including for example tamoxifen, raloxifene, aromatase inhibiting 4(5)-imidazoles, 4-hydroxytamoxifen, trioxifene, keoxifene, LY 117018, onapristone, and toremifene (Fareston®); anti-metabolites such as methotrexate and 5-fluorouracil (5-FU); folic acid analogs such as denopterin, methotrexate, pteropterin, and trimetrexate; aziridines such as benzodepa, carboquone, meturedepa, and uredepa; ethylenimines and methylmelamines including altretamine, triethylenemelamine, triethylenephosphoramide, triethylenethiophosphoramide and trimethylol melamine; folic acid replenisher such as folinic acid; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, and uracil mustard; nitrosoureas such as carmustine, chlorozotocin, fotemustine, lomustine, nimustine, and ranimustine; platinum analogs such as cisplatin and carboplatin; vinblastine; platinum; proteins such as arginine deiminase and asparaginase; purine analogs such as fludarabine, 6-mercaptopurine, thiamiprine, and thioguanine; pyrimidine analogs such as ancitabine, azacitidine, 6-azauridine, carmofur, cytarabine, dideoxyuridine, doxifluridine, enocitabine, floxuridine, 5-FU; taxanes such as paclitaxel (*e.g.*, sold under the trademark TAXOL®, Bristol-Myers Squibb Oncology, Princeton, N.J.) and docetaxel (*e.g.*, sold under the trademark TAXOTERE®, Rhone-Poulenc Rorer, Antony, France); topoisomerase inhibitor RFS 2000; thymidylate synthase inhibitor (such as Tomudex® chemotherapy drug); additional chemotherapeutics including aceglatone; aldophosphamide glycoside; aminolevulinic acid; amsacrine; bestrabucil; bisantrene; edatrexate; defosfamide; demecolcine; diaziquone; difluoromethylornithine (DFMO); eflornithine; elliptinium acetate; etoglucid; gallium nitrate; hydroxyurea; lentinan; lonidamine; mitoguazone; mitoxantrone; mopidamol; nitracrine; pentostatin; phenamet; pirarubicin; podophyllinic acid; 2-ethylhydrazide; procarbazine; PSK® medication; razoxane; sizofiran; spirogermanium; tenuazonic acid; triaziquone; 2,2', 2"-trichlorotriethylamine; urethan; vindesine; dacarbazine; mannomustine; mitobronitol; mitolactol; pipobroman; gacytosine; arabinoside ( Ara-C); cyclophosphamide; thiotepa; chlorambucil; gemcitabine; 6-thioguanine; mercaptopurine; methotrexate; etoposide (VP-16); ifosfamide; mitomycin C; mitoxantrone; vincristine; vinorelbine; Navelbine®; Novantrone; teniposide; daunomycin; aminopterin; Xeloda®; ibandronate; CPT-11; retinoic acid; esperamycins; capecitabine; and topoisomerase inhibitors such as irinotecan. Pharmaceutically acceptable salts, acids or derivatives of any of the above also can be used.

Chemotherapeutic agents include prodrugs, which include, but are not limited to, phosphate-containing prodrugs, thiophosphate-containing prodrugs, sulfate-containing prodrugs, peptide-containing prodrugs, D-amino acid-modified prodrugs, glycosylated prodrugs, beta-lactam-containing prodrugs, optionally substituted phenoxy acetamide-containing prodrugs or optionally substituted phenylacetamide-containing prodrugs, 5-fluorocytosine and other 5-fluorouridine prodrugs which can be converted into the more active cytotoxic free drug.

Other cargo include, for example, anti-angiogenic agents. Anti-angiogenic agents can be a small molecule or protein, such as an antibody, Fc fusion, and cytokine, that binds to a growth factor or growth factor receptor involved in promoting angiogenesis. Examples of anti-angiogenic agents include but are not limited to antibodies that bind to Vascular Endothelial Growth Factor (VEGF) or that bind to VEGF-R, RNA-based therapeutics that reduce levels of VEGF or VEGF-R expression, VEGF-toxin fusions, Regeneron's VEGF-trap, angiostatin (plasminogen fragment), antithrombin III, angiozyme, ABT-627, Bay 12-9566, BeneFin, bevacizumab, bisphosphonates, BMS-275291, cartilage-derived inhibitor (CDI), CAI, CD59 complement fragment, CEP-7055, Col 3, Combretastatin A-4, endostatin (collagen XVIII fragment), farnesyl transferase inhibitors, fibronectin fragment, GRO-beta, halofuginone, heparinases, heparin hexasaccharide

fragment, HMV833, human chorionic gonadotropin (hCG), IM-862, interferon alpha, interferon beta, interferon gamma, interferon inducible protein 10 (IP-10), interleukin-12, kringle 5 (plasminogen fragment), marimastat, metalloproteinase inhibitors (e.g., TIMPs), 2-methoxyestradiol, MMI 270 (CGS 27023A), plasminogen activator inhibitor (PAI), platelet factor-4 (PF4), prinomastat, prolactin 16 kDa fragment, proliferin-related protein (PRP), PTK 787/ZK 222594, retinoids, solimastat, squalamine, SS3304, SU5416, SU6668, SU11248, tetrahydrocortisol-S, tetrathiomolybdate, thalidomide, thrombospondin-1 (TSP-1), TNP470, transforming growth factor beta (TGF-β), vasculostatin, vasostatin (calreticulin fragment), ZS6126, and ZD6474.

Other cargo include tyrosine kinase inhibitors, which include, but are not limited to quinazolines, such as PD 153035, 4-(3-chloroanilino) quinazoline; pyridopyrimidines; pyrimidopyrimidines; pyrrolopyrimidines, such as CGP 59326, CGP 60261 and CGP 62706; pyrazolopyrimidines, 4-(phenylamino)-7H-pyrrolo(2,3-d) pyrimidines; curcumin (diferuloylmethane, 4,5-bis (4-fluoroanilino) phthalimide); tyrphostins containing nitrothiophene moieties; PD-0183805 (Warner-Lambert); antisense molecules (*e.g.*, those that bind to ErbB-encoding nucleic acid); quinoxalines (U.S. Pat. No. 5,804,396); tyrphostins (U.S. Pat. No. 5,804,396); PTK-787 (Novartis/Schering A G); pan-ErbB inhibitors such as C1-1033 (Pfizer); Affinitac™ cancer treatment (ISIS 3521; Isis/Lilly); Imatinib mesylate (STI571, Gleevec® chemotherapy medication; Novartis); PKI 166 (Novartis); GW2016 (Glaxo SmithKline); C1-1033 (Pfizer); EKB-569 (Wyeth); Semaxinib (Sugen); ZD6474 (AstraZeneca); PTK-787 (Novartis/Schering A G); IMC-1C11 (ImClone); or as described in any of the following patent publications: U.S. Pat. No. 5,804,396; PCT WO 99/09016 (American Cyanamid); PCT WO 98/43960 (American Cyanamid); PCT WO 97/38983 (Warner-Lambert); PCT WO 99/06378 (Warner-Lambert); PCT WO 99/06396 (Warner-Lambert); PCT WO 96/30347 (Pfizer, Inc.); PCT WO 96/33978 (AstraZeneca); PCT WO 96/33979 (AstraZeneca); PCT WO 96/33980 (AstraZeneca), gefitinib (Iressa® medication, ZD1839, AstraZeneca), and OSI-774 (Tarceva® medication, OSI Pharmaceuticals/Genentech).

Other cargo include immunomodulatory agents that increase or decrease production of one or more cytokines, up-or down-regulate self-antigen presentation, mask MHC antigens, or promote the proliferation, differentiation, migration, or activation state of one or more types of immune cells. Examples of immunomodulatory agents include but are not limited to non-steroidal anti-inflammatory drugs (NSAIDs) such as aspirin, ibuprofen, celecoxib, diclofenac, etodolac, fenoprofen, indomethacin, ketorolac, oxaprozin, nabumetone, sulindac, tolmetin, rofecoxib, naproxen, ketoprofen, and nabumetone; steroids (e.g., glucocorticoids, dexamethasone, cortisone, hydroxycortisone, methylprednisolone, prednisone, prednisolone, triamcinolone, azulfidine eicosanoids such as prostaglandins, thromboxanes, and leukotrienes; as well as topical steroids such as anthralin, calcipotriene, clobetasol, and tazarotene); cytokines such as TGFβ, IFNα, IFNβ, IFNγ, IL-2, IL-4, IL-10; cytokine, chemokine, or receptor antagonists including antibodies, soluble receptors, and receptor-Fc fusions, such as those against BAFF, B7, CCR2, CCR5, CD2, CD3, CD4, CD6, CD7, CD8, CD11, CD14, CD15, CD17, CD18, CD20, CD23, CD28, CD40, CD40L, CD44, CD45, CD52, CD64, CD80, CD86, CD147, CD152, complement factors (C5, D), CTLA4, eotaxin, Fas, ICAM, IFNα, IFNβ, IFNγ, IFNAR, IgE, IL-1, IL-2, IL-2R, IL-4, IL-5R, IL-6, IL-8, IL-9 IL-12, IL-13, IL-13R1, IL-15, IL-18R, IL-23, integrins, LFA-1, LFA-3, MHC, selectins, TGFβ, TNFα, TNFβ, TNF-R1, T-cell receptor, including Enbrel® (etanercept), Humira® (adalimumab), and Remicade® (infliximab) medications; heterologous antilymphocyte globulin; other immunomodulatory molecules such as 2-amino-6-aryl-5 substituted pyrimidines, anti-idiotypic antibodies for MHC binding peptides and MHC fragments, azathioprine, brequinar, Bromocriptine, cyclophosphamide, cyclosporine A, D-penicillamine, deoxyspergualin, FK506, glutaraldehyde, gold, hydroxychloroquine, leflunomide, malononitriloamides (e.g., leflunomide), methotrexate, minocycline, mizoribine, mycophenolate mofetil, rapamycin, and sulfasalazine.

Other cargo include cytokines which include, but are not limited to lymphokines, monokines, and traditional polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; Müllerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and-II; erythropoietin (EPO); osteoinductive factors; interferons such as interferon-alpha, -beta, and -gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL).

Other exemplary cargo include cytokines and other agents that stimulate cells of the immune system and enhance desired effector function. For example, agents that stimulate NK cells include IL-2; agents that stimulate macrophages include but are not limited to C5a, formyl peptides such as N-formyl-methionyl-leucyl-phenylalanine. Cargo include agents that stimulate neutrophils, such as, for example, G-CSF and GM-CSF. Additional agents include, but are not limited to, interferon gamma, IL-3 and IL-7.

Cargo include antibiotics, for treatment of infections, particularly for hard-to-treat bacterial infections, including

urinary tract infection, respiratory infections, particularly *Pseudomonas aeruginosa* or *Staphylococcus aureus* infections in subjects with cystic fibrosis, and sinus infections, which can be treated by local administration, such as by inhalation of aerosols containing the MEVs. The antibiotic treatments for pulmonary infections in subjects with cystic fibrosis can be combined with gene therapy using the same or different MEVs that comprise nucleic acid, DNA or RNA, encoding the cystic fibrosis transmembrane conductance regulator (CFTR) protein or providing a gene editing system to correct the defect in CFTR protein.

Antibiotics that can be loaded as cargo in the MEVs include but are not limited to: aminoglycoside antibiotics (*e.g.*, apramycin, arbekacin, bambermycins, butirosin, dibekacin, gentamicin, kanamycin, neomycin, netilmicin, paromomycin, ribostamycin, sisomicin, and spectinomycin), aminocyclitols (*e.g.*, spectinomycin), amphenicol antibiotics (*e.g.*, azidamfenicol, chloramphenicol, florfenicol, and thiamphenicol), ansamycin antibiotics (*e.g.*, rifamide and rifampin), carbapenems *(e.g.,* imipenem, meropenem, and panipenem); cephalosporins *(e.g.,* cefaclor, cefadroxil, cefamandole, cefatrizine, cefazedone, cefozopran, cefpimizole, cefpiramide, cefpirome, cefprozil, cefuroxime, cefixime, cephalexin, and cephradine), cephamycins (cefbuperazone, cefoxitin, cefminox, cefmetazole, and cefotetan); lincosamides *(e.g.,* clindamycin, and lincomycin); macrolide *(e.g.,* azithromycin, brefeldin A, clarithromycin, erythromycin, roxithromycin, and tobramycin), monobactams (e.g., aztreonam, carumonam, and tigemonam); mupirocin; Oxacephems *(e.g.,* flomoxef, latamoxef, and moxalactam); penicillins (*e.g.,* amdinocillin, amdinocillin pivoxil, amoxicillin, bacampicillin, benzylpenicillinic acid, benzylpenicillin sodium, epicillin, fenbenicillin, floxacillin, penamecillin, penethamate hydriodide, penicillin o-benethamine, penicillin O, penicillin V, penicillin V benzoate, penicillin V hydrabamine, penimepicycline, and phenethicillin potassium); polypeptides (*e.g.*, bacitracin, colistin, polymyxin B, teicoplanin, and vancomycin); quinolones (such as for example, amifloxacin, cinoxacin, ciprofloxacin, enoxacin, enrofloxacin, fleroxacin, flumequine, gatifloxacin, gemifloxacin, grepafloxacin, lomefloxacin, moxifloxacin, nalidixic acid, norfloxacin, ofloxacin, oxolinic acid, pefloxacin, pipemidic acid, rosoxacin, rufloxacin, sparfloxacin, temafloxacin, tosufloxacin, and trovafloxacin); rifampin; streptogramins (e.g., quinupristin, and dalfopristin); sulfonamides (sulfanilamide, and sulfamethoxazole); and tetracyclines (chlortetracycline, demeclocycline hydrochloride, demethylchlortetracycline, doxycycline, Duramycin, minocycline, neomycin, oxytetracycline, streptomycin, tetracycline, and vancomycin).

Cargo also include anti-fungal agents, which include, but are not limited to, amphotericin B, ciclopirox, clotrimazole, econazole, fluconazole, flucytosine, itraconazole, ketoconazole, miconazole, nystatin, terbinafine, terconazole, and tioconazole. In some examples, cargo-loaded MEVs described herein are administered with one or more antiviral agents, including but not limited to protease inhibitors, reverse transcriptase inhibitors, and others, including type I interferons, viral fusion inhibitors, neuraminidase inhibitors, acyclovir, adefovir, amantadine, amprenavir, clevudine, enfuvirtide, entecavir, foscamet, ganciclovir, idoxuridine, indinavir, lopinavir, pleconaril, ribavirin, rimantadine, ritonavir, saquinavir, trifluridine, vidarabine, and zidovudine.

In all instances, the form of the cargo includes proteins, and also, nucleic acid encoding the proteins, such as the plasmids, and also, mRNA. The nucleic acids can be operably linked to regulatory elements that are recognized in the particular subject, such as a mammal, in which they are to be delivered.

| Target organ/tissue | Exemplary indication | Exemplary route of administration |
|---|---|---|
| Lung | Cystic Fibrosis | Inhalation |
| Lung | Cystic Fibrosis | Inhalation |
| Lung | Idiopathic Pulmonary Fibrosis | Inhalation |
| Lung | Primary Ciliary Dyskinesia | Inhalation |
| Lung | Pulmonary Arterial Hypertension | Inhalation |
| Liver | Inborn error of metabolism | Intravenous or direct injection into the liver |
| Lung | Covid-19 (preventive or therapeutic) | Intranasal or Inhalation |
| Lymphatic | Covid-19 (vaccine) | Intravenous or intramuscular |
| Lung | Influenza (preventive or therapeutic) | Intranasal or Inhalation |
| Lymphatic | Influenza (vaccine) | Intravenous or intramuscular |
| Lymphatic | Viral pathogen | Intravenous or intramuscular |
| Lymphatic | Bacterial pathogen | Intravenous or intramuscular |

### 3. Generation of Payload-Loaded MEVs

As shown herein, the isolated *Chlorella* can be loaded with cargo for delivery to humans by any suitable route, including but not limited to intravenous, oral, topical, mucosal, inhalation, and any other routes known to those of skill in the art for delivery of vehicles, such as lipid nanoparticles, vectors, therapeutic bacteria, and therapeutic viruses. Upon administration, the MEVs are taken up by cells. Any cargo presently delivered in vectors, bacteria, exosomes, nanoparticles, and other such delivery vehicles can be loaded into the MEVs provided herein. The loaded cargo can be selected so that it only is expressed or produced in targeted cells, such as in instances in which the cargo is a plasmid encoding a therapeutic product. Transcription regulatory signals can be selected so that the encoded product is expressed in targeted cells. For example, for expression in the liver, the encoded product can be expressed under control of a liver-specific promoter, or the product can be targeted to a receptor or target expressed in targeted cells, such as in tumors or in the tumor microenvironment. Delivery methods, described above, and in the Examples below, include, but are not limited to:

**a. Electroporation**
**b. Sonication**
**c. Extrusion**
**d. Surfactants**
**e. Other Methods**

### 4. Exemplary Cargo and Exemplary Uses of the Exogenously Loaded MEVs

#### a. Cargo

As described above, the MEVs are loaded with cargo that can be used for any purpose of interest, including any for which other delivery vehicles are used. These uses include delivery of mRNA, such as mRNA encoding corona virus spike proteins and modified spike proteins to improve the immune response to the viruses, Anti-sense RNA, or anti-sense DNA (ASO), to silence genes, such as bacterial and viral pathogen virulence genes, antibiotic resistance genes, antimicrobial resistance genes, genes that suppress the immune system, tumor genes, such as oncogenes, and host factors for viral infection, such as targeting angiotensin-converting enzyme-2 (ACE2), transmembrane protein serine 2 (TMPRSS2), and other such genes. The cargo also can include any therapeutic antibodies. Therapeutic antibodies, include, but are not limited to, anti-cancer antibodies, antibodies to treat autoimmune or inflammatory disease, antibodies to treat transplant rejection, antibodies to treat graft-versus-host-disease (GVHD), and antibodies to treat infectious diseases.

#### 1) RNA Cargo (reference only)

The mechanism of RNA interference or RNAi was originally described as a process of sequence-specific silencing of gene expression in the nematode *Caenorhabditis elegans* (Fire et al. (1998) Nature 391(6669):806-11; Fire and Mello, 2006 Nobel Prize in Medicine awarded to Andrew Fire and Craig Mello). The process of small RNAs targeting (and silencing) messenger RNAs involves a particular RNAi machinery (including silencing factors, such as DICER and ARGONAUTE).

In the plant kingdom, RNAi is involved in antiviral defense mechanisms, and in defense mechanisms against phytopathogenic fungi and oomycetes. Small regulatory RNAs can be active in silencing genes inside bacterial cells, which lack the said RNAi machinery. The silencing activity of siRNA has been demonstrated to be interkingdom (see, *e.g.,* Singla, Navarro., 2019a, PCT / EP2019 / 072169; Singla, Navarro., 2019b, PCT / EP2019 / 072170; Singla et al. (2019c) bioRxiv, doi: doi.org/10.1101/863902).

RNAi-mediated regulation of gene expression has been exploited for several years in the field of biotechnology to confer resistance to viruses (Baulcombe (2015) Current Opinion in Plant Biology 26:141-146). The inter-kingdom RNAi has been used to characterize the function of genes of eukaryotic pathogens / parasites as well as to induce protection against these organisms.

In *Drosophila* and *Caenorhabditis,* RNAi plays a crucial role in antiviral defense by directly targeting viral RNAs via the small RNAs produced by the host in response to viruses. Recent work has shown that plant EVs naturally loaded (loaded by the plant cells producing the EVs) with small RNAs, from human edible plants, can modify the composition of the human gut microbiota and oral microbiota by silencing the expression of specific genes in certain commensal bacteria (Teng et al. (2018) Cell Host & Microbes 24:637-652; Sundaram et al. (2019) iScience 21:308-327).

Small interfering RNAs (siRNAs) and microRNAs (miRNAs) are noncoding RNAs with important roles in gene regulation. They have recently been investigated as novel classes of therapeutic agents for the treatment of a wide range of disorders including cancers and infections. Clinical trials of siRNA- and miRNA-based drugs have already been initiated.

siRNAs and miRNAs share many similarities, both are short duplex RNA molecules that exert gene silencing effects at the post-transcriptional level by targeting messenger RNA (mRNA), yet their mechanisms of action and clinical applications are distinct. The major difference between siRNAs and miRNAs is that the former are highly specific with only one mRNA target, whereas the latter have multiple targets. The siRNAs and miRNAs have a role in gene regulation, and serve as targets for drug discovery and development. Compared with conventional small therapeutic molecules, siRNAs and miRNAs offer the potential to be highly potent and able to act on "non-druggable" targets (for example, proteins which lack an enzymatic function); moreover, RNAi can be designed to target and/or affect expression of any gene of interest.

**2) Antibody Cargo**

Examples of anti-cancer antibodies and other antibodies, include, but are not limited to, anti-17-1A cell surface antigen antibodies such as the antibody sold or provided under the trademark Panorex® (edrecolomab); anti-4-1BB antibodies; anti-4Dc antibodies; anti-A33 antibodies such as A33 and CDP-833; anti-$\alpha$1 integrin antibodies such as natalizumab; anti-$\alpha 4\beta 7$ integrin antibodies such as LDP-02; anti-$\alpha V\beta 1$ integrin antibodies such as F-200, M-200, and SJ-749; anti-$\alpha V\beta 3$ integrin antibodies such as abciximab, CNTO-95, Mab-17E6, and Vitaxin® antibodies; anti-complement factor 5 (C5) antibodies such as 5G1.1; anti-CA125 antibodies such as sold or provided under the trademark OvaRex® (oregovomab); anti-CD3 antibodies such as those sold or provided under the trademark Nuvion® (visilizumab) and Rexomab®; anti-CD4 antibodies such as IDEC-151, MDX-CD4, OKT4A; anti-CD6 antibodies such as Oncolysin B and Oncolysin CD6; anti-CD7 antibodies such as HB2; anti-CD19 antibodies such as B43, MT-103, and Oncolysin B; anti-CD20 antibodies such as 2H7, 2H7.v16, 2H7.v114, 2H7.v115, the product sold or provided under the trademark Bexxar® (tositumomab), the antibody sold or provided under the trademark Rituxan® (rituximab), and the antibody sold or provided under the trademark Zevalin® (Ibritumomab tiuxetan); anti-CD22 antibodies such as the those sold or provided under the following generic names, tradenames, or trademarks: Lymphocide® (epratuzumab); anti-CD23 antibodies such as IDEC-152; anti-CD25 antibodies such as basiliximab and antibodies sold under the trademark Zenapax® (daclizumab); anti-CD30 antibodies such as AC10, MDX-060, and SGN-30; anti-CD33 antibodies such as those sold under the trademark Mylotarg® (gemtuzumab ozogamicin), Oncolysin M, and Smart M195; anti-CD38 antibodies; anti-CD40 antibodies such as SGN-40 and toralizumab; anti-CD40L antibodies such as 5c8, antibodies sold under the trademark Antova®, and IDEC-131; anti-CD44 antibodies such as bivatuzumab; anti-CD46 antibodies; anti-CD52 antibodies such as those sold under the trademark Campath® (alemtuzumab); anti-CD55 antibodies such as SC-1; anti-CD56 antibodies such as huN901-DM1; anti-CD64 antibodies such as MDX-33; anti-CD66e antibodies such as XR-303; anti-CD74 antibodies such as IMMU-110; anti-CD80 antibodies such as galiximab and IDEC-114; anti-CD89 antibodies such as MDX-214; anti-CD123 antibodies; anti-CD138 antibodies such as B-B4-DM1; anti-CD146 antibodies such as AA-98; anti-CD148 antibodies; anti-CEA antibodies such as cT84.66, labetuzumab, and those sold under the trademark Pentacea®; anti-CTLA-4 antibodies such as MDX-101; anti-CXCR4 antibodies; anti-EGFR antibodies such as ABX-EGF, those sold under the trademark Erbitux® (cetuximab), IMC-C225, and Merck Mab 425; anti-EpCAM antibodies such as Crucell's anti-EpCAM, ING-1, and KS-IL-2; anti-ephrin B2/EphB4 antibodies; anti-Her2 antibodies such as those sold under the trademark Herceptin®, MDX-210; anti-FAP (fibroblast activation protein) antibodies such as sibrotuzumab; anti-ferritin antibodies such as NXT-211; anti-FGF-1 antibodies; anti-FGF-3 antibodies; anti-FGF-8 antibodies; anti-FGFR antibodies, anti-fibrin antibodies; anti-G250 antibodies such as WX-G250 and those sold under the trademark Rencarex®; anti-GD2 ganglioside antibodies such as EMD-273063 and TriGem; anti-GD3 ganglioside antibodies such as BEC2, KW-2871, and mitumomab; anti-gpIIb/IIIa antibodies such as ReoPro; anti-heparinase antibodies; anti-Her2/ErbB2 antibodies such as Herceptin® (trastuzumab), MDX-210, and pertuzumab; anti-HLA antibodies such as those sold under the trademark Oncolym®, Smart 1D10; anti-HM1.24 antibodies; anti-ICAM antibodies such as ICM3; anti-IgA receptor antibodies; anti-IGF-1 antibodies such as CP-751871 and EM-164; anti-IGF-1R antibodies such as IMC-A12; anti-IL-6 antibodies such as CNTO-328 and elsilimomab; anti-IL-15 antibodies such as those sold under the trademark HuMax®-IL15; anti-KDR antibodies; anti-laminin 5 antibodies; anti-Lewis Y antigen antibodies such as Hu3S193 and IGN-311; anti-MCAM antibodies; anti-Muc1 antibodies such as BravaRex and TriAb; anti-NCAM antibodies such as ERIC-1 and ICRT; anti-PEM antigen antibodies such as Theragyn and Therex; anti-PSA antibodies; anti-PSCA antibodies such as IG8; anti-Ptk antibodies; anti-PTN antibodies; anti-RANKL antibodies such as AMG-162; anti-RLIP76 antibodies; anti-SK-1 antigen antibodies such as Monopharm C; anti-STEAP antibodies; anti-TAG72 antibodies such as CC49-SCA and MDX-220; anti-TGF-$\beta$ antibodies such as CAT-152; anti-TNF-$\alpha$ antibodies such as CDP571, CDP870, D2E7, those sold under the trademark Humira® (adalimumab), and those sold under the trademark Remicade® (infliximab); anti-TRAIL-R1 and TRAIL-R2 antibodies; anti-VE-cadherin-2 antibodies; and anti-VLA-4 antibodies (such as those sold under the trademark Antegren®). Furthermore, anti-idiotype antibodies including but not limited to the GD3 epitope antibody BEC2 and the gp72 epitope antibody 105AD7, can be used. In addition, bispecific antibodies including but not limited to the anti-CD3/CD20 antibody Bi20 can be used.

Additional exemplary cargo, uses and treatments that can be effected with cargo-loaded MEVs are described, by way of example, as follows.

**b. Diseases and Methods of Treatment**

As described above, the MEVs can be loaded with any desired cargo, including, but not limited to, nucleic acid molecules, detectable marker proteins and tags, small molecule drugs, gene editing systems, and others, and combinations thereof for delivering therapeutic molecules, serving as vaccines, and for use in human and other animal health, agricultural, cosmetic, dermatological and diagnostic applications, industrial uses, and other uses. The MEVs can deliver nutrients, or regulators of gene pathways to produce a beneficial product, gene editing systems, such as CRISPR/cas to effect gene editing, and gene therapy vectors and products.

MEVs can carry cargo, for example, for treating a disease characterized by a genetic defect that results in a deficiency of a functional protein, or for treating a disease characterized by overexpression of a polypeptide. Non-limiting examples of diseases that can be treated by silencing of a target gene, for example using siRNA or microRNA (see, *e.g.,* International Pub. No. WO 2013/048734) include cancer (*e.g.,* lung cancer, leukemia and lymphoma, pancreatic cancer, colon cancer, prostate cancer, glioblastoma, ovarian cancer, breast cancer, head and neck cancer, liver cancer, skin cancer, and uterine cancer), cardiovascular diseases, ocular diseases (*e.g*., age-related macular degeneration, herpes stromal keratitis, Glaucoma, dry eye syndrome, diabetic retinopathy, and conditions associated with ocular angiogenesis and ocular hypertension), neurological diseases (*e.g*., amyotrophic lateral sclerosis, Alzheimer's disease, myasthenic disorders, Huntington's disease, Spinocerebellar ataxia, frontotemporal dementia, Parkinson's disease, prion diseases, and Lafora disease, and those arising from ischemic or hypoxic conditions), kidney disorders, inflammatory or autoimmune diseases (*e.g*., ischemia or reperfusion injury, restenosis, Rheumatoid arthritis, inflammatory Bowel Disease, *e.g*., Crohn's Disease or Ulcerative Colitis, lupus, multiple sclerosis, diabetes, *e.g*., type II diabetes, and diabetic conditions, arthritis, *e.g.,* rheumatoid or psoriatic), respiratory diseases (*e.g.,* asthma, Chronic obstructive pulmonary diseases (COPD), cystic fibrosis, acute respiratory distress syndrome (ARDS), emphysema, and acute lung injury), hearing disorders, epilepsy, spinal cord injuries, oral mucositis, male infertility, uterine disorders, endometrial disorders or conditions, as well as conditions relating to metabolism (*e.g*., obesity), ischemia, stroke, alcohol metabolism and liver function (see, e.g., International Pub. Nos. WO 2006/029161, WO 2007/022470, WO 2007/130604, WO 2008/021157, WO 2009/104051, WO 2009/142822, WO 2019/217459, WO 2020/123083; European Pub. No. EP 2504435; and U.S. Patent Pub. Nos. U.S. 2011/0223665, U.S. 2012/0116360, U.S. 2012/0071540, U.S. 2016/0257956, U.S. 2015/0196648, and U.S. 2017/0304459). The RNAi molecule may target a gene that encodes, for example, an oncogene, a transcription factor, a receptor, an enzyme, a structural protein, a cytokine, a cytokine receptor, a lectin, a selectin, an immunoglobulin, a kinase and a phosphatase. In accordance with the claims, the cargo is not RNAi.

Other cargo and uses are contemplated. For example, MEVs can carry cargo, for example, for treating conditions resulting from trauma, such as wounds, burns, skin cuts, broken bones, hair loss, dermis exposure, mucosal exposure, fibrosis, lacerations, and ulcerations. MEVs can carry cargo, for example, for treating conditions resulting from natural or induced aging, in particular on the skin, or of the vision.

MEVs can be used to deliver cargo to treat, *e.g*., with gene silencing, or prevent, *e.g*., through vaccination, infectious diseases. For example, MEVs derived from antigen-pulsed macrophages or dendritic cells were shown to elicit an immune response when introduced into naïve animals (György et al. (2015) Annu. Rev. Pharmacol. Toxicol. 55:439-464). Gene silencing also can be used to target a pathogen-associated protein, such as a viral protein involved in immunosuppression of the host, replication of the pathogen, transmission of the pathogen, or maintenance of the infection; or a host protein which facilitates entry of the pathogen into the host, drug metabolism by the pathogen or host, replication or integration of the pathogen's genome, establishment or spread of infection in the host, or assembly of the next generation of pathogen. Pathogens can include, for example, RNA and DNA viruses such as arenaviruses, coronaviruses, influenza viruses, paramyxoviruses, flaviviruses (*e.g.,* West Nile virus), picornaviruses (*e.g.,* Coxsackievirus, Poliovirus, and Rhinovirus), rhabdoviruses, filoviruses, retroviruses (*e.g*., lentiviruses, and Rous sarcoma virus), adenoviruses, poxviruses, herpes viruses, human papilloma viruses, cytomegaloviruses, hepadnaviruses (*e.g*., Hepatitis B and C), rotaviruses, respiratory syncytial viruses, polyomaviruses, and others; bacteria; fungi; helminths; schistosomes; trypanosomes; parasites including plasmodiums (*e.g., Plasmodium malariae* and others); and mammalian transposable elements (see, *e.g.,* International Pub. Nos. WO 2010/141724, WO 2011/071860, WO 2011/072292, WO 2013/126803, WO 2020/035620, and WO 2020/097540; Australian Pub. Nos. AU 2004257373 A1, AU 2013203219 B2, and AU 2016225873 A1; European Pub. Nos. EP 2395012, and EP 2888240; U.S. Patent Pub. Nos. U.S. 2011/0223665, U.S. 2014/0256785, and U.S. 2019/0032051; Japanese Pub. No. JP 2018-197239A; and Taiwanese Pub No. TW 201204351A).

MEVs also can be used to deliver DNA or mRNA sequences that encode therapeutically useful polypeptides. For example, in cases where subjects lack a specific gene product, the gene can be encoded in a nucleic acid molecule, such as a DNA or RNA molecule. The nucleic acid molecule encoding the gene product can be loaded into a MEV and delivered to a subject lacking the gene product. For example, diseases that occur due to the absence or deficiency of a gene product, include, but are not limited to, lysosomal storage disorders, metabolic disorders of the urea cycle, SMN1-related spinal muscular atrophy (SMA); amyotrophic lateral sclerosis (ALS); GALT-related galactosemia; cystic fibrosis (CF); SLC3A1-related disorders including cystinuria; COL4A5-related disorders including Alport syndrome; galactocerebrosidase deficiencies; X-linked adrenoleukodystrophy and adrenomyeloneuropathy; Friedreich's ataxia; Pelizaeus-Merzbacher disease; TSC1 and TSC2-related tuberous sclerosis; Sanfilippo B syndrome (MPS IIIB); CTNS-related cystinosis; the FMR1-related disorders which include Fragile X syndrome, Fragile X-Associated Tremor/ Ataxia Syndrome and Fragile X Premature Ovarian Failure Syndrome; Prader-Willi syndrome; hereditary hemorrhagic telangiectasia; Niemann-Pick disease Type C1; the neuronal ceroid lipofuscinoses-related diseases including Juvenile Neuronal Ceroid Lipofuscinosis (JNCL), Juvenile Batten disease, Haltia-Santavuori disease, Jansky-Bielschowsky disease, and PTT-1 and TPP1 deficiencies; EIF2B1-, EIF2B2-, EIF2B3-, EIF2B4- and EIF2B5-related childhood ataxia with central nervous system hypomyelination/vanishing white matter; CACNA1A- and CACNB4-related Episodic Ataxia Type 2; the MECP2-related disorders including Classic Rett Syndrome, MECP2-related Severe Neonatal Encephalopathy and PPM-X Syndrome; CDKL5-related Atypical Rett Syndrome; Kennedy's disease (SBMA); Notch-3 related cerebral autosomal dominant arteriopathy with subcortical infarcts and leukoencephalopathy (CADASIL); SCN1A and SCN1B-related seizure disorders; the Polymerase G-related disorders, including Alpers-Huttenlocher syndrome, POLG-related sensory ataxic neuropathy, dysarthria, and ophthalmoparesis, and autosomal dominant and recessive progressive external ophthalmoplegia with mitochondrial DNA deletions; X-Linked adrenal hypoplasia; X-linked agammaglobulinemia; and Wilson's disease (see, e.g., International Pub. Nos. WO 2011/068810, WO 2019/243574, WO 2019/092287, and WO 2020/099682).

The MEVs may be loaded with a CRISPR/Cas system to effect gene editing. The clustered, regularly interspaced, short palindromic repeat (CRISPR) technology allows for the modification of the genome in a living organism, and is based on the bacterial CRISPR/Cas9 antiviral defense system. The system allows for DNA cleavage at a target site. The type II CRISPR system incorporates sequences from invading foreign nucleic acids, such as DNA from viruses or plasmids, between CRISPR repeat sequences encoded within the host genome. Transcripts from the CRISPR repeat sequences are processed into CRISPR RNAs (crRNAs). Each crRNAs harbors a variable sequence transcribed from the foreign DNA and a part of the CRISPR repeat. Each crRNA hybridizes with a second transactivating CRISPR RNA (tracrRNA) and these two RNAs complex with and direct the Cas9 nuclease to cleave the target DNA sequence. By delivering a Cas nuclease complexed with a synthetic guide RNA (gRNA), which consists of a fusion of a crRNA and a tracrRNA, into a cell, the cell's genome can be cut at a desired location, allowing existing genes to be removed and/or new ones added *in vivo* (Sander and Joung (2014) Nat. Biotechnol. 32(4):347-355). The CRISPR technology can be used with the Cas polypeptide or the single RNA guided endonuclease Cpf1 to effect genome modification, and can be delivered in lipid nanoparticles, EVs and other vesicles (see, *e.g*., International Pub. Nos. WO 2017/161010, WO 2019/238626, and WO 2020/097540).

MEVs also can be used to treat diseases, including but not limited to those listed above, by introduction of a payload in form of a therapeutic protein, polypeptide, or small organic molecule or compound to a target cell. Non-limiting examples of such therapeutically effective agents or drugs include oncology drugs (*e.g*., chemotherapy drugs, hormonal therapeutic agents, immunotherapeutic agents, and radiotherapeutic agents), lipid-lowering agents for treating lipid diseases, antiviral drugs, anti-fungal agents, anti-cholinergics, anti-inflammatory compounds, antidepressants, stimulants, analgesics, antibiotics, birth control medication, antipyretics, vasodilators, anti-angiogenics, cytovascular agents, anti-fibrotics, antihypertensives, aromatase or esterase inhibitors, signal transduction inhibitors, synthase inhibitors, cardiovascular drugs such as anti-arrhythmic agents, hormones or hormone antagonists, ion channel modifiers, antineoplastic agents, neuroactive agents, vasoconstrictors, cytotoxic agents, nucleolytic compounds, radioactive isotopes, pro-drug activating enzymes, and steroids (see, *e.g*., International Pub. Nos. WO2015110957A2 and WO2019018349A1; and U.S. Patent Pub. Nos. U.S. 2019/0032051, U.S. 2012/0315324, U.S. 2019/0388347, and U.S. 2019/0175506). The therapeutic also can be a biologic therapeutic agent selected from an allergen, adjuvant, antigen, or immunogen, antibody (*e.g*., whole antibodies, polyclonal, monoclonal and recombinant antibodies, fragments thereof, and further includes single-chain antibodies, humanized antibodies, murine antibodies, chimeric, mouse-human, mouse-primate, primate-human monoclonal antibodies, anti-idiotype antibodies, antibody fragments, such as, *e.g*., scFv, (scFv)2, Fab, Fab', and F(ab')2, F(ab)2, Fv, dAb, and Fd fragments, diabodies, and antibody-related polypeptides), cytokine, hormone, factor, cofactor, cell component protein, metabolic enzyme, immunoregulatory enzyme, interferon, interleukin, gastrointestinal enzyme, an enzyme or factor implicated in hemostasis, growth regulatory enzyme, vaccine, antithrombolytic, toxin, antitoxin, or diagnostic or imaging biologic agent (see, *e.g.,* International Pub. Nos. WO 2017/203260, WO 2018/102397, WO 2019/081474, WO 2019/155060, WO 2020/041720; Australian Pub. No. AU 2018365299A1; Singapore Pub. No. SG 11201811149TA; and U.S. Patent Pub. No. U.S. 2019/0202892). For example, MEV therapies can be used to treat Crohn's disease, ulcerative colitis, ankylosing spondylitis, rheumatoid arthritis, multiple sclerosis, systemic lupus erythematosus, sarcoidosis, idiopathic pulmonary fibrosis, psoriasis, tumor necrosis factor (TNF) receptor-associated periodic syndrome (TRAPS), deficiency of the interleukin-1 receptor antagonist (DIRA), endometriosis, autoimmune hepatitis, scleroderma, myositis, stroke, acute spinal cord injury, vasculitis, Guillain-Barre syndrome, acute myocardial infarction, acute respiratory distress syndrome (ARDS), sepsis, meningitis, encephalitis, liver failure, non-alcoholic steatohepatitis (NASH), non-alcoholic fatty liver disease (NAFLD), kidney failure, heart failure or any acute or chronic organ failure and the associated

underlying etiology, graft-vs-host disease, Duchenne muscular dystrophy and other muscular dystrophies, lysosomal storage diseases, neurodegenerative diseases, cancer-induced cachexia, anorexia, diabetes mellitus type 2, and cancers (*e.g.*, acute lymphoblastic leukemia (ALL), acute myeloid leukemia, adrenocortical carcinoma, AIDS-related cancers, AIDS-related lymphoma, anal cancer, appendix cancer, astrocytoma, cerebellar or cerebral, basal-cell carcinoma, bile duct cancer, bladder cancer, bone tumor, brainstem glioma, brain cancer, brain tumor (cerebellar astrocytoma, cerebral astrocytoma/malignant glioma, ependymoma, medulloblastoma, supratentorial primitive neuroectodermal tumors, visual pathway and hypothalamic glioma), breast cancer, bronchial adenomas/carcinoids, Burkitt's lymphoma, carcinoid tumor (childhood, gastrointestinal), carcinoma of unknown primary, central nervous system lymphoma, cerebellar astrocytoma/malignant glioma, cervical cancer, chronic lymphocytic leukemia, chronic myelogenous leukemia, chronic myeloproliferative disorders, colon Cancer, cutaneous T-cell lymphoma, desmoplastic small round cell tumor, endometrial cancer, ependymoma, esophageal cancer, extracranial germ cell tumor, extragonadal germ cell tumor, extrahepatic bile duct cancer, eye cancer (intraocular melanoma, retinoblastoma), gallbladder cancer, gastric cancer, gastrointestinal carcinoid tumor, gastrointestinal stromal tumor (GIST), germ cell tumor (extracranial, extragonadal, or ovarian), gestational trophoblastic tumor, glioma (glioma of the brain stem, cerebral astrocytoma, visual pathway and hypothalamic glioma), gastric carcinoid, hairy cell leukemia, head and neck cancer, heart cancer, hepatocellular (liver) cancer, hypopharyngeal cancer, intraocular melanoma, islet cell carcinoma (endocrine pancreas), kidney cancer (renal cell cancer), laryngeal cancer, leukemias (acute lymphoblastic, acute myeloid, chronic lymphocytic, chronic myelogenous, hairy cell leukemia), lip and oral cancer, cavity cancer, liposarcoma, liver cancer (primary), lung cancer (non-small cell, small cell), lymphomas, AIDS-related lymphoma, Burkitt lymphoma, cutaneous T-cell lymphoma, Hodgkin lymphoma, non-Hodgkin lymphoma, medulloblastoma, Merkel cell carcinoma, mesothelioma, metastatic squamous neck cancer with occult primary, mouth cancer, multiple endocrine neoplasia syndrome, multiple myeloma/plasma cell neoplasm, mycosis fungoides, myelodysplastic/myeloproliferative diseases, myelogenous leukemia, chronic myeloid leukemia, myeloma, nasal cavity and paranasal sinus cancer, nasopharyngeal carcinoma, neuroblastoma, oral cancer, oropharyngeal cancer, osteosarcoma/malignant fibrous histiocytoma of bone, ovarian cancer, ovarian epithelial cancer (surface epithelial-stromal tumor), ovarian germ cell tumor, ovarian low malignant potential tumor, pancreatic cancer, pancreatic islet cell cancer, parathyroid cancer, penile cancer, pharyngeal cancer, pheochromocytoma, pineal astrocytoma, pineal germinoma, pineoblastoma and supratentorial primitive neuroectodermal tumors, pituitary adenoma, pleuropulmonary blastoma, prostate cancer, rectal cancer, renal cell carcinoma (kidney cancer), retinoblastoma, rhabdomyosarcoma, salivary gland cancer, sarcoma (Ewing family of tumors sarcoma, Kaposi sarcoma, soft tissue sarcoma, uterine sarcoma), Sézary syndrome, skin cancer (nonmelanoma, melanoma), small intestine cancer, squamous cell, squamous neck cancer, stomach cancer, supratentorial primitive neuroectodermal tumor, testicular cancer, throat cancer, thymoma and thymic carcinoma, thyroid cancer, transitional cell cancer of the renal pelvis and ureter, urethral cancer, uterine cancer, uterine sarcoma, vaginal cancer, vulvar cancer, Waldenström macroglobulinemia, and/or Wilms' tumor) (see, *e.g.*, International Pub. Nos. WO 2017/203260 and WO 2019/155060A1; and U.S. Patent Pub. No. U.S. 2019/0388347).

### c. Agro-Veterinary Applications

MEVs carrying cargos of biomolecules can be used for agro-veterinary applications. For example, immune ribonucleic acid can be used for the treatment and prevention of poultry diseases and resistance to virulent pathogens can be enhanced in plants and animals by selective modulation of the miRNA pathway (see, International Pub. No. WO 2008/087562). Cargo-loaded MEVs can thus be used to treat diseases in animals, including livestock.

Although not forming part of the present invention, cargo-loaded MEVs can be used to treat plant diseases. As exemplified, the MEVs can be loaded with therapeutic molecules, such as siRNAs that target virulence genes in plant pathogens, including bacteria and virus, and delivered to the plants, such as by application or spraying onto leaves and/or other surfaces, to target the genes to eliminate or to control the pathogen.

### d. Cosmetic and Dermatological Applications

MEVs carrying payloads of pharmacological agents also can be used for cosmetic and dermatological applications. For example, skin care products such as creams, lotions, gels, emulsions, ointments, pastes, powders, liniments, sunscreens, and shampoos comprising EVs, particularly from stem cells, can be used to improve and/or alleviate symptoms and problems such as dry skin, elasticity, wrinkles, folds, ridges, and/or skin creases (see, *e.g.*, Singapore Pub. No. SG 11201811149TA). Stem cell EVs, which inherently carry cytokines, growth and transcription factors among their cargo, also have been shown to control inflammation, accelerate skin cell migration and proliferation, control wound scarring, improve angiogenesis, and ameliorate signs of skin aging. Although the exact mechanisms are being elucidated, the effect of stem cell EVs on wound healing may rely in the vertical transfer on microRNAs or proteins to skill cells. Angiogenesis, a part of wound healing, can be induced by stem cell EVs. Stem cell EVs also have beneficial effects for cellular matrix maintenance and collagen production, and have been shown to play a role in rejuvenating skin

cells (da Fonseca Ferreira, A. and Gomes, D. (2019) Bioengineering (Basel) 6(1):4). MEVs loaded with a desired cargo can thus be used for cosmetic and dermatological applications.

**e. Other Exemplary Cargo**

The MEVs can be exogenously loaded with any suitable heterologous cargo, including, but not limited to, nucleic acid molecules, including modified mRNA, proteins, polypeptides, and peptides, detectable marker proteins and tags, any therapeutic or prophylactic or vaccine polypeptide or peptide, gene editing systems, and others, and combinations thereof, provided that that cargo is not RNAi. The MEVs can deliver therapeutic molecules, can serve as vaccines, and can be used in human health, gene therapy applications, including delivery genes, modification of genes with gene editing systems, and gene silencing nucleic acids, cosmetic applications, dermatological applications, diagnostic applications, industrial uses, and others. The MEVs can deliver regulators of gene pathways to produce a beneficial product, and can be used to deliver gene editing systems, such as CRISPR/cas (see *e.g.,* SEQ ID NOs:73 and 74 for exemplary CRISPR/cas protein and encoding nucleic sequences, respectively) to effect gene editing.

Diseases and conditions that can be treated include any known to those of skill in the art, including but not limited to, cardiovascular diseases, metabolic diseases, infections, including respiratory infections, bladder infections and other urinary tract infections, infectious diseases, including viral disease, such as hepatitis, HIV, corona viruses, including SARS-CoV-2, CNS diseases, ocular diseases, and liver diseases. As discussed, delivered cargo includes protein products, such as, but not limited to, enzymes, regulatory factors, signaling proteins, antigens, antibodies and antigen-binding forms thereof, RNA products, such as mRNA, including modified mRNA, such as modified mRNA to increase stability for delivery.

An advantage of MEVs for delivery, is that the RNA is a labile molecule, and so, mRNAs delivered by other kinds of nanoparticles, like lipid nanoparticles (LNPs) have been modified to increase RNA stability. For delivery in MEVs, the mRNA does not necessarily have to be modified. Modified mRNA can be synthetic mRNA that comprises a translatable region that contains at least one nucleoside modification in which at least a percentage of the uridine nucleotides in the synthetic mRNA are modified (see, *e.g.,* U.S. Patent No. 9,464,124, which describes modified mRNA for delivery and translation; see, also, U.S. Patent No. 9,464,124). For example, at least one nucleoside modification can be pyridin-4-one ribonucleoside, 5-aza-uridine, 2-thio-5-aza-uridine, 2-thiouridine, 4-thio-pseudouridine, 2-thio-pseudouridine, 5-hydroxyuridine, 3-methyluridine, 5-carboxymethyl-uridine, 1-carboxymethyl-pseudouridine, 5-propynyl-uridine, 1-propynyl-pseudouridine, 5-taurinoethyluridine, 1-taurinomethyl-pseudouridine, 5-taurinomethyl-2-thio-uridine, 1-taurinomethyl-4-thio-uridine, 5-methyl-uridine, 1-methyl-pseudouridine, 4-thio-1-methyl-pseudouridine, 2-thio-1-methyl-pseudouridine, 1-methyl-1-deaza-pseudouridine, 2-thio-1-methyl-1-deaza-pseudouridine, dihydrouridine, dihydropseudouridine, 2-thio-dihydrouridine, 2-thio-dihydropseudouridine, 2-methoxyuridine, 2-methoxy-4-thiouridine, 4-methoxy-pseudouridine, 4-methoxy-2-thio-pseudouridine, 5-aza-cytidine, pseudoisocytidine, 3-methyl-cytidine, N4-acetylcytidine, 5-formylcytidine, N4-methylcytidine, 5-hydroxymethylcytidine, 1-methyl-pseudoisocytidine, pyrrolo-cytidine, pyrrolo-pseudoisocytidine, 2-thio-cytidine, 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-aza-zebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-methoxy-1-methyl-pseudoisocytidine, 2-aminopurine, 2, 6-diaminopurine, 7-deaza-adenine, 7-deaza-8-aza-adenine, 7-deaza-2-aminopurine, 7-deaza-8-aza-2-aminopurine, 7-deaza-2,6-diaminopurine, 7-deaza-8-aza-2,6-diaminopurine, 1-methyladenosine, N6-methyladenosine, N6-isopentenyladenosine, N6-(cis-hydroxyisopentenyl)adenosine, 2-methylthio-N6-(cis-hydroxyisopentenyl) adenosine, N6-glycinylcarbamoy-adenosine, N6-threonylcarbamoyladenosine, 2-methylthio-N6-threonyl carbamoyl-denosine, N6,N6-dimethyladenosine, 7-methyladenine, 2-methylthio-adenine, 2-methoxy-adenine, inosine, 1-methyl-inosine, wyosine, wybutosine, 7-deaza-guanosine, 7-deaza-8-aza-guanosine, 6-thio-guanosine, 6-thio-7-deaza-guanosine, 6-thio-7-deaza-8-aza-guanosine, 7-methyl-guanosine, 6-thio-7-methyl-guanosine, 7-methylinosine, 6-methoxy-guanosine, 1-methylguanosine, N2-methylguanosine, N2,N2-dimethylguanosine, 8-oxo-guanosine, 7-methyl-8-oxo-guanosine, 1-methyl-6-thio-guanosine, N2-methyl-6-thio-guanosine, and N2,N2-dimethyl-6-thio-guanosine. The mRNA can include a) a sequence of linked nucleotides, a 5' UTR, a 3' UTR, and at least one 5' cap structure. The mRNA also can include other regulatory sequences for translation and trafficking in a eukaryotic, such as mammalian, such as a human, host cell.

The MEVs can carry cargos that include reporter genes and proteins and other detectable products, such as, for example, a fluorescent protein, such as, but not limited to an enhanced green fluorescent protein (EGFP; SEQ ID NO:10), a luciferase gene (SEQ ID NO:11), luxA (SEQ ID NO:8), luxB (SEQ ID NO:9), and the Lux operon (luxCDABE and luxABCDE; SEQ ID NO:12).

Other cargos can target genes or products involved in diseases, such as, but not limited to, peptidyl-prolyl cis-trans isomerase FKBP4 or FKBP52 (SEQ ID NO:1); gamma-aminobutyric acid type B receptor subunit 1 (GABBR1; SEQ ID NO:3); oncogenes such as MYCN or NMYC (SEQ ID NO:38), RAS (H-RAS, N-RAS, and K-RAS; see SEQ ID NOs:39, 40, and 41, respectively), BCL2 (SEQ ID NO:43), and PLK1 (SEQ ID NO:44). Genes involved in diseases, such as

oncogenes, and checkpoints, can be modulated by cargo that encodes a product that inhibits or agonizes expression of a gene, or inhibits or agonizes a gene product. Exemplary of such modulators, are RNAis, such as, for example, siRNAs, miRNAs, shRNAs, peptides and/or tetratricopeptides. For example, siRNAs and ASOs targeting EGFP (SEQ ID NOs:5 and 6), firefly luciferase (SEQ ID NO:7), MYCN (SEQ ID NOs:13-19), RAS (SEQ ID NOs:20-27), BCL2 (SEQ ID NOs:29-31), and PLK1 (SEQ ID NOs:32-35), and microRNA-34A, which targets MYC and BCL2 (SEQ ID NO:28), are exemplified herein. In accordance with the claims, the cargo is not RNAi.

Although not forming part of the present invention, gene silencing using RNA interference, including siRNAs and miRNAs, can be used to silence developmental genes, such as, for example, adhesion molecules, cyclin kinase inhibitors, Wnt family members, Pax family members, Winged helix family members, Hox family members, cytokines/lymphokines and their receptors, growth/differentiation factors and their receptors, and neurotransmitters and their receptors; oncogenes; tumor suppressor genes; enzymes; genes associated with a pathological condition; genes associated with autoimmune diseases; anti-angiogenic genes; angiogenic genes; immunomodulator genes; genes associated with alcohol metabolism and liver function; genes associated with neurological disease; genes associated with tumorigenesis or cell transformation; and genes associated with metabolic diseases and disorders (see, *e.g.,* WO 2009/082606, JP 2014-240428A, WO 2011/072292A2, WO 2010/141724, and WO 2020/097540). These types of heterologous products can be delivered in or encoded in MEVs to activate genes or pathways or to provide therapeutic effects. Certain cytokines can be used to treat diseases/disorders, such as certain cancers, in which immune suppression plays a role.

Exo-loaded MEVs can be used to transfer therapeutic agents such as nucleic acids, such as mRNA, tRNA, rRNA, regulatory RNA, non-coding and encoding RNA, and DNA fragments (see, *e.g.,* CN105821081A and CN110699382A); nucleotides or amino acids comprising a detectable moiety; polypeptides (*e.g*., enzymes; see, U.S. Patent No. 10,195,290). Non-limiting examples of proteins that can be loaded into MEVs (or in some embodiments encoded by exo-loaded DNA), include, but are not limited to, antibodies, intrabodies, single chain variable fragments, affibodies, enzymes, transporters, tumor suppressors, viral or bacterial inhibitors, cell component proteins, DNA and/or RNA binding proteins, DNA repair inhibitors, nucleases, proteinases, integrases, transcription factors, growth factors, apoptosis inhibitors and inducers, toxins, structural proteins, neurotrophic factors, membrane transporters, nucleotide binding proteins, heat shock proteins, CRISPR-associated proteins, cytokines, cytokine receptors, caspases and any combination and/or derivatives thereof (see, *e.g*., AU2018365299).

Cargo bioactive molecules can target central nervous system diseases, such as neurodegenerative diseases, such as Alzheimer's Disease. Exemplary of such is FKBP52 and the tetratricopeptide derivative therefrom. The full sequence of human peptidyl-prolyl cis-trans isomerase FKBP4 is (SEQ ID NO:1):

```
MTAEEMKATESGAQSAPLPMEGVDISPKQDEGVLKVIKREGTGTEMPMIGDRVFVHYTGW

LLDGTKFDSSLDRKDKFSFDLGKGEVIKAWDIAIATMKVGEVCHITCKPEYAYGSAGSPP
KIPPNATLVFEVELFEFKGEDLTEEEDGGIIRRIQTRGEGYAKPNEGAIVEVALEGYYKD
KLFDQRELRFEIGEGENLDLPYGLERAIQRMEKGEHSIVYLKPSYAFGSVGKEKFQIPPN
AELKYELHLKSFEKAKESWEMNSEEKLEQSTIVKERGTVYFKEGKYKQALLQYKKIVSWL
EYESSFSNEEAQKAQALRLASHLNLAMCHLKLQAFSAAIESCNKALELDSNNEKGLFRRG
EAHLAVNDFELARADFQKVLQLYPNNKAAKTQLAVCQQRIRRQLAREKKLYANMFERLAE
EENKAKAEASSGDHPTDTEMKEEQKSNTAGSQSQVETEA
```

The tetratricopeptide repeat (TPR) domain 260-400 is (SEQ ID NO:2):

```
MNSEEKLEQSTIVKERGTVYFKEGKYKQALLQYKKIVSWLEYESSFSNEEAQKAQALRLA
SHLNLAMCHLKLQAFSAAIESCNKALELDSNNEKGLFRRGEAHLAVNDFELARADFQKVL
QLYPNNKAAKTQLAVCQQRI
```

For example, cargo can be delivered or encoded in MEVs for treatment of Alzheimer's Disease by preventing accumulation of Tau. An *in vitro* model is available and was developed by a group from Institut National de la Santé et de la Recherche Médicale, Université Paris XI (see, Chambraud et al. (2007) FASEB J. 21(11):2787-97; and Chabraud et al. (2010) Proc. Natl. Acad. Sci. U.S.A. 107(6):2658-63). The effect of depletion of FKBP52 in PC12 cultured cells was examined by introducing two different small, interfering RNA (siRNA) duplexes specific for rat FKBP52, named RNAi 1 and RNAi 2. The sense sequence of siRNAs and an oligonucleotide duplex with a scrambled sequence corresponding to RNAi 1 were used as negative control. In these experiments, the level of FKBP52 analyzed by Western blot was substantially reduced after 48 h and remained low 72 h post-transfection. Tubulin and FKBP52 staining was performed 72 h post transfections. In cells transfected with RNAi 1 or 2, FKBP52 staining was significantly lower than that observed in control cells, and tubulin staining revealed a change in the PC12 cell phenotype-in particular, the loss of FKBP52 in PC12 cells

results in these cells forming extensions. Therefore, these cells acquired a differentiated phenotype that could be compared with PC12 cells treated with NGF. No significant modification could be observed in cells transfected with controls. In another study, Chambraud et al. ((2010) Proc. Natl. Acad. Sci. U.S.A. 107(6):2658-63) reports that FKBP52 prevents Tau Accumulation and Neurite Outgrowth in PC12 Cells. The FKBP52-inducible expression system based on a tetracycline-responsive element was used. The system allows the generation of a stably transformed PC12 cell line to determine a cellular role for FKBP52. Among clones that were positively tested, one clone, so-called H7C2, was selected and used to study the effects of FKBP52 overexpression on PC12 cells and to further investigate the relationship between FKBP52 and Tau. Under basal conditions, H7C2 cells expressed endogenous FKBP52, and treatment with doxycycline (Dox) resulted in a marked increase of recombinant FKBP52 protein expression. FKBP52 induction in H7C2 cells was about fourfold after 5 days of Dox treatment. Next, the effect of FKBP52 on the accumulation of Tau was examined. The amount of Tau protein was determined by Western blotting of extracts from cultures of either PC12 cells or H7C2 cells, treated or not with nerve growth factor (NGF) (50 nM) for 5 days with or without Dox. In PC12 cells, FKBP52 expression was unchanged after treatment with NGF. As expected, in both PC12 and H7C2 cells an increase in Tau was observed after NGF treatment. When H7C2 cells were exposed to Dox in addition to NGF, so that they overexpress FKBP52, no additional accumulation of Tau protein occurred. An increase in Tau protein was still observed in PC12 cells treated with NGF and Dox, ruling out the possibility that Dox was responsible for the lack of decrease in Tau. The report concludes that FKBP52 prevents the accumulation of Tau induced by NGF in PC12 cells.

Because one role of Tau is to stimulate neurite outgrowth, the consequence of FKBP52 overexpression on neurite length in PC12 and H7C2 cells also was investigated. In the absence of NGF, no neurite outgrowth was observed in H7C2 cells, whether or not they were treated with Dox for a week. In H7C2 cells treated with 50 nM NGF and Dox, a 40% (±7) decrease in neurite length, compared to control (H7C2 not treated with Dox) was observed. The same effect of Dox on neurite length was observed in H7C2 cells treated with 10 or 20 nM NGF. Dox by itself was not involved in the process of neurite outgrowth because there was no difference in neurite length between Dox-treated and untreated PC12 cells observed. The inhibition of neurite outgrowth resulting from FKBP52 overexpression is in agreement with the previous report (Chambraud et al. (2010) Proc. Natl. Acad. Sci. U.S.A. 107(6):2658-63) showing that the loss of FKBP52 in PC12 cells results in the formation of neurite extensions. The FKBP52 effect on neurite length could be explained by the binding of Tau to FKBP52, removing Tau from microtubules. The prevention of Tau accumulation by overexpression of FKBP52 is consistent with the decrease of neurite length and is indicative of a role of this immunophilin in Tau function. Hence, the above target and sequences can be delivered or encoded in MEVs for treatment of Alzheimer's Disease by preventing accumulation of Tau.

Although not forming part of the present invention, the MEVs can deliver RNAi. Target sequences, in the form, for example, of siRNAs, miRNAs to modulate (inhibition or stimulation) of each of the marker genes, such as a beta-glucuronidase (GUS), green fluorescent protein (GFP) a eukaryotic luciferase, or a prokaryotic Luciferase, such as: Lux operon (luxCDABE) and lux operon (luxABCDE), can be used, for example for diagnostics and gene expression assessments (SEQ ID NOs: 75-80, 5, 6, 7, and 62-65, respectively). Exemplary siRNAs that target the encoding nucleic acid are set forth in the following table:

| Target gene | Type of sequence | Sequence(s) | SEQ ID NO: |
|---|---|---|---|
| **GUS** | siRNA[1] | GT1 sense: 5'-TACACTGTGGAATTGATCAGCGTTCAGATGACGCTGATCAATTCCACAGTTTTTTT | 75 |
| | | GT1 antisense: 5'-CTAGAAAAAAACTGTGGAATTGATCAGCGTCATCTGAACGCTGATCAATTCCACAGTGTA | 76 |
| | | GT2 sense: 5'-TACTTGATCAGCGTTGGTGGGATTCAGATGATCCCACCAACGCTGATCAATTTTTTT | 77 |
| | | GT2 antisense: 5'-CTAGAAAAAAATTGATCAGCGTTGGTGGGATCATCTGAATCCCACCAACGCTGATCAAGTA | 78 |
| | | GT3 sense: 5'-TACCTGTGGAATTGATCAGCGTTTCAGATGAACGCTGATCAATTCCACAGTTTTTTT | 79 |
| | | GT3 antisense: 5'-CTAGAAAAAAACTGTGGAATTGATCAGCGTTCATCTGAAACGCTGATCAATTCCACAGGTA | 80 |
| DEGFP | siRNA | sense: 5'-GCAAGCUGACCCUGAAGUUCAUUU-3'<br>antisense: 5'-AUGAACUUCAGGGUCAGCUUGCCG-3' | 83<br>87 |
| firefly luciferase | shRNA | 5'-CTGACGCGGAATACTTCGA-3' | 84 |
| luxA (Lux operon) | siRNA | sense: 5'-CAAACAGAGGUAAUGAAAUGGUUG-3'<br>antisense: 3'-CAACCAUUUCAUUACCUCUGUUUG-5' | 85<br>88 |
| luxB (Lux operon) | siRNA | sense: 5'-AUGUUAAGUUGAAUAAGUUCUGCA-3'<br>antisense: 3'-UGCUCUUGAAUAAGUUGAAUUGAU-5' | 86<br>89 |
| [1]Lu et al., (2004). RNA silencing in plants by the expression of siRNA duplexes. Nucleic Acids Research, 32(21):e171). | | | |

A GUS coding sequence is set forth in SEQ ID NO:81, and the encoded protein in SEQ ID NO:82 (see, Lu et al., (2004) Nucleic Acids Research 32(21):e171); also in SEQ ID NOs: 59 and 60.

Other reporters known to those of skill in the art can be delivered with exo-loaded MEVs.

Other heterologous cargo includes, for example, anti-angiogenic agents. Anti-angiogenic agents can be a protein, such as an antibody, Fc fusion, and cytokine, that binds to a growth factor or growth factor receptor involved in promoting angiogenesis. Examples of anti-angiogenic agents include but are not limited to antibodies that bind to Vascular Endothelial Growth Factor (VEGF) or that bind to VEGF-R, RNA-based therapeutics that reduce levels of VEGF or VEGF-R expression, VEGF-toxin fusions, Regeneron's VEGF-trap, angiostatin (plasminogen fragment), antithrombin III, angiozyme, ABT-627, Bay 12-9566, BeneFin, bevacizumab, bisphosphonates, BMS-275291, cartilage-derived inhibitor (CDI), CAI, CD59 complement fragment, CEP-7055, Col 3, Combretastatin A-4, endostatin (collagen XVIII fragment), farnesyl transferase inhibitors, fibronectin fragment, GRO-beta, halofuginone, heparinases, heparin hexasaccharide fragment, HMV833, human chorionic gonadotropin (hCG), IM-862, interferon alpha, interferon beta, interferon gamma, interferon inducible protein 10 (IP-10), interleukin-12, kringle 5 (plasminogen fragment), marimastat, metalloproteinase inhibitors (e.g., TIMPs), 2-methoxyestradiol, MMI 270 (CGS 27023A), plasminogen activator inhibitor (PAI), platelet factor-4 (PF4), prinomastat, prolactin 16 kDa fragment, proliferin-related protein (PRP), PTK 787/ZK 222594, retinoids, solimastat, squalamine, SS3304, SU5416, SU6668, SU11248, tetrahydrocortisol-S, tetrathiomolybdate, thalidomide, thrombospondin-1 (TSP-1), TNP470, transforming growth factor beta (TGF-β), vasculostatin, vasostatin (calreticulin fragment), ZS6126, and ZD6474.

Heterologous cargo includes immunomodulatory agents that increase or decrease production of one or more cytokines, up- or down-regulate self-antigen presentation, mask MHC antigens, or promote the proliferation, differentiation, migration, or activation state of one or more types of immune cells. Examples of immunomodulatory agents include

but are not limited to cytokines such as TGFβ, IFNα, IFNβ, IFNγ, IL-2, IL-4, IL-10; cytokine, chemokine, or receptor antagonists including antibodies, soluble receptors, and receptor-Fc fusions, such as those against BAFF, B7, CCR2, CCR5, CD2, CD3, CD4, CD6, CD7, CD8, CD11, CD14, CD15, CD17, CD18, CD20, CD23, CD28, CD40, CD40L, CD44, CD45, CD52, CD64, CD80, CD86, CD147, CD152, complement factors (C5, D), CTLA4, eotaxin, Fas, ICAM, IFNα, IFNβ, IFNγ, IFNAR, IgE, IL-1, IL-2, IL-2R, IL-4, IL-5R, IL-6, IL-8, IL-9 IL-12, IL-13, IL-13R1, IL-15, IL-18R, IL-23, integrins, LFA-1, LFA-3, MHC, selectins, TGFβ, TNFα, TNFβ, TNF-R1, T-cell receptor, including those sold under the trademarks Enbrel® (etanercept), Humira® (adalimumab), and Remicade® (infliximab); heterologous anti-lymphocyte globulin; other immunomodulatory molecules such as anti-idiotypic antibodies for MHC binding peptides and MHC fragments.

Other heterologous cargo includes cytokines which include, but are not limited to lymphokines, monokines, and polypeptide hormones. Included among the cytokines are growth hormones such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-alpha and -beta; Müllerian-inhibiting substance; mouse gonadotropin-associated peptide; inhibin; activin; vascular endothelial growth factor; integrin; thrombopoietin (TPO); nerve growth factors such as NGF-beta; platelet-growth factor; transforming growth factors (TGFs) such as TGF-alpha and TGF-beta; insulin-like growth factor-I and-II; erythropoietin (EPO); osteoinductive factors; interferons such as interferon-alpha, beta, and-gamma; colony stimulating factors (CSFs) such as macrophage-CSF (M-CSF); granulocyte-macrophage-CSF (GM-CSF); and granulocyte-CSF (G-CSF); interleukins (ILs) such as IL-1, IL-1alpha, IL-2, IL-3, IL-4, IL-5, IL-6, IL-7, IL-8, IL-9, IL-10, IL-11, IL-12; IL-15, a tumor necrosis factor such as TNF-alpha or TNF-beta; and other polypeptide factors including LIF and kit ligand (KL).

Other exemplary heterologous cargo includes cytokines and other agents that stimulate cells of the immune system and enhance desired effector function. For example, agents that stimulate NK cells include IL-2; agents that stimulate macrophages include but are not limited to C5a, formyl peptides such as N-formyl-methionyl-leucyl-phenylalanine. Heterologous cargo includes agents that stimulate neutrophils, such as, for example, G-CSF and GM-CSF. Additional agents include, but are not limited to, interferon gamma, IL-3 and IL-7.

The above cargo and others throughout the description are non-limiting examples of cargo that can be exo-loaded into MEVs for delivery. Exemplary of indications and routes of administration are as set forth in the table below. The skilled person, in light of the disclosure herein, can select other indications and targets and identify suitable routes of administration and formulations therefor.

| Target organ/tissue | Exemplary indication | Exemplary route of administration |
|---|---|---|
| Lung | Cystic Fibrosis | Inhalation |
| Lung | Cystic Fibrosis | Inhalation |
| Lung | Idiopathic Pulmonary Fibrosis | Inhalation |
| Lung | Primary Ciliary Dyskinesia | Inhalation |
| Lung | Pulmonary Arterial Hypertension | Inhalation |
| Liver | Inborn error of metabolism | Intravenous or direct injection into the liver |
| Lung | Covid-19 (preventive or therapeutic) | Intranasal or Inhalation |
| Lymphatic | Covid-19 (vaccine) | Intravenous or intramuscular |
| Lung | Influenza (preventive or therapeutic) | Intranasal or Inhalation |
| Lymphatic | Influenza (vaccine) | Intravenous or intramuscular |
| Lymphatic | Viral pathogen | Intravenous or intramuscular |
| Lymphatic | Bacterial pathogen | Intravenous or intramuscular |

## E. PHARMACEUTICAL COMPOSITIONS, FORMULATIONS, KITS, ARTICLES OF MANUFACTURE AND COMBINATIONS

### 1. Pharmaceutical Compositions and Formulations

The compositions containing the MEVs and loaded MEVs provided herein can be formulated as pharmaceutical compositions provided for administration by a desired route, such as oral, muscosal, intravenous, and others. Pharmaceutically acceptable compositions are prepared in view of approvals for a regulatory agency or other agency prepared in

accordance with generally recognized pharmacopeia for use in animals and in humans, and also, for agricultural applications, for plants. Typically, compounds are formulated into pharmaceutical compositions using techniques and procedures well-known in the art (see *e.g.,* Ansel Introduction to Pharmaceutical Dosage Forms, Fourth Edition, 1985, 126).

The pharmaceutical composition can be used for therapeutic, prophylactic, cosmetic, and/or diagnostic applications. The MEVs and cargo-loaded MEVs provided herein can be formulated with a pharmaceutical acceptable carrier or diluent. Generally, such pharmaceutical compositions include components that do not significantly impair the biological properties or other properties of the cargo. Each component is pharmaceutically and physiologically acceptable so that it is compatible with the other ingredients and not injurious to the subject to whom it is to be administered. The formulations can be provided in unit dosage form and can be prepared by methods well-known in the art of pharmacy, including but not limited to, tablets, pills, powders, liquid solutions or suspensions (*e.g*., including injectable, ingestible and topical formulations (*e.g*., eye drops, gels, pastes, creams, or ointments)), aerosols (*e.g*., nasal sprays, and inhalers), liposomes, suppositories, pessaries, injectable and infusible solution and sustained release forms. See, *e.g.,* Gilman, et al. (eds. 1990) Goodman and Gilman's: The Pharmacological Bases of Therapeutics, 8th Ed., Pergamon Press; and Remington's Pharmaceutical Sciences, 17th ed. (1990), Mack Publishing Co., Easton, Pa.; Avis, et al. (eds. 1993) Pharmaceutical Dosage Forms: Parenteral Medications Dekker, NY; Lieberman, et al. (eds. 1990) Pharmaceutical Dosage Forms: Tablets Dekker, NY; and Lieberman, et al. (eds. 1990) Pharmaceutical Dosage Forms: Disperse Systems Dekker, NY. When administered systemically, the therapeutic composition is sterile, pyrogen-free, generally free of particulate matter, and in a parenterally acceptable solution having due regard for pH, isotonicity, and stability. These conditions are known to those skilled in the art. Methods for preparing parenterally administrable compositions are well-known or will be apparent to those skilled in the art and are described in more detail in, *e.g., "*Remington: The Science and Practice of Pharmacy (Formerly Remington's Pharmaceutical Sciences)", 19th ed., Mack Publishing Company, Easton, Pa. (1995).

Pharmaceutical compositions provided herein can be in various forms, *e.g*., in solid, semi-solid, liquid, powder, aqueous, and lyophilized form. Examples of suitable pharmaceutical carriers are known in the art and include but are not limited to water, buffering agents, saline solutions, phosphate buffered saline solutions, various types of wetting agents, sterile solutions, alcohols, gum arabic, vegetable oils, benzyl alcohols, gelatin, glycerin, carbohydrates such as lactose, sucrose, amylose or starch, magnesium stearate, talc, silicic acid, viscous paraffin, perfume oil, fatty acid monoglycerides and diglycerides, pentaerythritol fatty acid esters, hydroxy methylcellulose, and powders, among others. Pharmaceutical compositions provided herein can contain other additives including, for example, antioxidants, preservatives, antimicrobial agents, analgesic agents, binders, disintegrants, coloring, diluents, excipients, extenders, glidants, solubilizers, stabilizers, tonicity agents, vehicles, viscosity agents, flavoring agents, emulsions, such as oil/water emulsions, emulsifying and suspending agents, such as acacia, agar, alginic acid, sodium alginate, bentonite, carbomer, carrageenan, carboxymethylcellulose, cellulose, cholesterol, gelatin, hydroxyethyl cellulose, hydroxypropyl cellulose, hydroxypropyl methylcellulose, methylcellulose, octoxynol-9, oleyl alcohol, povidone, propylene glycol monostearate, sodium lauryl sulfate, sorbitan esters, stearyl alcohol, tragacanth, xanthan gum, and derivatives thereof, solvents, and miscellaneous ingredients such as crystalline cellulose, microcrystalline cellulose, citric acid, dextrin, dextrose, liquid glucose, lactic acid, lactose, magnesium chloride, potassium metaphosphate, and starch, among others (see, generally, Alfonso R. Gennaro (2000) Remington: The Science and Practice of Pharmacy, 20th Edition. Baltimore, MD: Lippincott Williams & Wilkins). Such carriers and/or additives can be formulated by conventional methods and can be administered to the subject at a suitable dose. Stabilizing agents such as lipids, nuclease inhibitors, polymers, and chelating agents can preserve the compositions from degradation within the body.

The route of administration is in accord with known methods, *e.g*., injection or infusion by intravenous, intraperitoneal, intracerebral, intramuscular, subcutaneous, intraocular, intraarterial, intrathecal, inhalation or intralesional routes, topical, rectal, mucosal, and by sustained release systems. The MEVs or cargo-loaded MEVs can be administered continuously by infusion or by bolus injection. One can administer the MEVs or cargo-loaded MEVs in a local or systemic manner.

The MEVs or cargo-loaded MEVs can be prepared in a mixture with a pharmaceutically acceptable carrier. Techniques for formulation and administration of the compounds are known to one of skill in the art (see *e.g., "*Remington's Pharmaceutical Sciences," Mack Publishing Co., Easton, Pa.). This therapeutic composition can be administered intravenously or through the nose or lung, such as a liquid or powder aerosol (lyophilized). The composition also can be administered parenterally or subcutaneously as desired. When administered systematically, the therapeutic composition should be sterile, pyrogen-free and in a parenterally acceptable solution having due regard for pH, isotonicity, and stability. These conditions are known to those skilled in the art.

Pharmaceutical compositions suitable for use include compositions wherein the MEVs or cargo-loaded MEVs are contained in an amount effective to achieve their intended purpose. Determination of a therapeutically effective amount is well within the capability of those skilled in the art. Therapeutically effective dosages can be determined by using *in vitro* and *in vivo* methods, and/or by a skilled person.

Therapeutic formulations can be administered in many conventional dosage formulations. Dosage formulations

of MEVs and cargo-loaded MEVs provided herein are prepared for storage or administration by mixing the compound having the desired degree of purity with physiologically acceptable carriers, excipients, or stabilizers. Such materials are non-toxic to the recipients at the dosages and concentrations employed, and can include buffers such as Tris HCl, phosphate, citrate, acetate and other organic acid salts; antioxidants such as ascorbic acid; low molecular weight (less than about ten residues) peptides such as polyarginine, proteins, such as serum albumin, gelatin, or immunoglobulins; hydrophilic polymers such as polyvinylpyrrolidinone; amino acids such as glycine, glutamic acid, aspartic acid, or arginine; monosaccharides, disaccharides, and other carbohydrates including cellulose or its derivatives, glucose, mannose, or dextrins; chelating agents such as EDTA; sugar alcohols such as mannitol or sorbitol; counterions such as sodium and/or nonionic surfactants such as TWEEN (polysorbates), Pluronic, polyethylene glycol, and others.

In particular examples herein, provided are pharmaceutical compositions that contain a stabilizing agent. The stabilizing agent can be an amino acid, amino acid derivative, amine, sugar, polyol, salt or surfactant. In some examples, the stable co-formulations contain a single stabilizing agent. In other examples, the stable co-formulations contain 2, 3, 4, 5 or 6 different stabilizing agents. For example, the stabilizing agent can be a sugar or polyol, such as a glycerol, sorbitol, mannitol, inositol, sucrose or trehalose. In particular examples, the stabilizing agent is sucrose. In other examples, the stabilizing agent is trehalose. The concentration of the sugar or polyol is from or from about 100 mM to 500 mM, 100 mM to 400 mM, 100 mM to 300 mM, 100 mM to 200 mM, 200 mM to 500 mM, 200 mM to 400 mM, 200 mM to 300 mM, 250 mM to 500 mM, 250 mM to 400 mM, 250 mM to 300 mM, 300 mM to 500 mM, 300 mM to 400 mM, or 400 mM to 500 mM, each inclusive.

In examples, the stabilizing agent can be a surfactant that is a polypropylene glycol, polyethylene glycol, glycerin, sorbitol, poloxamer and polysorbate. For example, the surfactant can be a polypropylene glycol, polyethylene glycol, glycerin, sorbitol, poloxamer and polysorbate, such as a poloxamer 188, polysorbate 20 and polysorbate 80. In particular examples, the stabilizing agent is polysorbate 80. The concentration of surfactant, as a % of mass concentration (w/v) in the formulation, is between or about between 0.005% to 1.0%, 0.01% to 0.5%, 0.01% to 0.1%, 0.01% to 0.05%, or 0.01% to 0.02%, each inclusive.

When used for *in vivo* administration, the formulation should be sterile and can be formulated according to conventional pharmaceutical practice. This is readily accomplished by filtration through sterile filtration membranes, prior to or following lyophilization and reconstitution. The MEVs or cargo-loaded MEVs can be stored in lyophilized form or in solution; they can be frozen or refrigerated. Other vehicles such as naturally occurring vegetable oil like sesame, peanut, or cottonseed oil or a synthetic fatty vehicle like ethyl oleate can be included. Buffers, preservatives, and antioxidants can be incorporated according to accepted pharmaceutical practice.

The MEVs or cargo-loaded MEVs provided herein, can be provided at a concentration in the composition of from or from about 0.1 to 10 mg/mL or higher or lower amounts, depending upon the application and the subject, such as, for example a concentration that is at least or at least about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, 10 mg/mL or more. The volume of the solution can be at or about 1 to 100 mL, such as, for example, at least or about at least or 0.5, 1, 2, 3, 4, 5, 10, 15, 20, 25, 30, 35, 40, 45, 50, 55, 60, 65, 70, 75, 80, 85, 90, 95, 100 mL or more. In some examples, the MEVs or cargo-loaded MEVs are supplied in phosphate buffered saline.

The MEVs or cargo-loaded MEVs provided herein can be provided as a controlled release or sustained release composition. Polymeric materials are known in the art for the formulation of pills and capsules which can achieve controlled or sustained release of the MEVs and cargo-loaded MEVs provided herein (see, *e.g.,* Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Langer and Peppas (1983) J. Macromol. Sci. 23:61; see also Levy et al. (1985) Science 228:190; During et al. (1989) Ann. Neurol. 25:351; Howard et al. (1989) J. Neurosurg. 71:105; U.S. Pat. Nos. 5,679,377, 5,916,597, 5,912,015, 5,989,463, 5,128,326; and PCT Publication Nos. WO 99/15154 and WO 99/20253). Examples of polymers used in sustained release formulations include, but are not limited to, poly(2-hydroxy ethyl methacrylate), poly(methyl methacrylate), poly(acrylic acid), poly(ethylene-co-vinyl acetate), poly(methacrylic acid), polyglycolides (PLG), polyanhydrides, poly(N-vinyl pyrrolidone), poly(vinyl alcohol), polyacrylamide, poly(ethylene glycol), polylactides (PLA), poly(lactide-co-glycolides) (PLGA), and polyorthoesters. Generally, the polymer used in a sustained release formulation is inert, free of leachable impurities, stable on storage, sterile, and biodegradable. Any technique known in the art for the production of sustained release formulation can be used to produce a sustained release formulation containing the MEVs or cargo-loaded MEVs provided herein.

In some examples, the pharmaceutical composition contains the MEVs or cargo-loaded MEVs provided herein and one or more additional agents, such as an antibody or other therapeutic, for combination therapy.

### 2. Articles of Manufacture/Kits and Combinations

Pharmaceutical compositions of the MEVs or cargo-loaded MEVs can be packaged as articles of manufacture containing packaging material, a pharmaceutical composition which is effective for treating a disease or condition that can

be treated by administration of the particular MEVs or cargo-loaded MEVs, such as the diseases and conditions described herein or known in the art, and a label that indicates that the cargo, such as an antibody or nucleic acid molecule, is to be used for treating the infection, disease or disorder. The pharmaceutical compositions can be packaged in unit dosage forms containing an amount of the pharmaceutical composition for a single dose or multiple doses. The packaged compositions can contain a lyophilized powder of the pharmaceutical compositions containing the cargo-loaded MEVs which can be reconstituted (*e.g.*, with water or saline) prior to administration.

The articles of manufacture described herein contain packaging materials. Packaging materials for use in packaging pharmaceutical products are well-known to those of skill in the art (see, *e.g.,* U.S. Patent Nos. 5,323,907, 5,052,558 and 5,033,252). Examples of pharmaceutical packaging materials include, but are not limited to, blister packs, bottles, tubes, inhalers (*e.g.*, pressurized metered dose inhalers (MDI), dry powder inhalers (DPI), nebulizers (*e.g.*, jet or ultrasonic nebulizers) and other single breath liquid systems), pumps, bags, vials, containers, syringes, bottles, and any packaging material suitable for a selected formulation and intended mode of administration and treatment.

The MEVs or cargo-loaded MEVs can be provided as combinations and as kits. Kits optionally can include one or more components such as instructions for use, devices and additional reagents (*e.g.*, sterilized water or saline solutions for dilution of the compositions and/or reconstitution of lyophilized protein), and components, such as tubes, containers and syringes for practice of the methods. Exemplary kits can include the MEVs or cargo-loaded MEVs provided herein, and can optionally include instructions for use, a device for administering the MEVs or cargo-loaded MEVs to a subject, a device for detecting MEVs or cargo-loaded MEVs in samples obtained from a subject, and a device for administering an additional therapeutic agent to a subject.

The kit can, optionally, include instructions. Instructions typically include a tangible expression describing the MEVs or cargo-loaded MEVs, and, optionally, other components included in the kit, and methods for administration, including methods for determining the proper state of the subject, the proper dosage amount, dosing regimens, and the proper administration method for administering the MEVs or cargo-loaded MEVs. Instructions also can include guidance for monitoring the subject over the duration of the treatment time.

Kits also can include a pharmaceutical composition described herein and an item for diagnosis. For example, such kits can include an item for measuring the concentration, amount or activity of the MEVs and cargo-loaded MEVs, in a subject.

In some examples, the MEVs or cargo-loaded MEVs are provided in a diagnostic kit for the detection of the MEVs or cargo-loaded MEVs or cargo in an isolated biological sample (*e.g.*, tumor cells, such as circulating tumor cells obtained from a subject or tumor cells excised from a subject).

Kits described herein also can include a device for administering the MEVs to a subject. Any of a variety of devices known in the art for administering medications to a subject can be included in the kits provided herein. Exemplary devices include, but are not limited to, a hypodermic needle, an intravenous needle, a catheter, a nebulizer, and an inhaler. Typically the device for administering the compositions is compatible with the desired method of administration of the composition.

### 3. Combination Therapies

The cargo-loaded MEVs provided herein can be administered before, after, or concomitantly with one or more other therapeutic regimens or agents. The skilled medical practitioner can determine empirically, or by considering the pharmacokinetics and modes of action of the agents, the appropriate dose or doses of each therapeutic regimen or agent, as well as the appropriate timings and methods of administration. The additional therapeutic regimens or agents can improve the efficacy or safety or other properties of the cargo-loaded MEVs. In some examples, the additional therapeutic regimens or agents can treat the same disease or a comorbidity. In some examples, the additional therapeutic regimens or agents can ameliorate, reduce or eliminate one or more side effects known in the art or described herein that are associated with administration of the cargo-loaded MEVs or the cargo.

For example, the cargo-loaded MEVs described herein can be administered with chemotherapy, radiation therapy, or chemotherapy and radiation therapy, or for anti-viral or anti-bacterial or other pathogen therapy, the cargo-loaded MEVs can be administered with other anti-pathogen therapeutics and treatments. The cargo-loaded MEVs can be administered in combination with one or more other prophylactic or therapeutic agents, including but not limited to antibodies, cytotoxic agents, chemotherapeutic agents, cytokines, growth inhibitory agents, anti-hormonal agents, kinase inhibitors, anti-angiogenic agents, cardio-protectants, immunostimulatory agents, immunosuppressive agents, agents that promote proliferation of hematological cells, angiogenesis inhibitors, protein tyrosine kinase (PTK) inhibitors, FcγRIIb or other Fc receptor inhibitors, or other therapeutic agents.

The one or more additional agents can be administered simultaneously, sequentially or intermittently with the cargo-loaded MEVs. The agents can be co-administered, for example, as part of the same pharmaceutical composition or same method of delivery. In some examples, the agents can be co-administered at the same time as the cargo-loaded MEVs, but by a different means of delivery. The agents also can be administered at a different time than administration of

the cargo-loaded MEVs, but close enough in time to have a combined prophylactic or therapeutic effect. In some examples, the one or more additional agents are administered subsequent to or prior to the administration of the cargo-loaded MEVs separated by a selected time period. In some examples, the time period is 1 day, 2 days, 3 days, 4 days, 5 days, 6 days, 1 week, 2 weeks, 3 weeks, 1 month, 2 months, or 3 months. In some examples, the one or more additional agents are administered multiple times and/or the cargo-loaded MEVs provided herein are administered multiple times.

## F. ADMINISTRATION OF CARGO-LOADED MEVS AND ROUTES OF ADMINISTRATION

### 1. Routes of administration

The cargo-loaded MEVs provided herein can be administered to a subject by any method or route known in the art for the administration of pharmaceuticals, including biologics. The cargo-loaded MEVs can be administered by various routes. Routes of administration, include, but are not limited to, systemic, topical, and local administration. Routes of administration, as discussed, include, oral administration, enteral administration, parenteral, which includes intradermal, intramuscular, intraperitoneal, intravenous, subcutaneous, and intracavity administration, topical, epidural, mucosal, which includes topical, intranasal, vaginal, vulvovaginal, esophageal, oroesophageal, bronchial, rectal, and pulmonary.

The cargo-loaded MEVs can be administered externally to a subject, at the site of the disease for local or transdermal action. Compositions containing the cargo-loaded MEVs can be administered, as discussed above, by any route depending upon the target tissue or organ for the disease, disorder, or condition, treated. For targeting the spleen, for example, oral administration is employed. The MEVs for such administration are formulated as a liquid or solid. As shown in the Examples, following oral administration, the MEVs pass through the stomach and into the intestines, and, as shown, then are found in the spleen. This is a distinction and advantage of the MEVs compared to mammalian MEVs, which cannot be administered orally because they cannot survive the harsh conditions in the stomach. Administration can be by infusion, inhalation, by bolus injection, by absorption through epithelial or mucocutaneous linings (*e.g.*, topical, oral, vaginal, rectal and intestinal mucosa).

Compositions containing the cargo-loaded MEVs can be administered together with or sequentially with other biologically active agents. For example, the cargo-loaded MEVs are administered by infusion delivery, such as by infusion pump or syringe pump, and can be administered in combination with another therapeutic agent or as a monotherapy.

The method and/or route of administration can be altered to alleviate adverse side effects associated with administration provided herein. For example, if a patient experiences a mild or moderate (*i.e.,* Grade 1 or 2) infusion reaction, the infusion rate can be reduced (*e.g.*, reduced by 10%, 20%, 30%, 40%, 50%, 60%, 70%, 80%, 90% or more). If the patient experiences severe (*i.e.,* Grade 3 or 4) infusion reactions, the infusion can be temporarily or permanently discontinued.

In some examples, if the subject experiences an adverse side effect, such as severe skin toxicity, for example severe acneiform rash, treatment adjustments can be made. For example, after the occurrence of an adverse side effect, administration can be delayed, such as for 1 to 2 weeks or until the adverse side effect improves. In some examples, after additional occurrences of an adverse side effect, the dosage can be reduced. A particular regimen and treatment protocol can be established by the skilled physician or other skilled practitioner in the art.

Appropriate methods for delivery, can be selected by one of skill in the art based on the properties of the dosage amount of the cargo-loaded MEVs or the pharmaceutical composition containing the cargo-loaded MEVs. Such properties include, but are not limited to, solubility, hygroscopicity, crystallization properties, melting point, density, viscosity, flow, stability, and degradation profile. As detailed in the Examples, the route of administration of the MEVs determines their biodistribution. As shown, following administration, the MEVs distribute in tissues and organs that differ from mammalian and other EVs.

### 2. Compositions Containing MEVs as Drug Delivery Systems

As discussed above, and demonstrated in the Examples, the MEVs and compositions containing the MEVs can be considered drug delivery systems in which the MEVs are formulated for a particular route of administration for targeting an organ or tissue involved in a disease, disorder, or condition, that can be treated by the selected cargo. The compositions optionally can include additional components, such as another therapeutic agent for combination therapy and/or devices for administration.

A composition or drug delivery system, for example, contains extracellular vesicles (MEVs) formulated for oral delivery, intravenous delivery, intramuscular delivery, intranasal delivery, subcutaneous delivery, topical delivery, mucosal delivery, intraperitoneal delivery, intratumoral delivery, or inhalation delivery. The MEVs are isolated from the cell culture, and exogenously loaded with cargo. The MEVs are formulated for a route of delivery, whereby the cargo is delivered to a target organ or tissue. Target organs or tissues include lungs, liver, spleen, intestine, brain, spinal cord, peripheral nerves, lymphoid tissues, eyes, mucosal tissue, skin, hematopoietic tissues, pancreas, muscle, bones, heart, endocrine tissues

and kidneys. The target organ or tissue is mucosal tissue can be, for example, naso-buccal, ocular, urogenital, vaginal, or rectal. The compositions or drug delivery system containing the cargo-loaded MEVs can be formulated as a suspension or emulsion, which can be a nanoemulsion or is a microemulsion. The drug delivery system can be formulated as a tablet, capsules, gel capsule, powder, troche, granules, liquid for oral administration, oil, or is a suspension or emulsion for nasal administration or oral administration, or inhalation, or nebulization, or intratracheal administration. As above, exemplary of MEVs are *Chlorella* extracellular vesicles into which bioactive molecule cargo is loaded. *Chlorella* species include, but are not limited to, *Chlorella ellipsoidea, Chlorella pyrenoidosa, Chlorella sorokiniana, Chlorella vulgaris,* and *Chlorella variabilis.*

The exogenously-loaded cargo can be a therapeutic for treating or preventing a disease, disorder, or condition, or treating or preventing a symptom thereof, such as, for example, the cargo comprises or encodes a protein, or is a small molecule therapeutic that is a prophylactic for preventing or reducing the risk of getting a disease, disorder, or condition, or reducing the severity of a disease, disorder, or condition. The cargo can comprise nucleic acid, a protein, a small peptide, a peptide, a polypeptide, a small molecule drug, or any other molecule of interest, including detectable reagents for diagnostics. The cargo includes any described herein and any molecule(s), including complexes of interest for exo-loading into the MEVs, and/or known to those of skill in the art. For example, the exogenous cargo for the drug delivery systems, as well as the compositions and MEVs described in the sections above and below, can, for example, comprise an immunomodulatory agent to increase or decrease production of one or more cytokines; up- or down-regulate self-antigen presentation; mask MHC antigens; or promote the proliferation, differentiation, migration, or activation state of one or more types of immune cells. The exogenous cargo can comprise a hormone or a cytokine or a chemokine; or comprises nucleic acid encoding a hormone, or a cytokine, or a chemokine. Exemplary thereof is one or more of a hormone or cytokine or growth factor selected from among human growth hormone; N-methionyl human growth hormone; bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factors; fibroblast growth factors; prolactin; placental lactogen; tumor necrosis factor-alpha and -beta; Müllerian-inhibiting substance; gonadotropin-associated peptide; inhibin; activin; vascular endothelial growth factors; integrin; thrombopoietin (TPO); nerve growth factors, transforming growth factors (TGFs); insulin-like growth factor-I and-II; erythropoietin (EPO); osteoinductive factors; interferons such as interferon-alpha, -beta, and -gamma; colony stimulating factors (CSFs) such as macrophage-CSF (M-CSF); granulocyte-macrophage-CSF (GM-CSF); and granulocyte-CSF (G-CSF); or an interleukin (IL). The cargo can comprise for mRNA-mediated gene therapy, gene silencing, gene substitution, gene overexpression, and/or gene editing, can comprise one or more of peptides or proteins for gene regulation, gene substitution, gene overexpression, gene editing, regulation of cell metabolism, cell functions; and protein therapy. The mRNA-mediated gene therapy or protein therapy can be treating an inborn error of metabolism. The cargo can comprise a vaccine, such as a protein vaccine or mRNA vaccine. Vaccines, as described herein can be immunoprotective and/or prophylactic, and/or can treat a disease, disorder, or condition.

The MEV cargo can comprise a nucleic acid or protein or a nucleic acid encoding a protein that is a therapeutic product for treatment of cancer, or an infectious disease, or metabolic diseases, or a neurodegenerative disease or other CNS disorder, or aging, or an aging associated disease, or genetic diseases, or ophthalmic disorders, or immunological disorders, or involving internal organs urogenital organs, the cardiovascular system and associated organs and tissues, hematopoietic or lymphoid tissues, sensory organs and tissues, urogenital organs and tissues, muscle tissues, bones, and/or endocrine tissues. Internal organs include, for example, liver, or the pancreas, or spleen, or brain. The compositions and drug delivery systems that contain the MEVs can be formulated for oral administration, parenteral administration, topical administration, local administration, intratumoral administration, systemic administration, mucosal administration, intravenous administration, subcutaneous administration, intramuscular administration, intraperitoneal administration, transdermal administration, intranasal administration, inhalation, intratracheal administration. They can be formulated for oral delivery, or as an aerosol for intranasal, inhalation, or nebulization. The compositions provided herein can be used for delivering cargo in an MEV to an organ or tissue, wherein the route of administration is selected to target the organ or tissue of interest.

## G. BIODISTRIBUTION OF MEVs FOLLOWING ADMINISTRATION VIA VARIOUS ROUTES

The cargo-loaded MEVs provided herein can be administered to a subject by any method known in the art for the administration of polypeptides, including for example systemic and local administration. The cargo-loaded MEVs can be administered by routes, such as parenteral (*e.g.*, routes, such as intradermal, intramuscular, intraperitoneal, intravenous, subcutaneous, and intracavity), topical, epidural, or mucosal (*e.g.*, routes, such as topical, intranasal, oral, vaginally, vulvovaginal, esophageal, oroesophageal, bronchial, rectal, and pulmonary). The cargo-loaded MEVs can be administered externally to a subject, at the site of the disease for exertion of local or transdermal action. Compositions containing the cargo-loaded MEVs can be administered by any convenient route, for example by infusion, inhalation, by bolus injection, or by absorption through epithelial or mucocutaneous linings (*e.g.*, topical, oral, vaginal, rectal and intestinal

mucosa). Compositions containing the cargo-loaded MEVs can be administered together with or sequentially with other biologically active agents. For example, the cargo-loaded MEVs are administered by infusion delivery, such as by infusion pump or syringe pump, and can be administered in combination with another therapeutic agent or as a monotherapy.

The method and/or route of administration can be altered to alleviate adverse side effects associated with administration provided herein. For example, if a patient experiences a mild or moderate (*i.e.,* Grade 1 or 2) infusion reaction, the infusion rate can be reduced (*e.g.*, reduced by 10%, 20%, 30%, 40%, 50%, 60%, 70%, 80%, 90% or more). If the patient experiences severe (*i.e.,* Grade 3 or 4) infusion reactions, the infusion can be temporarily or permanently discontinued.

In some examples, if the subject experiences an adverse side effect, such as skin toxicity, for example severe acneiform rash, treatment adjustments can be made. For example, after the occurrence of an adverse side effect, administration can be delayed, such as for 1 to 2 weeks or until the adverse side effect improves. In some examples, after additional occurrences of an adverse side effect, the dosage can be reduced. A particular regimen and treatment protocol can be established by the skilled physician or other practitioner.

Appropriate methods for delivery, can be selected by one of skill in the art based on the properties of the dosage amount of the cargo-loaded MEVs or the pharmaceutical composition containing the cargo-loaded MEVs. Such properties include, but are not limited to, solubility, hygroscopicity, crystallization properties, melting point, density, viscosity, flow, stability and degradation profile.

### 1. Biodistribution of mammalian EVs

The biodistribution and pharmacokinetics of EVs of mammalian origin have been extensively studied. Treatments with mammalian cell-derived EVs generally are based on intravenous or intraperitoneal routes of administration. Primary target organs upon systemic administration of mammalian EVs are the liver, spleen and lungs. A comprehensive study (see, Wiklander et al. (2015) J. Extracellular Vesicles 4:26316) of the tissue distribution of fluorescently-labelled mammalian EVs from various cell sources demonstrated that 24 hours after intravenous (i.v.) injection in mice, the highest fluorescence signal was in the liver, followed by spleen, gastrointestinal tract and lungs. Furthermore, cell source, EV dose, and route of administration was shown to affect EV distribution; for example, injection of higher EV doses resulted in relatively lower liver accumulation compared to lower doses, possibly caused by saturation of the mononuclear phagocyte system (MPS). Comparison between intraperitoneal (i.p.), subcutaneous (s.c.) and i.v. administrations showed that intraperitoneal and subcutaneous doses resulted in reduced EV accumulation in liver and spleen and enhanced pancreas and gastrointestinal tract accumulation compared to i.v. injections. Systemically administered EVs are reported to be taken up by the mononuclear phagocyte system (MPS), particularly in the liver and spleen. The mechanism of clearance resembles that described for synthetic nanoparticles, such as liposomes (Van der Meel et al. (2014) J. Control. Release 195:72-85). The majority of splenic accumulation is caused by EV storage in the spleen rather than uptake by the spleen (Lai C.P. et al. (2014) ACS Nano 8:483-494). Biodistribution of mammalian EVs following other routes of administration also has been investigated. For targeting of the central nervous system, intranasal administration of curcumin-loaded mammalian EVs resulted in EV localization in the brain. Drug levels peaked at 1 hour after administration, and a significant amount detected after 12 hours with no toxic effects observed (Zhuang et al. (2011) Mol. Ther. 19:1769-1779).

In general mammalian EVs are not employed for oral delivery because of their low stability at various pH and temperatures, rapid degradation of biomolecules in the digestive tract, and the limitations of industrial scale production for oral dosing (Cheng et al. (2019) Protein Cell 10:295-299). The only exception so far are bovine milk-derived EVs, which upon oral delivery to mice have shown a pattern of distribution that, analyzed with whole-body in vivo imaging system (IVIS), included rapid accumulation in the intestine, where the EVs were detectable after 2 and 6 hours, followed by fluorescence signal observed in liver, spleen, lungs, kidney, heart, and the gastrointestinal tract at 24 hours. After 48 hours, the fluorescence signal subsided within most of the organs indicating the clearance of nanovesicles from the system (Samuel et al. (2021) Nat Commun 12:3950, doi.org/10.1038/s41467-021-24273-8). Thus, mammalian EVs (derived from sources other than milk) cannot be absorbed by the intestinal tract and from the intestines become bioavailable in target organs (Zhong et al. (2021) Biomaterials. 277:121126. doi: 10.1016/j.biomaterials.2021.121126).

As shown herein in the Examples, and discussed below, MEVs are stable in the harsh environment of the gastrointestinal tract compared to mammalian cell-derived EVs. Thus, the microalgae EVs, as described herein, are particularly suitable for oral administration and drug delivery.

### 2. Microalgae EVs Biodistribution

It is shown herein that MEVs, including those provided herein from *Chlorella,* have properties that are distinct from mammalian EVs, including bovine milk EVs. For example, a striking difference, discussed below, is that the MEVs can be administered orally, and that the primary target is the spleen, likely the white spleen.

The MEVs provided herein can deliver a variety of bioactive molecules, such as RNAs, such as mRNA; proteins;

peptides; and small molecules. These include products such as tissue-specific products and/or disease specific products. As discussed below, each route can be used to target particular organs and treat particular diseases. The MEVs can be formulated for administration by each route. Thus, provided are compositions containing MEVs that are for treating particular disease and for particular routes of administration.

It is shown herein, the route of administration determines the fate of the MEVs, and that the ultimate location of the MEVs is a function of the route of administration. Targets and endpoints of the MEVs include, but are not limited to, the liver, spleen, lungs, the intestines, and brain. Routes of administration include, but are not limited to, respiratory (nose, lungs), oral (digestive), intravenous, central nervous system (CNS), and topical. The selection of route depends upon the ultimate target and the payload. It is shown herein that intranasal administration goes to the lungs, intratracheal via a spray goes to the lung(s), intravenous accumulates in spleen and liver, oral (per os) goes to the digestive tract and spleen. In contrast, mammalian EVs cannot be taken orally.

MEVs are readily internalized by human cells. For example, *in vitro,* when administered to cells in culture, such as A549 cells, at a ratio of MEV/cell of 1000/1, 93% of the cells internalized the MEVS, and this occurred within 24 to 48 hours after contacting the cells with the MEVs.

DIR-labeled MEVs were administered to mice via four routes: intranasal (IN), intratracheal (IT), intravenous (IV), and oral, and, by full-body imaging as a function of time, the fate of the MEVs was visualized for 3 days, followed by sacrificing the mice to harvest organs for study. As shown in the examples, intravenous administration targets the liver at about 4-12 hours following administration, and the spleen, appearing to be in the red spleen, at 10-30 hours. Oral administration targets the intestine and spleen. It is shown herein that the MEVs are orally available; they resist passage through the stomach, and reach the intestine at 0.5 hour to 4 hours, and then the spleen at 0.5 hour to 10 hours. Of interest is the route to the spleen; there are two possible routes to the spleen, via the blood (to red spleen), and via lymphocytes (to white spleen), which has implications for targeting and delivering cargo to the immune system, accumulating from 4 hours to 28 hours. This can be effected by internalization by lymphocytes that are activated and end up in the spleen where they multiply, and/or by lymphocytes that phagocytose the MEVs, which are not activated, and go to the spleen (white spleen) from where they are disseminated through the immune system.

Thus, orally ingested MEVs go into the intestine, then, as shown, end up in the spleen, likely the white spleen. The spleen is responsible for initiating immune reactions to blood-borne antigens, and for filtering foreign material and old or damaged red blood cells from the blood. These functions are performed by two different compartments in the spleen: the white pulp (white spleen), and red pulp (red spleen). The two compartments are vastly different in structure, vascular organization, and cellular composition (see, *e.g.,* Cesta (2006) Toxicologic Pathology 34:455-465 for a review of the structure, function and histology of the spleen).

White blood cells, which are plentiful in the intestine, migrate to the white spleen. When ingested orally the MEVs can be internalized by intestinal cells and, as discussed below, can be internalized by intestinal lymphocytes, which carry the MEVs to the spleen. This is in contrast to mammalian vesicles, which cannot be administered orally. Thus, MEVs provide a delivery vehicle for agents for which the immune system is a target, such as for immune modulating cargo. As discussed above, the pathway to the white spleen can occur, for example, via activated lymphocytes and/or phagocytic lymphocytes. Lymphocytes can phagocytose the MEVs, and are homed to the spleen. The MEVs, unlike mammalian EVs, provide a way to orally deliver small molecule drugs and proteins and other therapeutics, such as nucleic acid therapeutics, that cannot be administered orally. In particular, orally administered MEVs provide a route for treatment of diseases, such as cancers and inflammatory diseases, in which the immune system is involved or in which the treatment can be effected by targeting the immune system. Such diseases include, but are not limited to, infectious disease, autoimmune diseases, cancers, and prevention of organ transplant rejection. These diseases are treated by suppressing or augmenting the activity of immune cells.

### a. Components of the Lymphatic System

The lymphatic system includes lymph, lymphatic vessels and lymphatic organs (see, discussion in Zgair et al., (2016) Targeting Immunomodulatory Agents to the Gut-Associated Lymphoid Tissue. In: Constantinescu C., Arsenescu R., Arsenescu V. (eds) Neuro-Immuno-Gastroenterology. Springer, Cham. (doi.org/10.1007/978-3-319-28609-9_14) and summarized below).

#### Lymph

Lymph is a generally clear and colorless fluid that drains from the interstitium, and contains recovered fluids and plasma proteins, and also can contain lipids, immune cells, hormones, bacteria, viruses, cellular debris, and cancer cells.

**Lymphatic Vessels**

The lymphatic system is the body's second circulatory system. The lymphatic system is a unidirectional, blind-ended and thin-walled system of capillary vessels where lymph is driven. Lymphatic capillaries drain in the afferent collecting vessels, which then pass through one or more gatherings of lymph nodes. Lymph fluid then passes through the efferent collecting vessels, larger trunks and then the lymphatic duct, which drain lymph to the systemic circulation. Primary lymphatic organs include the thymus gland and bone marrow, which produce mature lymphocytes, which identify and respond to antigens; secondary lymphatic organs include lymph nodes, spleen and mucosa-associated lymph tissues (MALTs). Within the secondary lymphatic organs, lymphocytes initiate immune responses. MALTs are distributed throughout mucous membranes and provide a defensive mechanism against a wide variety of inhaled or ingested antigens. MALTs are categorized according to their anatomical location as: bronchus-associated lymphoid tissue (BALT), nasal-associated lymphoid tissue (NALT), salivary gland duct-associated lymphoid tissue (DALT), conjunctiva-associated lymphoid tissue (CALT), lacrimal duct-associated lymphoid tissue (LDALT) and gut-associated lymphoid tissue (GALT).

**Gut-Associated Lymphoid Tissue (GALT)**

GALT is composed of effector and immune induction sites. Effector sites include lymphocytes distributed throughout the lamina propria (LP) and intestinal epithelium; induction sites involve tissues, such as such as mesenteric lymph nodes (MLN), PP and smaller isolated lymphoid follicles (ILF). Mesenteric lymph nodes (MLN), which occur in the base of the mesentary, are the largest gatherings of lymph nodes in the body. The structure of MLN is divided into two regions: the medulla and cortex. The cortex primarily is composed of T-cell areas and B-cell follicles. Within the T-cell area, circulating lymphocytes enter the lymph node, and dendritic cells (DC) present antigens to T-cells. Lymph (containing cells, antigens and chylomicrons) is collected from the intestinal mucosa and reaches the MLN via the afferent lymphatics. Lymph fluid subsequently leaves the MLN through efferent lymphatics to reach the thoracic duct that drains to the blood.

Peyer's patches (PP) are a collection of lymphoid nodules distributed in the mucosa and submucosa of the intestine. They contain a sub-epithelial dome area and B-cell follicles dispersed in a T-cell area. A single layer of epithelial cells, called follicle-associated epithelium (FAE), separates lymphoid areas of PP from the intestinal lumen. FAE is permeated by specialized enterocytes called microfold (M) cells. These cells are a gate for the transport of luminal antigens to PP.

Isolated lymphoid follicles (ILF) are a combination of lymphoid cells in the intestinal LP. ILF are composed of germinal centers covered by FAE containing M-cells. ILF is a complementary system to PP for the induction of intestinal immunity.

GALT is the largest lymphatic organ in the human body and contains more than half of the body's lymphocytes. GALT is exposed to more antigens in the form of commensal bacteria and alimentary antigens, in addition to those from invasive pathogens, than any other part of the body. Intestinal lymphatic transport avoids hepatic first-pass metabolic loss by diverting the absorption of lipophilic drugs towards intestinal lymphatics rather than the portal vein. The intestinal immune system must distinguish antigens that require a protective immune response and develop a state of immune hypo-responsiveness (oral tolerance) for harmless antigens. This is effected by sampling of luminal antigens in the intestinal epithelium by DC. Antigens can cross the epithelium through M-cells, which are specialized epithelial cells of the follicle-associated epithelium of the GI tract. The antigens interact with DC in the underlying sub-epithelial dome region. Antigens are presented to local T-cells in PP by DC.

DC also migrate to the draining MLN where they present antigens to local lymphocytes. Alternative pathways for antigen transport across the intestinal epithelial cells involve receptor-mediated transport, and direct sampling from the lumen by DC projections. Antigen-loaded DC then migrate to the MLN through afferent lymphatics where they present antigens to T-cells. Subsequently, differentiated lymphocytes migrate from MLN through the thoracic duct and blood stream and eventually accumulate in the mucosa for an appropriate immune response.

**b. Targeting GALT**

Orally administered MEVs can target gut-associated lymphoid tissue (GALT). Thus, GALT is a target (effective compartment) and/or a route through which MEVs and their therapeutic agent cargo can be used to deliver cargo to organs, tissues, and/or systemic circulation. GALT is an advantageous target for various pharmacological agents such as, for example, immunomodulators, chemotherapeutic agents, and anti-infective agents. The lymphatic system is a main pathway for intestinal and other tumor metastases; therefore, targeting cytotoxic drugs to the intestinal lymphatics can be used to treat tumor metastases. GALT is a delivery target for antiviral agents, as some viruses, such as, for example, human immunodeficiency virus (HIV), morbillivirus, canine distemper virus, severe acute respiratory syndrome (SARS)-associated coronaviruses, hepatitis B and hepatitis C, spread and develop within the lymphatic system.

Thus, MEVs, including the *Chlorella* MEVs exemplified herein, can be used to target immune cells upon oral

delivery. As described above, the microalgae MEVs show a distinct pattern of biodistribution when administered orally. This pattern includes initial intestine accumulation followed by targeting the spleen, where they are detectable up to 24 hours (see, *e.g.,* Fig. 7).

Since the microalgae MEVs are delivered to the spleen, the mechanism of this delivery can be based on cells of the immune system. Immune cells are abundant in the single-cell layer of intestinal epithelium and underlying lamina propria of the gut-associated lymphoid tissue (GALT). The immune cells include, T cells, plasma cells, mast cells, dendritic cells, and macrophages (Luongo et al. (2009) Current perspectives. International Reviews of Immunology 28(6):446-464, doi.org/10.3109/08830180903236486). Macrophages, dendritic cells, neutrophils, and also B cells perform phagocytosis. The immune cells in the gut, thus, can phagocytose the MEVs to deliver them to the spleen. After phagocytosis, the fate of the MEV cargo can depend upon the type of cargo. For example, macrophage and dendritic cells participate in antigen presentation, and present proteins delivered in the MEVS, or the products in the MEVs can be secreted, or the products, such RNA, can be translated.

Immune cells present in the intestinal epithelium and lamina propria of the intestine migrate to the spleen and back to the intestine. This homing to the spleen can be involved in MEV transfer from the gut to secondary lymphatic organs, especially to the spleen. T cells exhibit a specific lymphocyte recirculation pathway (Mackay et al. (1990) J Exp Med 171:801-17) that can be part of MEV trafficking to the spleen upon oral delivery. Therefore, cells of the immune system are targeted by orally-administered MEVs, and this phenomenon contributes to MEV localization in the spleen within hours post-administration.

As shown herein, upon oral administration the MEVs go the intestine and then migrate to the spleen. The route to the spleen can be via absorption into the blood and/or by internalization by immune cells in the intestine. The blood route is an unlikely route, because the MEVs then would appear in the liver as shown for intravenous administration. When MEVs are administered intravenously they primarily reach the liver (massively) and to a much lesser extent the spleen. It is shown herein that clearance of the MEVs from the spleen follows different kinetics depending upon their origin (oral or IV). The migration to the spleen following oral administration therefore uses a different a pathway from the MEVs administered intravenously. When MEVs are administered by mouth, they reach the spleen after having passed through the intestine. These results indicate that the MEVs are located in "different compartments" inside the spleen, depending on the route of arrival: either from the intestine or from the blood. As discussed, upon oral administration, the likely route is that the MEVs in the intestine are internalized by lymphocytes present in the GALT, and that the subsequent migration of the MEVs from the intestine/GALT to the spleen occurs because the MEVs are transported by the lymphocytes. Coming from the intestine/GALT, the MEVs end up in the white spleen compartment. Thus, the MEVs provide a way to deliver cargo to different organs from mammalian EVs, which cannot be administered orally.

### 3. Exemplary Diseases and conditions treated by MEVs

Based upon the targeted organs, a variety of diseases and disorders can be treated by MEVs. The MEVs can be loaded or produced to contain therapeutic agents for treating these diseases and conditions. The appropriate route of administration for the targeted organ and disease is selected. For example, for targeting the spleen and intestines, oral administration is selected; and for targeting the lungs, inhalation or nasal administration is selected. Based on the biodistribution and pharmacokinetic data the following organs can be targeted to treat diseases exemplified as follows.

**liver:** cancer, cancer metastases, metabolic syndrome, genetic disorders (delivery of gene therapy), alpha-antitrypsin (AAT) deficiency and other inborn errors of metabolism, hemophilia, hypercholesterolemia, liver inflammation, steatohepatitis, and other diseases and disorders that can be treated by delivery of a therapeutic to the liver;

**spleen:** diseases treated by immune modulation, including cancers, and immune cell disorders, and cancer, and other diseases that can be treated by administration to the spleen, particularly by immune cells that occur in or traffic to the white spleen;

**intestine:** diseases and disorders treated or prevented by vaccines, intestinal infections, microbiota modulation, Crohn's disease, cancer, ulcers, diseases treated by orally administered drugs, such as small molecules and proteins, and other such diseases, disorders, and conditions; and

**lungs:** infectious diseases, particularly respiratory diseases, chronic obstructive pulmonary disease (COPD), pulmonary hypertension, asthma, other inflammatory lung diseases, cystic fibrosis, ATT-deficiency, lung disease, cancer, cancer metastases, and other such diseases and disorders.

## H. EXAMPLES

The following examples are included for illustrative purposes only and are not intended to limit the scope of the invention.

**EXAMPLE 1**

**Production of *Chlorella* cells and isolation of Microalgae Extracellular Vesicles (MEVs)**

**A. Batch production of the inoculum**

*Chlorella vulgaris* of any strain can be used to produce MEVs. Exemplary strains include, but are not limited to, UTEX 265 strain, UTEX 395 strain, UTEX 26 strain, 15 UTEX 30 strain, UTEX 259 strain, UTEX 2219 strain, UTEX 2714 strain UTEX B 1811 (available from the UTEX Culture Collection), the strain designated CCAP 211/19, GEPEA, University of Nantes, France, and any other suitable strain, either transformed or not, can be used to produce the algal cell material. For exemplary purposes the UTEX 265 strain was used.

*Chlorella* was stored on nutrient agar slopes until flask/photobioreactor (PBR) inoculation. For different experiments, different scales of production, between 400 mL (flasks) to 170 L (several PBRs with different total volume) cultures, were used. This description relates to the highest volume of PBR used (170 L, HECTOR PBR ["Hector" photobioreactor designed by the Laboratory of Process Engineering - Environment - Agri-food (GEPEA) / CNRS for the culture of microalgae. Reference: 20160067_0017. Year of production: 2016. Maximum size: 56.43 x 37.66 cm/ 170 L / 300 dpi]).

A 5-Liter PBR was filled with 4 L of sterile BG11 medium (see, *e.g.,* utex.org/products/bg-11-medium for a description of its preparation and see table below (Table 1) for autotrophic growth), and inoculated directly from the stock algal slope on nutrient agar. Then, the *Chlorella* strain was grown as a batch culture in a bubble column using the following culture parameters: temperature of 23°C; medium pH 7.5-8.0; light intensity: 100 $\mu mol \cdot m^{-2} \cdot s^{-1}$; light cycle: continuous. Biomass concentration, specific growth rate and biomass productivity of *Chlorella* were estimated daily. Typically, after 6 days of continuous growth the cultures reached biomass concentrations of approx. 1.2 g/L. The total crop volume of 20 L was collected for subsequent production scale-up to the HECTOR PBR.

**Table 1. Composition of the BG11 medium**

| | **Conc** | **Ml** |
|---|---|---|
| Salt Mix | 100x | 10 |
| Iron* | 1000x | 1 |
| Nitrate* | 100x | 10 |
| Trace metals | 1000x | 1 |
| agar | | 14 |
| $H_2O$ | | up to 1 L |
| pH 7 | | |
| * In case of precipitation, add after sterilization. | | |

**Stock solutions for BG11 medium:**

| **Nitrate 100x** | **g/L** | **MW** | **μM** |
|---|---|---|---|
| $NaNO_3$ | 150 | 85 | 1764.71 |
| | | | |
| **Iron 1000x** | | | |
| Fe-EDTA | 3 | 344.06 | 8.72 |
| | | | |
| **Salt Mix 100x** | | | |
| $K_2PO_4$ | 4 | 173.17 | 23.10 |
| $MgSO_4 \cdot 7H_2O$ | 7.5 | 246.48 | 30.43 |
| $CaCl_2 \cdot 2H_2O$ | 3.6 | 147.01 | 24.49 |

(continued)

| Salt Mix 100x | | | |
|---|---|---|---|
| $Na_2CO_3$ | 2 | 105.99 | 18.87 |
| add HCl if needed | | | |
| | | | |
| **Trace Metals 1000x** | g/L | MW | μM |
| $H_3BO_3$ | 2.9 | 61.84 | 46.25 |
| $MnCl_2 \cdot 4H_2O$ | 1.8 | 197.91 | 9.15 |
| $ZnSO_4 \cdot 7H_2O$ | 0.2 | 287.56 | 0.77 |
| $Na_2MoO_4 \cdot 2H_2O$ | 0.4 | 241.95 | 1.61 |
| $CuSO_4 \cdot 5H_2O$ | 0.1 | 249.68 | 0.32 |
| $CoCl_2 . 6H_2O$ | 0.3 | 237.93 | 0.14 |

**B. Production scale-up in a semi-industrial Photobioreactor (PBR)**

The *Chlorella* cells were cultured further in a 170-Liter photobioreactor system (HECTOR). The inoculum was added to sterile BG11 medium (see Table 1) to the total volume of 150 L and the cells were grown autotrophically as a semi-batch culture with bubble column mixing. The following culture parameters were used: temperature of 18±4°C; medium pH 8.0±0.05; light intensity: 150-300 $\mu mol \cdot m^{-2} \cdot s^{-1}$; between 150 μmol·m-2·s-1 the three first days of each batch, 250 μmol·m-2·s-1 days four and five and 300 μmol·m-2·s-1 days six and seven before the harvesting as light cycle: continuous, with gradual increase in light intensity (see FIG. 1).

Biomass concentration, growth rate and biomass productivity of *Chlorella* were estimated daily. On the $6^{th}$ day of the cultivation, at the biomass concentration of approx. 1.5 g/L Chlorella production then was performed.

**C. Production of 3 consecutive batches of *Chlorella***

The *Chlorella* production was performed in 3 semi-batches of 130 L, from which about 80% of the culture volume was aseptically removed for downstream treatment and supplemented with sterile BG11 medium. Following the harvest, the light intensity was lowered to 140 $\mu mol \cdot m^{-2} \cdot s^{-1}$ in order to avoid excessive photon intake. A seeding line was set up to go from 100 mL of culture to 150 L of culture. Three consecutive batches lasting 6-7 days were carried out with the aim of extracting a vesicle concentrate devoid of microalgae.

**Culture parameters monitoring**

**1. Determination of the protein content**

The protein content of cultures was determined by elemental analysis, resorting to Vario el III (Vario EL, Elementar Analyser systeme, GmbH, Hanau, Germany), according to the procedure provided by the manufacturer. The final protein content was calculated by multiplying the percentage of nitrogen given by the elemental analysis by 6.25.

**2. Estimation of chlorophyll content**

Culture samples were centrifuged at 2547 g for 15 min using a Hermle centrifuge (HERMLE Labortechnik GmbH, Wehingen, Germany). Pigments were extracted from the resulting pellet by bead milling in acetone. The full absorbance spectrum of the extract was obtained with a Genesys™ 10S UV-VIS spectrophotometer (Thermo Scientific, Massachusetts, USA) and iteratively decomposed to the standard pigment spectra in order to obtain the total chlorophyll content.

**3. Growth estimation**

Dry weight was obtained by filtration of culture samples using pre-weighed 0.7 μm GF/C 698 filters (VWR, Pennsylvania, USA) and dried at 120°C until constant mass was obtained using a DBS 60-30 electronic moisture analyser (KERN & SOHN GmbH, Balingen, Germany). All dry weight samples were washed with demineralized water to remove growth medium salts.

**D. Isolation of Microalgae Extracellular Vesicles: Production of concentrated MEV preparation (Down-Stream Processing: clarification and concentration step)**

The culture harvested from the photobioreactor was centrifuged at 2,700 g for 5 minutes at room temperature for cell removal. The supernatant was transferred into fresh bottles and centrifuged again at 2,700 g for 5 minutes at room temperature. The clear MEV-containing solution was then subjected to membrane filtering using a 1.2 μm cut-off cartridge filter. The filtrate was concentrated with the use of a 100 kDa MWCO tangential filtration system. At each isolation step the material was analyzed spectrophotometrically for chlorophyll and particulate matter. Dry weight of the final product was <0.01 g/L and the concentration factor relative to the initial volume of the processed culture was approx. 20. Thus, obtained MEV solution was stored at - 50°C in 1-1.2 L pockets for further purification.

**E. Detailed Protocol for Purification of *Chlorella* Microalgae Extracellular Vesicles**

1. Thaw the product in a cold room at 4°C, overnight, the MEVs, and then the preparation previously clarified and concentrated and stored (1.0 -1.2 L) as described in section D.
2. When the preparation is thawed, harvest the biomass by centrifugation (set the temperature to 4°C): 2 x 10000g for 10' at 4°C.
3. Collect the supernatant (MEVs) and filter by vacuum filter onto 0.65 μm filters to get rid of the remaining cells.
4. The MEVs are concentrated and purified by tangential flow filtration (TFF) using Sartorius VivaFlow systems.

    a. The membrane is washed by running water at ≈100 ml/minute, as described by the manufacturer. After that, the circuit is washed with cell-free medium (BG-11 medium) at ≈200 ml/minute (pressure reading at 2/2,5 bars).
    b. The MEV preparation (supernatant) is run in the circuit at ≈200 ml/minute (pressure reading at 2/2,5 bars). When the residual volume in the circuit plus the reservoir is about 200mL, the TFF is used to diafiltrate and change the medium from BG-11 to PBS using 1L of PBS.
    c. When the residual volume in the circuit plus the reservoir is about 200 ml in PBS, slow the flow to ≈100 ml/minute (20 minutes, 1 bar).
    d. From 30-60 ml of residual volume, slow the flow to a speed lower than 50 ml/ minutes and allow the MEVs in PBS to recirculate for 30 minutes in order to recover the particles trapped on the membrane surface.
    e. MEVs are then filtered using 0.45 μm filters and purified by ultracentrifugation.
    f. The filtered MEVs are loaded on the ultracentrifuge tubes and centrifuged for 1h at 4°C, at 100000g (27400rpm) (acceleration and deceleration at 10 max), for example in a SorVall™ WX ultra 80 TST 28.38. Pellets containing the MEVs are resuspended in 1-2 ml of PBS buffer and sterilized by filtration using a 0.2 μm filter and analyzed the particles by nanoparticle tracking analysis (NTA; dilute up to 1:1000 before the NTA analysis).

**F. Protocol for Purification of *Chlorella* Microalgae Extracellular Vesicles by Size Exclusion Chromatography (SEC)**

When higher purity of MEVs preparations is needed, a last step of purification is added. The MEVs previously concentrated by TFF and purified by ultracentrifugation and formulated in PBS at concentration of 10Exp11 to 10Exp13 per mL are seeded in a pre-packed column qEV1 from IZON. The MEVs are eluted with PBS solution. The elution fractions of 0.5 mL are collected. MEVs are recovered in the first fractions as shown in FIG. 2.

MEVs concentrations in the initial sample and in the fractions collected throughout the elution are evaluated with the ZetaView® engine (Nanoparticle Tracking Analyzer from Particle Metrix) as the quantity of proteins by Bradford assay. The most concentrated fractions (4-5) are pooled and stored at 4°C before use.

**EXAMPLE 2**

**MEV characterization**

**A. Nanoparticle Tracking Analysis (NTA)**

MEVs were analyzed for size and dispersity (size distribution) using a NanoSight NS500 system (Malvern Panalytical Instruments). The instrument was equipped with a 488 nm laser, a high sensitivity sCMOS camera and a syringe pump. The MEV samples were diluted in particle-free PBS (0.02 μm filtered) to obtain a concentration within the recommended measurement range (1-10$\times 10^8$ particles/mL), corresponding to dilutions of from 1/1000 to 1/10000

depending on the initial sample concentration.

For each sample, 5 experiment videos of 60 seconds duration were analyzed using NTA 3.4 Build 3.4.003 (camera level 15-16) with syringe pump speed 30. A total of 1500 frames were examined per sample, which were captured and analyzed by applying instrument-optimized settings using a suitable detection threshold so that the observed particles are marked with a red cross and that no more than 5 blue crosses are seen. Further settings were set to "automatic" and viscosity to "water".

For characterization the MEVs samples are produced and purified as described in Example 1 as follows: MEV is concentrated by tangential flow filtration (TFF), diafiltration and ultracentrifugation, purified by SEC and sterilized by 0.22 μm filtration. After SEC purification and filtration, MEVs are diluted 10,000 times in PBS (1X) and measured in ZetaView® (Particle Metrix GmbH, Ammersee, Germany).

Table 2, below, shows the results of 3 independent measurements.

| SAMPLES (MEVs after SEC) | Concentration (MEVs/mL) | MEVs Median size (nm) | MEVs Zeta potential (mV) |
|---|---|---|---|
| #1 | 2.9Exp12 | 159.9 | -20 |
| #2 | 2.9Exp12 | 163.6 | -22.58 |
| #3 | 2.9Exp12 | 161.7 | -22.3 |
| MEAN | 2.9Exp12 | 161.6 | -21.63 |
| SD | 5.7Exp8 | 2.6 | -2.3 |

### B. Transmission Electron Microscopy (TEM)

To verify the presence of intact MEVs, the preparations were analyzed using transmission electron microscopy (TEM). Fixed (4% formaldehyde, 0.2% glutaraldehyde) MEV samples were allowed to attach to Formvar/carbon-coated grids for 15-20 min, washed again with PBS followed by distilled water and finally stained with 0.4% uranyl acetate/1.8% methyl cellulose and then dried. The preparations were observed using a JEOL-JEM 1230 (JEOL Ltd., Tokyo, Japan) at 80 kV and images were acquired using a Morada digital camera and iTEM software (Olympus, Münster, Germany).

The TEM imaging, presented in the Figure 3, demonstrates that the MEVs are round shaped vesicles sized ~50-250 nm in diameter. MEVs are enveloped in a single lipid bilayer membrane, their lumen has slightly higher electron density, and the thickness of the membrane was estimated as ~5-10 nm.

### C. DiR fluorescent labelling

For further characterization studies, MEVs were labelled with DiR, a lipophilic carbocyanine derivative (1,1'-Dioctadecyl-3,3,3',3'-Tetramethylindotricarbocyanine Iodide; ThermoFisher Scientific) that has low fluorescence in water, but becomes highly fluorescent upon membrane incorporation, and diffuses laterally within the plasma membrane. Fresh samples of the P40 fraction (prepared as above) were re-suspended in 1 ml of BG11 culture medium. 5 μl of 1 mg/ml DiR solution was added to the samples, following incubation at 37°C for 1 hour. Then, the samples were ultra-centrifuged at 100,000 g for 30 min using a Kontron TST 55.5 rotor at 28,100 rpm. The supernatant was removed, while the pellets were washed twice with 1 ml of PBS and centrifugation at 100,000g for 30 min. Finally, the pellet was re-suspended in 1 ml of PBS. The DiR-labelled MEVs were stored at 4°C and used promptly to ensure highest possible fluorescent intensity. DiR fluorescence of the labelled MEVs was measured using a SpectraMax® fluorescence microplate reader (Molecular Devices, USA) with excitation at 750 nm and emission at 780 nm.

### D. PKH26 fluorescent labelling

For uptake and internalization studies, MEVs are labelled with PKH26 (Sigma-Aldrich), a fluorochrome in the red spectrum with peak excitation (551 nm) and emission (567 nm) that may also be excited by a 488 nm laser. Fresh samples of the P40 fraction (prepared as above) are re-suspended in 1 ml of Diluent C from the PKH26 kit. 6 μl of PKH26 dye is added to the samples, followed by continuous mixing for 30 seconds by gentle pipetting. After 5-minute incubation at room temperature, the samples are quenched by adding 2 ml of 10% BSA in 1× PBS. The volume is brought up to 8.5 ml in media and 1.5 ml of 0.971 M sucrose solution is added by pipetting slowly and carefully into the bottom of the tube, making sure not to create turbulence. The PKH26-labelled MEVs remain on top of a sucrose cushion. Then, the samples are ultra-centrifuged at 190,000 g for 2 hours at 2-8°C using a Kontron TST 55.5 rotor. The supernatant is removed, while the pellets are washed with 1× PBS by gentle pipetting and centrifuged again at 100,000g for 30 min. Finally, the pellet is re-suspended in 1 ml of 1× PBS. The PKH26-labelled MEVs are stored at 4°C and filtered with 0.45 μm filter before adding to cells.

### E. Flow cytometry experiments

The analyses were conducted using LSRII flow cytometer with CellQuest Pro software (BD Biosciences). Latex beads of 0.3 and 1.1 μm diameters were prepared and used according to the manufacturer's recommendation to define the MEV gate. Since latex beads typically have higher refractive index and thus lower limits of size detection by flow cytometry than MEVs, the thresholds for forward and side scatter were adjusted to avoid background noise during acquisition. The predefined MEV gate was applied to all samples during analysis.

### F. Identification of proteins present in *Chlorella* MEVs

#### 1. Proteomic analysis

In order to determine the identity and the sequence of proteins associated to *Chlorella* MEVs, a MEV sample was submitted to SDS-PAGE separation and main protein bands were cut into small pieces and washed several times with a 1/1 (v/v) solution of acetonitrile / 100 mM ammonium bicarbonate pH 8. The proteins were then reduced with 100 mM dithiothreitol in ammonium bicarbonate pH 8 for 45 min at 56°C and then cysteine residues were alkylated with 55 mM iodoacetamide in 100 mM ammonium bicarbonate pH 8 for 30 min at room temperature and in the dark. The gel pieces were then digested by trypsin (PROMEGA, reference V511A in a ratio 1: 20) in 100 mM ammonium bicarbonate pH 8 and placed at 4°C for 45 min prior to incubation overnight at 37°C. Tryptic peptides were analyzed by LC-MS/MS on a mass spectrometer and protein were identified by comparison of peptide sequences deduced from MS/MS sequencing with the proteome of *Chlorella vulgaris.* Proteins exhibiting sequence coverage higher than 10% were identified.

#### 2. Sequence Homology (by BLAST) of proteins from the proteomic analysis

The proteins below were individually "blasted" using NCBI-Blast Protein. The identified proteins either were previously characterized as occurring *Chlorella vulgaris,* or they presented medium or strong homologies with many proteins from other Chlorella species: *Chlorella variabilis, Chlorella desiccata* and *Chlorella sorokiniana.*

- Protein CHLNCDRAFT_26010: annotated as «Acetyl-coenzyme A synthetase », with acetate-CoA ligase, AMP binding and ATP binding activities (MF), involved in acetyl-CoA biosynthetic process from acetate (Glycolysis, Gluconeogenesis, Pyruvate, Glyoxylate, Dicarboxylate and Propanoate metabolisms) (BP).
- Protein CHLNCDRAFT_56016: annotated as « Clp R domain-containing protein », with ATP binding and ATP hydrolysis activities (MF).
- Protein atpA: annotated as «ATP synthase subunit alpha, chloroplastic », component of chloroplast thylakoid membrane (CC), with ATP binding, proton-transporting ATP synthase activity, proton-transporting ATPase and hydrolase activities (MF), possibly involved in Oxidative phosphorylation and Photosynthesis (BP).
- Protein atpB: annotated as «ATP synthase subunit beta, chloroplastic », component of chloroplast thylakoid membrane (CC), with ATP binding, proton-transporting ATP synthase activity, proton-transporting ATPase and hydrolase activities (MF), possibly involved in Oxidative phosphorylation and Photosynthesis (BP).
- Protein tufA: annotated as «Elongation factor Tu », component of chloroplast (CC), with GTP binding, GTPase and translation elongation factor activities (MF).
- Hypothetical protein D9Q98_001761 (coverage 48%). Highly homolog to hypothetical protein D9Q98_001760 from *Chlorella vulgaris* (85,6%) and to other proteins from different species of *Chlorella* (*variabilis, desiccata* and *sorokiniana*), 3 of these homologs being characterized in *Chlorella sorokiniana:* "chlorophyll a b-binding", "chlorophyll a b-binding of LHCII type 1 chloroplastic" and "MYST-like histone acetyltransferase 1", with respectively 73%, 84% and 89% of sequence homology.
- Hypothetical protein D9Q98_002614 (coverage 33%). Highly homolog to hypothetical protein D9Q98_001760 (72%) and D9Q98_001761 (70%) from *Chlorella vulgaris* and to other proteins from different species of *Chlorella* (*variabilis, desiccata* and *sorokiniana*), 2 of these homologs being characterized in *Chlorella sorokiniana:* "chlorophyll a b-binding", "chlorophyll a b-binding of LHCII type chloroplastic", with respectively 89% and 70% of sequence homology; and 1 of these homologs characterized in *Chlorella desiccata*: putative "chlorophyll a b-binding of LHCII type 1 chloroplastic", with 60% of sequence homology.
- Hypothetical protein D9Q98_006265 (coverage 26%). No homology in *Chlorella vulgaris.* Only one homology with "DNA replication licensing factor MCM3" from *Chlorella sorokiniana,* with 56% of sequence homology.
- Hypothetical protein D9Q98_000265 (coverage 19%). No homology in *Chlorella vulgaris.* Only two homologies with

two proteins from *Chlorella desiccata* and *Chlorella sorokiniana,* with only one characterized: "flagellar associated protein", with 58% of sequence homology (*Chlorella sorokiniana*).

- Hypothetical protein D9Q98_007183 (coverage 19%). No homology in *Chlorella vulgaris.* Only 5 homologies with 5 proteins from *Chlorella variabilis, Chlorella desiccata,* and *Chlorella sorokiniana,* whose only one characterized: "Fe-assimilating 1", with 47% of sequence homology, *Chlorella sorokiniana*).
- Hypothetical protein D9Q98_009617 (coverage 15%). Mostly homolog to hypothetical proteins D9Q98_000026 and D9Q98_009618 from *Chlorella vulgaris* (with respectively 55% and 33% of sequence homology), and with 11 other proteins from *Chlorella variabilis, Chlorella desiccata,* and *Chlorella sorokiniana,* whose 5 are characterized in *Chlorella sorokiniana*: 2 "lytic transglycosylases", 2 "peptidoglycan-binding" and 1 "spore coat assembly domain" with respectively 52%, 34%, 42%, 42% and 55% of sequence homology.
- Hypothetical protein D9Q98_005959 (coverage 12%). No homology in *Chlorella vulgaris.* Only 4 homologies with 4 proteins from *Chlorella variabilis, Chlorella desiccata,* and *Chlorella sorokiniana,* whose only 2 characterized: "elongation factor mitochondrial" (87% of sequence homology, *Chlorella sorokiniana*) and "putative elongation factor Tu mitochondrial" (86% of sequence homology, *Chlorella desiccata*).
- Hypothetical protein D9Q98_003812 (coverage 11%). No homology in *Chlorella vulgaris.* Only 3 homologies with 3 proteins from *Chlorella variabilis* and *Chlorella desiccata,* whose only one characterized: "putative iron uptake system component EfeO" (58% of sequence homology, *Chlorella desiccata*).
- Hypothetical protein D9Q98_010620, partial [*Chlorella vulgaris*] (coverage 11%). Mostly homolog with 9 proteins from *Chlorella vulgaris*: D9Q98_004806, D9Q98_004582, D9Q98_004143, D9Q98_002326, D9Q98_002347, D9Q98_008273, D9Q98_008254, D9Q98_008251 and D9Q98_008376 (with respectively 63%, 59%, 47%, 49%, 48%, 41%, 41%, 44% and 47% of sequence homology). Only 7 homologies with 7 proteins from *Chlorella variabilis* and *Chlorella sorokiniana,* whose only 3 are characterized: "cellulosome anchoring cohesin region," "nascent polypeptide-associated complex subunit alpha isoform X1" and "Protein CBG24242" (with respectively 43%, 42% and 58% of sequence homology, *Chlorella sorokiniana*).
- Hypothetical protein D9Q98_009216 (coverage 11%). Highly homolog to hypothetical protein D9Q98_009214 and D9Q98_009215 from *Chlorella vulgaris* (with respectively 98% and 98% of sequence homology). Only 2 homologies with 2 proteins from *Chlorella sorokiniana,* both characterized: "mechanosensitive ion channel 10" and "beta-Ig-H3 fasciclin" (with respectively 52% and 53% of sequence homology).

Proteins containing sites susceptible to N-glycosylation are reported in Table 3, below.

**Table 3.** List of proteins identified by a proteomic approach. Sequence coverage and number of potential N-glycosylation sites are indicated.

| Protein | Sequence coverage | Potential Glycosite | Name of the putative protein relevant to the identified fragment |
|---|---|---|---|
| D9Q98_001761 | 48% | 1 | chlorophyll a/b-binding protein of LHCII type, chloroplastic |
| D9Q98_002614 | 33% | 1 | chlorophyll a/b-binding protein of LHCII type, chloroplastic |
| D9Q98_006265 | 26% | 1 | n/a |
| D9Q98_007183 | 19% | 1 | n/a |
| D9Q98_000265 | 19% | 3 | n/a |
| D9Q98_009617 | 15% | 0 | n/a |
| D9Q98_005959 | 12% | 2 | translation elongation factor Tu, mitochondrial |
| D9Q98_003812 | 11% | 4 | n/a |
| D9Q98_010620 | 11% | 1 | n/a |
| D9Q98_009216 | 11% | 4 | n/a |

**G. Characterization of RNAs present in Chlorella MEVs**

With the aim to inventory the RNAs present in the extracellular vesicles of *Chlorella vulgaris* we performed the sequencing of total RNA sequencing (small RNAs and long RNAs). Two types of sequencing were performed in this study: total RNA sequencing (which is based on reverse transcription from short random primers) and small RNA sequencing (which is based on reverse transcription from RNA-ligated adapters).

### 1. RNA extraction

Total RNAs were extracted from the "MEVs" sample using the Direct-zol™ RNA Microprep kit (Ozyme, Ref.: ZR2061) from 300 μL. The concentration of the RNAs and their quality was determined by fluorometry on a Qubit with the Qubit RNA Assay Kit (Fisher Scientific, Ref.: Q32852) and Qubit RNA IQ Kit (Fisher Scientific, Ref.: Q33221) kits, respectively. After isolation a concentration of about 80ng of total native RNA per $10^9$ MEV was quantified.

### 2. Preparation of total RNA library

Total RNA was produced using the NEBNext® Ultra™ II Directional RNA library prep kit (New England Biolabs, Ref.: E7760S) following the manufacturer's protocol which was adapted for this study. Thus, in step 1.1.1, the 10 μl volume of nuclease-free water was adapted for this study.

The PCR products obtained were analyzed by capillary electrophoresis on Qiaxcel (QIAGEN, QIAxcel DNA Screening Kit, Ref.: 929004). The migration profile showed an amplification of fragments whose size was between 81 and 200 base pairs (see FIG. 4).

The library then was purified using the GeneJET PCR purification kit (Thermo Fisher Scientific, Ref.: K0702). All the fragments whose size was between 125 and 175 bp were purified by electrophoresis on PippinHT (Sage Science) using a 3% agarose cassette, marker 30G (Sage Sciences, Ref.: HTG3010). Following purification, the size of the fragments constituting the purified pool was checked by capillary electrophoresis and its concentration was determined by fluorometric assay on Qubit 4.0 before sequencing.

### 3. Sequencing on MiSeq and on NovaSeq 6000 System (Illumina)

To validate the quality of the TotalRNA library, the sequencing of the PCR products was performed on a MiSeq System (Illumina) in 2 X 250 bp using the MiSeq Reagent Kit v2 (500 cycles) (Illumina; Ref.: MS-102 -2003). The mix of libraries was loaded at a concentration making it possible to obtain a theoretical density of clusters between 800 and 1000 $K/mm^2$. The results from positive quality control allowed follow-up on the sequencing on the NovaSeq 6000 Instrument. Sequencing of the Total RNA library was performed on NovaSeq 6000 system in paired end reads (2X150 bp). The total read count was 94,850,242.

The small RNA library was sequenced on MiSeq (Illumina) in 2 X 250 bp using the MiSeq Reagent Kit v2 (500 cycles) (Illumina; Ref.: MS-102-2003). The mix of libraries was loaded at a concentration making it possible to obtain a theoretical density of clusters between 800 and 1000 $K/mm^2$.

### H. Bioinformatics analysis

The quality of the reads was evaluated with the FastQC tool (TotalRNA.R1.fastqc.html and TotalRNA.R2.fastqc.html files). Poor quality adapter sequences and external bases were removed from reads using the cutadapt tool. Reads were then aligned to the *Chlorella vulgaris* reference genome (cvul assembly, GenBank No: GCA_023343905.1) using Hisat2 (using default settings). Forty reads aligned with the *Chlorella vulgaris* genome. The counting of the different alignments was carried out with Stringtie (default parameters) from annotations of the cvul genome containing only the genes (with corresponding annotations) to which the reads obtained was aligned with *Chlorella vulgaris* genome. The available annotations, coming from public data, do not allow assigning an RNA type to each sequence.

The same alignment work was carried out with the genome of the mitochondria and that of the chloroplast of *Chlorella vulgaris.* None of the reads aligned with these two genomes.

The bioinformatic analysis of the total library shows about 40 RNAs between 140 and 250 nucleotides that blast with the *Chlorella vulgaris* genome and encode proteins that were not identified.

The quality of the reads was evaluated with the FastQC tool (SmallRNA.R1.fastqc.html and SmallRNA.R2.fastqc.html files). Poor quality adapter sequences and external bases were removed from reads using the cutadapt tool. Reads were then aligned to the *Chlorella vulgaris* reference genome (cvul assembly, GenBank No: GCA_023343905.1) using Hisat2 (using default settings). The counting of the different alignments was carried out with Stringtie (default parameters) from annotations of the cvul genome. The SmallRNA_quantitative_measures_filtred.xisx file which lists only the genes to which the reads obtained aligned contains only one gene.

As the MEVs used for the analysis contained a mixture of siRNAs against luciferase an alignment on the luciferase sequence was carried out using the bowtie2 tool from sequences of at least 18 bp. About 3.7% of the sequences align with the luciferase sequence.

### I. MEV Lipidomics Analysis

## 1. Sample processing

Samples were slowly thawed on ice; 1.5 mL of chloroform/methanol (2/1, v/v) solution was added, followed by 0.5 mL of pure water. Samples were vortexed for 1 min, sonicated at 4 °C for 30 min and centrifuged at 3000 rpm for 10 min. The organic phase was removed to a clean test tube, dried with nitrogen and reconstituted with 200 μL isopropanol/methanol (1/1, v/v). After adding 5 μL of internal standard (125 μg/mL LPC (12:0)), samples were centrifuge again at 12000 rpm, 4 °C for 10 min. The supernatant was transferred into the injection bottle.

## 2. LC-MS detection

Analysis platform: LC- MS (Waters, UPLC; Thermo, Q Exactive); column:
(ACQUITY UPLC BEH C18 (2.1*100mm 1.7 μm)); chromatographic separation conditions:

- Column temperature: 40 °C; Flow rate: 0.3 mL/min;
- Mobile phase A: Acetonitrile /water (6:4, v/v, with 10mM Ammonium formate);
- Mobile phase B: Iso-Propyl alcohol / Acetonitrile (9:1, v/v, with 10 mM Ammonium formate);
- Injection volume: 3 μL (positive ion); 3 μL (negative ion); Automatic injector temperature: 4°C.

**Table 4.** The gradient of mobile phase used in LC-MS detection method.

| Time (min) | Flow rate (mL/min) | A (%) | B (%) |
|---|---|---|---|
| 0 | 0.3 | 70 | 30 |
| 10.50 | 0.3 | 0 | 100 |
| 12.50 | 0.3 | 0 | 100 |
| 12.51 | 0.3 | 70 | 30 |
| 16.00 | 0.3 | 70 | 30 |

## 3. Mass spectrometry detection parameters:

- ESI+:Heater Temp 300°C; Sheath Gas Flow rate, 45 arb; Aux Gas Flow Rate, 15 arb; Sweep Gas Flow Rate, 1arb; spray voltage, 3.0KV; Capillary Temp, 350°C; S-Lens RF Level, 30%.
- ESI-:Heater Temp 300°C, Sheath Gas Flow rate,45arb; Aux Gas Flow Rate, 15arb; Sweep Gas Flow Rate, 1arb; spray voltage, 3.2KV; Capillary Temp 350°C; S-Lens RF Level,60%.
- Scanning mode: Full Scan (M/Z 70~1050) and data-dependent mass spectrometry (DD-MS2, TopN = 10); Resolution:70,000 (primary mass spectrometry) & AMP;
- Collision mode: High energy collision dissociation (HCD)

## 4. Lipidomics analysis results

**Table 5.** ESI+ lipidomics analysis results for MEVs.

| LipidIon | LipidGroup | Class | Rt | IonFormula | Peak Area |
|---|---|---|---|---|---|
| LPC(12:0)+H | LPC(12:0)+H | LPC | 0.97 | C20 H43 O7 N1 P1 | 13265407632.00 |
| LPC(12:0)+Na | LPC(12:0)+Na | LPC | 0.93 | C20 H42 O7 N1 P1 Na1 | 2093451784.00 |
| MG(16:0)+H | MG(16:0)+H | MG | 2.72 | C19 H39 O4 | 627421076.70 |
| TG(36:3e)+H | TG(36:3e)+H | TG | 9.64 | C39 H71 O5 | 597367444.40 |
| DG(18:0_16:0)+NH4 | DG(34:0)+NH4 | DG | 9.76 | C37 H76 O5 N1 | 545916845.40 |
| Cer(d16:0_16:0)+H | Cer(d32:0)+H | Cer | 8.67 | C32 H66 O3 N1 | 434951801.90 |

(continued)

| LipidIon | LipidGroup | Class | Rt | IonFormula | Peak Area |
|---|---|---|---|---|---|
| DG(16:0_16:0)+NH4 | DG(32:0)+NH4 | DG | 9.16 | C35 H72 O5 N1 | 430757024.40 |
| DG(18:0_16:0)+NH4 | DG(34:0)+NH4 | DG | 9.64 | C37 H76 O5 N1 | 408179029.10 |
| MG(18:0)+H | MG(18:0)+H | MG | 3.81 | C21 H43 O4 | 402213163.90 |

**Table 6.** ESI- lipidomics analysis results for MEVs.

| LipidIon | LipidGroup | Class | Rt[c-1] | IonFormula | Peak Area |
|---|---|---|---|---|---|
| Cer(d35:1+O)-H | Cer(d35:1+O)-H | Cer | 7.28 | C35 H68 O4 N1 | 31988627.10 |
| Cer(d20:0_23:0)+HCOO | Cer(d43:0)+HCOO | Cer | 10.63 | C44 H88 O5 N1 | 12403541.83 |
| Cer(d18:0_18:1)-H | Cer(d36:1)-H | Cer | 8.41 | C36 H70 O3 N1 | 5523529.96 |
| Cer(m18:0_13:0)+HCOO | Cer(m31:0)+HCOO | Cer | 6.75 | C32 H64 O4 N1 | 4409871.47 |
| Cer(d18:0_18:1)-H | Cer(d36:1)-H | Cer | 8.27 | C36 H70 O3 N1 | 3956710.64 |
| Cer(d15:1_18:1)+HCOO | Cer(d33:2)+HCOO | Cer | 6.38 | C34 H64 O5 N1 | 2457056.42 |
| Cer(d15:1_18:1)+HCOO | Cer(d33:2)+HCOO | Cer | 6.51 | C34 H64 O5 N1 | 2373869.91 |
| Cer(d20:2_13:0)+HCOO | Cer(d33:2)+HCOO | Cer | 4.22 | C34 H64 O5 N1 | 2255771.34 |
| BiotinylPE(31:1)-H | BiotinylPE(31:1)-H | BiotinylPE | 5.43 | C46 H83 O10 N3 S1 P1 | 1767937.80 |
| Cer(d15:0_18:2)+HCOO | Cer(d33:2)+HCOO | Cer | 4.11 | C34 H64 O5 N1 | 1324275.01 |

The major lipids species extracted are: Lyso-phosphatidyl-choline (LPC), monoacylglycerol (MG), diacylglycerol (DG), triacylglycerol (TG), and ceramides (Cer). Total extract of Chlorella cells has a different lipid composition (phosphatidyl-choline (PC), phosphatidyl-ethanolamine (PE), phosphatidyl-glycerol (PG) and diacylglycerol (DG) (Cecchin et al. (2019) The Plant Journal,100:1289-1305).

With regard to fatty acid composition, MEVs from *Chlorella vulgaris* showed a different composition to *Chlorella* total cell extract. MEVs have longer fatty acids and are more unsaturated than cell extract. These results are summarized in the tables herein.

**J. MEV Metabolomics Analysis**

**Table 7.** ESI+ metabolomics analysis results for MEVs.

| Name | Formula | Molecular Weight | m/z | RT [min] | Peak Area |
|---|---|---|---|---|---|
| [Similar to: 5-chloro-8-hydroxy-6-methoxy-3-methyl-3,4-dihydro-1H-2-benzopyran-1-one; AMass: -130.0268 Da] | C6 H5 C1 | 112.01 | 113.02 | 2.79 | 2275517912.00 |
| Benzothiazole | C7 H5 N S | 135.01 | 136.02 | 5.58 | 884980308.30 |
| N,N-Dimethyldodecylamine | C14 H31 N | 213.25 | 214.25 | 11.70 | 834705736.40 |
| Palmitic Acid | C16 H32 O2 | 273.27 | 274.27 | 9.56 | 574595945.20 |
| Indole | C8 H7 N | 100.03 | 101.04 | 2.80 | 541857154.80 |
| n/a | n/a | 101.04 | 102.05 | 2.79 | 514881461.80 |
| 2-(Methylthio)benzothiazole | C8 H7 N S2 | 181.00 | 182.01 | 7.96 | 448808384.80 |
| 1-Lauroyl-2-hydroxy-sn-glycero-3-phosphocholine | C20 H42 N O7 P | 439.27 | 440.28 | 7.65 | 346845832.20 |

(continued)

| Name | Formula | Molecular Weight | m/z | RT [min] | Peak Area |
|---|---|---|---|---|---|
| [Similar to: 2-(Methylthio)benzothiazole; AMass: -15.0236 Da] | C3 H7 N2 P S2 | 165.98 | 166.99 | 7.96 | 321761664.00 |
| 2-Amino-1,3,4-octadecanetriol | C18 H39 N O3 | 317.29 | 318.30 | 9.69 | 286436435.80 |

**Table 8.** ESI- metabolomics analysis results for MEVs.

| Name | Formula | Molecular Weight | m/z | RT [min] | Peak Area |
|---|---|---|---|---|---|
| IS_2-chloro-phenylalanine | C9 H10 Cl N O2 | 199.04 | 198.03 | 2.76 | 4768994771.00 |
| LPE 14:0; [M-H]- | C19 H40 N O7 P | 425.25 | 424.25 | 7.65 | 1457941932.00 |
| 2-Mercaptobenzothiazole | C7 H5 N S2 | 166.99 | 165.98 | 5.83 | 613566495.90 |
| n/a | n/a | 93.93 | 92.93 | 0.72 | 567261536.20 |
| n/a | n/a | 179.98 | 178.98 | 14.66 | 480949549.40 |
| [Similar to: 3'-Dephosphocoenzyme A; AMass: -589.1732 Da] | n/a | 97.98 | 96.97 | 0.75 | 392534007.80 |
| n/a | C25 H32 O8 | 460.21 | 459.20 | 8.39 | 304244723.20 |
| Xanthine | C5 H4 N4 O2 | 152.03 | 151.03 | 0.98 | 269743132.80 |
| DL-β-Leucine | C6 H13 N O2 | 131.09 | 130.09 | 1.16 | 266337552.60 |
| n/a | C2 H4 N2 O4 S | 151.99 | 150.98 | 14.67 | 246714926.90 |

The results indicate that the MEVs naturally carry a number of small-molecule components, such as aromatic heterocyclic compounds (including purine base and organosulfur compounds), fatty acids, cationic detergents, amino acids and derivatives thereof.

## EXAMPLE 3

### Loading of biomolecule cargo into the Microalgae Extracellular Vesicles (MEVs)

### A. Passive and surfactant-assisted loading of biomolecules (*e.g.*, proteins, peptides, mRNA, Antisense Oligonucleotides (ASOs), plasmids, complexes) using plasmid DNA (pDNA) and GFP (protein) as exemplary biomolecules

Purified MEVs, as described in Example 1, were diluted in PBS to a specific concentration (10^8, 10^9 or 10^10). Next, different concentrations of cargos (0.2, 1 or 2 μg/ml for pDNA; 2 or 20 μg/ml for GFP) were added to the MEV suspension (total volume of 500-1000 μl), and incubated for 1 or 24 hours for pDNA and 0, 1 or 6 hours for GFP (passive loading). For surfactant-assisted loading, the mixture of MEVs and pDNA was supplemented with 0.2% or 0.5% of saponin and incubated at room temperature for 5 or 30 min, with or without an agitation at 700 rpm.

Table 9. Protocol for passive and surfactant-assisted loading of DNA (plasmids, ASOs); parameters and values used as variables for sensitivity analysis.

| MEV concentration (per ml) | DNA concentration (μg/ml) | saponin concentration (%) | incubation temperature | incubation time | agitation (rpm) |
|---|---|---|---|---|---|
| 4.00E+08<br>4.00E+09<br>4.00E+10 | 0.05<br>0.15<br>0.25<br>0.35 | 0<br>0.2<br>0.5 | RT<br>37°C | 5 min<br>30 min<br>1h | 0<br>700 |

(continued)

| MEV concentration (per ml) | DNA concentration (μg/ml) | saponin concentration (%) | incubation temperature | incubation time | agitation (rpm) |
|---|---|---|---|---|---|
| | 0.50 | | | 24 h | |
| | 0.70 | | | | |

Table 10. Protocol optimization for passive and surfactant-assisted loading of proteins (oligopeptides, polypeptides); parameters and values used as variables for sensitivity analysis.

| MEV concentration (per ml) | protein concentration (μg/ml) | saponin concentration (%) | incubation temperature | incubation time | agitation (rpm) |
|---|---|---|---|---|---|
| 6.00E+08 | 0.1 | 0 | | 5 min | |
| 6.00E+09 | 0.5 | 0.2 | RT | 30 min | 0 |
| 1.40E+10 | 1.0 | | 37°C | 1 h | 700 |
| 6.00E+10 | 2.0 | 0.5 | | 24 h | |

**B. Freeze-thaw cycles loading of biomolecules (*e.g.*, proteins, peptides, mRNA, ASOs, plasmids, complexes) using plasmid DNA (pDNA), GFP (protein) and mRNA (encoding eGFP) as exemplary biomolecules**

Purified MEVs, as described in Example 1, were diluted in PBS to a specific concentration (10^8, 10^9, 10^10, 10^11 and 10^12). Next, various concentrations of cargos (0.2, 1, 2 or 40 μg/ml for pDNA; 0,2 and 2 μg/ml for mRNA) were added to the MEV suspension (total volume of 500 μl). The mixture of MEVs and the cargo was frozen at -80°C or immersed into liquid nitrogen (-196°C). Then the sample was thawed at 37°C in a water bath. This freeze-thaw cycle was repeated 2 or 4 times for each sample.

Table 11. Protocol optimization for freeze-thaw cycles loading of DNA (plasmids, ASOs); parameters and values used as variables for sensitivity analysis.

| MEV concentration (per ml) | DNA concentration (μg/ml) | freezing temperature | thawing temperature | number of cycles |
|---|---|---|---|---|
| 8.00E+07 | | | | |
| 1.00E+08 | | | | |
| 8.00E+08 | | | | |
| 9.00E+08 | 0.2 | | | |
| 1.00E+09 | 1.0 | -80°C | | 2 |
| 6.00E+09 | 2.0 | -196°C | 37°C | 4 |
| 8.00E+09 | 20 | | | |
| 9.00E+09 | | | | |
| 6.00E+10 | | | | |
| 9.00E+10 | | | | |
| 9.00E+11 | | | | |

Table 12. Protocol optimization for freeze-thaw cycles loading of RNA (mRNA, siRNA, miRNA); parameters and values used as variables for sensitivity analysis.

| MEV concentration (per ml) | RNA concentration (μg/ml) | freezing temperature | thawing temperature | number of cycles |
|---|---|---|---|---|
| 8.00E+07 | | | | |
| 1.00E+08 | | | | |
| 8.00E+08 | | | | |
| 9.00E+08 | | | | |
| 1.00E+09 | 0.2 | | | |

(continued)

| MEV concentration (per ml) | RNA concentration (μg/ml) | freezing temperature | thawing temperature | number of cycles |
|---|---|---|---|---|
| 6.00E+09<br>8.00E+09<br>9.00E+09<br>6.00E+10<br>8.00E+10<br>9.00E+010<br>9.00E+11 | 1.0<br>2.0<br>20 | -80°C<br>-196°C | 37°C | 2<br>4 |

Table 13. Protocol optimization for freeze-thaw cycles loading of proteins (oligopeptides, polypeptides); parameters and values used as variables for sensitivity analysis.

| MEV concentration (per ml) | protein concentration (μg/ml) | freezing temperature | thawing temperature | number of cycles |
|---|---|---|---|---|
| 8.00E+07<br>1.00E+08<br>8.00E+08<br>9.00E+08<br>1.00E+09<br>6.00E+09<br>8.00E+09<br>9.00E+09<br>6.00E+10<br>8.00E+10<br>9.00E+10<br>9.00E+11 | 0.2<br>1.0<br>2.0<br>20 | -80°C<br>-196°C | 37°C | 2<br>4 |

**C. Sonication loading of biomolecules (proteins, peptides, mRNA, ASOs, plasmids, complexes) using plasmid DNA (pDNA), mRNA (encoding eGFP) and GFP (protein) as exemplary biomolecules**

Purified MEVs, as described in Example 1, were diluted in PBS to a predetermined concentration (10^8, 10^9, 10^10, 10^11 and 10^12 MEVs/mL. Next, various concentrations of cargos (0.2, 1, 2 or 20 μg/ml for pDNA; 2 and 20 μg/ml for mRNA; 2 or 20 μg/ml for GFP) were added to the MEV suspension (total volume of 500-1000 μl). The mixture of MEVs and the cargo was sonicated using Fisherbrand Model 50 Sonic Dismembrator (frequency: 20 kHz, wattage: 50 W). Before sonication, the signal amplitude was set to 20%, 40%, 50% or 60%. Each sample underwent a sonication for 30 seconds followed by a pause during 30 seconds on ice, or 4 seconds followed by a rest period of 120 seconds on ice. This cycle was repeated 2, 6 or 8 times for each sample.

Table 14. Protocol optimization for sonication loading of DNA (plasmids, ASOs); parameters and values used as variables for sensitivity analysis.

| MEV concentration (per ml) | DNA concentration (μg/ml) | amplitude (Pa) | cycle timing | number of cycles |
|---|---|---|---|---|
| 8.00E+08<br>8.00E+09<br>9.00E+10<br>1.50E+10<br>1.50E+11 | 0.2<br>1.0<br>2.0<br>20 | 20<br>40<br>50<br>60 | 4 s ON, 2 s OFF, 2 min on ice 30 s ON, 30 s OFF, 2 min on ice 30 s ON, 30 s OFF on ice | 2<br>6<br>8 |

Table 15. Protocol optimization for sonication loading of RNA (mRNA, siRNA, miRNA); parameters and values used as variables for sensitivity analysis.

| MEV concentration (per ml) | RNA concentration (μg/ml) | amplitude (Pa) | cycle timing | number of cycles |
|---|---|---|---|---|
| 8.00E+08<br>8.00E+09<br>9.00E+10<br>1.40E+10<br>1.50E+10 | 0.2<br>1.0<br>2.0<br>20 | 20<br>40<br>50<br>60 | 30 s ON, 30 s OFF, 2 min on ice 4 s ON, 2 s OFF, 2 min on ice 30 s ON, 30 s OFF on ice | 2<br>6<br>8 |

Table 16. Protocol optimization for sonication loading of proteins (oligopeptides, polypeptides); parameters and values used as variables for sensitivity analysis.

| MEV concentration (per ml) | protein concentration (μg/ml) | amplitude (Pa) | cycle timing | number of cycles |
|---|---|---|---|---|
| 1.40E+10<br>1.50E+10<br>1.50E+11<br>2.00E+11<br>6.00E+11<br>1.50E+12 | 2.0<br>20 | 20<br>50<br>70 | 30 s ON, 30 s OFF on ice | 4<br>6<br>8 |

Table 17. Protocol optimization for sonication loading of small molecules (hydrophilic molecules); parameters and values used as variables for sensitivity analysis.

| MEV concentration (per ml) | protein concentration (μg/ml) | amplitude (Pa) | cycle timing | number of cycles |
|---|---|---|---|---|
| 1.40E+10<br>1.50E+10<br>1.50E+11<br>2.00E+11<br>6.00E+11<br>1.50E+12 | 2.0<br>20 | 20<br>50<br>70 | 30 s ON, 30 s OFF on ice | 4<br>6<br>8 |

**D. Extrusion loading of biomolecules (*e.g.*, proteins, peptides, mRNA, ASOs, plasmids, complexes) using plasmid DNA (pDNA), mRNA and GFP as exemplary biomolecules**

Purified MEVs, as described in Example 1, were diluted in PBS to a specific concentration (10^10). Next, different concentrations of cargos (2 or 20 μg/ml for pDNA; 0.1, 1, 2 and 10 μg/ml for mRNA; 2 or 20 μg/ml for GFP) were added to the MEV suspension (total volume of 500-1000 μl). The mixture of MEVs and the cargo was extruded through a syringe-based hand-held mini-extruder (SKU: 610023-1 EA, Avanti® Polar Lipids). The sample was extruded from 10 to 15 times across a membrane filter with 100 nm diameter pores, using two facing syringes. In some experiments, the sample was extruded sequentially through a 200 nm, a 100 nm and 50 nm diameter pore membranes. The extrusion was performed at room temperature or at 65°C.

Table 18. Protocol optimization for extrusion loading of DNA (plasmids, ASOs); parameters and values used as variables for sensitivity analysis.

| MEV concentration (per ml) | DNA concentration (μg/ml) | temperature | extrusion cycles | membrane pore diameter (nm) |
|---|---|---|---|---|
| 4.00E+08<br>9.00E+08<br>4.00E+09 | 0.2 | | 10 | 100 |

(continued)

| MEV concentration (per ml) | DNA concentration (μg/ml) | temperature | extrusion cycles | membrane pore diameter (nm) |
|---|---|---|---|---|
| 4.00E+10<br>6.00E+10<br>8.00E+10<br>9.00E+10 | 1.0<br>2.0<br>20 | RT | 11<br>20 | 200 |

Table 19. Protocol optimization for extrusion loading of RNA (mRNA, siRNA, miRNA); parameters and values used as variables for sensitivity analysis.

| MEV concentration (per ml) | RNA concentration (μg/ml) | temperature | extrusion cycles | membrane pore diameter (nm) |
|---|---|---|---|---|
| 4.00E+08<br>9.00E+08<br>4.00E+09<br>7.00E+09<br>1.50E+10<br>4.00E+10<br>6.00E+10<br>8.00E+10<br>9.00E+10 | 0.2<br>1.0<br>2.0<br>20 | RT<br>65°C | 10<br>11<br>20 | 100<br>200 |

Table 20. Protocol optimization for extrusion loading of proteins (oligopeptides, polypeptides); parameters and values used as variables for sensitivity analysis.

| MEV concentration (per ml) | protein concentration (μg/ml) | temperature | extrusion cycles | membrane pore diameter (nm) |
|---|---|---|---|---|
| 6.00E+08<br>6.00E+09<br>1.50E+10<br>6.00E+10<br>9.00E+10 | 0.1<br>0.5<br>1.0<br>2.0<br>10<br>20 | RT 65°C | 10<br>11<br>20 | 50<br>100<br>200 |

**E. Sonication and extrusion-assisted active loading (SEAL) of biomolecules (*e.g.*, proteins, peptides, mRNA, ASOs, plasmids, complexes) loading mRNA and GFP as exemplary biomolecules**

Purified MEVs, as described in Example 1, were diluted in PBS to a specific concentration (10^11). Next, different concentrations of cargos (2 or 20 μg/ml for mRNA; 2 or 20 μg/ml for GFP) were added to the MEV suspension (total volume of 500-600 μl). The mixture of MEVs and the cargo was sonicated for 8 min (30 sec on/30 sec off, on ice) at 20% amplitude by a Fisherbrand Model 50 Sonic Dismembrator (frequency: 20 kHz, wattage: 50 W). Then the sonicated MEVs were further extruded with the cargo through 100 nm polycarbonate (PC) membranes for ten cycles at room temperature.

**Table 21.** Protocol optimization for SEAL loading of RNA (mRNA, siRNA, miRNA); parameters and values used as variables for sensitivity analysis.

| MEV concentration (per ml) | RNA concentration (μg/ml) | amplitude (Pa) | cycle timing | number of cycles | temperature | extrusion cycles (per membrane) | membrane pore diameter (nm) |
|---|---|---|---|---|---|---|---|
| 1.50E+10 | 0.2<br>2.0<br>20 | 20 | 30 s ON, 30 s OFF on ice | 8 | RT | 9<br>10 | 200, followed by 100 |

Table 22. Protocol optimization for SEAL loading of proteins (oligopeptides, polypeptides); parameters and values used as variables for sensitivity analysis.

| MEV concentration (per ml) | protein concentration (μg/ml) | amplitude (Pa) | cycle timing | number of cycles | temperature | extrusion cycles (per membrane) | membrane pore diameter (nm) |
|---|---|---|---|---|---|---|---|
| 1.50E+10 | 2.0<br>20 | 20 | 30 s ON, 30 s OFF on ice | 8 | RT | 10 | 100 |

**F. Enzymatic activity of MEV-loaded catalase**

The loading efficiency for MEV formulations loaded as above was assessed by catalase activity assay using hydrogen peroxide decomposition. 1 mL of PBS (pH 7.4) was mixed into a quartz cuvette with 1-4 μL $H_2O_2$ (7.5-30% v/w) and 2 μL of catalase (0.06-0.5 mg/ml) or samples of cargo-loaded MEVs, respectively. Enzymatic activity was measured by monitoring the absorbance at 240 nm using Lambda 25 UV VIS Spectrophotometer (Perkin Elmer Instruments). Catalase stability in the loaded MEVs was evaluated by incubation with pronase (0.1-0.2 mg/mL) for 3 hours at 37°C. The samples were subsequently assayed for catalytic activity as described above. Stability of catalase was expressed as the residual activity vs. initial activity of catalase.

**G. Active dialysis of biomolecules (*e.g.*, proteins, peptides, mRNA, ASOs, plasmids, complexes) using catalase or rhodamine B as exemplary biomolecules**

Hypotonic dialysis is performed by transferring MEVs and rhodamine B (Sigma-Aldrich) into dialysis membranes (cellulose ester, molecular weight cut-off 100-500 Da, Spectrum Labs) placed in 200 mL of 10 mM phosphate buffer (pH 7.4) and stirred at room temperature for 4 h. Samples are subsequently purified from free biomolecules (cargo) molecules using 300 kDa molecular weight cut-off centrifuge filters with washing with 500 μL of TE buffer (10 mM Tris, pH 7.5 and 1 mM EDTA, pH 8.0) and centrifuging at 5,000 g at 4°C for 5 min, repeated three times.

Hypotonic dialysis is performed by transferring MEVs and catalase into dialysis membranes (cellulose ester, molecular weight cut-off 100-500 Da, Spectrum Labs) placed in 200 mL of 10 mM phosphate buffer (pH 7.4) and stirred at room temperature for 4 h. Samples are subsequently purified from free cargo molecules using 300 kDa molecular weight cut-off centrifuge filters with washing with 500 μL of TE buffer (10 mM Tris, pH 7.5 and 1 mM EDTA, pH 8.0) and centrifuging at 5,000 g at 4°C for 5 min, repeated three times.

**H. Electroporation of biomolecules (*e.g.*, proteins, peptides, mRNA, ASOs, plasmids, complexes) using plasmid DNA (pDNA) and mRNA as exemplary biomolecules**

Purified MEVs, as described in Example 1, were diluted in PBS to a specific concentration (10^8, 10^9 or 10^10). Next, different concentrations of cargos (1, 2, 5, 10 or 20 μg/ml for pDNA; 2 and 20 μg/ml for mRNA) were added to the MEV suspension (total volume of 110-150 μl). The mixture of MEVs and the cargo was transferred into a 100 μl electroporation cuvette and placed into a Super Electroporator NEPA 21 (NEPAGENE) device. The following parameters were set for each electroporation: the voltage (50 V, 100 V, or 200 V), the pulse length (5 ms, 10 ms, or 15 ms), the pulse interval (set to 50 ms), the decay rate (set to 10%), the polarity (set to positive), the number of pulse (1, 9 or 15), and the presence or absence

of a transfer pulse (5 pulses of 20 V during 50 ms with 50 ms interval and 10 % decay rate). To assess aggregation, 20 mM of EDTA or 10 mM of citrate have been added before electroporation for each condition.

Table 23. Protocol optimization for electroporation loading of DNA (plasmids, ASOs); parameters and values used as variables for sensitivity analysis.

| MEV concentration (per ml) | DNA concentration (μg/ml) | voltage (V) | length (ms) | number of pulses | transfer pulse(s) |
|---|---|---|---|---|---|
| 8.00E+08 | 0.2 | | | | |
| 9.00E+08 | 1.0 | 50 | 5 | 1 | 0 |
| 1.50E+09 | 2.0 | 100 | 10 | 9 | 1 |
| 8.00E+09 | 10 | 200 | 15 | | 6 |
| 9.00E+09 | 20 | | | | |
| 1.50E+10 | | | | | |
| 6.00E+10 | | | | | |
| 8.00E+10 | | | | | |
| 9.00E+10 | | | | | |
| 8.00E+11 | | | | | |
| 9.00E+11 | | | | | |

Table 24. Protocol optimization for electroporation loading of RNA (mRNA, siRNA, miRNA); parameters and values used as variables for sensitivity analysis.

| MEV concentration (per ml) | RNA concentration (μg/ml) | voltage (V) | length (ms) | number of pulses | transfer pulse(s) |
|---|---|---|---|---|---|
| 8.00E+08 | | | | | |
| 9.00E+08 | | | | | |
| 1.50E+09 | | | | | |
| 8.00E+09 | 0.2 | | | | |
| 9.00E+09 | 0.1 | 50 | 5 | | 0 |
| 1.50E+10 | 2.0 | 100 | 10 | 1 | 1 |
| 6.00E+10 | 10 | 200 | 15 | 9 | 6 |
| 8.00E+10 | 20 | | | | |
| 9.00E+10 | | | | | |
| 8.00E+11 | | | | | |
| 9.00E+11 | | | | | |

Table 25. Protocol optimization for electroporation loading of proteins (oligopeptides, polypeptides); parameters and values used as variables for sensitivity analysis.

| MEV concentration (per ml) | protein concentration (μg/ml) | voltage (V) | length (ms) | number of pulses | transfer pulse(s) |
|---|---|---|---|---|---|
| | | | 5 | 1 | 0 |
| 6.00E+10 | 1.0 | 100 | 10 | 9 | 1 |
| | 10 | | 15 | | 6 |

**I. DNase Treatment:**

After each loading method, a part of the samples had undergone a treatment with a Deoxyribonuclease I (DNase) (10104159001, Roche) to eliminate all free nucleic acids (non-encapsulated by the loading). The DNase and a DNase buffer (Tris-HCl 10 mM, $MgCl_2$ 2.5 mM, $CaCl_2$ 0.5 mM, pH 7.6) were mixed with the sample and incubated for 40 minutes in an incubator at 37°C. Finally, Ethylenediaminetetraacetic acid (EDTA) (20 mM) was added to inhibit DNase action.

Samples were then stored at 4°C.

**J. RNase Treatment:**

After each loading method, a portion of the samples were treated with ribonuclease I (RNase) (EN0601, Thermo Scientific) to remove any free (non-loading encapsulated) ribonucleic acids. The RNase was mixed with the sample and incubated for 15 minutes in a 37°C incubator. Finally, SUPERase-In (AM2694, Invitrogen), an RNAase inhibitor, was added to inhibit the action of RNase. The samples were then incubated for 4 hours in a 37°C incubator. Finally, the samples were stored at 4°C.

**EXAMPLE 4**

**MEV characterization after loading the biomolecule cargo**

**A. DNA extraction:**

QIAprep Spin Miniprep Kit (QIAGEN) was used to extract nucleic acid encapsulated by the MEVs according to the manufacturer's recommendations. At the end of the protocol, nucleic acids were eluted in 50 μL of nuclease-free water.

**B. RNA extraction:**

The RNeasy Micro Kit (50) (QIAGEN) was used to extract the ribonucleic acid encapsulated by the MEVs according to the manufacturer's recommendations. At the end of the protocol, the nucleic acids were eluted in 14 μL of nuclease-free water.

**C. Reverse Transcription:**

RNA was isolated from loading methods of MEVs with CleanCap mRNA coding for eGFP (Tebu-bio) (to which 50 ng of an RNA (mCherry (Tebu-bio)) has been added) by the single-step purification method with RNeasy Micro Kit (ref No. 74004, Qiagen GmbH, Germany) described by the manufacturer's protocol (Qiagen-RNeasy Micro Handbook). At the end of the protocol, the nucleic acids were eluted in 14 μL of nuclease-free water.

Reverse transcription and real-time PCR: Quantitative RT-PCR (RT-qPCR) was performed as follows: 4μl of extracted total RNA was reverse transcribed using SuperScript™ II Reverse Transcriptase (ThermoFisher Scientific cat. No. 18064-071, manufacturer's protocol: Part no. 18064.pps, MAN0001342) in a 16 μl reaction volume (constituted with hexamer random primer, dNTP Mix, Buffer, DTT, pure RNA, SuperScript™ II RT and RnaseOut). The reaction was mixed by very gentle pipetting to ensure that all reagents were thoroughly mixed. After mixing Hexamer Random primer, dNTP Mix, RNA and water, samples were spun down and incubated for 5 minutes at 65°C, put on ice and finally incubated 12 minutes at 25°C where buffer, DTT, RnaseOut and SuperScript™ II RT were added. Then incubate the reaction at 42°C for 50 min. For the last step of the mRNA reverse transcription protocol, the Reverse Transcriptase was heat-inactivated for 15 minutes at 70°C. The obtained volume of cDNA after RT was 16 μl.

After the RT reaction, 56μl of $H_2O$ were added to the sample to obtain in total a 72μl cDNA starting solution per sample. Before Real-time PCR measurements, the needed dilution factor of the 72μl cDNA starting solution was determined via prescreening experiments as X1000. Defining the right dilution factor ensures that the measured Cq output values will have values between 20 and 30. This Cq 20-30 range assures better linearity and avoids the signal's noise region which is expected to start at Cqs higher than 30.

**D. qPCR:**

To quantify the plasmid extracted from the MEVs, quantitative Polymerase Chain Reaction (qPCR) have been performed. DNA concentration was measured in the samples using a NanoDrop™ 2000 (Thermo Fischer Scientific,) and the samples were diluted if necessary to be correctly amplified during the qPCR (volumes for each condition are listed in annexes). A plasmid range was realized from 0.4 ng x $10^{-6}$ ng to 5.1 x $10^{-6}$ ng of pDNA. A master mix was prepared with 5 μL of PowerUp™ SYBR™ Green per well (A25776, Fisher Scientific™ Applied Biosystems™), 0.5 μL of forward primer per well (concentration = 10 μM), and 0.5 μL of reverse primer per well (concentration = 10 μM). Primers complementary of the resistance gene sequence of the pDNA have been previously designed to amplify only the plasmid of interest (primers sequences are in annexes). A MicroAmp™ Optical 384-Well reaction plate (10411785, Fisher Scientific™, Applied Biosystems™) was used, 6 μL of master mix were put in each well, followed by 4 μL of the sample. Two negative controls were made: nuclease-free water with SYBR™ green, and nuclease-free water with SYBR™ green and primers. All

conditions were done in duplicates. The plate was sealed and briefly centrifuged. The thermocycler was a CFX384 Touch Real Time PCR detection system (Bio-Rad). The three-step cycling, followed by the melt curve protocols are summarized in the tables below.

Table 26. Three-step cycling protocol for qPCR

| Step | Temperature (°C) | Time | Number of cycles |
|---|---|---|---|
| Dual lock DNA polymerase | 95 | 10 minutes | 1 |
| Denaturation | 95 | 15 seconds | 40 |
| Annealing | 60 | 1 minute | |

Table 27. Melt curve protocol for qPCR

| Step | Ramp rate | Temperature (°C) | Time |
|---|---|---|---|
| 1 | 1.6°C/second | 95 | 15 seconds |
| 2 | 1.6°C/second | 60 | 1 minute |
| 3 | 0.15°C/second | 95 | 15 seconds |

For the RNA detection after RT, real-time qPCR was performed in technical duplicates in qPCR plates with a rapid thermal cycler system (LightCycler® 480 II, 384 (well)). The protocol was the following: 3 µl cDNA (with defined dilution factor of X1000 were mixed with one forward and one reverse primer (added at optimized concentrations usually between 200 nM and 1.0 µM), mix Takyon® No ROX SYBR 2X MasterMix blue dTTP (Eurogentec, UF-NSMT-B0701) with dNTPs, $MgCl_2$, Taq DNA polymerase and buffer, constituting a volume of 10 µl in total. Then, this mix was placed in LightCycler plates.

The amplification protocol started with an initial incubation at 95°C for 10 min (serving for activation of Taq DNA polymerase), followed by 45 amplification cycles composed of two steps: step I) a 95°C denaturation for 10 s, step II) 60°C primer annealing and extension for 40 s, (the detection of the fluorescent product was performed at the end of this 60°C extension period with a single acquisition mode). The amplification protocol is followed by the step to perform the melting curve with one cycle of 95°C denaturation for 5 s, 60°C annealing for 40 s, and a 95°C end point (achieved by a temperature raise with a ramp rate of 0.04°C/s from 60°C to 95°C and accompanied by continuous detection of the fluorescent product). The amplification protocol ended by cooling at 37°C for 10 seconds.

For plasmid quantification (dilution by 1000), the same protocol (mix and cycles) of the LightCycler® is used as with cDNA quantification. Only the primers are modified.

The qPCR's primers and their concentrations are listed below: RNA Primers (SEQ ID NOs: 66 and 67):

- eGfP(1-1)For3 AGCAAAGACCCCAACGAGA
- eGfP(1-1)Rev3 TCGTCCATGCCGAGAGTG
- eGfP(1-1) For3Rev3: 400 nM

RNA reference Primers (SEQ ID NO:68 and 69):

- mCherry(1-1)F1: GACCACCTACAAGGCCAAGA
- mCherry(1-1)R1: CCGCTCGTACTGCTCCAC
- mCherry(1-1) FR1: 500 nM

Plasmid (pDNA) pcDNA3.1+ Primers (SEQ ID NOs:70 and 71):

- Left primer PCDNA3.1+1: GACCACCAAGCGAAACATGG

(continued)

| | |
|---|---|
| • Right primer PCDNA3.1+1: | CCATGGGTCACGACGAGATC |
| • PCDNA3.1 LR1(Left primer PCDNA3.1+1, Right primer PCDNA3.1+1): | 700 nM |

**Controls for RT-qPCR assays**

To confirm the correct functioning of our RT-qPCR analysis, two different controls were included in the experiments: a negative control and an efficacy and quantification control. All two controls were applied on each qPCR plate.

One negative control, consisting of RNA free sample ($H_2O$ only), was added to each qPCR plate and mixed with the according primers on the qPCR plate. This negative $H_2O$ control allowed the estimation of the Cq background levels for each amplicon, that hybridizing primers create.

Efficacy and quantification controls were employed on each qPCR plate to control the primers' annealing efficacies at different cDNA concentrations and quantified.

For the "RNA efficacy and quantification control" sample, a mix of CleanCap mRNA coding for eGFP was prepared. Subsequently, 9 different concentrations (to which 50 ng of an RNA (mCherry (Tebu-bio)) has been added) were prepared with RNAse/DNase free water (1000 ng, 500 ng, 100 ng, 50 ng, 10 ng, 5 ng, 1 ng, 0.5 ng, 0.1 ng), extracted, reverse transcribed, and then diluted (identically as the samples, dilution factor of X1000) and mixed with the according primers used on a qPCR plate.

Efficacy and quantification controls were employed on each qPCR plate to control the primers' annealing efficacies at different DNA concentrations and quantified.

For the "DNA efficacy and quantification control" sample, a mix of Plasmid (pDNA) pcDNA3.1+ was prepared. Subsequently, 9 different concentrations were prepared with RNAse/Dnase-free water (1000 ng, 500 ng, 100 ng, 50 ng, 10 ng, 5 ng, 1 ng, 0.5 ng, 0.1ng), extracted and diluted (identically as the samples, dilution factor of X1000) and mixed with the according primers used on a qPCR plate.

**E. Fluorescence readout by ZetaView® nanoparticle tracking analysis (NTA) instrument.**

ZetaView® NTA instrument (engine) from Particle Metrix is a Nanoparticle Tracking Analysis engine for measuring hydrodynamic particle size, zeta potential, concentration and fluorescence. It was calibrated before the experiment according to the manufacturer's recommendations with polystyrene beads. The PBS used for the day's experiments was evaluated with the ZetaView® engine (normal average number of particles on screen in PBS: 0-5). Samples were diluted with PBS to be measured by ZetaView® within the manufacturer's recommended reading range (50-200 particles per frame). Dilutions were made in PBS, then the sample was vortexed and placed in a 1 mL syringe for the analysis.

The samples were analyzed with ZetaView® engine in scatter mode (laser 488 nm) to determine the number and size distribution of the particles. The samples were then analyzed with the ZetaView® engine in fluorescence using a laser at 488 nm and a fluorescence filter at 500 nm at different percentages of sensitivity (95% or 90% ). The analog view of the ZetaView® engine was activated during the fluorescence analysis to visualize the background noise.

**F. Enzymatic activity of catalase-loaded MEVs**

The loading efficiency for MEV formulations loaded as above was assessed by catalase activity assay using hydrogen peroxide decomposition. Briefly, 1 mL of PBS (pH 7.4) was mixed into a quartz cuvette with 1-4 μL $H_2O_2$ (7.5-30% v/w) and 2 μL of catalase (0.06-0.5 mg/ml) or samples of cargo-loaded MEVs, respectively. Enzymatic activity was measured by monitoring the absorbance at 240 nm using Lambda 25 UV VIS Spectrophotometer (PerkinElmer Instruments). Catalase stability in the loaded MEVs was evaluated by incubation with pronase (0.1-0.2 mg/mL) for 3 hours at 37°C. The samples were subsequently assayed for catalytic activity as described above. Stability of catalase was expressed in the residual activity vs. initial activity of catalase.

**G. Quantification of the rhodamine B cargo**

The cargo carrying MEVs loaded as above were lysed to release the cargo by adding 5 μL of 0.4% SDS into 95 μL of MEVs mixture, thorough pipetting and incubation in a thermocycler for 15 min at 85°C. Next, the samples were transferred into black-walled clear bottom non-treated polystyrene 96-well plates. Control samples contained 0.1, 0.5, 1.0, 5.0 nmol of rhodamine B alone. The sample fluorescence was measured using a SpectraMax® fluorescence microplate reader (Molecular Devices, USA) with excitation at 540 nm and emission at 625 nm.

**H. Quantification of the siRNA cargo**

The cargo loaded MEVs were lysed as above. Next, the siRNA cargo was detected using Quant-iT® PicoGreen® Assay kit (Life Technologies), able to quantify nucleic acids with picogram sensitivity. Briefly, 100 μL of dye reagent working solution was added to 100 μL of each sample to make final volume of 200 μl. A control sample prepared in the same buffer contained 10 pmol of siRNA alone. Samples were transferred into black-walled clear bottom non-treated polystyrene 96-well plates and incubated in the dark for 10 min at room temperature. The sample fluorescence was measured using a SpectraMax® fluorescence microplate reader (Molecular Devices, USA) with excitation at 480 nm and emission at 520 nm.

**I. Quantification of total proteins**

Total proteins were quantified by BCA or Bradford test.

**J. Statistical analysis:**

Screening and optimized experimental designs were generated with NemrodW software. The results have been analyzed using R Studio (1.4.1717 version) and Prism (8.3 version). Significance of the variables compared to the controls was evaluated with unpaired parametric t tests. "ns" stands for "not significant" meaning a p-value superior to 0.05, "*" stands for a p-value inferior to 0.05, "**" stands for a p-value inferior to 0.01, and "***" stands for a p-value inferior to 0.001. Loading efficiency were calculated based on the pDNA quantity obtained by qPCR compared to the initial pDNA quantity for each condition. Linear regression modeling was realized to evaluate the effect of tested variables on plasmid encapsulation.

**K. Exo-loading Efficiency**

The efficiency of exo-loading of MEV obtained, purified, characterized, loaded, and internalization of payload determine as described in examples 1-4 is shown in the following Table 28. The efficiency of exo-loading using the different loading methods for different payloads is calculated using the parameters: (i) loading efficacy, and/or (ii) loading capacity, or (iii) the percentage of loaded MEV.

Table 28:

| Payload | Method | Tested Conditions | Loading Efficiency | Loading Capacity |
|---|---|---|---|---|
| **DNA (plasmids / ASOs)** | **A** Incubation | 9 | (0) | (0) |
| | **B** Saponification | 12 | (+) | (++) |
| | **C** Freeze thaw cycle | 28 | (++) | (+++) |
| | **D** Electroporation | 46 | (+++) | (+++) |
| | **E** Sonication | 23 | (++) | (++) |
| | **F** Extrusion | 25 | (+) | (+) |
| | **G** Serial Extrusion | 12 | (+) | (+) |
| | **H** SEAL | (-) | ND | ND |
| **RNA (mRNA / siRNA / miRNA)** | **A** Incubation | (-) | ND | ND |
| | **B** Saponification | (-) | ND | ND |
| | **C** Freeze thaw cycle | 6 | (++) | (++) |
| | **D** Electroporation | 12 | (++) | (++) |
| | **E** Sonication | 6 | (++) | (++) |
| | **F** Extrusion | 24 | (++) | (++) |
| | **G** Serial Extrusion | 21 | (+++) | (+++) |
| | **H** SEAL | 10 | (0) | (0) |

| Payload | Method | Tested Conditions | Loading Efficiency | % of positive (fluorescent) MEV |
|---|---|---|---|---|
| | **A** Incubation | 9 | (+) | (+) |
| | **B** Saponification | 12 | (++) | (++) |

(continued)

| Payload | Method | Tested Conditions | Loading Efficiency | % of positive (fluorescent) MEV |
|---|---|---|---|---|
| **Proteins (peptides / GFP / other proteins)** | **C** Freeze thaw cycle | 12 | (++) | (++) |
| | **D** Electroporation | 12 | (++) | (++) |
| | **E** Sonication | 40 | (+++) | (+++) |
| | **F** Extrusion | 14 | (++) | (++) |
| | **G** Serial Extrusion | 14 | (++) | (++) |
| | **H** SEAL | 15 | (++) | (++) |

Key: Loading (0) very low; (+) low; (++) acceptable; (+++) best results; ND not determined, where the parameters are as follows:

- Internalized payload is the total amount of payload molecules (DNA, RNA, protein, or other molecules) measure into the MEVs after the loading reaction. The internalized payload is determined by a specific quantification method (such as qPCR, RT-qPCR, determination of proteins, and other), after the elimination of the remaining payload molecules in the loading reaction medium at the end of reaction by specific enzymatic treatment (such as DNAse, RNAse, protease, other).
- Copy number of payloads: Number of copies of payload molecule (DNA or RNA) in the either initial amount or the internalized payload amount. The number of copies is obtained using the following calculation:

$$\text{Number of copies}=(\text{ng}\times[6.022\text{Exp}23])/(\text{length}\ \times[1\text{Exp}9]\times 650)$$

where:

ng is the amount of nucleic acids (plasmid, RNA.)
6.022Exp23 = Avogadro's number.
Length is the length of your DNA fragment in base pairs multiply by 1000 (kb) Multiply by 1Exp9 to convert to nanograms.

- Loading efficiency is the percentage of the initial amount of payload molecule, in the loading reaction medium at the initial timepoint, internalized into extracellular vesicles from microalgae (MEVs).
- Loading capacity is the copy number of payload internalized per MEV. The loading capacity calculated as the ratio between the internalized payload copies and the number of MEVs in the reaction.
- As an example: if in a loading reaction are initially 3.6Exp10 copy of DNA and 1Exp9 MEVs, and by qPCR is determined that 1.8Exp9 copies of DNA are internalized into the MEVs. For this loading reaction the loading parameter are the following:

$$-\quad \text{loading efficiency} = (1{,}8\text{Exp}9\ \text{DNA copies}/3{,}6\text{Exp}10\ \text{DNA copies})\ {*}100 = 5\%$$

- loading capacity = 1,8Exp9 DNA copies/1Exp9 MEV = 1.8 DNA copies / MEV.

- Percentage of loading MEVs: is the number of fluorescent MEV per total number of MEVs per 100. The fluorescence is determined using ZetaView® device at f90 and f95 are sensitivity values. Sensitivity values are expressed in arbitrary units ranges from 0 to 100. It represents the sensitivity of the camera sensor in the ZetaView® device. This sensitivity setting is comparable to the sensitivity setting of a camera used in digital photography. The number of detected particles in the field is associated to the sensitivity camera: increase of sensitivity -> increase of the number of particles detected in each sample. Sensitivity is calibrated before the measurement based on the negative control chose the highest sensitivity for which no event is detectable.

**Table 29,** below, shows different payloads loaded into the MEV with acceptable results.

**Table 29:**

| Payload | | SEQUENCE ID/formula | SEQ ID NO. |
|---|---|---|---|
| Proteins & Peptides | GFP | MSKGEELFTGVVPILVELDGDVNGHKFSVSGEGEGDATYGKLTLKFICTTGKLPVPWPTLVTTFSYGVQCFSRYPDHMKQHDFFKSAMPEGYVQERTIFFKDDGNYKTRAEVKFEGDTLVNRIELKGIDFKEDGNILGHKLEYNYNSHNVYIMADKQKNGIKVNFKIRHNIEDGSVQLADHYQQNTPIGDGPVLLPDNHYLSTQSALSKDPNEKRDHMVLLEFVTAAGITHGMDELYK | 111 |
| | luciferase | MEDAKNIKKGPAPFYPLEDGTAGEQLHKAMKRYALVPGTIAFTDAHIEVNITYAEYFEMSVRLAEAMKRYGLNTNHRIVVCSENSLQFFMPVLGALFIGVAVAPANDIYNERELLNSMNISQPTVVFVSKKGLQKILNVQKKLPIIQKIIIMDSKTDYQGFQSMYTFVTSHLPPGFNEYDFVPESFDRDKTIALIMNSSGSTGLPKGVALPHRTACVRFSHARDPIFGNQIIPDTAILSVVPFHHGFGMFTTLGYLICGFRVVLMYRFEEELFLRSLQDYKIQSALLVPTLFSFFAKSTLIDKYDLSNLHEIASGGAPLSKEVGEAVAKRFHLPGIRQGYGLTETTSAILITPEGDDKPGAVGKVVPFFEAKVVDLDTGKTLGVNQRGELCVRGPMIMSGYVNNPEATNALIDKDGWLHSGDIAYWDEDEHFFIVDRLKSLIKYKGYQVAPAELESILLQHPNIFDAGVAGLPDDDAGELPAAVVVLEHGKTMTEKEIVDYVASQVTTAKKLRGGVVFVDEVPKGLTGKLDARKIREILIKAKKGGKSKL | 112 |
| | catalase | MADSRDPASDQMQHWKEQRAAQKADVLTTGAGNPVGDKLNVITVGPRGPLLVQDVVFTDEMAHFDRERIPERVVHAKGAGAFGYFEVTHDITKYSKAKVFEHIGKKTPIAVRFSTVAGESGSADTVRDPRGFAVKFYTEDGNWDLVGNNTPIFFIRDPILFPSFIHSQKRNPQTHLKDPDMVWDFWSLRPESLHQVSFLFSDRGIPDGHRHMNGYGSHTFKLVNANGEAVYCKFHYKTDQGIKNLSVEDAARLSQEDPDYGIRDLFNAIATGKYPSWTFYIQVMTFNQAETFPFNPFDLTKVWPHKDYPLIPVGKLVLNRNPVNYFAEVEQIAFDPSNMPPGIEASPDKMLQGRLFAYPDTHRHRLGPNYLHIPVNCPYRARVANYQRDGPMCMQDNQGGAPNYYPNSFGAPEQQPSALEHSIQYSGEVRRFNTANDDNVTQVRAFYVNVLNEEQRKRLCENIAGHLKDAQIFIQKKAVKNFTEVHPDYGSHIQALLDKYNAEKPKNAIHTFVQSGSHLAAREKANL | 113 |

(continued)

| Payload | | SEQUENCE ID/formula | SEQ ID NO. |
|---|---|---|---|
| | ovalbumin | MGSIGAASMEFCFDVFKELKVHHANENIFYCPIAIMSALA MVYLGAKDSTRTQINKVVRFDKLPGFGDSIEAQCGTSVN VHSSLRDILNQITKPNDVYSFSLASRLYAEERYPILPEYLQC VKELYRGGLEPINFQTAADQARELINSWVESQTNGIIRNVL QPSSVDSQTAMVLVNAIVFKGLWEKAFKDEDTQAMPFRV TEQESKPVQMMYQIGLFRVASMASEKMKILELPFASGTMS MLVLLPDEVSGLEQLESIINFEKLTEWTSSNVMEERKIK VYLPRMKMEEKYNLTSVLMAMGITDVFSSSANLSGISSAE SLKISQAVHAAHAEINEAGREVVGSAEAGVDAASVSEEFR ADHPFLFCIKHIATNAVLFFGRCVSP | 114 |
| | Flagellin peptide (flg22) | QRLSTGSRINSAKDDAAGLQIA | 115 |
| | GUS | MARGSAVAWAALGPLLWGCALGLQGGMLYPQESPSREC KELDGLWSFRADFSDNRRRGFEEQWYRRPLWESGPTVD MPVPSSFNDISQDWRLRHFVGWVWYEREVILPERWTQDL RTRVVLRIGSAHSYAIVWVNGVDTLEHEGGYLPFEADISN LVQVGPLPSRLRITIAINNTLTPTTLPPGTIQYLTDTSKYPK GYFVQNTYFDFFNYAGLQRSVLLYTTPTTYIDDITVTTSVE QDSGLVNYQISVKGSNLFKLEVRLLDAENKVVANGT GTQGQLKVPGVSLWWPYLMHERPAYLYSLEVQLTAQTSL GPVSDFYTLPVGIRTVAVTKSQFLINGKPFYFHGVNKHED ADIRGKGFDWPLLVKDFNLLRWLGANAFRTSHYPYAEEV MQMCDRYGIVVIDECPGVGLALPQFFNNVSLHHHMQVM EEVVRRDKNHPAVVMWSVANEPASHLESAGYYLKMVIA HTKSLDPSRPVTFVSNSNYAADKGAPYVDVICLNSYYSW YHDYGHLELIQLQLATQFENWYKKYQKPIIQSEYGAETI AGFHQDPPLMFTEEYQKSLLEQYHLGLDQKRRKYVVGEL IWNFADFMTEQSPTRVLGNKKGIFTRQRQPKSAAFLLRER YWKIANETRYPHSVAKSQCLENSLFT | 116 |
| DNA | Plasmid pcDNA3.1 | GACGGATCGGGAGATCTCCCGATCCCCTATGGTGCACTCTCA GTACAATCTGCTCTGATGCCGCATAGTTAAGCCAGTATCTGCT CCCTGCTTGTGTGTTGGAGGTCGCTGAGTAGTGCGCGAGCAA AATTTAAGCTACAACAAGGCAAGGCTTGACCGACAATTGCAT GAAGAATCTGCTTAGGGTTAGGCGTTTTGCGCTGCTTCGCGAT GTACGGGCCAGATATACGCGTTGACATTGATTATTGACTAGT TATTAATAGTAATCAATTACGGGGTCATTAGTTCATAGCCCAT ATATGGAGTTCCGCGTTACATAACTTACGGTAAATGGCCCGC CTGGCTGACCGCCCAACGACCCCCGCCCATTGACGTCAATAA TGACGTATGTTCCCATAGTAACGCCAATAGGGACTTTCCATTG ACGTCAATGGGTGGAGTATTTACGGTAAACTGCCCACTTGGC AGTACATCAAGTGTATCATATGCCAAGTACGCCCCCTATTGA CGTCAATGACGGTAAATGGCCCGCCTGGCATTATGCCCAGTA | 117 |

(continued)

| Payload | | SEQUENCE ID/formula | SEQ ID NO. |
|---|---|---|---|
| | | CATGACCTTATGGGACTTTCCTACTTGGCAGTACATCTACGTATTAGTCATCGCTATTACCATGGTGATGCGGTTTTGGCAGTACATCAATGGGCGTGGATAGCGGTTTGACTCACGGGGATTTCCAAGTCTCCACCCCATTGACGTCAATGGGAGTTTGTTTTGGCACCAAAATCAACGGGACTTTCCAAAATGTCGTAACAACTCCGCCCCATTGACGCAAATGGGCGGTAGGCGTGTACGGTGGGAGGTCTATATAAGCAGAGCTCTCTGGCTAACTAGAGAACCCACTGCTTACTGGCTTATCGAAATTAATACGACTCACTATAGGGAGACCCAAGCTGGCTAGCGTTTAAACTT<br><br>AAGCTTGGTACCGAGCTCGGATCCACTAGTCCAGTGTGGTGGAATTCTGCAGATATCCAGCACAGTGGCGGCCGCTCGAGTCTAGAGGGCCCGTTTAAACCCGCTGATCAGCCTCGACTGTGCCTTCTAGTTGCCAGCCATCTGTTGTTTGCCCCTCCCCCGTGCCTTCCTTGACCCTGGAAGGTGCCACTCCCACTGTCCTTTCCTAATAAAATGAGGAAATTGCATCGCATTGTCTGAGTAGGTGTCATTCTATTCTGGGGGGTGGGGTGGGGCAGGACAGCAAGGGGGAGGATTGGGAAGACAATAGCAGGCATGCTGGGGATGCGGTGGGCTCTATGGCTTCTGAGGCGGAAAGAACCAGCTGGGGCTCTAGGGGGTATCCCCACGCGCCCTGTAGCGGCGCATTAAGCGCGGCGGGTGTGGTGGTTACGCGCAGCGTGACCGCTACACTTGCCAGCGCCCTAGCGCCCGCTCCTTTCGCTTTCTTCCCTTCCTTTCTCGCCACGTTCGCCGGCTTTCCCCGTCAAGCTCTAAATCGGGGGCTCCCTTTAGGGTTCCGATTTAGTGCTTTACGGCACCTCGACCCCAAAAAACTTGATTAGGGTGATGGTTCACGTAGTGGGCCATCGCCCTGATAGACGGTTTTTCGCCCTTTGACGTTGGAGTCCACGTTCTTTAATAGTGGACTCTTGTTCCAAACTGGAACAACACTCAACCCTATCTCGGTCTATTCTTTTGATTTATAAGGGATTTTGCCGATTTCGGCCTATTGGTTAAAAAATGAGCTGATTTAACAAAAATTTAACGCGAATTAATTCTGTGGAATGTGTGTCAGTTAGGGTGTGGAAAGTCCCCAGGCTCCCCAGCAGGCAGAAGTATGCAAAGCATGCATCTCAATTAGTCAGCAACCAGGTGTGGAAAGTCCCCAGGCTCCCCAGCAGGCAGAAGTATGCAAAGCATGCATCTCAATTAGTCAGCAACCATAGTCCCGCCCCTAACTCCGCCCATCCCGCCCCTAACTCCGCCCAGTTCCGCCCATTCTCCGCCCCATGGCTGACTAATTTTTTTTATTTATGCAGAGGCCGAGGCCGCCTCTGCCTCTGAGCTATTCCAGAAGTAGTGAGGAGGCTTTTTTGGAGGCCTAGGCTTTTGCAAAAAGCTCCCGGGAGCTTGTATATCCATTTTCGGATCTGATCAAGAGACAGGATGAGGATCGTTTCGCATGATTGAACAAGATGGATTGCACGCAGGTTCTCCGGCCGCTTGGGTGGAGAGGCTATTCGGCTATGACTGGGCACAACAGACAATCGGCTGCTCTGATGCCGCCGTGTTCCGGCTGTCAGCGCAGGGGCGCCCGGTTCTTTTTGTCAAGACCGACCTGTCCGGTGCCCTGAATGAACTGCAGGACGAGGCAGCGCGGCTATCGTGGCTGGCCACGACGGGCGTTCCTTGCGCAGCTGTGCTCGACGTTGTCACTGAAGCGGGAAGGGACTGGCTGCTATTGGGCGAAGTGCCGGGGCAGGATCTCCTGTCATCTCACCTTGCTCCTGCCGAGAAAGTATCCATCATGGCTGATGCAATGCGGCGGCTGCATACGCTTGATCCGGCTACCTGCCCATTCGACCACCAAGCGAAACATCGCATCGAGCGAGCACGTACTCGGATGGAAGCCGGTCT | |

(continued)

| Payload | | SEQUENCE ID/formula | SEQ ID NO. |
|---|---|---|---|
| | | TGTCGATCAGGATGATCTGGACGAAGAGCATCAGGGGCTCGC GCCAGCCGAACTGTTCGCCAGGCTCAAGGCGCGCATGCCCGA CGGCGAGGATCTCGTCGTGACCCATGGCGATGCCTGCTTGCC GAATATCATGGTGGAAAATGGCCGCTTTTCTGGATTCATCGA CTGTGGCCGGCTGGGTGTGGCGGACCGCTATCAGGACATAGC GTTGGCTACCCGTGATATTGCTGAAGAGCTTGGCGGCGAATG GGCTGACCGCTTCCTCGTGCTTTACGGTATCGCCGCTCCCGAT TCGCAGCGCATCGCCTTCTATCGCCTTCTTGACGAGTTCTTCT GAGCGGGACTCTGGGGTTCGAAATGACCGACCAAGCGACGC CCAACCTGCCATCACGAGATTTCGATTCCACCGCCGCCTTCTA TGAAAGGTTGGGCTTCGGAATCGTTTTCCGGGACGCCGGCTG GATGATCCTCCAGCGCGGGGATCTCATGCTGGAGTTCTTCGC CCACCCCAACTTGTTTATTGCAGCTTATAATGGTTACAAATAA AGCAATAGCATCACAAATTTCACAAATAAAGCATTTTTTTCA CTGCATTCTAGTTGTGGTTTGTCCAAACTCATCAATGTATCTT ATCATGTCTGTATACCGTCGACCTCTAGCTAGAGCTTGGCGTA ATCATGGTCATAGCTGTTTCCTGTGTGAAATTGTTATCCGCTC ACAATTCCACACAACATACGAGCCGGAAGCATAAAGTGTAA AGCCTGGGGTGCCTAATGAGTGAGCTAACTCACATTAATTGC GTTGCGCTCACTGCCCGCTTTCCAGTCGGGAAACCTGTCGTGC CAGCTGCATTAATGAATCGGCCAACGCGCGGGGAGAGGCGG TTTGCGTATTGGGCGCTCTTCCGCTTCCTCGCTCACTGACTCG CTGCGCTCGGTCGTTCGGCTGCGGCGAGCGGTATCAGCTCAC TCAAAGGCGGTAATACGGTTATCCACAGAATCAGGGGATAAC GCAGGAAAGAACATGTGAGCAAAAGGCCAGCAAAAGGCCAG GAACCGTAAAAAGGCCGCGTTGCTGGCGTTTTTCCATAGGCT CCGCCCCCCTGACGAGCATCACAAAAATCGACGCTCAAGTCA GAGGTGGCGAAACCCGACAGGACTATAAAGATACCAGGCGT TTCCCCCTGGAAGCTCCCTCGTGCGCTCTCCTGTTCCGACCCT GCCGCTTACCGGATACCTGTCCGCCTTTCTCCCTTCGGGAAGC GTGGCGCTTTCTCATAGCTCACGCTGTAGGTATCTCAGTTCGG TGTAGGTCGTTCGCTCCAAGCTGGGCTGTGTGCACGAACCCC CCGTTCAGCCCGACCGCTGCGCCTTATCCGGTAACTATCGTCT TGAGTCCAACCCGGTAAGACACGACTTATCGCCACTGGCAGC AGCCACTGGTAACAGGATTAGCAGAGCGAGGTATGTAGGCG GTGCTACAGAGTTCTTGAAGTGGTGGCCTAACTACGGCTACA CTAGAAGAACAGTATTTGGTATCTGCGCTCTGCTGAAGCCAG TTACCTTCGGAAAAAGAGTTGGTAGCTCTTGATCCGGCAAAC AAACCACCGCTGGTAGCGGTTTTTTTGTTTGCAAGCAGCAGA TTACGCGCAGAAAAAAAGGATCTCAAGAAGATCCT<br><br>TTGATCTTTTCTACGGGGTCTGACGCTCAGTGGAACGAAAAC TCACGTTAAGGGATTTTGGTCATGAGATTATCAAAAAGGATC TTCACCTAGATCCTTTTAAATTAAAAATGAAGTTTTAAATCAA TCTAAAGTATATATGAGTAAACTTGGTCTGACAGTTACCAAT GCTTAATCAGTGAGGCACCTATCTCAGCGATCTGTCTATTTCG TTCATCCATAGTTGCCTGACTCCCCGTCGTGTAGATAACTACG ATACGGGAGGGCTTACCATCTGGCCCCAGTGCTGCAATGATA CCGCGAGACCCACGCTCACCGGCTCCAGATTTATCAGCAATA AACCAGCCAGCCGGAAGGGCCGAGCGCAGAAGTGGTCCTGC AACTTTATCCGCCTCCATCCAGTCTATTAATTGTTGCCGGGAA | |

(continued)

| Payload | | SEQUENCE ID/formula | SEQ ID NO. |
|---|---|---|---|
| | | GCTAGAGTAAGTAGTTCGCCAGTTAATAGTTTGCGCAACGTT GTTGCCATTGCTACAGGCATCGTGGTGTCACGCTCGTCGTTTG GTATGGCTTCATTCAGCTCCGGTTCCCAACGATCAAGGCGAG TTACATGATCCCCCATGTTGTGCAAAAAAGCGGTTAGCTCCTT CGGTCCTCCGATCGTTGTCAGAAGTAAGTTGGCCGCAGTGTT ATCACTCATGGTTATGGCAGCACTGCATAATTCTCTTACTGTC ATGCCATCCGTAAGATGCTTTTCTGTGACTGGTGAGTACTCAA CCAAGTCATTCTGAGAATAGTGTATGCGGCGACCGAGTTGCT CTTGCCCGGCGTCAATACGGGATAATACCGCGCCACATAGCA GAACTTTAAAAGTGCTCATCATTGGAAAACGTTCTTCGGGGC GAAAACTCTCAAGGATCTTACCGCTGTTGAGATCCAGTTCGA TGTAACCCACTCGTGCACCCAACTGATCTTCAGCATCTTTTAC TTTCACCAGCGTTTCTGGGTGAGCAAAAACAGGAAGGCAAAA TGCCGCAAAAAAGGGAATAAGGGCGACACGGAAATGTTGAA TACTCATACTCTTCCTTTTTCAATATTATTGAAGCATTTATCAG GGTTATTGTCTCATGAGCGGATACATATTTGAATGTATTTAGA AAAATAAACAAATAGGGGTTCCGCGCACATTTCCCCGAAAAG TGCCACCTGACGTC | |
| | Plasmid pEGFP-N1 | TAGTTATTAATAGTAATCAATTACGGGGTCATTAGTTCATAGC CCATATATGGAGTTCCGCGTTACATAACTTACGGTAAATGGC CCGCCTGGCTGACCGCCCAACGACCCCCGCCCATTGACGTCA ATAATGACGTATGTTCCCATAGTAACGCCAATAGGGACTTTC CATTGACGTCAATGGGTGGAGTATTTACGGTAAACTGCCCAC TTGGCAGTACATCAAGTGTATCATATGCCAAGTACGCCCCCT ATTGACGTCAATGACGGTAAATGGCCCGCCTGGCATTATGCC CAGTACATGACCTTATGGGACTTTCCTACTTGGCAGTACATCT ACGTATTAGTCATCGCTATTACCATGGTGATGCGGTTTTGGCA GTACATCAATGGGCGTGGATAGCGGTTTGACTCACGGGGATT TCCAAGTCTCCACCCCATTGACGTCAATGGGAGTTTGTTTTGG CACCAAAATCAACGGGACTTTCCAAAATGTCGTAACAACTCC GCCCCATTGACGCAAATGGGCGGTAGGCGTGTACGGTGGGAG GTCTATATAAGCAGAGCTGGTTTAGTGAACCGTCAGATCCGC TAGCGCTACCGGACTCAGATCTCGAGCTCAAGCTTCGAATTC TGCAGTCGACGGTACCGCGGGCCCGGGATCCACCGGTCGCCA CCATGGTGAGCAAGGGCGAGGAGCTGTTCACCGGGGTGGTGC CCATCCTGGTCGAGCTGGACGGCGACGTAAACGGCCACAAGT TCAGCGTGTCCGGCGAGGGCGAGGGCGATGCCACCTACGGCA AGCTGACCCTGAAGTTCATCTGCACCACCGGCAAGCTGCCCG TGCCCTGGCCCACCCTCGTGACCACCCTGACCTACGGCGTGC AGTGCTTCAGCCGCTACCCCGACCACATGAAGCAGCACGACT TCTTCAAGTCCGCCATGCCCGAAGGCTACGTCCAGGAGCGCA CCATCTTCTTCAAGGACGACGGCAACTACAAGACCCGCGCCG AGGTGAAGTTCGAGGGCGACACCCTGGTGAACCGCATCGAGC TGAAGGGCATCGACTTCAAGGAGGACGGCAACATCCTGGGG CACAAGCTGGAGTACAACTACAACAGCCACAACGTCTATATC ATGGCCGACAAGCAGAAGAACGGCATCAAGGTGAACTTCAA GATCCGCCACAACATCGAGGAC<br><br>GGCAGCGTGCAGCTCGCCGACCACTACCAGCAGAACACCCCC ATCGGCGACGGCCCCGTGCTGCTGCCCGACAACCACTACCTG | 118 |

(continued)

| Payload | | SEQUENCE ID/formula | SEQ ID NO. |
|---|---|---|---|
| | | AGCACCCAGTCCGCCCTGAGCAAAGACCCCAACGAGAAGCG CGATCACATGGTCCTGCTGGAGTTCGTGACCGCCGCCGGGAT CACTCTCGGCATGGACGAGCTGTACAAGTAAAGCGGCCGCGA CTCTAGATCATAATCAGCCATACCACATTTGTAGAGGTTTTAC TTGCTTTAAAAAACCTCCCACACCTCCCCCTGAACCTGAAAC ATAAAATGAATGCAATTGTTGTTGTTAACTTGTTTATTGCAGC TTATAATGGTTACAAATAAAGCAATAGCATCACAAATTTCAC AAATAAAGCATTTTTTTCACTGCATTCTAGTTGTGGTTTGTCC AAACTCATCAATGTATCTTAAGGCGTAAATTGTAAGCGTTAA TATTTTGTTAAAATTCGCGTTAAATTTTTGTTAAATCAGCTCA TTTTTTAACCAATAGGCCGAAATCGGCAAAATCCCTTATAAA TCAAAAGAATAGACCGAGATAGGGTTGAGTGTTGTTCCAGTT TGGAACAAGAGTCCACTATTAAAGAACGTGGACTCCAACGTC AAAGGGCGAAAAACCGTCTATCAGGGCGATGGCCCACTACGT GAACCATCACCCTAATCAAGTTTTTTGTGCCGTAAAGCACTAA ATCGGAACCCTAAAGGGAGCCCCCGATTTAGAGCTTGACGGG GAAAGCCGGCGAACGTGGCGAGAAAGGAAGGGAAGAAAGC GAAAGGAGCGGGCGCTAGGGCGCTGGCAAGTGTAGCGGTCA CGCTGCGCGTAACCACCACACCCGCCGCGCTTAATGCGCCGC TACAGGGCGCGTCAGGTGGCACTTTTCGGGGAAATGTGCGCG GAACCCCTATTTGTTTATTTTTCTAAATACATTCAAATATGTA TCCGCTCATGAGACAATAACCCTGATAAATGCTTCAATAATA TTGAAAAAGGAAGAGTCCTGAGGCGGAAAGAACCAGCTGTG GAATGTGTGTCAGTTAGGGTGTGGAAAGTCCCCAGGCTCCCC AGCAGGCAGAAGTATGCAAAGCATGCATCTCAATTAGTCAGC AACCAGGTGTGGAAAGTCCCCAGGCTCCCCAGCAGGCAGAA GTATGCAAAGCATGCATCTCAATTAGTCAGCAACCATAGTCC CGCCCCTAACTCCGCCCATCCCGCCCCTAACTCCGCCCAGTTC CGCCCATTCTCCGCCCCATGGCTGACTAATTTTTTTTATTTATG CAGAGGCCGAGGCCGCCTCGGCCTCTGAGCTATTCCAGAAGT AGTGAGGAGGCTTTTTTGGAGGCCTAGGCTTTTGCAAAGATC GATCAAGAGACAGGATGAGGATCGTTTCGCATGATTGAACAA GATGGATTGCACGCAGGTTCTCCGGCCGCTTGGGTGGAGAGG CTATTCGGCTATGACTGGGCACAACAGACAATCGGCTGCTCT GATGCCGCCGTGTTCCGGCTGTCAGCGCAGGGGCGCCCGGTT CTTTTTGTCAAGACCGACCTGTCCGGTGCCCTGAATGAACTGC AAGACGAGGCAGCGCGGCTATCGTGGCTGGCCACGACGGGC GTTCCTTGCGCAGCTGTGCTCGACGTTGTCACTGAAGCGGGA AGGGACTGGCTGCTATTGGGCGAAGTGCCGGGGCAGGATCTC CTGTCATCTCACCTTGCTCCTGCCGAGAAAGTATCCATCATGG CTGATGCAATGCGGCGGCTGCATACGCTTGATCCGGCTACCT GCCCATTCGACCACCAAGCGAAACATCGCATCGAGCGAGCAC GTACTCGGATGGAAGCCGGTCTTGTCGATCAGGATGATCTGG ACGAAGAGCATCAGGGGCTCGCGCCAGCCGAACTGTTCGCCA GGCTCAAGGCGAGCATGCCCGACGGCGAGGATCTCGTCGTGA CCCATGGCGATGCCTGCTTGCCGAATATCATGGTGGAAAATG GCCGCTTTTCTGGATTCATCGACTGTGGCCGGCTGGGTGTGGC GGACCGCTATCAGGACATAGCGTTGGCTACCCGTGATATTGC TGAAGAGCTTGGCGGCGAATGGGCTGACCGCTTCCTCGTGCT TTACGGTATCGCCGCTCCCGATTCGCAGCGCATCGCCTTCTAT CGCCTTCTTGACGAGTTCTTCTGAGCGGGACTCTGGGGTTCGA | |

(continued)

| Payload | | SEQUENCE ID/formula | SEQ ID NO. |
|---|---|---|---|
| | | AATGACCGACCAAGCGACGCCCAACCTGCCATCACGAGATTT CGATTCCACCGCCGCCTTCTATGAAAGGTTGGGCTTCGGAAT CGTTTTCCGGGACGCCGGCTGGATGATCCTCCAGCGCGGGGA TCTCATGCTGGAGTTCTTCGCCCACCCTAGGGTGAAACACGG AAGGAGACAATACCGGAAGGAACCCGCGCTTAAAAAGACAG AATAAAACGCACGGTGTTGGGTCGTTTGTTCATAAACGCGGG GTTCGGTCCCAGGGCTGGCACTCTGTCGATACCCCACCGAGA CCCCATTGGGGCCAATACGCCCGCGTTTCTTCCTTTTCCCCAC CCCACCCCCCAAGTTCGGGTGAAGGCCCAGGGCTCGCAGCCA ACGTCGGGGCGGCAGGCCCTGCCATAGCCTCAGGTTACTCAT ATATACTTTAGATTGATTTAAAACTTCATTTTTAATTTAAAAG GATCTAGGTGAAGATCCTTTTTGATAATCTCATGACCAAAATC CCTTAACGTGAGTTTTCGTTCCACTGAGCGTCAGACCCCGTAG AAAAGATCAAAGGATCTTCTTGAGATCCTTTTTTTCTGCGCGT AATCTGCTGCTTGCAAACAAAAAAACCACCGCTACCAGCGGT GGTTTGTTTGCCGGATCAAGAGCTACCAACTCTTTTTCCGAAG GTAACTGGCTTCAGCAGAGCGCAGATACCAAATACTGTCCTT CTAGTGTAGCCGTAGTTAGGCCACCACTTCAAGAACTCTGTA GCACCGCCTACATACCTCGCTCTGCTAATCCTGTTACCAGTGG CTGCTGCCAGTGGCGATAAGTCGTGTCTTACCGGGTTGGACT CAAGACGATAGTTACCGGATAAGGCGCAGCGGTCGGGCTGA ACGGGGGGTTCGTGCACACAGCCCAGCTTGGAGCGAACGACC TACACCGAACTGAGATACCTACAGCGTGAGCTATGAGAAAGC GCCACGCTTCCCGAAGGGAGAAAGGCGGACAGGTATCCGGT AAGCGGCAGGGTCGGAACAGGAGAGCGCACGAGGGAGCTTC CAGGGGGAAACGCCTGGTATCTTTATAGTCCTGTCGGGTTTC GCCACCTCTGACTTGAGCGTCGATTTTTGTGATGCTCGTCAGG GGGGCGGAGCCTATGGAAAAACGCCAGCAACGCGGCCTTTTT ACGGTTCCTGGCCTTTTGCTGGCCTTTTGCTCACATGTTCTTTC CTGCGTTATCCCCTGATTCTGTGGATAACCGTATTACCGCCAT GCAT | |
| | ASO k-ras | GCTATTAGGAGTCTTT | 119 |
| | ASO c-myc | AACGTTGAGGGGCAT | 120 |
| RNA | mRNA (eGFP) | AUGGUGAGCA AGGGCGAGGA GCUGUUCACC GGGGUGGUGC CCAUCCUGGU CGAGCUGGAC GGCGACGUAA ACGGCCACAA GUUCAGCGUG UCCGGCGAGG GCGAGGGCGA UGCCACCUAC GGCAAGCUGA CCCUGAAGUU CAUCUGCACC ACCGGCAAGC UGCCCGUGCC CUGGCCCACC CUCGUGACCA CCCUGACCUA CGGCGUGCAG UGCUUCAGCC GCUACCCCGA CCACAUGAAG CAGCACGACU UCUUCAAGUC CGCCAUGCCC GAAGGCUACG UCCAGGAGCG CACCAUCUUC UUCAAGGACG ACGGCAACUA CAAGACCCGC GCCGAGGUGA AGUUCGAGGG CGACACCCUG GUGAACCGCA UCGAGCUGAA GGGCAUCGAC UUCAAGGAGG ACGGCAACAU CCUGGGGCAC | 121 |

(continued)

| Payload | | SEQUENCE ID/formula | SEQ ID NO. |
|---|---|---|---|
| | | AAGCUGGAGU ACAACUACAA CAGCCACAAC GUCUAUAUCA UGGCCGACAA GCAGAAGAAC GGCAUCAAGG UGAACUUCAA GAUCCGCCAC AACAUCGAGG ACGGCAGCGU GCAGCUCGCC GACCACUACC AGCAGAACAC CCCCAUCGGC GACGGCCCCG UGCUGCUGCC CGACAACCAC UACCUGAGCA CCCAGUCCGC CCUGAGCAAA GACCCCAACG AGAAGCGCGA UCACAUGGUC CUGCUGGAGU UCGUGACCGC CGCCGGGAUC ACUCUCGGCA UGGACGAGCU GUACAAGUAA | |
| | mRNA (luciferase) | ATGGAAGACGCCAAAAACATAAAGAAAGGCCCGGCGCCATTCTATCCTCTAGAGGATGGAACCGCTGGAGAGCAACTGCATAAGGCTATGAAGAGATACGCCCTGGTTCCTGGAACAATTGCTTTTACAGATGCACATATCGAGGTGAACATCACGTACGCGGAATACTTCGAAATGTCCGTTCGGTTGGCAGAAGCTATGAAACGATATGGGCTGAATACAAATCACAGAATCGTCGTATGCAGTGAAAACTCTCTTCAATTCTTTATGCCGGTGTTGGGCGCGTTATTTATCGGAGTTGCAGTTGCGCCCGCGAACGACATTTATAATGAACGTGAATTGCTCAACAGTATGAACATTTCGCAGCCTACCGTAGTGTTTGTTTCCAAAAAGGGGTTGCAAAAAATTTTGAACGTGCAAAAAAAATTACCAATAATCCAGAAAATTATTATCATGGATTCTAAAACGGATTACCAGGGATTTCAGTCGATGTACACGTTCGTCACATCTCATCTACCTCCCGGTTTTAATGAATACGATTTTGTACCAGAGTCCTTTGATCGTGACAAAACAATTGCACTGATAATGAATTCCTCTGGATCTACTGGGTTACCTAAGGGTGTGGCCCTTCCGCATAGAACTGCCTGCGTCAGATTCTCGCATGCCAGAGATCCTATTTTTGGCAATCAAATCATTCCGGATACTGCGATTTTAAGTGTTGTTCCATTCCATCACGGTTTTGGAATGTTTACTACACTCGGATATTTGATATGTGGATTTCGAGTCGTCTTAATGTATAGATTTGAAGAAGAGCTGTTTTTACGATCCCTTCAGGATTACAAAATTCAAAGTGCGTTGCTAGTACCAACCCTATTTTCATTCTTCGCCAAAAGCACTCTGATTGACAAATACGATTTATCTAATTTACACGAAATTGCTTCTGGGGGCGCACCTCTTTCGAAAGAAGTCGGGGAAGCGGTTGCAAAACGCTTCCATCTTCCAGGGATACGACAAGGATATGGGCTCACTGAGACTACATCAGCTATTCTGATTACACCCGAGGGGGATGATAAACCGGGCGCGGTCGGTAAAGTTGTTCCATTTTTTGAAGCGAAGGTTGTGGATCTGGATACCGGGAAAACGCTGGGCGTTAATCAGAGAGGCGAATTATGTGTCAGAGGACCTATGATTATGTCCGGTTATG | 122 |

(continued)

| Payload | | SEQUENCE ID/formula | SEQ ID NO. |
|---|---|---|---|
| | | TAAACAATCCGGAAGCGACCAACGCCTTGATTGACAAG GATGGATGGCTACATTCTGGAGACATAGCTTACTGGGA CGAAGACGAACACTTCTTCATAGTTGACCGCTTGAAGT CTTTAATTAAATACAAAGG ATATCAGGTGGCCCCCGCTGAATTGGAATCGATATTGTT ACAACACCCCAACATCTTCGACGCGGGCGTGGCAGGTC TTCCCGACGATGACGCCGGTGAACTTCCCGCCGCCGTT GTTGTTTTGGAGCACGGAAAGACGATGACGGAAAAAG AGATCGTGGATTACGTCGCCAGTCAAGTAACAACCGCG AAAAAGTTGCGCGGAGGAGTTGTGTTTGTGGACGAAGT ACCGAAAGGTCTTACCGGAAAACTCGACGCAAGAAA AATCAGAGAGATCCTCATAAAGGCCAAGAAGGGCGGA AAGTCCAAATTGTAA | |
| | mRNA (GUS) | AUGUUACGUCCUGUAGAAACCCCAACCCGUGAAAUCA AAAAACUCGACGGCCUGUGGGCAUUCAGUCUGGAUCG CGAAAACUGUGGAAUUGAUCAGCGUUGGUGGGAAAG CGCGUUACAAGAAA<br>GCCGGGCAAUUGCUGUGCCAGGCAGUUUUAACGAUCA GUUCGCCGAUGCAGAUAUUCGUAAUUAUGCGGGCAAC GUCUGGUAUCAGCGCGAAGUCUUUAUACCGAAAGGUU GGGCAGGCCAGC<br>GUAUCGUGCUGCGUUUCGAUGCGGUCACUCAUUACGG CAAAGUGUGGGUCAAUAAUCAGGAAGUGAUGGAGCA UCAGGGCGGCUAUACGCCAUUUGAAGCCGAUGUCACG CCGUAUGUUAUUG<br>CCGGGAAAAGUGUACGUAUCACCGUUUGUGUGAACAA CGAACUGAACUGGCAGACUAUCCCGCCGGGAAUGGUG AUUACCGACGAAAACGGCAAGAAAAAGCAGUCUUACU UCCAUGAUUUCUUUAACUAUGCCGGAAUCCAUCGCAG CGUAAUGCUCUACACCACGCCGAACACCUGGGUGGAC GAUAUCACCGUGGUGACGCAUGUCGCGCAAGACUGUA ACCACGCGUCUGUUGACUGGCAGGUGGUGGCCAAUGG UGAUGUCAGCGUUGAACUGCGUGAUGCGGAUCAACAG GUGGUUGCAACUGGACAAGGCACUAGCGGGACUUUGC AAGUGGUGAAUCCGCACCUCUGGCAACCGGGUGAAGG UUAUCUCUAUGAACUGUGCGUCACAGCCAAAAGCCAG ACAGAGUGUGAUAUCUACCCGCUUCGCGUCGGCAUCC GGUCAGUGGCAGUGAAGGGCCAACAGUUCCUGAUUAA CCACAAACCGUUCUACUUUACUGGCUUUGGUCGUCAU GAAGAUGCGGACUUACGUGGCAAAGGAUUCGAUAACG UGCUGAUGGUGCACGACCACGCAUUAAUGGACUGGAU UGGGGCCAACUCCUACCGUACCUCGCAU | 123 |

(continued)

| Payload | | SEQUENCE ID/formula | SEQ ID NO. |
|---|---|---|---|
| | | UACCCUUACGCUGAAGAGAUGCUCGACUGGGCAGAUG<br>AACAUGGCAUCGUGGUGAUUGAUGAAACUGCUGCUGU<br>CGGCUUUAACCUCUCUUUAGGCAUUGGUUUCGAAGCG<br>GGCAACAAGCCGAAAGAACUGUACAGCGAAGAGGCAG<br>UCAACGGGGAAACUCAGCAAGCGCACUUACAGGCGAU<br>UAAAGAGCUGAUAGCGCGUGACAAAAACCACCCAAGC<br>GUGGUGAUGUGGAGUAUUGCCAACGAACCGGAUACCC<br>GUCCGCAAGUGCACGGGAAUAUUUCGCCACUGGCGGA<br>AGCAACGCGUAAACUCGACCCGACGCGUCCGAUCACC<br>UGCGUCAAUGUAAUGUUCUGCGACGCUCACACCGAUA<br>CCAUCAGCGAUCUCUUUGAUGUGCUGUGCCUGAACCG<br>UUAUUACGGAUGGUAUGUCCAAAGCGGCGAUUUGGA<br>AACGGCAGAGAAGGUACUGGAAAAAGAACUUCUGGCC<br>UGGCAGGAGAAACUGCAUCAGCCGAUUAUCAUCACCG<br>AAUACGGCGUGGAUACGUUAGCCGGGCUGCACUCAAU<br>GUACACCGACAUGUGGAGUGAAGAGUAUCAGUGUGCA<br>UGGCUGGAUAUGUAUCACCGCGUCUUUGAUCGCGUCA<br>GCGCCGUCGUCGGUGAACAGGUAUGGAAUUUCGCCGA<br>UUUUGCGACCUCGCAAGGCAUAUUGCGCGUUGGCGGU<br>AACAAGAAAGGGAUCUUCACUCGCGACCGCAAACCGA<br>AGUCG<br>GCGGCUUUUCUGCUGCAAAAACGCUGGACUGGCAUGA<br>ACUUCGGUGAAAAACCGCAGCAGGGAGGCAAACAA | |
| | siRNA k-ras (reference only) | GUCUCUUGGAUAUUCUCGA (Sense)<br>UCGAGAAUAUCCAAGAGAC (Antisense) | 124<br>125 |
| | miRNA c-myc (reference only) | CAAAAACCGCAAUUACUUUUGCA (Sense)<br>UGCAAAAGUAAUUGAGAUUUUUG (Antisense) | 126<br>127 |
| | siRNA mixed population (reference only) | Mixture of synthetic siRNAs generated by T7 polymerase from *cfa6* gen and *hrpL* gen (see example 12) | |
| Small molecule | Rhodamine | 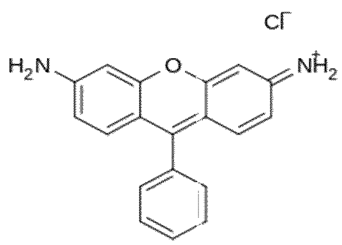 | |

**EXAMPLE 5**

**A. *In Vivo* Biodistribution of MEVs**

Experiments were performed to determine the pathway and fate of when administered via various routes.

### 1. MEV labelling using DiR fluorescent

As described in example 2, the near IR fluorescent, lipophilic carbocyanine DiR (1,1-dioctadecyl-3,3,3,3-tetramethylindotricarbocyanine iodide) is weakly fluorescent in water but highly fluorescent and photostable when incorporated into membranes and can be tracked *in vivo* (see, Example 2 above; Thermo Fisher Scientific). The DiR labelled (MEVs) are incubated with human cells. Their uptake by human cells is measured by fluorimetry analysis (fluorescence spectroscopy) on microplate readers. DiR has an excitation of 750 nm and an emission of 780 nm.

The methodology for the biodistribution studies is summarized as follows: *Chlorella* cells were grown in the photobioreactor as described above (120L per batch), were isolated and labeled with DIR, and labelling efficiency was assessed as described in Examples 1 and 2.

### 2. Biodistribution of MEVs in mice

The labeled MEVs were administered to the mouse model animals via one of four routes, via: intranasal, intratracheal, oral, and intravenous administration, to determine the pathways and fate of the MEVs by each route. The mice were subjected to full body imaging as a function of time, and at day 3 mice were sacrificed and the organs were imaged *ex vivo,* and by histology in several mice organs after different routes of application.

### Animal model

MEV pharmacokinetics and biodistribution were studied in Specific Pathogen Free (SPF), 7-week old male Balb/cByJ mice (Charles River Laboratories). The animals were labelled with unique ear ID tags and acclimatized for at least 2 days. Background (control) mice were housed individually, while MEV-treated mice were housed collectively in disposable standard cages in ventilated racks at 21±3°C (temperature recorded and controlled) with a 12hr-12hr light/dark cycle. Filtered water and low fluorescence laboratory food for rodents were provided *ad libitum.* All mice were euthanized at the end of the *in vivo* experiments.

### *In vivo* administration of DiR labelled MEV for biodistribution studies

The DiR-labelled MEVs (prepared as above in Example 2) were used to determine the biodistribution of the MEVs in the whole animal body or in several mice organs after different routes of application.

Prior to the imaging, the fur of each animal was clipped using an electric clipper in the following areas: abdomen, thorax, head, and whole back. Care was taken in order to clip the fur as homogeneously as possible. Next, DiR-labelled MEV preparations were re-suspended by vortexing before filling the syringes for injection.

### Routes of administration

The following routes were used to administer the MEVs to the animals: **Intravenous (*IV*):** 50 μL of MEV suspension containing $0.6x10^{12}$ MEV/mL were injected in a tail vein by disposable plastic syringe and appropriate needle. Dosage of MEV administered: $3x10^{10}$ MEV/mouse.

**Intranasal *(IN):*** Animals were induced and maintained under anesthesia during IN administration by a mixture of isoflurane and oxygen as a carrier gas. A volume of 100 μL of MEV suspension containing $0.3x10^{12}$ MEVs/mL was administered into the nostrils of the mouse using a thin pipette cone. Dosage of MEV administered: $3x10^{10}$ MEV/mouse.

***Per os (PO):*** 100 μL of MEV suspension containing $0.3x10^{12}$ MEVs/mL was administered orally using a disposable plastic syringe and an appropriate feeding probe. Dosage of MEV administered: $3x10^{10}$ MEV/mouse.

**Intratracheal (*IT*):** Animals were induced and maintained under anesthesia during IT administration by a mixture of isoflurane and oxygen as a carrier gas. Once adequate anesthesia was observed, animals were placed on a mouse intubation platform, suspended from the front incisors in the supine position, to maximize view of the trachea. Then, a cold light was placed on the skin near the trachea localization in order to backlight the trachea. If needed, a laryngoscope was inserted to guide the syringe of Microsprayer® sprayer into the trachea. An injection of 50 μL of MEV suspension containing $0.6\times10^{12}$ MEVs/mL was instilled into the trachea. Anesthesia was maintained throughout the procedure. After injection, the mice were maintained in the same position on the intubating platform for at least 30 seconds, before being replaced in their cage. Dosage of MEV administered: $3x10^{10}$ MEV/mouse.

### *In vivo* imaging for biodistribution studies

Fluorescence acquisitions were performed with the optical imaging system IVIS Spectrum of PerkinElmer. Bidimensional (2D) fluorescence imaging was performed by sensitive detection of light emitted by DiR-labelled MEVs.

Mice were anesthetized and imaged 1 hour before MEV administration. Next, fluorescence of the material to be administered was confirmed with an *in vitro* acquisition performed on at least 1 drop of a minimum of 20 μL of MEV suspensions, deposited in 2 different Petri dishes. The emission of fluorescence was measured in order to check the actual emission of fluorescence and detection with the selected parameters (see below). Finally, *in vivo* fluorescence acquisitions were performed on mice administered MEVs. The animals were induced and maintained under anesthesia with a mixture of isoflurane and oxygen. Mice were positioned in dorsal recumbency to obtain ventral images and in ventral recumbency to obtain dorsal images. At least 2 acquisitions/mouse/timepoint were taken and mice were imaged in groups of maximum 3 mice/acquisition. Images were analyzed and fluorescence quantified on 6 organs (liver, spleen, lung, kidney, intestine, and brain).

The following timepoints were used (T0=MEV administration):

Day 0 - 6 timepoints: T0 -1h ± 10 min; T0 +30 min ± 5 min; T0 +2 h ± 15 min; T0 +4 h ± 30 min; T0 +8 h ± 30 min; T0 +10 h ± 30 min
Day 1 - 1 timepoint: T0 + 24 h ± 3 h
Day 2 - 1 timepoint: T0 + 48 h ± 3 h
Day 3 - 1 timepoint: T0 + 72 h ± 3 h

The data were analyzed with the IVIS software (Living Image Software for IVIS).

The following parameters were used for the in vivo and ex-vivo fluorescence acquisitions:

| | | |
|---|---|---|
| Field of View (FOV) | 14 x 14 cm (FOV C) | |
| Image number: | *In vivo:* | For each mouse at each applicable timepoint, at least 2 acquisitions (ventral and dorsal) were performed -3 mice (at maximum) were imaged in one acquisition. |
| *Ex-vivo:* | the organs of at least one mouse were imaged in one acquisition | |
| Image format | TIFF format | |
| Fluorescent probe | DiR | |
| Excitation wavelength | 745 nm | |
| Emission wavelength | 800 nm | |
| Exposition time | Automatic, depending on the fluorescence signal detected | |
| Minimum counts | 20000 | |
| Binning | Between 16 and 1 (depending on the fluorescence signal detected) | |
| F/STOP | 2 (in case of fluorescence signal saturation it was | |
| automatically increased to 4 or it | was decreased to 1 in case of weak signal) | |
| Subject height | *In vivo:* | 1.5 cm |
| | *Ex-vivo:* | 0.5 cm |

***Ex vivo* imaging for biodistribution studies**

Production, purification, characterization, labelling and administration (by different routes) of MEVs was as described above. On Day 3 after administration, mice were euthanized and the organs of interest of each mouse were sampled and positioned in a Petri dish in order to perform *ex vivo* acquisitions. The following organs were sampled: liver, spleen, lungs, kidneys, brain, and intestine. The data were analyzed with the IVIS software (Living Image Software for IVIS).

Figure 5 shows representative patterns of biodistribution according to the route of administration, for the Intravenous, Intratracheal and Per os routes.

### Results, Discussion and Conclusions

**1.** Biodistribution - Full body imaging is depicted in Figures 6-9, which show *in vivo* full body imaging of a representative animal administered via intravenous (FIG. 6), Per os (FIG. 7), intranasal (FIG. 8) and Intratracheal (FIG. 9) administration.

### Pharmacokinetics

The pharmacokinetic curves demonstrating the accumulation as a percent of baseline over time (hours) after intravenous, Per os, intranasal, and intratracheal administration are set forth in Figures 10-13, respectively.

### Biodistribution - isolated organs- *ex vivo* imaging

The biodistribution by *ex vivo* fluorescence analysis (total radiant efficiency) in liver; spleen; lungs; and brain after intravenous, Per os, intranasal, and intratracheal administration is set forth in Figures 14A-D.

### 3. Summary of results by each route

### a. Intravenous administration

- Organs targeted by unmodified MEVs - **liver and spleen**
- PK parameters:
    - Peak/plateau time: 2-24 h (liver), 4-24 h (spleen)
    - % at t=day 3 compared to peak/plateau approx. 62% (liver), approx. 61% (spleen)
- Longer presence or duration compared to mammalian EVs (see, *e.g.,* Kang et al., Biodistribution of extracellular vesicles following administration into animals: A systematic review. J Extracell Vesicles. 2021;10:e12085. (doi.org/10.1002/jev2.12085)).
- No visible signs of toxicity

### b. Per os (oral) administration

- Organs targeted by following oral administration: **intestine and spleen**
- Oral availability:
    - MEVs are orally available; they resist the passage through the stomach, reach the intestine and are subsequently appear in the spleen.
    - Mammalian EVs, with the exception of bovine milk EVs, are not orally available.
- PK parameters:
    - Peak/plateau time: 1-6 h (intestine), 1-10 h (spleen)
    - % at t=10h compared to peak/plateau: approx. 33% (intestine), approx. 44% (spleen)
    - % at t=48h compared to peak/plateau: 0% (intestine), 0% (spleen)
- No visible signs of toxicity

### c. Distribution in organs following intravenous and oral administration.

The distribution of the MEVs depends upon the route of administration. For the liver, there is a different distribution profile dependent on whether the administration is intravenous or oral. Following intravenous administration, the liver is the

primary target organ; following oral administration, the liver is marginally targeted. Similarly, for the spleen, the biodistribution profile and pharmacokinetics depends upon the route of administration. The pharmacokinetics profile depends on whether administration is intravenous or oral (per os). Intravenous administration is longer lasting. At day 3, 50-70% of the peak is still remaining. Oral administration is shorter lasting; at 48 hours, 0% remains.

**d. Intranasal administration**

- Organs targeted by the unmodified MEVs are the **lungs**
- Preliminary PK parameters:

    - Peak/plateau time: 4-28 h
    - % at t=day 3 compared to peak/plateau: 70-80%

- No visible signs of toxicity

**e. Intratracheal administration**

- Organs targeted by the MEVs are the **lungs**

    - PK parameters:

        - Peak/plateau time: 2-72 h
        - % at t=day 3 compared to peak/plateau: approx. 80%

    - No visible signs of toxicity

**4. Biodistribution of MEVs labelled with PKH26 after per os (PO) administration in BALB/cByJ mouse.**

When MEVs are orally administrated to the mice, they follow through the digestive tract. MEVs first reach the stomach, where they resist the stringent conditions of the gastric juice. At about 30 minutes after administration, they reach the intestine, where they stay for several hours.

Once in the lumen of the intestine, MEVs pass through the epithelial layer into the GALT. The GALT is a lymphoid structure associated to the digestive tract *(gut-associated lymphoid tissues).* It is located all along the intestine. GALT is a dense tissue composed of germinal centers with B and T lymphocytes, plasmocytes and innate immune cells including dendritic cells and macrophages. In the intestine, fluorescence is observed mostly in the GALT. Fluorescence appears concentrated in discrete spots around nucleus meaning that MEVs are localized in the plasma of cells occupying all the space in the plasma. This allows revealing the shape of cells. Cells with MEVs inside correspond to histiocytes (resident macrophages) or dendritic cells, respectively, and are located mainly at the periphery of GALT and in the center of the GALT. Representative images are displayed in Figures 15A and B.

No visible MEVs appear in the liver at all times evaluated (30 min to 24h). A few hours after injection, cells with MEVs inside move from the GALT to the spleen and stay in the spleen for several hours. MEVs reach principally the red pulp (blood cells and some Th and B cells) and to a lesser extent the white pulp of the spleen (lymphoid cells) (see FIG. 16).

In the spleen, fluorescence is visible in several areas corresponding to the white and to the red pulps. The intensity of the fluorescence decreases in a gradient from the red pulps to the white pulps. Fluorescence is detected in spots inside the cell's cytoplasm. Cells with MEVs inside are round or reticular and have similar size; they may respectively be histiocytes and dendritic cells or macrophages.

Cells can move from the GALT into the bloodstream directly or they can join it after they have first passed through the lymph stream. GALT cells carrying MEV inside eventually arrived to the liver by the portal vein but are invisible to the liver as they have been internalized by GALT cells that naturally transport them to the spleen (see Figure 17).

**EXAMPLE 6**

## Evaluation of toxicity of MEVs in mice

Experiments were performed to assess the signs of MEV toxicity *in vivo* using Balb/C mouse model after oral and intratracheal administration.

### A. Analysis of MEV toxicity *in vivo:*

Toxicity was evaluated in several ways: clinical signs, body weights, hematological analysis, biochemical analysis, histological analysis on main organs (liver, spleen, kidney, lung and brain). The MEV samples were stored at -80°C and thawed just prior to in vivo administrations. After thawing, each sample was mixed by vigorous vortexing for 1-2 minutes. Male Balb/C mice at 5 weeks of age and with a mass about 20 g each, were used. Animals were acclimatized. Animals were housed in polyethylene cages (<5 animals/cage), in a controlled environment with 12:12 light-dark at the temperature of 24±1°C (mean ± SD) and fed once daily with an adapted pelleted feed. Water was offered *ad libitum.* The animals were randomly assigned to experimental groups and acclimatized for at least 7 days before the initiation of the designed study. The experimental groups are described in Table 30, below.

The experiment was designed to determine the eventual toxicity of the test compound after its administration in mice through exemplary routes including oral, and respiratory tract (intratracheal) routes. As described above, male Balb/C mice, with a mass about 20 g each, were used. Animals were acclimatized. After a test item is administered, all mice are closely monitored for 10 days.

**Table 30.** Experimental groups in MEV toxicity study.

| Group no. | Group type | Route of administration | MEV dosage |
|---|---|---|---|
| 1 | Vehicle control (PBS) | PO (oral) | - |
| 2 | Experimental | PO (oral) | 4x10exp11 per animal |
| 3 | Experimental | PO (oral) | 1x10exp12 per animal |
| 4 | Vehicle control (PBS) | IT (intratracheal) | - |
| 5 | Experimental | IT (intratracheal) | 4x10exp11 per animal |

## Clinical examination

Daily clinical examination of all animals, behavior, signs of suffering (cachexia, weakening, difficulty for moving or feeding, etc.); test item toxicity (hunching, convulsions), and other such parameters. Determination of body weight once a week for each animal, a body weight curve was designed (Mean + SD). Observation of acute reactions was done after administration of the tested compounds.

## Clinical Pathology Investigations

After the end of the in-life phase, all animals were euthanized. Clinical pathology investigations were performed at experiment termination.

## Blood collection

The blood samples were collected from mice by intracardiac puncture into different vials. Aliquots of blood were collected for various clinical pathology investigations into tubes containing anticoagulants: for hematology analysis with K2 EDTA and for biochemistry analysis with lithium heparin.

## Clinical chemistry

Plasma was separated after centrifugation of whole blood samples, 45000 rpm for 15 minutes and analyzed for the following parameters at the end of treatment for all animals. Clinical chemistry analysis parameters are indicated as: Alanine Aminotransferase (ALT), Aspartate Aminotransferase (AST), and Gamma-glutamyl transferase (GGT) in units per liter (U/L); Urea in g/L and Creatinine in mg/L.

## Hematology

The following hematological parameters were determined at the end of treatment of all animals: Hemoglobin (HGB) in g/L; Hematocrit (HCT) in L/L; Mean Corpuscular Volume (MCV) in fL; Eosinophils (EO) and MHCH (BASO) as $10^9$/L & %.

When animals are euthanatized, an autopsy is performed, and a careful macroscopic evaluation is performed. If any organs look abnormal, a detailed description and analysis is performed.

## Histological analysis

Histological analysis is performed for the 5 following organs: Lung, Spleen, Liver, Kidney, and Brain. The organs are collected, weighed, observed macroscopically, fixed in 4% Paraformaldehyde (PFA) and paraffin-embedded, cut into 5-7 μM sections and then observed under a fluorescence microscope.

## Histopathological scoring

Each H&E (hematoxylin- and eosin-stained) section was thoroughly examined histologically, and lesions observed were recorded in an Excel spreadsheet. Severity of lesions were graded (minimal, mild, moderate, or severe). Their distribution also was characterized (focal, multifocal, focally extensive, or diffuse), as well as, their localization.

## B. Toxicity study results: Clinical examinations

## Clinical chemistry

As shown below, two parameters Urea and Creatinine did not change in all treated groups compared to control groups. The enzymes Alanine Aminotransferase (ALT) and Aspartate Aminotransferase (AST) shown random non-significant deviations. The parameter Gamma-glutamyl transferase values is detected at low level (<6) in all groups indicating a protocol error and are not presented. Figure 18 presents these results.

The results of the clinical chemistry parameters do not indicate any toxicity at all tested doses in the mice either by oral or intratracheal administration.

## Hematology

The measured parameters were not significantly changed compared to controls. Figures 18A-I present the results in which MEV toxicity was evaluated by (1) chemistry parameters: ALAT, ASAT, urea and creatine; and (2) by hematology parameters in four groups of mice: Group 1 mice were administered 100 μl of PBS by PO delivery; Group 2 mice were administered 100 μl of $4*10^{11}$ MEV/ mouse by PO delivery (bar with black and white tiles); Group 3 mice were administered 100 μl of $4*10^{12}$ MEV/ mouse by PO delivery (bars with vertical lines); Group 4 mice were administered 100 μl of $4*10^{11}$ MEV/ mouse by IT delivery.

The results present blood parameters that can be altered when there is one or more of blood-, kidney-, and liver-related toxicity. Hematocrit, hemoglobin, eosinophil levels, RBC count, and volume (MCV) are hematologic parameters; urea and creatine are biochemical markers of kidney injury; ALAT (or ALT) and ASAT (or AST) are biochemical markers of liver injury. Alanine transaminase (ALT), also called alanine aminotransferase (ALAT), is a transaminase enzyme that occurs in plasma and in various body tissues, but most commonly in the liver. Significantly elevated levels of ALAT can be related to liver-related problems, such as hepatitis and/or liver damage. Aspartate transaminase (AST), also known as aspartate aminotransferase (ASAT), is another transaminase enzyme important in amino acid metabolism. Beyond liver toxicity, AST can be elevated also in diseases affecting other organs. Serum ALT level, serum AST level, and their ratio (AST/ALT ratio) are used clinically as biomarkers for liver health. In the instant study, no statistically-significant differences between experimental groups in ALT and AST levels were observed.

Eosinophils (eosinophiles) are a variety of white blood cells and one of the immune system components responsible for combating multicellular parasites and certain infections in vertebrates. Eosinophils usually account for less than 7% of the circulating leukocytes. Beyond causes related to infection or parasitic infestation, elevated eosinophil levels (also known as eosinophilia) can be also a sign of allergic or atopic reactions. In the instant study no statistically-significant differences between experimental groups in eosinophil levels were observed.

## Histology

The main organs were collected, fixed in 4% PFA, paraffin-embedded, cut into sections and stained with H&E.

*Lung*

Histopathological changes observed in lung sections are primarily limited to minimal to mild focal to multifocal alveolar hemorrhages in 8/10 lung sections examined in animals receiving intratracheal negative control material and test item. Changes were relatively subtle and limited in some of the alveolar spaces. This observation was observed at comparable incidence and severity in negative control and treated animals.

*Kidney*

Presence of a few basophilic tubules were observed in 1 animal treated PO with $4x10^{11}$ MEV dose. This finding was minimal in severity and often is observed spontaneously at low incidence and severity in laboratory rodent animals. It is therefore considered as incidental in origin in the present study.

*Spleen, Liver, and Brain*

No histopathological changes were observed in spleen, brain, or liver in all sections examined.

*Summary*

The pulmonary changes observed in most animals receiving the experimental material intra-tracheally were considered not treatment-related. There was no treatment-related observed in all organs examined, indicating that MEVs are not toxic to animals under the experimental conditions.

**C. Conclusion**

Regarding to the clinical aspects, all the animals, at each time of the study, exhibited normal behaviors, body weight, water and food consumption. The clinical findings indicate general tolerance in the mouse model of the test MEV products. Under the experimental conditions, both negative controls (PBS) and MEV at concentration of $4*10^{11}$ MEV/ mouse or $4*10^{12}$ MEV/ mouse by oral administration and at concentration of $4*10^{11}$ MEV/ mouse by IT administration did not induce any abnormal, toxic effect on animals.

**EXAMPLE 7 (reference only)**

***In vivo* delivery and expression of exogenously loaded siRNA**

This study assesses and demonstrates the efficacy of MEVs loaded with siRNA against genes of prokaryotic and eukaryotic microorganisms. The different bacterial strains (*E. coli, P. aeruginosa, S. aureus*) and yeast (*Candida albicans*) are genetically modified to carry the bacterial Luciferase gene Lux operon (luxCDABE) for Gram-negative species, (luxABCDE) for Gram-positive species, and the luciferase gene from the copepod *Gaussia* sp. for the yeast species. These microorganisms provide *in vivo* infection models as follows:

- *P. aeruginosa, S. aureus* / respiratory system,
- *P. aeruginosa, S. aureus, and Candida albicans* / skin,
- *E. coli* / digestive system,
- *E. coli* / urogenital, and

*Candida albicans* / urogenital system and skin.

Real-time monitoring of the interference activity of MEVs loaded with siRNA against the bacterial luciferase gene Lux operon or eukaryotic luciferase provides a way to follow, among other things: the capacity of the MEVs to penetrate infecting bacteria and yeast *in vivo;* to monitor the spread of MEVs in infection sites by different routes of applications and tissues; to monitor the capacity of MEVs to interfere with coding genes; and to assess the anti-microbial action of MEVs that deliver an anti-microbial therapeutic, such as an siRNA.

**1. *In vivo* imaging**

The animals are the male Balb/C mice as described above. After infection mice were closely monitored and imaged by IVIS bioluminescence to detect low levels of light emitted by luciferase *in vivo,* and bioluminescent bacteria that can be localized in specific tissues in living animals, thus allowing temporal monitoring of the infection process. The mice

were treated after with $1x10^{10}$ particles/g of MEVs and closely monitored and imaged by IVIS bioluminescence to follow the effectiveness of interference antimicrobial treatment, as represented by the interference activity of different siRNA populations against luciferase mRNA. The data were analyzed with the IVIS software (Living Image Software for IVIS).

## EXAMPLE 8

In vivo delivery and expression of exogenously loaded mRNA Ocular distribution following a single ocular topical administration in albino rabbits

### A. Material and Methods

Rabbit, New Zealand White (albino) is one of the species most used for ocular biodistribution. Eight 3-month-old males weighing between 2-2.5 kg were used in the ocular topical administration study.

All animals were ear-tagged at their arrival and the identification numbers were also marked in ears using indelible ink following the inclusion examination.

### B. Clinical examination and health status

Animals were daily observed for signs of illness and particular attention was paid to their eyes. Only healthy animals without visible ocular defect were used in the study.

### C. Housing

Animals were housed individually in standard cages. The temperature was held at 18+/-3°C and the relative humidity at 45-80%. Rooms were continuously ventilated (15-20 air volumes per hour). Animals were routinely exposed (in-cage) to 10-200 1x light in a 12-hour light (from 7:00 a.m. to 7:00 p.m.) and darkness-controlled cycle.

### D. Food and water

Animals had free access to food (90 g/day) and water available *ad libitum* from plastic bottles.

### E. Procedure

A 50-μL single instillation on both eyes was administered to all animals, using an appropriate micropipette, at a dose of $2X10^8$ MEVs loaded with mRNA-eGFP. Fluorophotometry was performed as follows: at several time-points (0, 6, 24, 30, 48, 54, and 72-hours post-administration) animals were anesthetized and pupils were dilated; measurements of ocular fluorescence were performed with FM-2 Fluorotron Master ocular fluorophotometer in both eyes. A series of 148 scans with a step size of 0.25 mm were recorded from the cornea to the retina along the optical axis.

### F. Results:

The fluorescence signal in various ocular tissues (choroid/ retina, vitreous, lens, anterior chamber, cornea) was obtained at data points that will be 0.25 mm apart along an optical axis by the fluorophotometer. Any detectable fluorescence was measured in the vitreous or the anterior chambre. The results of all animals are summarized in Figure 19.

## EXAMPLE 9

### *In vitro* delivery of different payloads

### A. To neuronal cells

PC12 neuronal cells are used as a model for *in vitro* evaluation of neuroprotective effects. Briefly, PC12 cells are seeded into 96-well plate (50,000 cell/well) and cultured for three days. Next, the catalase carrying MEV (loaded as described above) are stained with DiR (5 μg/mL) and added to the wells in serial dilutions for various times. Following the incubation, the cells are washed three times with ice-cold PBS and solubilized in 1% Triton X-100. The sample fluorescence is measured using a SpectraMax® fluorescence microplate reader (Molecular Devices, USA) with excitation at 750 nm and emission at 780 nm. The amount of MEV accumulated in neuronal cells is normalized for total protein content

and expressed as the number of MEVs per mg of protein. All MEV formulations are prepared at the same level of fluorescence, and a separate calibration curve is used for each MEV formulation.

**B. To neuronal cells - confocal microscopy**

The catalase loaded MEVs (100 μg/mL total protein) are sonicated, stained with DiR (5 μg/mL) and incubated with PC12 cells grown on chamber slides ($1x10^5$ cells/chamber) for various time intervals. Following the incubation, the cells are washed with PBS, fixed in 4% paraformaldehyde and permeabilized with 0.1% Triton X-100. Fluorescent staining is performed using anti-actin antibody (Abcam ab179467, 1:100) with goat anti-rabbit IgG Alexa Fluor 488 (Abcam ab150077, 1:1000) as the secondary antibody. DAPI (4',6-diamidino-2-phenylindole) is used as nuclear counterstain prior to the imaging. Accumulation of fluorescently labelled MEVs is visualized by a LSM700 laser scanning confocal microscope (Zeiss) and images are processed with LSM Image Browser.

**C. Neuroprotective effects of the loaded MEVs**

The protection of PC12 cells against 6-OHDA-induced cytotoxicity is assessed by MTT assay. For this purpose, PC12 cells ($1x10^5$ cells/mL) are seeded into a 96-well plate and allowed to attach overnight. Then, the cells are exposed to 200 μM 6-hydroxydopamine (6-OHDA) and different catalase-loaded MEV formulations, or catalase alone, or empty MEVs for four hours. Following the incubation, the cells are washed 3 times with ice-cold PBS, and incubated with the corresponding catalase loaded MEV formulations, or catalase alone, or empty MEVs for another 24 hours. Following the treatment, 20 μL of MTT tetrazolium dye solution (5 mg/mL) is added into each well. After 3 hours of incubation at 37°C, the MTT-containing medium is removed and 100 μL DMSO is added into each well to dissolve the purple formazan precipitate. Absorbance is measured at 570 nm using a SpectraMax® microplate reader (Molecular Devices, USA) and cell viability is expressed as a percentage of viable cells in the treated groups compared to the untreated control group.

**D. Cancer cells**

The HeLa cell line of human cervix epithelioid carcinoma is used to study uptake of GFP-loaded MEVs. For flow cytometry analysis, the cells are seeded in 24-well plates (15,000 cells/well). After 24 h, cells are incubated with the cargo-loaded MEVs for 4 h and subsequently washed with PBS, washed again with acid wash buffer (0.5 M NaCl, 0.2 M acetic acid) to remove membrane-bound MEVs and once more with PBS. Cells are detached with trypsin, fixed in 0.2% paraformaldehyde in PBS and analyzed using LSRII flow cytometer with CellQuest Pro software (BD Biosciences). For confocal microscopy analysis, HeLa cells are seeded in 16-well chamber slides (Lab-Tek) at 4,000 cells/well. After 24 h, cells are incubated with the cargo-loaded MEVs for 4 h and subsequently washed with PBS, washed again with acid wash buffer (0.5 M NaCl, 0.2 M acetic acid) to remove membrane-bound MEVs and once more with PBS. Cells are fixed with 4% paraformaldehyde in PBS at room temperature for 20 min. Slides are then washed with PBS and mounted using FluorSave™ (Calbiochem). Confocal fluorescent imaging is performed using a LSM700 laser scanning confocal microscope (Zeiss) and images are processed with LSM Image Browser.

**E. Delivery of GFP (green fluorescent protein) to normal human hepatocytes**

**1. Isolation and culture of primary human hepatocytes (PHH)**

Histologically normal liver tissue samples are obtained from subjects undergoing partial hepatectomy for the treatment of colorectal cancer metastases or primary liver cancer. All samples are seronegative for the hepatitis C virus (HCV), the hepatitis B virus (HBV) and the human immunodeficiency virus (HIV). Briefly, the liver fragment is initially perfused with a pre-warmed (37°C) calcium-free buffer supplemented with 5 mmol/L ethylene glycol tetra-acetic acid (Sigma-Aldrich) followed by perfusion with a pre-warmed (37°C) buffer containing 6 mmol/L calcium ($CaCl_2$) and 0.05% collagenase (Sigma-Aldrich). The liver fragment is then gently shaken to disperse liver cells in Hepatocyte Wash Medium (Life Technologies). The resulting cellular suspension is filtered through a gauze-lined funnel. Cells are then centrifuged at low speed (50 g), the supernatant removed, and pelleted hepatocytes are re-suspended in Hepatocyte Wash Medium. The count of viable cells is determined using Trypan Blue exclusion. Freshly isolated hepatocytes are suspended in William's E medium (Life Technologies) containing 10% fetal calf serum (Eurobio), penicillin (200 U/ml)-streptomycin (200 μg/ml; Life Technologies), fungizone (2.5 μg/ml; Life Technologies) and insulin (0.1 U/ml; Sigma-Aldrich). The cells are then seeded in 12-, 24- and 96-well plates pre-coated with type I collagen at a density of $0.78\times10^6$, $0.4\times10^6$ and $0.5\times10^5$ viable cells/well, respectively, and incubated at 37°C in 5% $CO_2$ overnight. The medium is replaced with fresh complete hepatocyte medium supplemented with 1 μmol/L hydrocortisone hemisuccinate (SERB) and cells are maintained in this medium until incubation with cargo loaded MEVs.

**2. Effect of MEVs on PHH viability**

PHHs ($1x10^5$ cells/mL) are seeded into a 96-well plate and allowed to attach overnight. Then, the cells are exposed to different cargo loaded MEV formulations or empty MEVs for four hours. Following the incubation, 20 μL of MTT tetrazolium dye solution (5 mg/mL) is added into each well. After 3 hours of incubation at 37°C, the MTT-containing medium is removed and 100 μL DMSO is added into each well to dissolve the purple formazan precipitate. Absorbance is measured at 570 nm using a SpectraMax® microplate reader (Molecular Devices, USA) and cell viability is expressed as a percentage of viable cells in the treated groups compared to the untreated control group.

**3. MEV internalization in PHH**

The internalization in PHH of the GFP-protein-cargo-loaded MEVs was determined by flow cytometry. For flow cytometry analysis, PHHs are seeded in 24-well plates (15,000 cells/well). After 24 h, cells are incubated with the MEVs for 4 h and subsequently washed with PBS, washed again with acid wash buffer (0.5 M NaCl, 0.2 M acetic acid) to remove membrane bound MEVs and once more with PBS. Cells are detached with trypsin, fixed in 0.2% paraformaldehyde in PBS and analyzed using LSRII flow cytometer with CellQuest Pro software (BD Biosciences). For confocal microscopy analysis, PHHs are seeded in 16-well chamber slides (Lab-Tek) at 4,000 cells/well. After 24 h, cells are incubated with the MEVs for 4 h and subsequently washed with PBS, washed again with acid wash buffer (0.5 M NaCl, 0.2 M acetic acid) to remove membrane bound MEVs and once more with PBS. Cells are fixed with 4% paraformaldehyde in PBS at room temperature for 20 min. Slides are then washed with PBS and mounted using FluorSave™ (Calbiochem). Confocal fluorescent imaging is performed using a LSM700 laser scanning confocal microscope (Zeiss) and images are processed with LSM Image Browser.

**4. Gene expression studies**

The internalization and biological activity in PHH of the GFP-mRNA-cargo-loaded MEVs is determined by total RNA extracted from the PPHs using the RNeasy Minikit (Qiagen) according to the manufacturer's instructions. Then, 500 ng of total RNA is reverse transcribed into cDNA using a reverse transcriptase (Promega) and real-time PCR is performed using the LightCycler® 480 SYBR Green I Master Kit on an LC480 device (both from Roche Diagnostics). The mRNA level is calculated by normalizing the threshold cycle (CT) of target genes to the CT of the 28S ribosomal RNA housekeeping gene. The primers are designed using primer software from Roche Diagnostics and are purchased from Eurogentec.

**5. Protein expression studies**

Additionally, the targeted protein expression is verified using SDS-PAGE followed by Western blot analysis. Cells are lysed on ice in RIPA buffer supplemented with β-mercaptoethanol (Sigma-Aldrich). Then, 30 μg of proteins are boiled and subjected to SDS-PAGE and transferred to nitrocellulose membranes (Bio-Rad). The blots are blocked with Tris buffered saline (TBS), 0.1% Tween-20 containing 5% BSA and incubated overnight at 4°C with the following antibodies. Subsequently, the blots are incubated with the relevant HRP-conjugated secondary antibody for 1 h at room temperature. Signals are revealed by chemiluminescence (Thermo Fisher Scientific).

**F. Human monocytes and keratinocytes**

Human primary cells were used as an *in vitro* model to study payload delivery with MEVs loaded with a fluorescent probe (GFP) or GFP coding RNA. Cell penetration and fluorescence effect was assayed in human monocytes and/or Normal Human Epidermal Keratinocytes (NHEK).

**1. Materials:**

Peripheral Blood Mononuclear Cells (PBMC) from healthy volunteers were obtained from l'Etablissement Français du Sang (EFS). Cell culture medium Macrophage-SFM (M-SFM) was purchased from Gibco. NHEK cells and culture medium (KGM-Gold Bullet kit) were purchased from Lonza.

**2. Monocyte isolation, cell cultures:**

PBMCs were obtained from healthy blood donor buffy coats by a standard Ficoll-Hypaque gradient method. Monocytes were isolated from PBMCs by adherence to plastic for 2 hours in serum-free medium (M-SFM) optimized for macrophage culture, at 37°C in a humidified atmosphere containing 5% $CO_2$. NHEK were cultured following manufacturer's (Lonza) recommendations and seeded in 96-wells plate the day before the assay.

**3. MEV penetration assay:**

MEVs either loaded with GFP protein (MEV-GFP) or mRNA eGFP (MEV-mRNA) were incubated with NHEK and monocytes in 96-well plates for 4h, 8h and 24h.

The doses used were:

| For the GFP protein: | For the mRNA eGFP: |
|---|---|
| D1= $1x10^7$ MEVs/well | D1= $1x10^7$ MEVs/well |
| D2= $5x10^6$ MEVs/well | D2= $5x10^6$ MEVs/well |
| D3= $1x10^6$ MEVs/well | D3= $2.5x10^6$ MEVs/well |

Then the cells were washed with PBS two times and fresh medium was added. The plates were then placed in the Incucyte apparatus and images were done at 0, 6, 12, 24 and 48h for Prot-GFP and 0, 12, 24, 48 and 72h for mRNA-GFP.

**4. Image analysis:**

Images were analyzed using the Incucyte software. The number of cells was quantified first, followed by the number of cells that contained green fluorescence by applying a threshold on the fluorescence level. The same threshold was applied on all the images. Then the percentage of cells exhibiting green fluorescence was calculated as ratio = (cells containing green fluorescence / number of total cells) x 100.

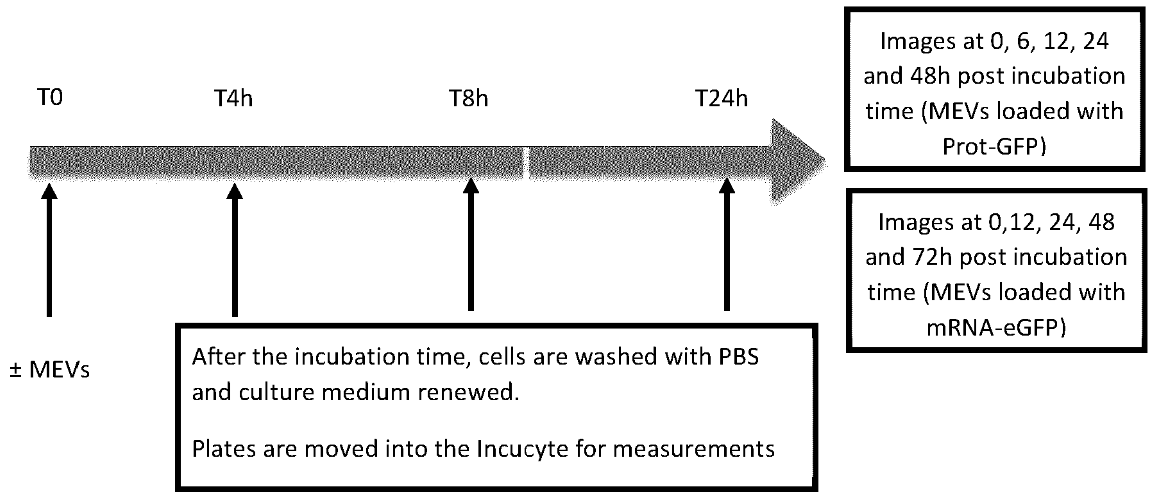


**5. Monocytes internal delivery:**

Green-fluorescent cells were observed at all times of incubation, for an interval of 48hs fluorescence following by Incucyte. This observation demonstrates the penetration of MEVs to human monocytes and the delivery of green fluorescence protein (GFP) from MEVs into monocytes.

The optimal incubation time was 8h based on the results at the first fluorescence measurement. It was observed a dose effect following different quantities of vesicles (between $1x10^6$ to $5x10^7$) incubated with the cells. Figure 20 shows the results after 8h and 24h of incubation (in a kinetics of 48h after wash).

**6. Keratinocytes internal delivery:**

Green-fluorescent cells were observed at all times of incubation, for an interval of 48hs fluorescence following by Incucyte. This observation demonstrates the penetration of MEVs to human keratinocytes and the delivery of green fluorescence protein (GFP) from MEVs into keratinocytes.

The optimal incubation times are between 8h to 24h based on the results at the first fluorescence measurement. It was observed a dose effect following different quantities of vesicles (between $1x10^6$ to $5x10^7$) incubated with the cells.

Figure 21 shows the results after 8h and 24 h of incubation (in a kinetics of 48h after wash)

G. **To human hepatocytes**

**1. Material and methods**

• Isolation of human hepatocytes

PHH (Primary Human Hepatocytes) are isolated from normal liver tissue obtained from patients undergoing partial hepatectomy. Dissociation is based on a two-step collagenase perfusion method as previously described (L. Aoudjehane, DMM, 2020). The cells are then separated by centrifugation over gradient. Hepatocytes are isolated from the pellet, and non-parenchymal cells (NPCs) from the supernatant with different gradients. Cell viability is determined by Trypan blue dye exclusion, and freshly isolated PHH were then seeded in 12-, 96-well plates pre-coated with type I collagen at a density of $1x10^6$, $6x10^4$ viable cells/well, respectively, and incubated at 37°C in 5% $CO_2$, overnight.

• Cell culture conditions

Cell lines Huh7 and HepG2 human hepatocytes cell lines were cultured in DMEM medium and incubated at 37°C, 5% $CO_2$ until confluence. The medium of PHH isolated cells was replaced with fresh complete hepatocyte medium supplemented with hydrocortisone and cells were maintained in this medium until incubation between D3 and D5 with MEV with different conditions at 37°C in 5% $CO_2$ for 24h and 48h. Huh7 and HepG2 ($2x10^6$ cells per well in 12-well plates) were seeded in triplicate on plastic in DMEM (10% FCS) and allowed to attach for 24 h. Then, in all experiments, the medium was replaced with free FCS-DMEM and the cells were cultured for 24h, 48h without MEV (for negative controls) or with the different MEV studied.

• Cell Viability Evaluation

Cell viability was determined by 3-(4,5- dimethylthiazol-2-yl)-5-(3-carboxymethoxyphenyl)-2-(4-sulfophenyl)-2H-tetrazolium (MTT) colorimetric assay, which tests the ability of viable cells to convert a soluble tetrazolium salt, MTT, into a blue formazan end product by mitochondrial dehydrogenase enzymes. MTT (0.5 mg/mL) was added to each well and incubation at 37°C was prolonged for an additional 2 hours. The medium was then discarded and DMSO was added to each well to solubilize the colored formazan product. Absorbance was read at 550 nm on a scanning microtiter spectrophotometer plate reader.

• Fluorescence imaging and confocal imaging:

The fluorescence images of PHHs and cell lines treated with MEV were viewed with Olympus microscope (IX83, Olympus) acquired with CellSens V1.6 and analyzed with FIJI software.

• Reverse Transcriptase-Polymerase Chain Reaction (RT-PCR) for mRNA Detection

Total RNA was prepared using the RNeasy minikit (Qiagen SA, Courtaboeuf, France) according to the manufacturer's recommendations. cDNA synthesis was carried out for 90 min at 42°C in a reaction mixture containing two units of RNase inhibitor (Promega, Charbonnieres, France), three units of avian myeloblastosis virus reverse transcriptase (Promega), 120 ng of random hexamer primers (Sigma-Genosys Ltd, Saint-Quentin Fallavier, France) and 1mM dNTP (Promega), for 500 ng of total RNA.

• Real-Time Quantitative PCR for Collagens and $\alpha$-SMA evaluation

Collagen-1 and $\alpha$-SMA gene expressions in HLMFs were analyzed by RT-PCR using the LightCycler® 480 SYBR Green I Master (2x con) Kit (Roche, Grenoble, France), with LC480 instruments and technology (Roche Diagnostics). The reaction was ensured using SYBR Green I Master (2x con) Kit (Roche Diagnostics), with LC480 instruments and technology (Roche Diagnostics). PCR amplification was performed in a total volume of 10 $\mu$l, containing 5 $\mu$l of SYBR green I Master Mix (2x), 10 ng of each primer (Sigma-Genosys Ltd; see Table) and 2 $\mu$l of cDNA (1/10eme). The PCR amplification protocol consisted in one step of initial denaturation for 10 min at 94° C, followed by 40 cycles: denaturation (95° C for 10 s), annealing (60°C for 10 s) and extension (72°C for 10 s).

**Table 31.** Primers used for quantitative real-time PCR.

| Gene | Sequence of forward (F) and reverse (R) prim ers | |
|---|---|---|
| eGFP | F:5'-AGCAAAGACCCCAACGAGA-3'<br>R:5'-TCGTCCATGCCGAGAGTG-3' | SEQ ID NO:66<br>SEQ ID NO:67 |
| Albumin (ALB) | F: 5'-TTTTATGCCCCGGAACTCCT-3'<br>R: 5'-TTGGAGACTGGCACACTTGA-3' | SEQ ID NO:128<br>SEQ ID NO:129 |
| Cytokeratin 18 (CK18) | F: 5'-CATAGACAAGGTACGGTTCCTG-3'<br>R: 5'-TGTTGTCCATGTTGCTTCGAGC-3' | SEQ ID NO:130<br>SEQ ID NO:131 |
| 28S (RN28S1) | F:5'- TTGAAAATCCGGGGGAGAG -3'<br>R:5'- ACATTGTTCCAACATGCCAG-3' | SEQ ID NO:132<br>SEQ ID NO:133 |

• Western blot analysis

Cells and tissues were lysed on ice directly in supplemented RIPA buffer (Sigma Aldrich). 20 μg of proteins was boiled and subjected to SDS-PAGE and transferred to nitrocellulose membranes (Bio-Rad, Hercules, CA). The blots were blocked with TBS, 0.1% Tween-20 containing 5% non-fat dry milk, and incubated with the following antibodies: Anti-Albumin (Dako), anti-eGFP (Roche), overnight at 4°C and with secondary HRP-link antibody (1/2000, GE Healthcare Europe GmbH), for 1h at room temperature. Signals were revealed by chemiluminescence (ThermoScientific, France).

**2. Results:**

Table 32 shows the results of mRNA expression by RT-qPCR analysis for the different experimental conditions.

**Table 32.** Results of gene expression analysis using quantitative real-time PCR in MEV internalization study with human hepatocytes.

| Experimental Conditions | eGFP | 28S | ALB | CK18 |
|---|---|---|---|---|
| After 24 hs | | | | |
| PHH (negative control) | (-) | (+++) | (+++) | (++) |
| PHH ( non-loaded MEV) | (-) | (+++) | (+++) | (++) |
| PHH ( GFP protein-loaded MEV) | (-) | (+++) | (+++) | (++) |
| PHH ( mRNA eGFP-loaded MEV) | (+++) | (+++) | (+++) | (+++) |
| Huh7 cells (negative control) | (-) | (+++) | (+++) | (+++) |
| Huh7 cells ( non-loaded MEV) | (-) | (+++) | (+++) | (+++) |
| Huh7 cells ( GFP protein-loaded MEV) | (-) | (+++) | (+++) | (+++) |
| Huh7 cells ( mRNA eGFP-loaded MEV) | (++) | (+++) | (+++) | (++) |
| Hep-G2 cells (negative control) | (-) | (+++) | (+++) | (++) |
| Hep-G2 cells (non-loaded MEV) | (-) | (+++) | (+++) | (++) |
| Hep-G2 cells ( GFP protein-loaded MEV) | (-) | (+++) | (+++) | (++) |
| Hep-G2 cells ( mRNA eGFP-loaded MEV) | (++) | (+++) | (+++) | (++) |
| After 48 hs | | | | |
| PHH (negative control) | (-) | (+++) | (+++) | (++) |
| PHH ( non-loaded MEV) | (-) | (+++) | (+++) | (++) |
| PHH ( GFP protein-loaded MEV) | (-) | (+++) | (+++) | (++) |
| PHH ( mRNA eGFP-loaded MEV) | (++) | (+++) | (+++) | (++) |
| Huh7 cells (negative control) | (-) | (+++) | (+++) | (++) |
| Huh7 cells (non-loaded MEV) | (-) | (+++) | (+++) | (++) |
| Huh7 cells ( GFP protein-loaded MEV) | (-) | (+++) | (+++) | (++) |
| Huh7 cells ( mRNA eGFP-loaded MEV) | (+) | (+++) | (+++) | (++) |
| Hep-G2 cells (negative control) | (-) | (+++) | (+++) | (++) |
| Hep-G2 cells (non-loaded MEV) | (-) | (+++) | (+++) | (++) |
| Hep-G2 cells ( GFP protein-loaded MEV) | (-) | (+++) | (+++) | (++) |

(continued)

| | | | | |
|---|---|---|---|---|
| After 48 hs<br>Hep-G2 cells ( mRNA eGFP-loaded MEV) | (+) | (+++) | (+++) | (++) |
| mRNA eGFP-loaded MEV (positive control) | (+++) | (-) | (-) | (-) |
| *References: (-) negative result (non mRNA present); (+) positive result (low ); (++) positive result (acceptable); (+++) positive result (best); (++++) positive result (mRNA eGFP-loaded MEV / positive control)* | | | | |

Table 33 shows the results of GFP expression by fluorescence expression after confocal analysis for the different experimental conditions. The results synthetize number of cells and fluorescence intensity. Further imaging results are presented in Figures 22 and 23.

**Table 33.** Results of protein expression analysis using confocal microscopy in MEV internalization study with human hepatocytes.

| Experimental Conditions | 100 loaded MEV per cell | 200 loaded MEV per cell | 400 loaded MEV per cell |
|---|---|---|---|
| Huh7 cells (negative control) | (-) | (-) | (-) |
| Huh7 cells ( non-loaded MEV) | (-) | (-) | (-) |
| Huh7 cells ( GFP protein-loaded MEV) | (+) | (+++) | (+++) |
| Huh7 cells ( mRNA eGFP-loaded MEV) | (++) | (+++) | (+++) |
| Hep-G2 cells (negative control) | (-) | (-) | (-) |
| Hep-G2 cells ( non-loaded MEV) | (-) | (-) | (-) |
| Hep-G2 cells ( GFP protein-loaded MEV) | (+) | (++) | (+++) |
| Hep-G2 cells ( mRNA eGFP-loaded MEV) | (++) | (++) | (+++) |
| *References: (-) negative result (non GFP fluorescence detected); (+) positive result (GFP fluorescence detected); (++) positive result (acceptable); (+++) positive result (best).* | | | |

Table 34, below, shows the viability results by MTT colorimetric assay. Nontoxicity was observed after the incubation with large quantities of MEVs (loaded or non-loaded MEVs).

**Table 34.** Cell viability assay results in MEV internalization study with human hepatocytes.

| Experimental Conditions | % of viability |
|---|---|
| Huh7 cells (negative control) | **100%** |
| Huh7 cells (10,000 MEV) | **80%** |
| Huh7 cells ( 20,000 MEV) | **80%** |
| Huh7 cells ( 40,000 MEV) | **80%** |
| Hep-G2 cells (negative control) | **100%** |
| Hep-G2 cells (10,000 MEV) | **100%** |
| Hep-G2 cells (20,000 MEV) | **100%** |
| Hep-G2 cells (20,000 MEV) | **100%** |

**H. Evaluation of the penetration of MEVs and delivering of payloads in different human cell models (fibroblasts)**

**1. Fluorescence Microscopy:**

Fibroblasts (ATCC human dermal CRL 2522 batch: 70027155) were seeded at $2x10^4$ cells per well into a 96 well plate. The plates used provide optimal resolution and the reference is Thermo Fisher Nunclon Delta Surface Disposable plate for cell culture, 96 wells. Cat No. 167425. After 20 h cells were incubated overnight with the different conditions (ratio of MEVs/cells is between 100 to 1000) of MEVs loaded with green fluorescent protein (GFP) or mRNA coding for green fluorescent protein (mRNA-eGFP) in non-complete medium. After 16h cells were rinsed two times and fresh complete medium was added. Hoechst solution at a dilution of 1:2000 was added to the medium to stain the nucleus. After that,

around the wells of the plate 1 mL of water was put in the compartments made for this. The images were taken by a laser autofocus camera at t=20h, t=48h and t=72h by Cellinsight. The Cellinsight culture condition are the same as the incubator at 37°C. Photos were taken with confocal module with 20X objective. Figure 24 show the results of 3 independent experiments at ratio MEVs/cells of 100.

**2. Flow Cytometry:**

Fibroblasts (ATCC human dermal CRL 2522 batch: 70027155) were seeded at $2x10^6$ cells by well into a 6 well plate. After 20 h cells were incubated overnight with the different conditions (ratio of MEVs/cells is between 100 to 1000) of MEVs loaded with green fluorescent protein (GFP) or mRNA coding for green fluorescent protein (mRNA-eGFP) in non-complete medium. After 16h cells were rinsed two times and fresh complete medium was added. At different times cells were rinsed and treated with trypsin, centrifuged at 1,200 g and washed one time with PBS and then resuspended in 1mL of PBS.

**3. Fibroblasts Fluorescence-activated cell sorting**

GFP expression in cultured fibroblast after incubation or not with MEVs loaded with GFP-protein or with MEVs loaded with mRNA-eGFP was assessed by FACS analysis. Fibroblasts cells were cultured in 96-well plates at 2000 cells per well. After 24h fibroblasts were incubated with or without MEVs (different conditions). At 24 h, after cells were detached from wells by trypsinization and washed two times with PBS, living cells were analyzed for GFP expression fluorescence cytometry.

Figures 25A-D show green fluorescent positive cells. FIG. 25A depicts fibroblasts incubated without MEVs. FIGs. 25B- and 25C- depicts fibroblasts incubated with MEVs loaded with mRNA-eGFP by different methods (freezing and thawing or electroporation, respectively). FIG. 25D shows fibroblasts incubated with MEVs loaded with GFP protein by sonication. The results show a percentage of positive cells over the control of 5% in B and D conditions and 8% in C.

**I. Studies on MEVs loaded with antibacterial peptide**

Isolated MEVs are loaded using sonication as above with antimicrobial peptide LL-37 (LLGDFFRKSKEKIGKEFKRIVQRIKDFLRNLVPRTES; SEQ ID NO:37), synthesized commercially by ProteoGenix (Lille, France) with 90% purity. Release of the antimicrobial peptide is monitored through dialysis and fluorescamine assay (Thermo Scientific) according to manufacturer's protocol. One mL of the sample is placed in a dialysis device (Float-A-Lyzer® G2100 kDa MWCO, Spectrum Laboratories Inc.) and allowed to dialyze in 20 mL of 20 mM sodium acetate buffer pH 5.5 at room temperature. Samples of 200 μL are withdrawn after 0, 1, 2, 4, 6 and 24 hours of dialysis. Peptide quantification is performed with fluorescamine assay using a microplate reader (Molecular Devices, USA) with a standard curve showing good linearity in the range 2.5-150 μg/mL of the peptide.

Proteolytic protection of peptide LL-37 is investigated after incubation of the MEV dispersions with *Pseudomonas aeruginosa* elastase (PE) or human neutrophil elastase (HNE). Peptide-loaded MEVs (total amount of 2 μg peptide) are incubated for 6 hours at 37°C in 10 mM TRIS buffer at pH 7.4 together with 0.2 μg PE (BioCol) or 0.4 μg HNE (Calbiochem) in a volume of 15 μL. For control samples, MEVs are lysed prior to incubation with enzymes to release the biomolecular cargo by adding 5 μL of 0.4% SDS into 95 μL of MEVs mixture, thorough pipetting and incubation in a thermocycler for 15 min at 85°C. Peptide degradation is visualized with Coomassie brilliant blue staining following SDS-PAGE. Band intensities are quantified by Molecular Imager Gel DOC with Image Lab Software (Bio-Rad).

Radial diffusion assays are performed on the MEV formulations before and after incubation with the enzymes, using *Escherichia coli* (ATCC 25922) and *Staphylococcus aureus* (ATCC 25923) to evaluate the bactericidal properties after proteolysis. $4 \times 10^6$ CFU is added to an underlay agarose gel casted in 85 mm Petri dishes and 4 mm diameter wells are punched after solidification. Six μL of the samples are added to each well and the plates are incubated at 37°C for 3 hours. Lysed MEVs are used as control samples. The underlay gel is thereafter covered with molten overlay agarose gel and incubated at room temperature overnight. Antimicrobial activity of the samples is visualized as a clear zone around each well. The results are presented as mean diameters of the clear zones formed by different formulations.

**EXAMPLE 10 (reference only)**

**In vitro delivery and expression of exogenously loaded siRNA in cancer cells**

**A. Cell cultures**

Human cell lines (ATCC) are grown in DMEM (Dulbecco's modified Eagle's medium) supplemented with 10%

fetal bovine serum (FBS), 50 units/ml penicillin, and 50 μg/ml streptomycin. The cells are kept in culture at 37°C and 5% $CO_2$, and the medium is changed every three days. To keep the cells at optimal proliferating conditions, they are passaged at 80% confluence and seeded at 20% confluence. The following cell lines have been used in the study:

1) *MYC* oncogene-expressing HT29 (colon cancer), PANC-1 (pancreatic cancer) and MYC-negative LN-18 (malignant glioma);
2) *RAS* oncogene-expressing SCLC-21H (small-cell lung cancer), MCF-7 (breast cancer) and *RAS*-negative PC3 (prostate cancer);
3) *BCL-2* oncogene-expressing AsPC-1 (pancreatic cancer), U-937 (pro-monocytic myeloid leukemia) and *BCL*-2-negative LM-MEL-53 (malignant melanoma);
4) *PLK-1* oncogene-expressing HepG2 (hepatoma), PC3 (prostate cancer) and *PLK-1-negative* AsPC-1 (pancreatic cancer).

**B. RT-PCR analysis and gel electrophoresis**

The cells ($1\times10^6$) are grown in culture plates for 24 h. Next, the siRNA carrying MEVs (loaded via electroporation as described above), or empty MEVs, or siRNA alone, are added to the wells in serial dilutions and incubated for 1-24 hours. Following the incubation, the medium is changed for the normal culture medium. 72 h after the MEV treatment, total RNA is extracted from the cultured cells using Trizol reagent. The RNA precipitate is centrifuged and dissolved in 20 μl of RNase-free water. UV spectrophotometer analysis at 260/280 nm and agarose gel electrophoresis are used to confirm the quality of purified RNA. One μg of total RNA is reverse transcribed to synthesize cDNA at 42°C for 1 h; this cDNA is then subjected to PCR amplification with specific primers in 25-μl reactions. Next, the PCR products are resolved on 2% agarose gel, stained with ethidium bromide and observed with a UV trans-illuminator. The digital images are quantified with ImageJ analysis software and the results are expressed as the relative expression level of relevant genes over GAPDH.

**C. Gene expression analysis with quantitative RT-PCR**

The cells treated with the MEVs and cultured for a total of 72 h are trypsinized and washed with PBS. Total RNA is extracted from the cell pellets using the RNeasy Minikit (Qiagen) according to the manufacturer's instructions. Then, 500 ng of total RNA is reverse transcribed into cDNA using a reverse transcriptase (Promega) and real-time PCR is performed using the LightCycler® 480 SYBR Green I Master Kit on an LC480 device (both from Roche Diagnostics). The mRNA level is calculated by normalizing the threshold cycle (CT) of target genes to the CT of the 18S ribosomal RNA housekeeping gene. The primers are designed using primer software from Roche Diagnostics and are purchased from Eurogentec.

**D. Western blot analysis**

The cells ($2\times10^6$) treated with the MEVs and cultured for a total of 72 h are trypsinized, washed with PBS and lysed on ice in RIPA buffer supplemented with β-mercaptoethanol (Sigma-Aldrich). Then, 50 μg samples of total protein are subjected to 10% standard sodium dodecyl sulfate-polyacrylamide gel electrophoresis (SDS-PAGE), with pre-stained molecular weight markers run in parallel. Subsequently, the resolved proteins are electro-transferred onto PVDF membranes. After extensive washing, the membranes are incubated with the relevant horseradish peroxidase-conjugated antibody (Sigma-Aldrich) for 1 h at room temperature, and developed with a chemiluminescence detection kit (Thermo Fisher Scientific). Membranes probed for the oncoproteins are re-probed for β-actin to normalize for loading and/or quantification errors and to allow comparisons of target protein expression. The protein expression is quantified with a gel analysis system (Bio-Rad).

**E. Cell proliferation assay**

The cells are seeded into 96-well plates (10,000 cells/well) and allowed to attach for 18 h. Next, the cells are treated with the MEVs as above; at 24, 48, and 72 h after the treatment, 10 μl of 10 mg/ml MTT is added to the cells in each well. The cells are incubated for 4 h, after which 100 μl DMSO is added, and the cells are allowed to lyse for 15 min. All of the analyses are carried out in triplicate. Optical density at 492 nm is determined with a SpectraMax® microplate reader (Molecular Devices, USA). To determine the inhibitor rate, the absorbance values are then normalized to the values obtained from the blank control cells.

**1. Apoptotic cell morphology observation**

The cell morphology related to apoptotic cell death are established by staining the cell nuclei with the DNA-binding fluorochrome Hoechst 33258 and assessing the chromatin condensation by fluorescence microscopy. Briefly, the cells are seeded in 24-well plates with glass slides on the bottom of the wells, and treated with the MEVs as above. Afterwards, the slides are gently washed with cold PBS, fixed with 4% paraformaldehyde for 1 h and washed three times with PBS. The slides are stained with 0.5 ml Hoechst 33258 (10 μg/ml) at 37°C for 10 min in a dark room. In each group, five microscopic fields are randomly selected, and one hundred cells are counted. The apoptotic cell level is then calculated as the percentage of apoptotic cells over the total number of fluorescent cells.

**2. Fluorescent analysis of necrotic/apoptotic cell death**

For flow cytometry analysis, the cells are seeded in 24-well plates (15,000 cells/well). After 24 h, cells are treated with the MEVs as above. Subsequently, the cells are washed with PBS and detached with trypsin. Following fixation in 0.2% paraformaldehyde in PBS, the cells are labeled with Annexin V FITC/PI (Thermo Fisher Scientific) and analyzed using LSRII flow cytometer with CellQuest Pro software (BD Biosciences).

**EXAMPLE 11 (reference only)**

**Targeting neuronal cells with Microalgae Extracellular Vesicles (MEVs) Biological activity of siRNA- and mRNA-loaded Microalgae Extracellular**

**Vesicles (MEVs) in neurons *in vitro***

**1. RNAi-induced knockdown in mouse neuronal cultures**

Murine pyramidal cells in DIV10- 14 hippocampal dissociated cultures are analyzed using whole-cell patch clamp technique, recording of spontaneous excitatory activity (sEPSCs). Cultures are either untreated or preincubated with siRNA loaded MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess knock-down efficacy. The analysis includes sEPSCs frequency, amplitude and membrane parameters (Rm, Rs, Cm).

**2. Overexpression/Rescue in KO neuronal mouse culture**

Pyramidal cells in DIV10- 14 hippocampal dissociated cultures from GABAB1a$^{-/-}$ mice are analyzed using whole-cell patch clamp technique, recording of spontaneous excitatory activity (sEPSCs). Cultures are either untreated or preincubated with MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess rescue efficacy. The analysis includes sEPSCs frequency, amplitude and membrane parameters (Rm, Rs, Cm).

**3. RNAi-induced knockdown in human neuronal cultures**

Human glutamatergic neuronal cells derived from iPSCs are analyzed using whole-cell patch clamp technique, recording of spontaneous excitatory activity (sEPSCs). Cultures are either untreated or preincubated with MEVs at different concentrations (from $10^6$ to $5x10^{10}$ particles) or preincubation periods (from 0.5 to 24 hours). For each cell, baseline signal is recorded for 3 mins and compared with post-application baclofen (20 μM) recording to assess knock-down efficacy. The analysis includes sEPSCs frequency, amplitude, and membrane parameters (Rm, Rs, Cm).

**EXAMPLE 12**

**Materials and methods for Examples 13-16**

**A. Total RNA extraction (*Chlorella*)**

200-800 ml of liquid *Chlorella* culture ($5 x10^6$ - $1 x 10^7$ cells/ml) were harvested by centrifugation (Beckman rotor JA18, 10000g, 10', 4°C), the pellet washed in 1X PBS and flash frozen in liquid nitrogen. The frozen pellet was ground to a fine powder in liquid nitrogen, using a mortar and pestle. Total RNA extraction was performed using Tri-Reagent (Sigma, St. Louis, MO) according to manufacturer's instructions, using about 100 mg of powder.

**B. Stomatal reopening assay**

To ensure full expansion of stomata, plants (4/5 weeks old, 8h/16h light/dark photoperiod) were kept under light (100 μE/$m^2$/s) for at least 3 hours before subjecting them to any treatment. Intact young leaf sections, at least 6 per condition, were immersed in water or bacterial suspension (at a concentration of $10^8$ cfu/ml, $OD_{600}$ =0.2). One hour prior to the bacterial infection, the sections were treated with either the MEVs (from ≈10 pM) or total RNAs (20 μg). At 3 hours of infection with the bacteria, the leaf sections were labelled for 10 minutes with Propidium Iodide (10 ng/ml in $H_2O$), washed for 5 minutes in $H_2O$ and observed under a SP5 laser scanning confocal microscope. For each condition, about 10-15 pictures were taken from different leaf surface regions. From the pictures, at least 60 stomata per condition were manually measured using ImageJ 1.53c to obtain their width and length. The width/length ratio was calculated using excel and statistical analysis performed using the Analysis of Variance (ANOVA) test.

**C. Stomatal closure assay**

Plants (4/5 weeks old, 8h/16h light/dark photoperiod) were kept under light (100 μE/$m^2$/s) for at least 3 hours before subjecting them to any treatment to ensure full expansion of stomata. Intact young leaf sections, at least 6 per condition, were immersed in water and treated with either the MEVs (from ≈10 pM) or flg22 1 μM (QRLSTGSRINSAKD-DAAGLQIA, SEQ ID NO:50, provided from GeneScript). At 1 hour post treatment, the sections were stained with Propidium Iodide, washed in water and observed under SP5 laser scanning confocal microscope. All these steps, including the data analysis were performed as described in the stomatal reopening assay protocol.

**D. Seedling growth-inhibition assay**

Seedlings were grown for 7-8 days on plates containing 13 Murashige Skoog (MS) medium (Duchefa), 1% sucrose, and 1% agar under a 16h/8h photoperiod at 22°C. Seedlings then were transferred into MS liquid medium (one seedling per 500 μl of medium in wells of 24-well plates) containing MEVs loaded with the flg22 peptide (QRLSTGSRINSAKDDAAGLQIA, SEQ ID NO:50, provided from arGeneScript), the flg22 peptide alone, water or control MEVs. The fresh weight of control *versus* treated seedlings from Col-0 genotypes was measured 7-8 days after.

**E. RT-qPCR quantification of Pattern Triggered Immunity (PTI) marker genes**

To quantify the expression of PTI markers in *Arabidopsis,* leaves of 4/5 weeks old plants were infiltrated with *Chlorella* MEVs loaded with fig22, flg22 1 μM, water or control MEVs. Following the infiltration, 3, 6 and 9 hours after the treatments the leaves were collected and frozen in liquid nitrogen. The frozen leaves were ground to powder with mortar and pestle before performing the RNA extraction with the NucleoSpin RNA Plant kit (Macherey-Nagel, 740949.250), according to manufacturer's instructions and using about 100 mg of powder. Total RNA was quantified using a NanoDrop system and 500 ng digested with the DNAse I (RQ1 RNase-free DNase, Promega, M6101) to eliminate all contaminating genomic DNA. The DNAse treatment was performed according to the manufacturer's protocol. The digested RNA was then retro-transcribed using the qScript cDNA SuperMix (Quantabio, 733-1178) as described by the manufacturer. The resulting cDNA was diluted 1:3 in water and 1 μl used to set up a qRT-PCR reaction using the appropriate gene-specific oligonucleotides and the Takyon No ROX SYBR 2X MasterMix blue dTTP (Eurogentec, UF-NSMT-B0701) mix. The RT-qPCR was performed with a "two-step" protocol with a first denaturation step at 95°C for 1' followed by 45 cycles of: 95°C for 10s and 60°C for 40s. A final step of melting curve generation was performed at the end of the reaction. Expression was normalized to that of the *Arabidopsis Ubiquitin* gene (Pfaffl (2001) Nucleic Acids Res. 29(9):e45). The PTI-marker genes analyzed were: *WRKY29* (Fw: TATCCAACGGATCAAGAGCTGA; Rv: TTTTCTTGCCAAACACCCTTTT), *FRK1* (Fw: TATCTTGAGCTGGGAAGAGAGG; Rv: AGTCGAATAGTACTCGGGGTCA) and *WRKY53* (Fw: CAGACGGGGATGCTACGG; Rv: GGCGAGGCTAATGGTGGTG) (SEQ ID NOs: 51, 52, 53, 54, 55, and 56, respectively).

**F. Cell death assay by Trypan Blue staining**

About 16-20 leaves infiltrated with *Chlorella* MEVs containing GUS or AvrRpm1 proteins (SEQ ID NOs: 60 and 61, respectively) were transferred in the Lactophenol Trypan Blue Solution (10 ml lactic acid [DL Sigma L-1250], 10 g glycerol, 10 ml $H_2O$ and 10 mg trypan blue) diluted in ethanol 1:1 and boiled for 1-2 min. The leaves were then destained overnight in chloral hydrate (2.5g/ml) and washed several times with water (5-6 times, 10') before the observation under an Olympus Macro Zoom System microscope. Trypan blue staining was quantified using ImageJ 1.53c.

### G. Bacterial strains

The *Pto* DC3000Δ*hrpL* strain was a gift from Dr. Cayo Ramos. The *Pto* DC3000-WT-HrpL and -mut-HrpL strain, the *Pto* DC3000ΔhrpL strain, express the NPTIIpro: WT-HrpL and NPTIIpro: mut-HrpL plasmids, respectively, and were previously characterized (International PCT application No PCT/EP2019/072169 and PCT/EP2019/072170, published as International PCT Publication Nos. WO2020035619 and WO2020035620, respectively).

### H. dsRNAs and sRNAs *in vitro* synthesis

The templates for the *in vitro* synthesis were amplified by PCR, introducing the T7 promoters at both the 5' and 3' ends of the oligonucleotide sequences. PCR products were gel-purified using the NucleoSpin® Gel and PCR Clean up kit (Macherey-Nagel) to eliminate any parasite amplification, and the purified PCR products were quantified and directly used for the *in vitro* RNA synthesis. The *in vitro* synthesis was performed using the MEGAscript® RNAi Kit (Life Technologies, Carlsbad, CA), following the manufacturer's instructions. 2 μg of PCR product were incubated for ON at 37°C with 2 μl of T7 Polymerase (T7 Enzyme Mix), 2 μl of 10X T7 Reaction Buffer and 2 μl of ribonucleotides (ATP, CTP; GTP and UTP, each at 75 mM) in a total reaction volume of 20 μl (adjusted with Nuclease-free water).

After the transcription reaction, the dsRNAs were treated with 2 μm of DNAse I, 2 μl of RNAse and 5 μl of Reaction Buffer to eliminate the DNA template and the ssRNAs. The dsRNAs were purified with filter cartridges provided with the kit and the long dsRNAs obtained after this step were quantified with the Nanodrop and directly used for the subsequent experiences. siRNAs were obtained by digesting the purified long dsRNAs with the ShortCut® RNAse III (NEB, Ipswich, MA). The digestion was performed for 30' at 37°C and the siRNAs purified using the mirVana™ miRNA Isolation kit (Life Technologies, Carlsbad, CA). The purified siRNAs were directly used for the subsequent experiences. At each step of the protocol, gel electrophoresis (TAE 1X, 1% agarose for DNA and 2% for RNAs) was performed to check the quality of the different nucleic acids.

### I. Infection Assays with *Fusarium graminearum* spores

For all *in vitro* infection assays, $5 \times 10^4$ conidia per $mL^{-1}$ were used for each of infection on leaves and on PDA (Potato Dextrose Agar) plates. Assays on PDA plates were performed by incubating for 2 hours at RT (about 25°C) the *F. graminearum* spores with different amount of *cyp51* dsRNAs-loaded *Chlorella* EVs. A total volume of 300 μl was deposited onto the PDA plate surface and dried in sterile conditions. To determine the efficacy of growth inhibition, the exogenously loaded vesicles were compared with total RNAs and *Chlorella* MEVs from a stable cell line, designated *Chlorella cyp51* IR transgenic lines (IT19), and a reference fungicide. Pictures of the plates at 24, 48 and 72 hours were taken, and the amount of developed mycelium was measured in order to determine the growth inhibition. Inoculation of *Arabidopsis* was done by wound inoculation on detached leaves with *F. graminearum.* About twenty rosette leaves of 15 different 4/5 weeks old Wt plants were detached and transferred in square Petri plates containing 1% agar. The exogenously loaded MEVs were deposed onto the surface of each leaf and let briefly dry. Inoculation of *F. graminearum* spores was carried out as previously described (Koch A et al., (2012) J Phytopathol 160(10):606-610). The progression of disease symptoms was determined by measuring the lesion size (in centimeters) at 3 dpi from the digital pictures of the petri dishes using the ImageJ 1.53c. These measures allowed to calculate the percentage of leaf area showing infection symptoms relative to the non-inoculated leaf.

### J. β-glucuronidase (GUS) staining assay

For this assay, about 20 leaves from 4/5 weeks old plants were infiltrated with the MEVs suspension at different concentrations. About 24/48 hours post infiltration, the leaves were detached, immersed in the GUS staining solution and vacuum infiltrated 2/3 times for 15' each. The plates containing the leaves are sealed with parafilm and incubated ON at 37°C. The following day several washes with 75% EtOH were performed until the leaves becomes completely transparent and only the blue GUS staining was visible. The GUS staining solution contains 1mM X-Gluc, 50 mM PBS pH 7.2, 10 mM EDTA pH 8, 0.5% Triton X-100. Pictures of the stained leaves are taken and the blue signal quantified using ImageJ 1.53c.

## EXAMPLE 13 (reference only)

### Determining the biological activity of *Chlorella* MEVs exogenously loaded with siRNAs targeting the Pto DC3000 hrpL virulence factor

The endogenous loading of MEVs was tested by generating transgenic *Chlorella* lines expressing an inverted repeat (IR) transgene carrying sequence homology with two major virulence factors of *Pto* DC3000. The first targeted *Pto*

DC3000 virulence factor is the coronafacic acid polyketide synthase I (*cfa6*) gene, which encodes a major structural component of the phytotoxin coronatine (COR) (Brooks et al. (2004) Mol Plant Microbe Interact. 17(2):162-174). The second one is *hrpL,* which encodes an alternative sigma factor that is known to directly control the expression of type III-secretion system associated genes, and to indirectly regulate the expression of COR biosynthesis genes (Fouts et al. (2004) Proc. Natl. Acad. Sci. U.S.A. 99:2275-2280; Sreedharan et al. (2006) Molecular plant-microbe interactions MPMI 19:768-779).

To demonstrate that the exogenous loading of siRNAs in *Chlorella* MEVs can be effective, small RNA duplexes directed against *Pto* DC30000 *hrpL* (SEQ ID NO:57) were synthesized *in vitro* to load exogenously into *Chlorella* MEVs, and to test their biological efficacy by monitoring their ability to suppress *Pto* DC3000-induced stomatal reopening. In this assay, the efficacy in inhibiting the stomatal reopening events by siRNA-loaded MEVs was compared with the events triggered by MEVs from stable *Chlorella* transgenic lines expressing siRNAs directed against *cfa6* and *hrpL* (described in International PCT application Nos. PCT/EP2019/072169 and PCT/EP2019/072170, published as International PCT Publication Nos. WO2020035619 and WO2020035620, respectively). Synthetic siRNA duplexes (1 μg) were loaded into *Chlorella* MEVs (sample 12). To exclude the possibility that the observed effects depend on siRNAs contamination, the samples were treated with Micrococcal nuclease (MNase), a broad range RNAse able to digest all unprotected RNAs that are either in a single-stranded or double-stranded forms. Empty MEVs were used as negative control and MNase-treated MEVs from the transgenic line IT20-3, expressing siRNAs targeting the *Pto* DC3000 *cfa6* and *hrpL* genes, were used as positive control. For the assays, all the vesicles were used at a concentration of about 20 pM (≈1,2E10 particles/ml). The results revealed that the siRNA-loaded MEVs are able to fully inhibit the stomatal reopening to the same extent as MEVs from the stable IT20-3 transgenic line (Figure 26). Furthermore, the sample maintained its biological activity upon the MNase treatment, indicating that the *Chlorella* MEVs protect siRNA from enzymatic degradation. These results further indicate that a sufficient quantity of exogenously-loaded antibacterial siRNAs are located inside the EVs to elicit the expected biological activity. Overall, the findings prove that *Chlorella* MEVs can be exogenously loaded with biologically-active siRNAs and that they can protect such small RNAs from enzymatic degradation, and that these siRNAs are delivered into the cell in sufficient quantity to trigger the specific biological activity.

Stomatal reopening assay at 3 hpi on leaf sections incubated with different MEVs one-hour prior infection. As controls, empty MEVs (Ctl-) and MEVs from the *Chlorella* stable transgenic line IT20-3 (Ctl+) that expresses siRNAs targeting the *Pto* DC3000 virulence genes *cfa6* and *hrpL,* were used. The siRNAs sample 12 corresponds to MEVs exogenously loaded with the synthetic siRNA duplexes targeting the *cfa6* and *hrpL* genes. The MNase treatment was performed for 15' at 37°C on the siRNAs sample 12 as well as on the Ctl+ vesicles. N, total number of analyzed stomata. Statistical analyses were performed using one-way ANOVA and comparing the mean of each condition with the means of all other conditions (p-value: ns >0.05; ***<0.001; ****<0.0001).

## EXAMPLE 14

### Determining the biological activity of *Chlorella* MEVs exogenously loaded with dsRNAs to target the *cyp51* genes in the plant pathogenic fungi *Fusarium graminearum*

*F. graminearum* is a pathogenic ascomycete fungus that causes devastating diseases in cereal crops: the *Fusarium* head blight (FHB) and the root rot. It has been recently shown that Host Induced Gene Silencing (HIGS) strategies using dsRNAs targeting three paralog cytochrome genes, *cyp51A, B* and *C*, are effective in limiting the growth of this fungal pathogen in *in vitro* conditions (Koch et al. (2013) Proc. Natl. Acad. Sci. U.S.A. 110:19324-19329). In the study reported in Koch *et al.,* the authors demonstrated that the same fungal growth inhibition can be achieved by spraying the dsRNA onto the leaf surface prior to the fungal infection (Spray Induced gene Silencing; see, Koch et al. (2016) PLOS Pathogens 12(10): e1005901). To demonstrate that antifungal dsRNAs can be exogenously loaded into *Chlorella* MEVs, *in vitro* synthesis of dsRNAs bearing sequence homologies against *cyp51A, B* and C was performed to load them into *Chlorella* MEVs, and to further test their antifungal activity *in vitro* and *in planta* assays. To perform this test, two *in vitro* approaches were employed: an analysis of *F. graminearum* growth onto PDA plates and on detached *Arabidopsis* leaves. In both cases, a MEV concentration carrying the equivalent of 800, 80 and 8 ng/ml of *cyp51* (SEQ ID NO:58) dsRNA were used and, as controls, naked *cyp51* dsRNAs, the total RNAs and MEVs from a transgenic *Chlorella* line (IT19) and a reference fungicide also were applied.

## EXAMPLE 15

### Determining the biological activity of *Chlorella* MEVs exogenously loaded with mRNAs and proteins

Two different approaches to characterize the biological activity of the loaded *Chlorella* MEVs with mRNAs or proteins are: 1) the GUS staining assay in *Arabidopsis* leaves using the GUS mRNA and protein (SEQ ID NOs: 59 and 60,

respectively); and 2) the plant cell death assay, also known as the hypersensitive response (HR). This assay makes use of the protein encoded by the bacterial effector *AvrRpm1* (SEQ ID NO:61), which is known to be recognized by the intracellular host immune receptor RPM1 and to trigger a potent HR in *Arabidopsis* accessions expressing a functional copy of RPM1 (*e.g.,* the Col-0 reference accession). For the first approach, we syringe-infiltrated different concentrations of MEVs loaded with either the GUS mRNA or protein directly in detached *Arabidopsis* leaves. A single mRNA-embedded MEVs concentration, equivalent to 100 ng/μl of mRNA, and three different amounts of protein-loaded MEVs, equivalent to 1, 5 and 10 μg/ μl of GUS protein was used. After the MEVs infiltration, the leaves were vacuum infiltrated with the GUS staining solution and left in incubation overnight for one night. The quantification of the blue signal resulting from the activity of the GUS enzyme allowed the direct comparison between the mRNA and protein delivery efficiency. For the second approach, the MEVs loaded with the effector protein AvrRpm1 were infiltrated in *Arabidopsis* leaves. The macro-HR was monitored by Trypan blue staining on at least 30 *Arabidopsis* Col-0 leaves treated with either MEVs carrying the GUS (negative control) or the AvrRpm1 proteins.

**EXAMPLE 16**

**Determining the biological activity of *Chlorella* MEVs exogenously loaded with peptides**

Small peptides ranging from 10 to 50 amino acids are potent effectors able to mediate a variety of responses in cell-to-cell communication, plant development, plant immune responses, and other processes and pathways. Such classes of molecules also can trigger the first layer of the immune defense through the Pathogen Associated Molecular Patterns (PAMPs) response, a mechanism that relies on the recognition of molecules released from the pathogen, including peptides, from the cell surface receptor of the host. In this context, 22 amino acid synthetic peptides that mimic the conserved N-terminal region of the bacterial flagellin, which is sensed by the surface receptor Flagellin Sensing 2 (FLS2), thereby triggering a potent immune response in various plant species, are used. Different concentrations of the synthetic peptides flg22 (10 nM, 100 nM and 1μM; SEQ ID NO:50) are loaded in *Chlorella* MEVs and the ability of these MEVs to trigger hallmarks of PAMP-triggered immunity, such as flg22-induced stomatal closure, flg22-induced growth inhibition and/or flg22-triggered induction of PTI marker genes is assessed. As negative controls, empty MEVs and the flg22 peptide at 1 nM, for which no biological activity was expected, were used in the assay, whilst as positive controls, 3 different concentrations of the synthetic peptide flg22 (10 nM, 0.1 and 1 μM) were used. Two additional controls were also included in order to eliminate any possible flg22 peptide contamination still present after the MEVs preparation and to test whether *Chlorella* MEVs are indeed able to protect the peptide from the protease activity. To this aim, the flg22-loaded MEVs were treated either with proteinase K (ProK) alone (+ProK condition), or in combination with Triton X100 (+ProK +Triton), which is a detergent proven to disrupt plant EVs (Rutter and Innes, 2017). The findings revealed that *Chlorella* MEVs exogenously loaded with flg22 peptides are able to induce stomata closure even after the proteinase K treatment. These data indicate that, when loaded, the flg22 peptide is bioactive, efficiently delivered to the host cells, and protected from enzymatic degradation by the EVs (Figure 27). The additional Triton treatment abolished such biological activity, likely by altering the integrity of *Chlorella* MEVs, thereby allowing ProK to degrade the flg22 peptide. This indicates that *Chlorella* MEVs protect the flg22 peptides located inside MEVs from ProK-triggered degradation. The results show that the biological effects on the stomata triggered by the flg22-loaded EVs are comparable to at least 10 nM of free flg22 peptide. Based on the concentration of MEVs used (about 16 pM (≈1E10 particles/ml)), it is estimated that each vesicle was loaded with more than $10^2$ peptide molecules in order to trigger the observed effect on stomata. Overall, the results show that *Chlorella* MEVs can be exogenously loaded with biologically active peptides, that the MEVs can protect the peptides from enzymatic degradation, and that these peptides are delivered into the cell in sufficient quantity to trigger the specific biological activity.

Stomatal closure assay at 1.5 hours post-treatment on *Arabidopsis thaliana* (accession Columbia-0 (Col-0)) leaf sections incubated with the flg22 peptides and MEVs loaded with the same molecule. As controls, empty EVs (Ctl-) and 1 nM of the flg22 peptide were used. Different concentrations of the flg22 peptide (10 nM, 0.1 and 1 μM) were used as positive control. The flg22-loaded EVs were either left untreated (-ProK) or incubated with the proteinase K alone (+ProK) or in combination with 1% of Triton X100 (+ProK +Triton), in order to remove possible flg22 contaminations (ProK alone) and/or affect the vesicle integrity. N represents the total number of analyzed stomata. Statistical analyses were performed using one-way ANOVA, comparing the mean of each condition with the means of all other conditions. Letters a and b denote different statistical groups determined by analyzing the pairwise table from the ANOVA analysis (statistical visualization using the compact letter display format).

The results in these examples and description show that microalgal MEVs, particularly *Chlorella* MEVs, can be exogenously loaded with bioactive cargo that can be delivered to cells and tissues, and the cargo is active. Based on this result and the other data and results provided herein, *Chlorella* MEVs can be loaded with bioactive cargo exogenously and delivered to cells and tissues, where the bioactive cargo is active.

## Claims

1. A composition, comprising isolated microalgae extracellular vesicles (MEVs), wherein:

   the microalgae is a species of the family Chlorellaceae;
   the composition is formulated for administration to a subject;
   the MEVs in the composition comprise heterologous cargo;
   the cargo is not RNAi; and
   the subject is an animal.

2. The composition of claim 1, wherein the cargo is not siRNA or miRNA.

3. The composition of claim 1 or claim 2, wherein the heterologous cargo has been exogenously loaded into the MEVs following purification of the MEVs.

4. The composition of any of claims 1-3, wherein:

   the cargo is a biomolecule or a small molecule; and
   the composition is formulated for administration to a human subject.

5. The composition of any of claims 1-4, wherein the microalgae is a species of the genus *Chlorella,* optionally, wherein the *Chlorella* is a species of *Chlorella* selected from among *Chlorella ellipsoidea, Chlorella pyrenoidosa, Chlorella sorokiniana, Chlorella vulgaris,* and *Chlorella variabilis*; optionally, wherein the *Chlorella* is *Chlorella vulgaris.*

6. The composition of any of claims 1-5, wherein:

   the composition is formulated for systemic, local, topical, parenteral, or enteral administration; or
   the composition is formulated for oral administration, intravenous administration, intramuscular administration, subcutaneous administration, intranasal administration, intratracheal administration, administration by inhalation or nebulization, topical administration, local administration, intratumoral administration, systemic administration, mucosal administration, enteral administration, or intraperitoneal administration.

7. The composition of any of claims 1-6 that is formulated as a tablet, or as powder, or as a liquid, or in a capsule, or in a troche.

8. The composition of any of claims 1-7, wherein:

   a) the cargo comprises a nucleic acid molecule, a polypeptide, a protein, a plasmid, an aptamer, or an antisense oligonucleotide; or
   b) the cargo comprises DNA, or RNA that encodes a product, or double-stranded RNA; or
   c) the cargo is mRNA or modified mRNA; or
   d) the cargo comprises a gene editing system; or
   e) the cargo comprises a plasmid; or
   f) the cargo comprises a viral vector; or
   g) the cargo comprises a therapeutic or diagnostic protein or polypeptide; or
   h) the cargo comprises a protein complex; or
   i) the cargo comprises a vaccine; or
   j) the cargo comprises or encodes a protein that is an antibody or antigen-binding fragment thereof; or
   k) the cargo is a product that stimulates the immune system of a subject treated with the composition; or
   l) the cargo is a cosmeceutical or a cosmetic or a cosmetically active product; or
   m) the cargo comprises a small molecule bioactive molecule; or
   n) the cargo is a chemotherapeutic agent; or
   o) the cargo is an immunotherapeutic agent; or
   p) the cargo comprises a diagnostic marker or detectable product; or
   q) the cargo comprises a prodrug; or
   r) the cargo comprises an antibody, or an scFv or other antigen-binding fragment of an antibody, or a bi-specific antibody; or
   s) the cargo comprises a diagnostic marker that comprises a luciferase or nucleic acid encoding a luciferase, a

fluorescent protein or nucleic acid encoding a fluorescent protein, or a luciferase operon; or
t) the cargo is for treatment or diagnosis of a disease, disorder or condition, of an animal.

**9.** The composition of claim 8 wherein:
the cargo comprises an antibody or antigen-binding fragment or other form thereof, or is a checkpoint inhibitor antibody or antigen-binding fragment thereof, or a tumor antigen-specific antibody or antigen-binding fragment thereof, or an anti-oncogene specific antibody or antigen-binding fragment thereof, or is a tumor-specific receptor or signaling molecule antibody or antigen-binding fragment thereof; or optionally the cargo comprises an antibody or antigen-binding fragment thereof that specifically binds to and inhibits one or more of CTLA-4, PD-1, PD-L1, PD-L2, the PD-1/PDL1 pathway, the PD-1/PDL2 pathway, HER2, EGFR, TIM-3, LAG-3, BTLA-4, HHLA-2, CD28, or other immune checkpoints, or immune suppressors, or tumor antigens.

**10.** The composition of any of claims 1-9, wherein:

the cargo is for treating or diagnosing or monitoring a disease, disorder, or condition selected from among: a cancer; and a disease, disorder, or condition of or involving the respiratory system, or involving the central nervous system or the nervous system, or involving the skin and exposed epithelia or mucosa, or involving the digestive tract; or
the cargo comprises a nucleic acid or protein or a nucleic acid encoding a protein that is a therapeutic product for treatment of a disease, disorder, or condition selected from among: a cancer; an infectious disease; a neurodegenerative or other CNS disease, disorder, or condition; or aging; an aging-associated disease; a skin condition; an ophthalmic disease, disorder, or condition; and an immunological disease, disorder, or condition.

**11.** The composition of any of claims 1-10, wherein:

the composition is formulated for intranasal administration, and the cargo comprises a therapeutic for treatment of a disease, disorder, or condition of the brain or involving the brain; or
the composition is formulated as drops for administration to the eye, and the cargo comprises a therapeutic for treatment of an ophthalmic disease, disorder, or condition; or
the composition is formulated for oral administration, and the cargo comprises a therapeutic for treatment of an immunological disease, disorder, or condition.

**12.** The composition of any of claims 1-11, wherein:

the MEVs contain cargo that comprises an immunostimulatory protein and/or an antigen and/or encodes an immunostimulatory protein; and
the cargo is immunostimulating and/or elicits an innate or adaptive immune response.

**13.** The composition of claim 12 that is formulated for oral administration.

**14.** The composition of claim 12 or claim 13, wherein the cargo comprises an antigen, such as an antigen for immunizing against a pathogen.

**15.** The composition of claim 10, wherein:

the condition is a skin condition or condition affecting vision; or
the condition is wrinkles or is discoloration of the skin; or is a macular degeneration or cataracts; or is a condition resulting from diabetic retinopathy.

**16.** A method of preparing the MEVs in the composition of claim 1 or claim 2, comprising:

culturing the microalgae;
isolating or purifying MEVs; and then
introducing the cargo into the MEVs following isolation or purification of the MEVs, wherein the cargo is loaded into the MEVs, for example, by a method selected from one or more of electroporation, sonication, extrusion, and surfactants.

**17.** The composition of any of claims 1-15 for use for treatment or prevention of a disease, disorder, or condition in an

animal.

**18.** The composition for use of claim 17, wherein the composition is formulated for oral administration, intravenous administration, intramuscular administration, subcutaneous administration, intranasal administration, intraocular administration, intratracheal administration, inhalation administration, nebulization, topical administration, local administration, intratumoral administration, systemic administration, mucosal administration, or intraperitoneal administration.

**19.** The composition for use of claim 17 or claim 18, wherein:

a) the disease, disorder, or condition is selected from among a cancer; a genetic disorder; a liver disease; a disease involving the immune system or immune cell; an infectious disease; a respiratory disease; a lung disease; a neurodegenerative disease; a gastrointestinal disease; a brain cancer; and a disease, disorder, or condition involving internal organs, the cardiovascular system and associated organs and tissues, hematopoietic or lymphoid tissues, sensory organs and tissues, urogenital organs and tissues, muscle tissues, bones, and/or endocrine tissues; or
b) the disease, disorder, or condition is a neurodegenerative disorder, or a cognitive disorder, or a brain disorder, or a nervous system disorder, or a genetic disease, or a multifactorial disease, or cancer, or high blood pressure, or high cholesterol, or schizophrenia, or bipolar disorder, or arthritis, or a metabolic disease, or Gaucher disease, or Hunter syndrome, or Krabbe disease, or maple syrup urine disease, or metachromatic leukodystrophy, or mitochondrial encephalopathy, lactic acidosis, stroke-like episodes (MELAS), or Niemann-Pick disease, or phenylketonuria (PKU), or porphyria, or Tay-Sachs disease, or Wilson's disease, or a disease of the respiratory system, or chronic bronchitis, or emphysema, or lung cancer, or cystic fibrosis, or bronchiectasis, or pneumonia, or a disease of the digestive tract, or inflammatory bowel disease (IBD), or gastroesophageal reflux disease (GERD), or celiac disease, or diverticulitis, or a disease of the digestive tract, or a disease of the mucosa, or a disease of the muscular or neuromuscular tissue, or a disease of the bones, or a disease of the endocrine tissue, or a disease of haemopoietic tissue, or a disease, disorder, or condition of the lymphoid tissue(s).

**20.** The composition for use of any of claims 17-19, wherein:

a) the disease, disorder, or condition is a neurodegenerative disease, or a brain disorder, or a nervous system disorder;

the composition is formulated for intranasal administration; and
the cargo comprises a therapeutic for treatment of the disease, disorder, or condition; and/or

b) the disease, disorder, or condition is a neurodegenerative disease, or a brain
disorder, or a nervous system disorder, or a psychiatric disorder; and/or
c) the disease, disorder, or condition involves or the cargo targets the gastrointestinal tract, or the immune system, or the white spleen for treatment; and
the composition is formulated for oral administration; and/or
d) the disease, disorder, or condition involves the immune system or is a disease, disorder, or condition that can be treated by delivering a therapeutic to cells of the immune system.

**21.** The composition for use of any of claims 17-20, wherein:

a) the composition is formulated for oral administration; and
the disease is treated by immune modulation; or
b) the composition is formulated for oral administration; and
the organs and tissues involved in the disease, disorder, or condition are accessible through the blood stream; or
c) the disease is an intestinal infection, Crohn's disease, or cancer; and
the composition is formulated for oral administration; or
d) the composition is formulated for intratracheal administration, or inhalation, or nebulization; and the cargo targets the respiratory tract and/or the lungs for treatment of respiratory and/or lung diseases, disorders, or conditions.

**22.** The composition for use of claim 21, wherein:

the disease is selected from among a chronic obstructive pulmonary disease (COPD), pulmonary hypertension, asthma, other inflammatory lung disease, cystic fibrosis, alpha-anti-trypsin (AAT) deficiency, an inborn error of metabolism, lung disease, cancer, and cancer metastases involving the lungs and respiratory system; and
the composition is formulated for intratracheal administration, or inhalation, or nebulization.

**23.** The composition for use of claim 17, wherein the composition is formulated for administration to the eye as eye drops; and
the disease, disorder, or condition is an ophthalmic disease, disorder, or condition.

**24.** The composition for use of any of claims 17-23, wherein the disease, disorder, or condition involves an infectious agent; and optionally the infectious agent is one or more of a bacterium, a virus, an oomycete, and a fungus, and/or optionally the composition is formulated for oral administration.

**25.** The composition of any of claims 1-15 or the composition for use of any of claims 17-24, wherein the microalgae is a *Chlorella* species, such as *Chlorella vulgaris.*

**Patentansprüche**

**1.** Zusammensetzung, umfassend isolierte extrazelluläre Vesikel von Mikroalgen (MEVs), wobei:

die Mikroalge eine Spezies aus der Familie der Chlorellaceae ist;
die Zusammensetzung zur Verabreichung an ein Subjekt formuliert ist;
die MEVs in der Zusammensetzung heterologe Fracht umfassen;
die Fracht nicht RNAi ist; und
das Subjekt ein Tier ist.

**2.** Zusammensetzung nach Anspruch 1, wobei die Fracht nicht siRNA oder miRNA ist.

**3.** Zusammensetzung nach Anspruch 1 oder Anspruch 2, wobei die heterologe Fracht nach der Reinigung der MEVs exogen in die MEVs eingebracht wurde.

**4.** Zusammensetzung nach einem der Ansprüche 1-3, wobei:

die Fracht ein Biomolekül oder ein kleines Molekül ist; und
die Zusammensetzung zur Verabreichung an ein menschliches Subjekt formuliert ist.

**5.** Zusammensetzung nach einem der Ansprüche 1-4, wobei die Mikroalge eine Spezies der Gattung *Chlorella* ist, wobei die *Chlorella* optional eine Spezies von *Chlorella* ist, ausgewählt aus *Chlorella ellipsoidea, Chlorella pyrenoidosa, Chlorella sorokiniana, Chlorella vulgarisund Chlorella variabilis;* wobei die *Chlorella* optional *Chlorella vulgaris* ist.

**6.** Zusammensetzung nach einem der Ansprüche 1-5, wobei:

die Zusammensetzung zur systemischen, lokalen, topischen, parenteralen oder enteralen Verabreichung formuliert ist; oder
die Zusammensetzung zur oralen Verabreichung, intravenösen Verabreichung, intramuskulären Verabreichung, subkutanen Verabreichung, intranasalen Verabreichung, intratrachealen Verabreichung, Inhalations- oder Zerstäubungsverabreichung, topischen Verabreichung, lokalen Verabreichung, intratumoralen Verabreichung, systemischen Verabreichung, mukosalen Verabreichung, enteralen Verabreichung oder intraperitonealen Verabreichung formuliert ist.

**7.** Zusammensetzung nach einem der Ansprüche 1-6, die als Tablette, oder als Pulver, oder als Flüssigkeit, oder in einer Kapsel oder in einer Lutschtablette formuliert ist.

**8.** Zusammensetzung zur Verwendung nach einem der Ansprüche 1-7, wobei:

a) die Fracht ein Nukleinsäuremolekül, ein Polypeptid, ein Protein, ein Plasmid, ein Aptamer oder ein Antisense-Oligonukleotid umfasst; oder

b) die Fracht DNA oder RNA, die ein Produkt codiert, oder doppelsträngige RNA umfasst; oder
c) die Fracht mRNA oder modifizierte mRNA ist; oder
d) die Fracht ein Genbearbeitungssystem umfasst; oder
e) die Fracht ein Plasmid umfasst; oder
f) die Fracht einen viralen Vektor umfasst; oder
g) die Fracht ein therapeutisches oder diagnostisches Protein oder Polypeptid umfasst; oder
h) die Fracht einen Proteinkomplex umfasst; oder
i) die Fracht einen Impfstoff umfasst; oder
j) die Fracht ein Protein, das ein Antikörper oder ein Antigen bindendes Fragment davon ist, umfasst oder codiert; oder
k) die Fracht ein Produkt ist, das das Immunsystem eines mit der Zusammensetzung behandelten Subjekts stimuliert; oder
l) die Fracht ein Kosmezeutikum oder ein kosmetisches oder kosmetisch wirksames Produkt ist; oder
m) die Fracht ein niedermolekulares bioaktives Molekül umfasst; oder
n) die Fracht ein Chemotherapeutikum ist; oder
o) die Fracht ein Immuntherapeutikum ist; oder
p) die Fracht einen diagnostischen Marker oder ein nachweisbares Produkt umfasst; oder
q) die Fracht ein Pro-Pharmakon umfasst; oder
r) die Fracht einen Antikörper oder ein scFv oder ein anderes Antigen bindendes Fragment eines Antikörpers oder einen bispezifischen Antikörper umfasst; oder
s) die Fracht einen diagnostischen Marker umfasst, der eine Luciferase oder eine Nukleinsäure, die eine Luciferase codiert, ein fluoreszierendes Protein oder eine Nukleinsäure, die ein fluoreszierendes Protein codiert, oder ein Luciferase-Operon umfasst; oder
t) die Fracht zur Behandlung oder Diagnose einer Erkrankung, Störung oder eines Zustands eines Tieres dient.

**9.** Zusammensetzung nach Anspruch 8, wobei:
die Fracht einen Antikörper oder ein Antigen bindendes Fragment oder eine andere Form davon umfasst, oder ein Checkpoint-Inhibitor-Antikörper oder ein Antigen bindendes Fragment davon, oder ein Tumor-Antigen-spezifischer Antikörper oder ein Antigen bindendes Fragment davon, oder ein Anti-Onkogen-spezifischer Antikörper oder ein Antigen bindendes Fragment davon, oder ein Tumor-spezifischer Rezeptor- oder Signalmolekül-Antikörper oder ein Antigen bindendes Fragment davon ist; oder die Fracht optional einen Antikörper oder ein Antigen bindendes Fragment davon umfasst, der spezifisch an einen oder mehrere von CTLA-4, PD-1, PD-L1, PD-L2, den PD-1/PDL1-Signalweg, den PD-1/PDL2 Signalweg, HER2, EGFR, TIM-3, LAG-3, BTLA-4, HHLA-2, CD28 oder andere Immun-Checkpoints oder Immunsuppressoren oder Tumor-Antigene gebunden ist und diese hemmt.

**10.** Zusammensetzung nach einem der Ansprüche 1-9, wobei:

die Fracht der Behandlung oder Diagnose oder Überwachung einer Erkrankung, Störung oder eines Zustands dient, ausgewählt aus: Krebs; und einer Erkrankung, Störung oder einem Zustand des Atmungssystems oder das zentrale Nervensystems oder des Nervensystems betreffend, oder die Haut und die freiliegenden Epithelien oder Schleimhäute oder des Verdauungstrakts betreffend; oder
die Fracht eine Nukleinsäure oder ein Protein oder eine Nukleinsäure umfasst, die ein Protein codiert, das ein therapeutisches Produkt zur Behandlung einer Erkrankung, Störung oder eines Zustands ist, ausgewählt aus: Krebs; einer Infektionskrankheit; einer neurodegenerativen oder anderen Erkrankung, Störung oder einem Zustand des zentralen Nervensystems; oder dem Altern; einer altersbedingten Krankheit; einer Hauterkrankung; einer Augenerkrankung, Störung oder einem Zustand des Auges; und einer immunologischen Erkrankung, Störung oder einem Zustand des Immunsystems.

**11.** Zusammensetzung nach einem der Ansprüche 1-10, wobei:

die Zusammensetzung zur intranasalen Verabreichung formuliert ist und die Fracht ein Therapeutikum zur Behandlung einer Erkrankung, Störung oder eines Zustands des Gehirns oder eines das Gehirn betreffenden Zustands umfasst; oder
die Zusammensetzung als Tropfen zur Verabreichung an das Auge formuliert ist, und die Fracht ein Therapeutikum zur Behandlung einer Augenkrankheit, -störung oder eines Augenzustands umfasst; oder
die Zusammensetzung zur oralen Verabreichung formuliert ist und ein Therapeutikum zur Behandlung einer immunologischen Erkrankung, Störung oder eines immunologischen Zustands umfasst.

**12.** Zusammensetzung nach einem der Ansprüche 1-11, wobei:

die MEVs Fracht enthalten, die ein immunstimulierendes Protein und/oder ein Antigen umfasst und/oder ein immunstimulierendes Protein codiert; und
die Fracht immunstimulierend ist und/oder eine angeborene oder adaptive Immunantwort hervorruft.

**13.** Zusammensetzung nach Anspruch 12, die zur oralen Verabreichung formuliert ist.

**14.** Zusammensetzung nach Anspruch 12 oder Anspruch 13, wobei die Fracht ein Antigen, wie beispielsweise ein Antigen zur Immunisierung gegen einen Krankheitserreger umfasst.

**15.** Zusammensetzung nach Anspruch 10, wobei:

der Zustand ein Hautzustand oder ein Zustand ist, der das Sehvermögen beeinträchtigt; oder
der Zustand Falten oder Verfärbungen der Haut ist; oder eine Makuladegeneration oder Katarakte ist; oder ein Zustand ist, der aus der diabetischen Retinopathie erfolgt.

**16.** Verfahren zur Herstellung der MEVs in der Zusammensetzung nach Anspruch 1 oder Anspruch 2, umfassend:

Kultivieren der Mikroalgen;
Isolieren oder Reinigen von MEVs; und danach
Einbringen der Fracht in die MEVs nach der Isolierung oder Reinigung der MEVs, wobei die Fracht in die MEVs zum Beispiel durch ein Verfahren geladen wird, das aus einem oder mehreren von Elektroporation, Beschallung, Extrusion und Tensiden ausgewählt ist.

**17.** Zusammensetzung nach einem der Ansprüche 1-15 zur Verwendung bei der Behandlung oder Vorbeugung einer Erkrankung, Störung oder eines Zustands bei einem Tier.

**18.** Zusammensetzung zur Verwendung nach Anspruch 17, wobei die Zusammensetzung zur oralen Verabreichung, intravenösen Verabreichung, intramuskulären Verabreichung, subkutanen Verabreichung, intranasalen Verabreichung, intraokularen Verabreichung, intratrachealen Verabreichung, inhalativen Verabreichung, Zerstäubung, topischen Verabreichung, lokalen Verabreichung, intratumoralen Verabreichung, systemischen Verabreichung, mukosalen Verabreichung oder intraperitonealen Verabreichung formuliert ist.

**19.** Zusammensetzung zur Verwendung nach Anspruch 17 oder Anspruch 18, wobei:

a) die Erkrankung, Störung oder der Zustand aus Krebs; einer genetischen Störung; einer Lebererkrankung; einer Erkrankung des Immunsystems oder der Immunzellen; einer Infektionskrankheit; einer Atemwegserkrankung; einer Lungenerkrankung; einer neurodegenerativen Erkrankung; einer Magen-DarmErkrankung; einem Hirntumor; und einer Erkrankung, Störung oder einem Zustand ausgewählt ist, der innere Organe, das Herz-Kreislauf-System und zugehörige Organe und Gewebe, blutbildende oder lymphatische Gewebe, Sinnesorgane und -gewebe, urogenitale Organe und -gewebe, Muskelgewebe, Knochen und/oder endokrine Gewebe betrifft; oder
b) die Erkrankung, Störung oder der Zustand eine neurodegenerative Erkrankung oder eine kognitive Störung oder eine Hirnerkrankung oder eine Erkrankung des Nervensystems oder eine genetische Erkrankung oder eine multifaktorielle Erkrankung oder Krebs oder Bluthochdruck oder hoher Cholesterinspiegel oder Schizophrenie oder bipolare Störung oder Arthritis oder eine Stoffwechselerkrankung oder Morbus Gaucher oder Hunter-Syndrom oder Morbus Krabbe oder Ahornsirupkrankheit oder metachromatische Leukodystrophie oder mitochondriale Enzephalopathie, Laktatazidose, schlaganfallähnliche Episoden (MELAS) oder Niemann-Pick-Krankheit oder Phenylketonurie (PKU) oder Porphyrie oder Tay-Sachs-Krankheit oder Morbus Wilson oder eine Erkrankung der Atemwege oder chronische Bronchitis oder Emphysem oder Lungenkrebs oder Mukoviszidose oder Bronchiektasen oder Lungenentzündung oder eine Erkrankung des Verdauungstrakts oder eine entzündliche Darmerkrankung (IBD), oder gastroösophageale Refluxkrankheit (GERD), oder Zöliakie, oder Divertikulitis, oder eine Erkrankung des Verdauungstraktes, oder eine Erkrankung der Schleimhaut, oder eine Erkrankung des Muskel- oder Nervengewebes, oder eine Knochenerkrankung, oder eine Erkrankung des endokrinen Gewebes, oder eine Erkrankung des blutbildenden Gewebes, oder eine Erkrankung, Störung oder ein Zustand der (des) lymphatischen Gewebe(s) ist.

**20.** Zusammensetzung zur Verwendung nach einem der Ansprüche 17-19, wobei:

a) die Erkrankung, Störung oder der Zustand eine neurodegenerative Erkrankung, eine Gehirnstörung oder eine Störung des Nervensystems ist;

die Zusammensetzung zur intranasalen Verabreichung formuliert ist; und
die Fracht ein Therapeutikum zur Behandlung der Erkrankung, Störung oder des Zustands umfasst; und/oder

b) die Erkrankung, Störung oder der Zustand eine neurodegenerative Erkrankung, eine Gehirnstörung, eine Störung des Nervensystems oder eine psychiatrische Störung ist; und/oder
c) die Erkrankung, Störung oder der Zustand betrifft oder die Fracht auf den Magen-Darm-Trakt, das Immunsystem oder die weiße Milz zur Behandlung abzielt; und
die Zusammensetzung zur oralen Verabreichung formuliert ist; und/oder
d) die Erkrankung, Störung oder der Zustand das Immunsystem betrifft oder eine Erkrankung, Störung oder ein Zustand ist, der durch Abgabe eines Therapeutikums an Zellen des Immunsystems behandelt werden kann.

**21.** Zusammensetzung zur Verwendung nach einem der Ansprüche 17-20, wobei:

a) die Zusammensetzung zur oralen Verabreichung formuliert ist; und die Erkrankung durch Immunmodulation behandelt wird; oder
b) die Zusammensetzung zur oralen Verabreichung formuliert ist; und
die an der Erkrankung, Störung oder dem Zustand beteiligten Organe und Gewebe über den Blutkreislauf erreichbar sind; oder
c) die Erkrankung eine Darminfektion, Morbus Crohn oder Krebs ist; und die Zusammensetzung ist zur oralen Verabreichung formuliert ist; oder
d) die Zusammensetzung zur intratrachealen Verabreichung, Inhalation oder Zerstäubung formuliert ist; und die Fracht auf die Atemwege und/oder die Lunge zur Behandlung von Atemwegserkrankungen und/oder Lungenerkrankungen, -störungen oder -zustände abzielt.

**22.** Zusammensetzung zur Verwendung nach Anspruch 21, wobei:

die Erkrankung aus einer chronischen obstruktiven Lungenerkrankung (COPD), pulmonaler Hypertonie, Asthma, anderen entzündlichen Lungenerkrankungen, Mukoviszidose, Alpha-Antitrypsin- (AAT) Mangel, einem angeborenen Stoffwechseldefekt, Lungenerkrankungen, Krebs und Krebsmetastasen, die die Lunge und die Atemwege betreffen, ausgewählt ist; und
die Zusammensetzung zur intratrachealen Verabreichung, Inhalation oder Zerstäubung formuliert ist.

**23.** Zusammensetzung zur Verwendung nach Anspruch 17, wobei die Zusammensetzung zur Verabreichung an das Auge als Augentropfen formuliert ist; und
die Erkrankung, Störung oder der Zustand eine Augenkrankheit, Störung oder einen Augenzustand ist.

**24.** Zusammensetzung zur Verwendung nach einem der Ansprüche 17-23, wobei die Erkrankung, Störung oder der Zustand einen Infektionserreger betrifft; und der Infektionserreger optional eines oder mehrere von einem Bakterium, einem Virus, einem Oomycet und/oder einem Pilz ist, und/oder die Zusammensetzung zur oralen Verabreichung formuliert ist.

**25.** Zusammensetzung nach einem der Ansprüche 1-15 oder Zusammensetzung zur Verwendung nach einem der Ansprüche 17-24, wobei die Mikroalge eine *Chlorella*-Spezies, wie zum Beispiel *Chlorella vulgaris* ist.

## Revendications

**1.** Composition comprenant des vésicules extracellulaires de microalgues isolées (MEV), dans laquelle :

la microalgue est une espèce de la famille des Chlorellaceae ;
la composition est formulée pour être administrée à un sujet ;
les MEV dans la composition comprennent une charge hétérologue ;

la charge n'est pas de l'ARNi ; et
le sujet est un animal.

**2.** Composition selon la revendication 1, dans laquelle la charge n'est ni un ARNsi ni un ARNmi.

**3.** Composition selon la revendication 1 ou la revendication 2, dans laquelle la charge hétérologue a été chargée de manière exogène dans les MEV après purification des MEV.

**4.** Composition selon l'une quelconque des revendications 1-3, dans laquelle :

la charge est une biomolécule ou une petite molécule ; et
la composition est formulée pour être administrée à un sujet humain.

**5.** Composition selon l'une quelconque des revendications 1-4, dans laquelle la microalgue est une espèce du genre *Chlorella,* optionnellement, dans laquelle la *Chlorella* est une espèce de *Chlorella* choisie parmi *Chlorella ellipsoidea, Chlorella pyrenoidosa, Chlorella sorokiniana, Chlorella vulgariset Chlorella variabilis;* optionnellement, dans laquelle la *Chlorella* est une *Chlorella vulgaris.*

**6.** Composition selon l'une quelconque des revendications 1-5, dans laquelle :

la composition est formulée pour une administration systémique, locale, topique, parentérale ou entérale ; ou
la composition est formulée pour une administration orale, intraveineuse, intramusculaire, sous-cutanée, intranasale, intratrachéale, par inhalation ou nébulisation, topique, locale, intratumorale, systémique, muqueuse, entérale ou intrapéritonéale.

**7.** Composition selon l'une quelconque des revendications 1-6 formulée sous forme de comprimé, de poudre, de liquide, de gélule ou de pastille.

**8.** Composition selon l'une quelconque des revendications 1-7, dans laquelle :

a) la charge comprend une molécule d'acide nucléique, un polypeptide, une protéine, un plasmide, un aptamère ou un oligonucléotide antisens ; ou
b) la charge comprend de l'ADN, ou de l'ARN codant pour un produit, ou de l'ARN double brin ; ou
c) la charge est de l'ARNm ou de l'ARNm modifié ; ou
d) la charge comprend un système de manipulation génique ; ou
e) la charge comprend un plasmide ; ou
f) la charge comprend un vecteur viral ; ou
g) la charge comprend une protéine ou un polypeptide thérapeutique ou diagnostique ; ou
h) la charge comprend un complexe protéique ; ou
i) la charge comprend un vaccin ; ou
j) la charge comprend ou code une protéine qui est un anticorps ou un fragment de celui-ci se liant à un antigène ; ou
k) la charge est un produit qui stimule le système immunitaire d'un sujet traité avec la composition ; ou
l) la charge est un produit cosméceutique, cosmétique ou à activité cosmétique ; ou
m) la charge comprend une petite molécule bioactive ; ou
n) la charge est un agent chimiothérapeutique ; ou
o) la charge est un agent immunothérapeutique ; ou
p) la charge comprend un marqueur diagnostique ou un produit détectable ; ou
q) la charge comprend un promédicament ; ou
r) la charge comprend un anticorps, ou un scFv ou autre fragment de liaison à l'antigène d'un anticorps, ou un anticorps bispécifique ; ou
s) la charge comprend un marqueur diagnostique qui comprend une luciférase ou un acide nucléique codant pour une luciférase, une protéine fluorescente ou un acide nucléique codant pour une protéine fluorescente, ou un opéron de luciférase ; ou
t) la charge est destinée au traitement ou au diagnostic d'une maladie, d'un trouble ou d'une affection d'un animal.

**9.** Composition selon la revendication 8, dans laquelle :
la charge comprend un anticorps ou un fragment de liaison à un antigène, ou toute autre forme de celui-ci ; il s'agit d'un

anticorps inhibiteur de point de contrôle immunitaire ou d'un fragment de liaison à un antigène de celui-ci ; il s'agit d'un anticorps spécifique d'un antigène tumoral ou d'un fragment de liaison à un antigène de celui-ci ; il s'agit d'un anticorps anti-oncogène spécifique ou d'un fragment de liaison à un antigène de celui-ci ; il s'agit d'un anticorps ou d'un fragment de liaison à un antigène de celui-ci ciblant un récepteur ou une molécule de signalisation tumorale ; ou, de manière optionnelle, la charge comprend un anticorps ou un fragment de liaison à un antigène qui se lie spécifiquement à un ou plusieurs des éléments suivants et les inhibe : CTLA-4, PD-1, PD-L1, PD-L2, le PD-1/PDL1 voie, le PD-1/PDL2 voie, HER2, EGFR, TIM-3, LAG-3, BTLA-4, HHLA-2, CD28, ou autres points de contrôle immunitaire, ou immunosuppresseurs, ou antigènes tumoraux.

**10.** Composition de l'une quelconque des revendications 1-9, dans laquelle :

la charge est destinée au traitement, au diagnostic ou au suivi d'une maladie, d'un trouble ou d'une affection choisis parmi : un cancer ; et une maladie, un trouble ou une affection du système respiratoire, du système nerveux central ou du système nerveux, de la peau et des épithéliums ou muqueuses exposés, ou du tube digestif ; ou

la charge comprend un acide nucléique ou une protéine, ou un acide nucléique codant pour une protéine, qui est un produit thérapeutique destiné au traitement d'une maladie, d'un trouble ou d'une affection choisis parmi : un cancer; une maladie infectieuse ; une maladie neurodégénérative ou autre maladie, trouble ou affection du SNC ; ou le vieillissement ; une maladie liée au vieillissement ; une affection cutanée ; une maladie, un trouble ou une affection ophtalmique ; et une maladie, un trouble ou une affection immunologique.

**11.** Composition selon l'une quelconque des revendications 1-10, dans laquelle :

la composition est formulée pour une administration intranasale et la charge comprend un agent thérapeutique destiné au traitement d'une maladie, d'un trouble ou d'une affection du cerveau ou impliquant le cerveau ; ou

la composition est formulée sous forme de gouttes pour administration oculaire, et la charge comprend un agent thérapeutique destiné au traitement d'une maladie, d'un trouble ou d'une affection ophtalmique ; ou

la composition est formulée pour une administration orale et la charge comprend un agent thérapeutique destiné au traitement d'une maladie, d'un trouble ou d'une affection immunologique.

**12.** Composition selon l'une quelconque des revendications 1-11, dans laquelle :

les MEV contiennent une charge composée d'une protéine immunostimulante et/ou un antigène et/ou code pour une protéine immunostimulante ; et

la charge est immunostimulante et/ou provoque une réponse immunitaire innée ou adaptative.

**13.** Composition selon la revendication 12 formulée pour une administration orale.

**14.** Composition selon la revendication 12 ou la revendication 13, dans laquelle la charge comprend un antigène, tel qu'un antigène servant à immuniser contre un agent pathogène.

**15.** Composition selon la revendication 10, dans laquelle :

l'affection est une affection cutanée ou une affection affectant la vision ; ou

l'affection se manifeste par des rides ou une décoloration de la peau ; ou par une dégénérescence maculaire ou une cataracte ; ou par une affection résultant d'une rétinopathie diabétique.

**16.** Procédé de préparation des MEV dans la composition de la revendication 1 ou de la revendication 2, comprenant :

la culture des microalgues ;

l'isolation ou la purification des MEV ; puis

l'introduction de la charge dans les MEV après isolation ou purification des MEV, dans lequel la charge est chargée dans les MEV, par exemple, par un procédé choisi parmi un ou plusieurs des procédés suivants : électroporation, sonication, extrusion et tensioactifs.

**17.** Composition selon l'une quelconque des revendications 1-15 pour une utilisation dans le traitement ou la prévention d'une maladie, d'un trouble ou d'une affection chez un animal.

**18.** Composition à utiliser selon la revendication 17, dans laquelle la composition est formulée pour une administration orale, intraveineuse, intramusculaire, sous-cutanée, intranasale, intraoculaire, intratrachéale, par inhalation, par nébulisation, topique, locale, intratumorale, systémique, muqueuse ou intrapéritonéale.

**19.** Composition destinée à être utilisée selon l'une des revendications 17 ou 18, dans laquelle :

a) la maladie, le trouble ou l'affection est choisie) parmi un cancer ; une maladie génétique ; une maladie du foie ; une maladie impliquant le système immunitaire ou les cellules immunitaires ; une maladie infectieuse ; une maladie respiratoire ; une maladie pulmonaire ; une maladie neurodégénérative ; une maladie gastro-intestinale ; un cancer du cerveau ; et une maladie, un trouble ou une affection impliquant les organes internes, le système cardiovasculaire et les organes et tissus associés, les tissus hématopoïétiques ou lymphoïdes, les organes et tissus sensoriels, les organes et tissus urogénitaux, les tissus musculaires, les os, et/ou les tissus endocriniens ; ou
b) la maladie, le trouble ou l'affection est une maladie neurodégénérative, un trouble cognitif, une maladie cérébrale, un trouble du système nerveux, une maladie génétique, une maladie multifactorielle, un cancer, une hypertension artérielle, un taux de cholestérol élevé, une schizophrénie, un trouble bipolaire, une arthrite, une maladie métabolique, la maladie de Gaucher, le syndrome de Hunter, la maladie de Krabbe, la leucinose (maladie des urines à odeur de sirop d'érable), la leucodystrophie métachromatique, l'encéphalopathie mitochondriale, l'acidose lactique, le syndrome MELAS (épisodes pseudo-AVC), la maladie de Niemann-Pick, la phénylcétonurie (PCU), la porphyrie, la maladie de Tay-Sachs, la maladie de Wilson, une maladie du système respiratoire, une bronchite chronique, un emphysème, un cancer du poumon, la mucoviscidose, une bronchectasie, une pneumonie, une maladie du tube digestif, une maladie inflammatoire chronique de l'intestin (MICI) ou un reflux gastro-œsophagien (RGO), une maladie cœliaque, une diverticulite, une maladie du tube digestif, une maladie de la muqueuse, une maladie du tissu musculaire ou neuromusculaire, une maladie des os, une maladie du tissu endocrinien, une maladie du tissu hématopoïétique, une maladie, un trouble ou une affection du ou des tissus lymphoïdes.

**20.** Composition destinée à utiliser selon l'une quelconque des revendications 17-19, dans laquelle :

a) la maladie, le trouble ou l'affection est une maladie neurodégénérative, un trouble cérébral ou un trouble du système nerveux ;

la composition est formulée pour une administration intranasale ; et
la charge comprend un produit thérapeutique destiné au traitement de la maladie, du trouble ou de l'affection ; et/ou

b) la maladie, le trouble ou l'affection est une maladie neurodégénérative, un trouble cérébral, un trouble du système nerveux ou un trouble psychiatrique ; et/ou
c) la maladie, le trouble ou l'affection implique ou la charge cible le tractus gastro-intestinal, le système immunitaire ou la rate blanche pour le traitement ; et
la composition est formulée pour une administration orale ; et/ou
d) la maladie, le trouble ou l'affection implique le système immunitaire ou est une maladie, un trouble ou une affection qui peut être traitée en administrant un traitement aux cellules du système immunitaire.

**21.** Composition destinée à être utilisée selon l'une quelconque des revendications 17-20, dans laquelle :

a) la composition est formulée pour une administration orale ; et la maladie est traitée par immunomodulation ; ou
b) la composition est formulée pour une administration orale ; et
les organes et les tissus impliqués dans la maladie, le trouble ou l'affection sont accessibles par la circulation sanguine ; ou
c) la maladie est une infection intestinale, la maladie de Crohn ou un cancer ; et la composition est formulée pour une administration orale ; ou
d) la composition est formulée pour une administration intratrachéale, par inhalation ou par nébulisation ; et la charge cible les voies respiratoires et/ou les poumons pour le traitement des affections respiratoires et/ou maladies, troubles ou affections pulmonaires.

**22.** Composition destinée à être utilisée selon la revendication 21, dans laquelle :

la maladie est choisie parmi les suivantes : bronchopneumopathie chronique obstructive (BPCO), hypertension pulmonaire, asthme, autres maladies pulmonaires inflammatoires, mucoviscidose, déficit en alpha-antitrypsine (AAT), erreur innée du métabolisme, maladie pulmonaire, cancer et métastases cancéreuses touchant les poumons et le système respiratoire ; et
la composition est formulée pour une administration intratrachéale, par inhalation ou par nébulisation.

**23.** Composition destinée à être utilisée selon la revendication 17, dans laquelle la composition est formulée pour être administrée dans l'œil sous forme de gouttes ophtalmiques ; et
la maladie, le trouble ou l'affection est une maladie, un trouble ou une affection ophtalmique.

**24.** Composition destinée à être utilisée selon l'une quelconque des revendications 17-23, dans laquelle la maladie, le trouble ou l'affection implique un agent infectieux ; et, en option, l'agent infectieux est un ou plusieurs éléments parmi des bactéries, virus, oomycètes et champignons, et/ou la composition est formulée, en option, pour une administration orale.

**25.** Composition selon l'une quelconque des revendications 1-15 ou composition destinée à être utilisée selon l'une quelconque des revendications 17-24, dans laquelle la microalgue est une espèce de *Chlorella,* telle que la *Chlorella vulgaris.*

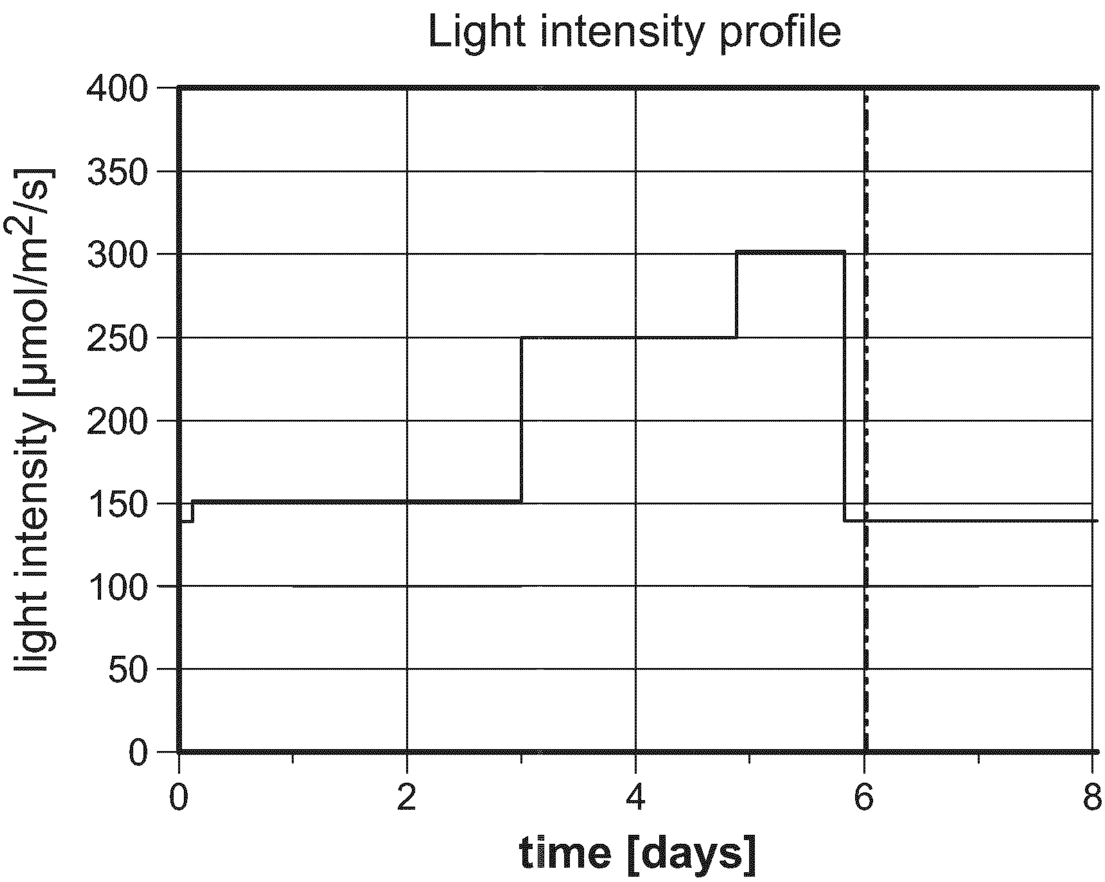
Light intensity profile
light intensity [µmol/m2/s]
400
350
300
250
200
150
100
50
0
0
2
4
6
8
time [days]

FIG. 1

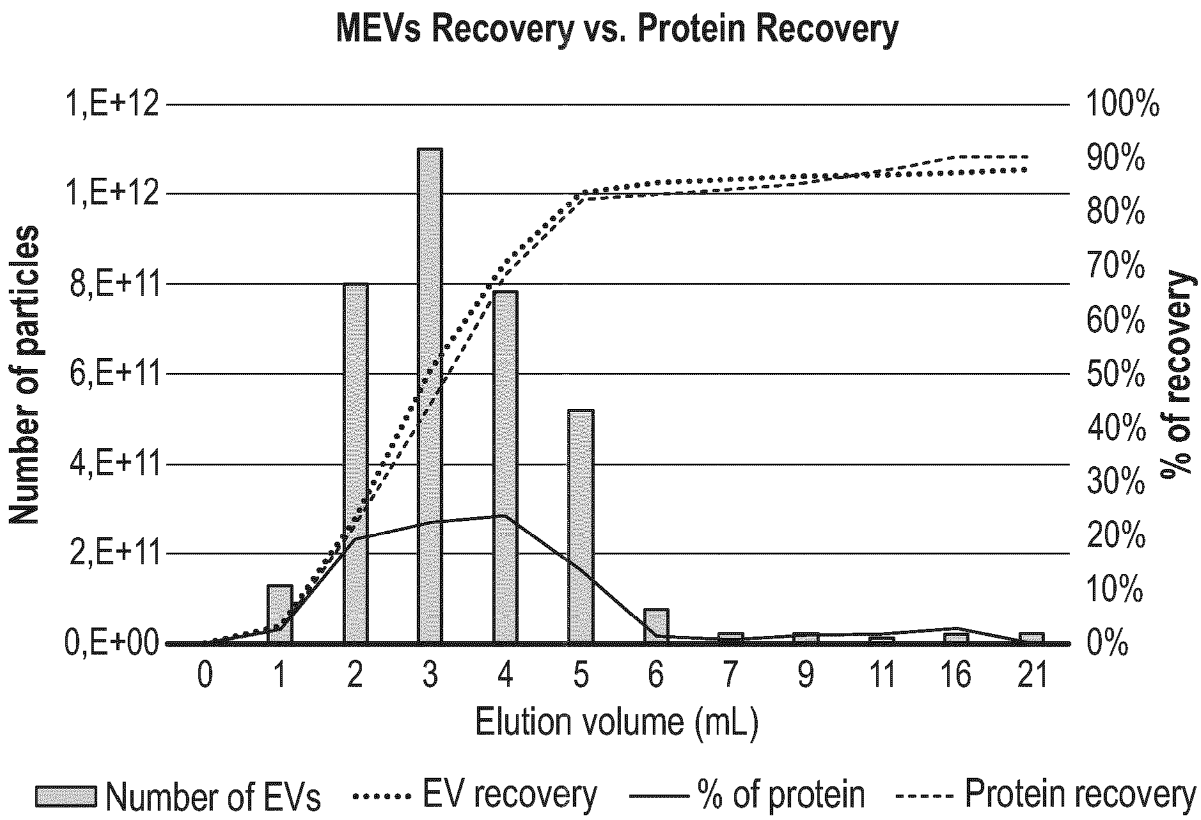
MEVs Recovery vs. Protein Recovery
1,E+12
1,E+12
8,E+11
6,E+11
4,E+11
2,E+11
0,E+00
Number of particles
100%
90%
80%
70%
60%
50%
40%
30%
20%
10%
0%
% of recovery
0
1
2
3
4
5
6
7
9
11
16
21
Elution volume (mL)
Number of EVs
EV recovery
% of protein
Protein recovery

FIG. 2

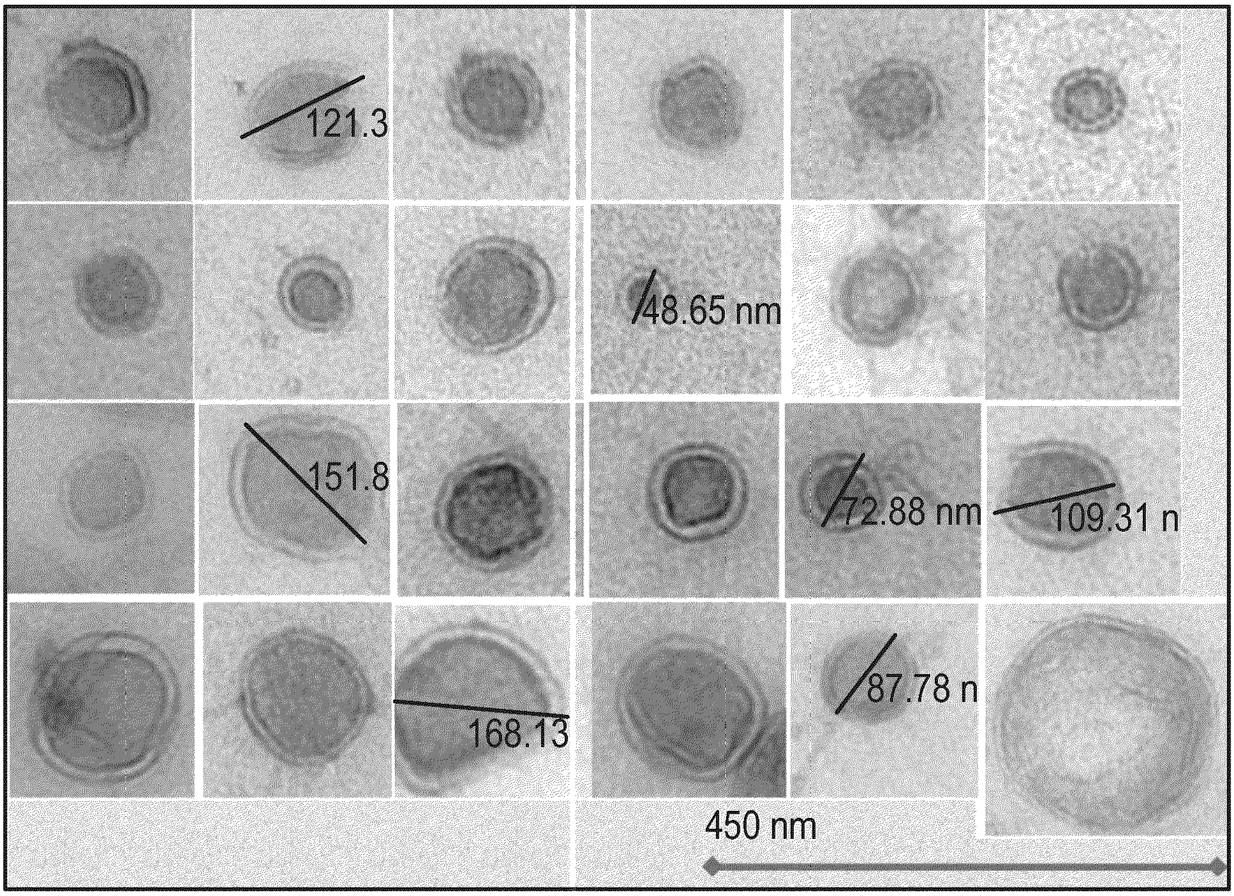


FIG. 3

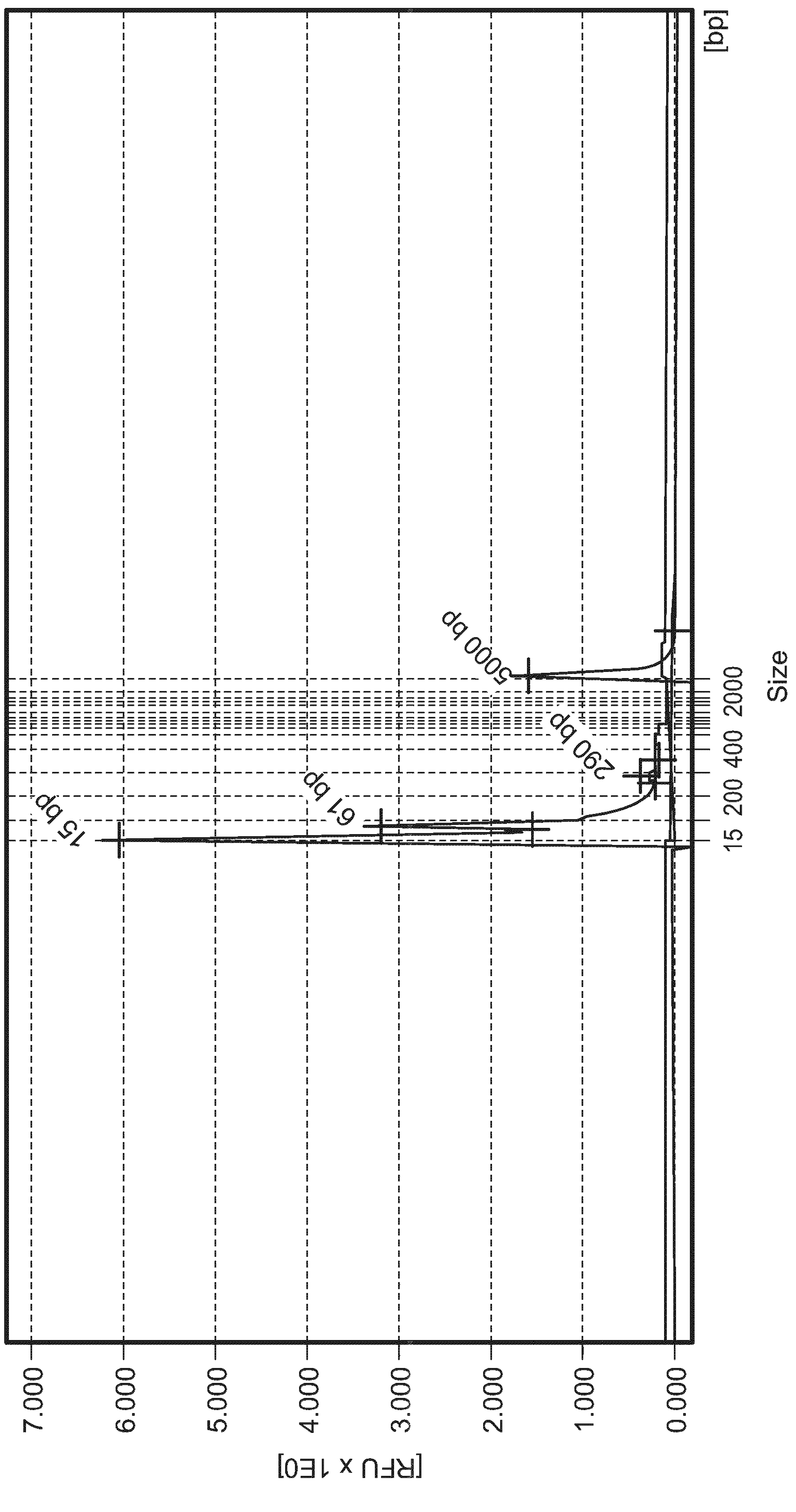
7.000
6.000
5.000
4.000
3.000
2.000
1.000
0.000
[RFU x 1E0]
15 bp
61 bp
290 bp
5000 bp
15
200
400
2000
Size
[bp]

FIG. 4

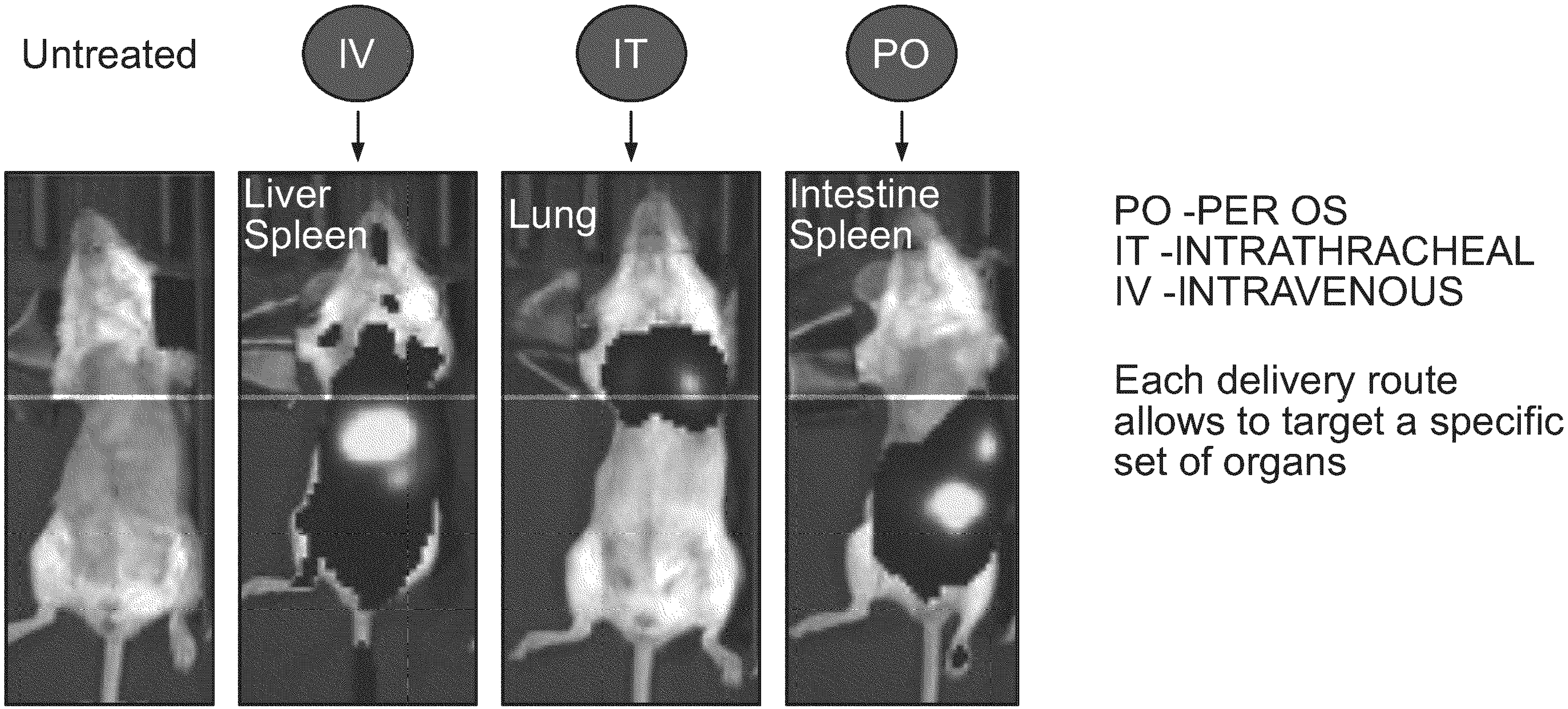
Untreated
IV
IT
PO
Liver
Spleen
Lung
Intestine
Spleen
PO -PER OS
IT -INTRATHRACHEAL
IV -INTRAVENOUS
Each delivery route
allows to target a specific
set of organs

FIG. 5

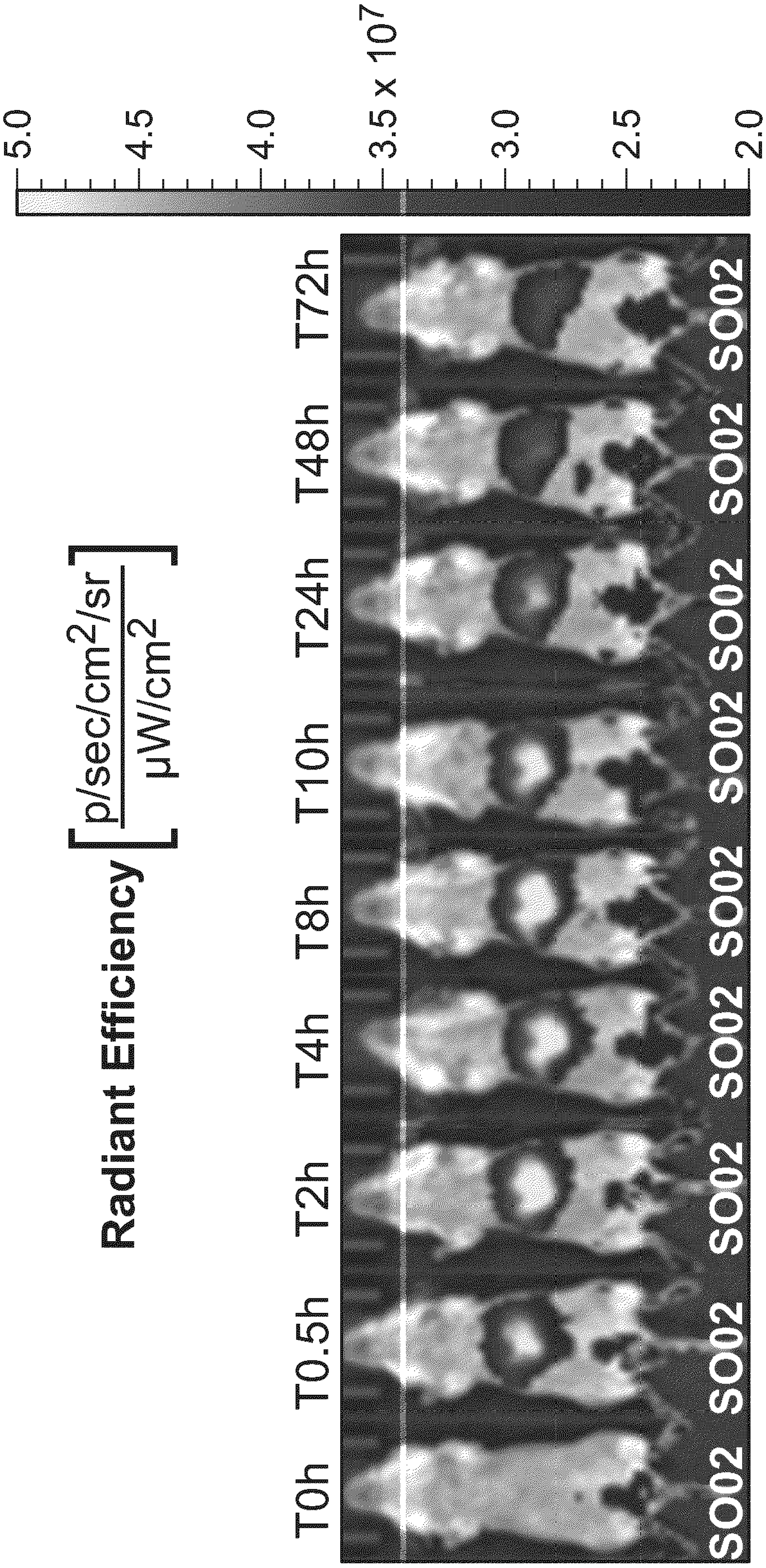
Radiant Efficiency [p/sec/cm²/sr / µW/cm²]
5.0
4.5
4.0
3.5 x 10⁷
3.0
2.5
2.0
T0h
T0.5h
T2h
T4h
T8h
T10h
T24h
T48h
T72h
SO02
SO02
SO02
SO02
SO02
SO02
SO02
SO02
SO02

FIG. 6

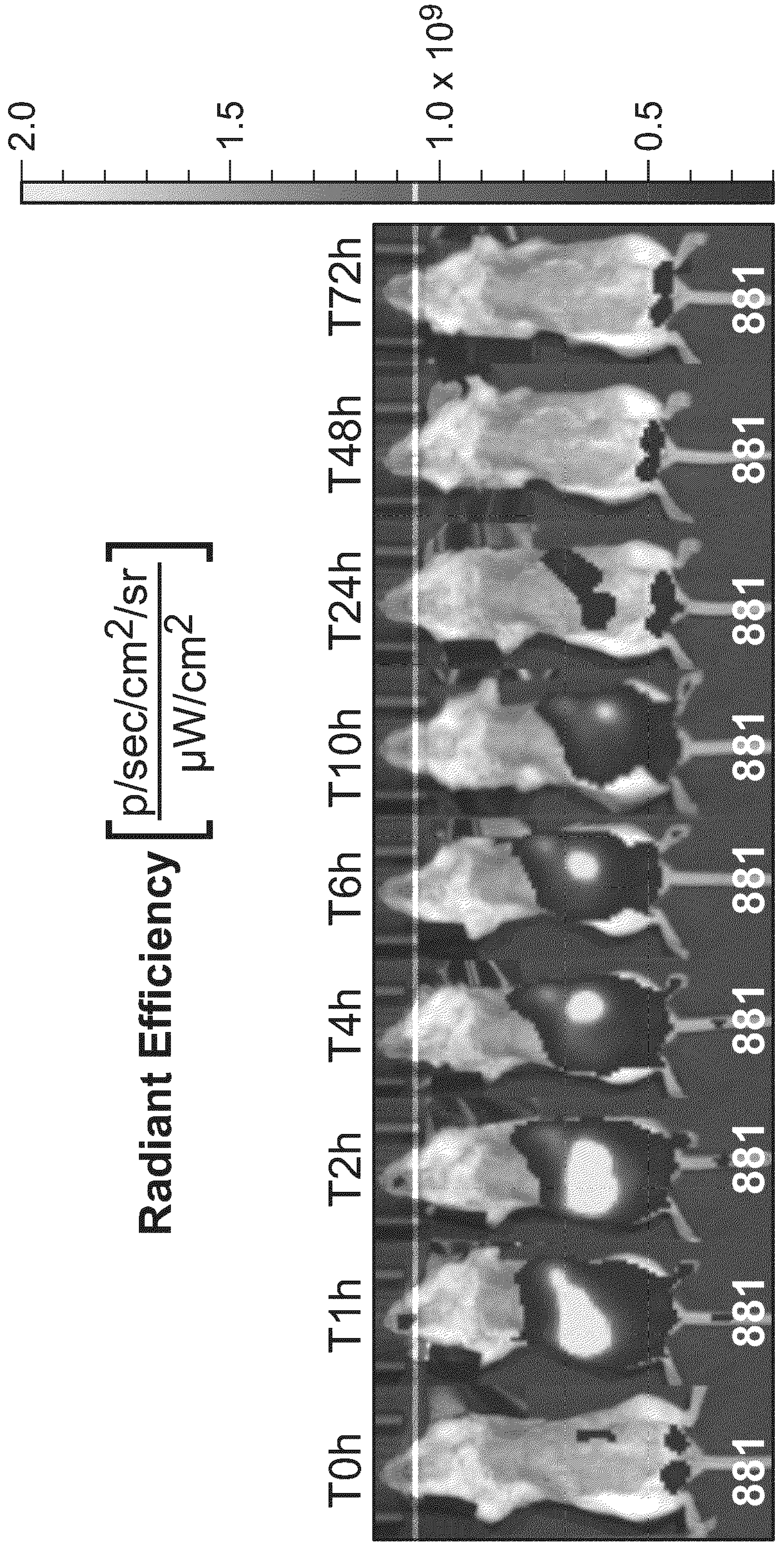
Radiant Efficiency
p/sec/cm2/sr
μW/cm2
2.0
1.5
1.0 x 10^9
0.5
T0h
T1h
T2h
T4h
T6h
T10h
T24h
T48h
T72h
881

FIG. 7

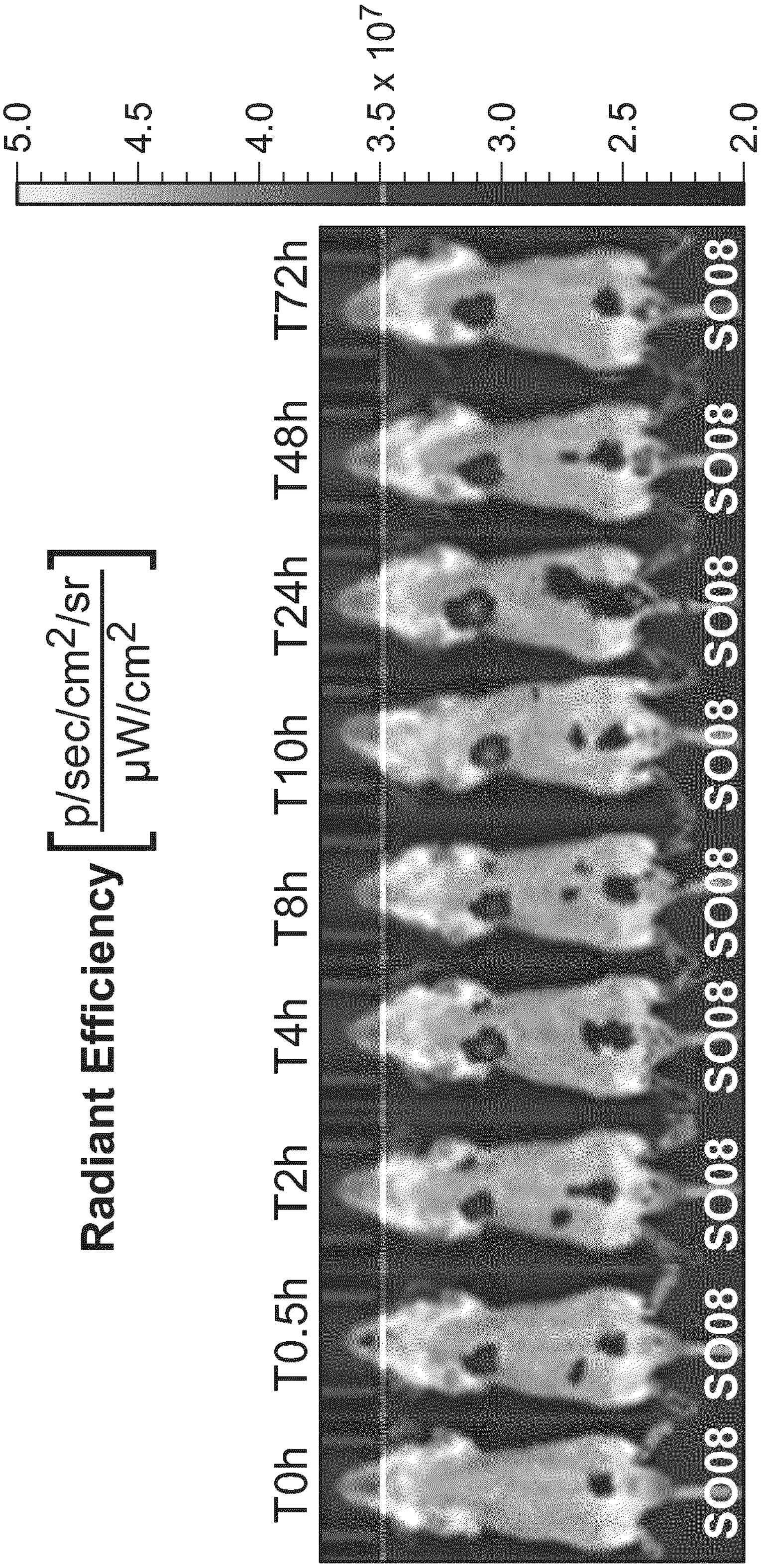
Radiant Efficiency
p/sec/cm²/sr
μW/cm²
5.0
4.5
4.0
3.5 x 10⁷
3.0
2.5
2.0
T0h
T0.5h
T2h
T4h
T8h
T10h
T24h
T48h
T72h
SO08
SO08
SO08
SO08
SO08
SO08
SO08
SO08
SO08

FIG. 8

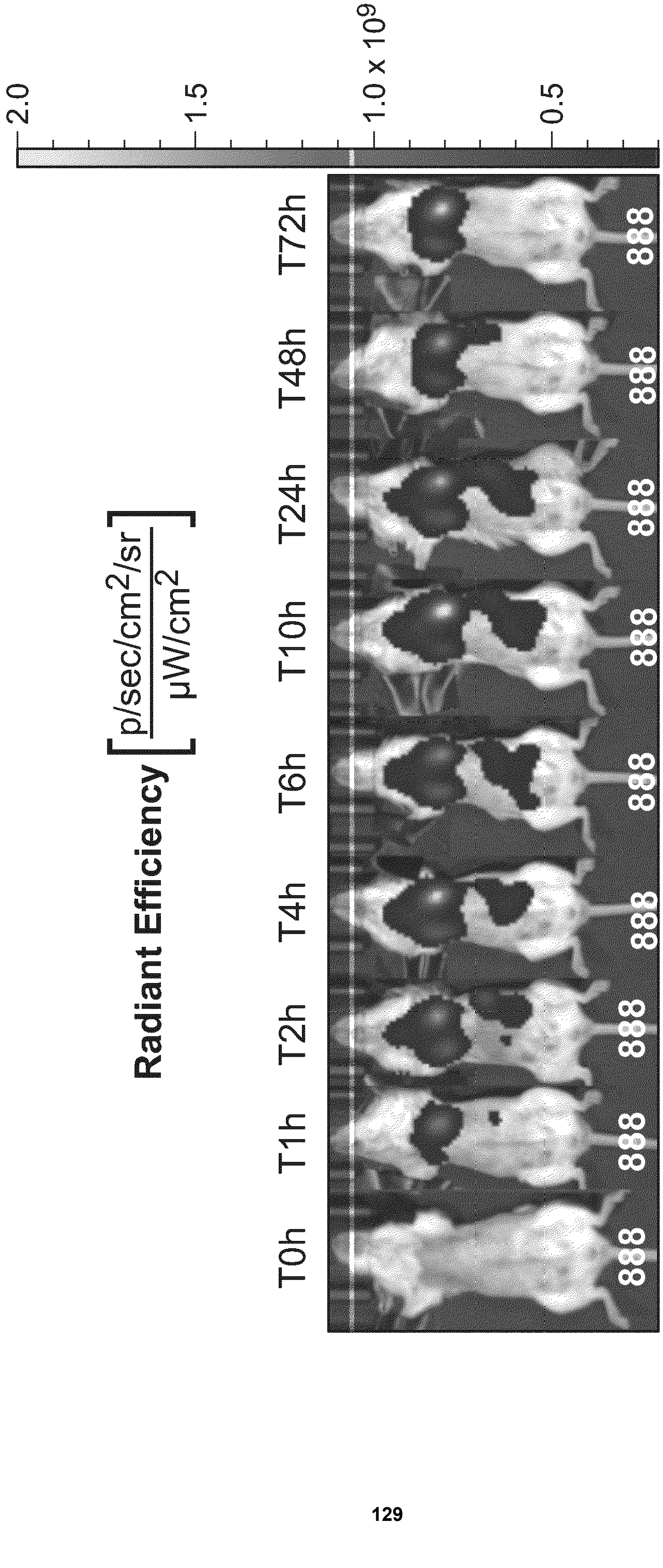

Radiant Efficiency
p/sec/cm²/sr / μW/cm²
2.0
1.5
1.0 x 10^9
0.5
T0h
T1h
T2h
T4h
T6h
T10h
T24h
T48h
T72h
888


FIG. 9

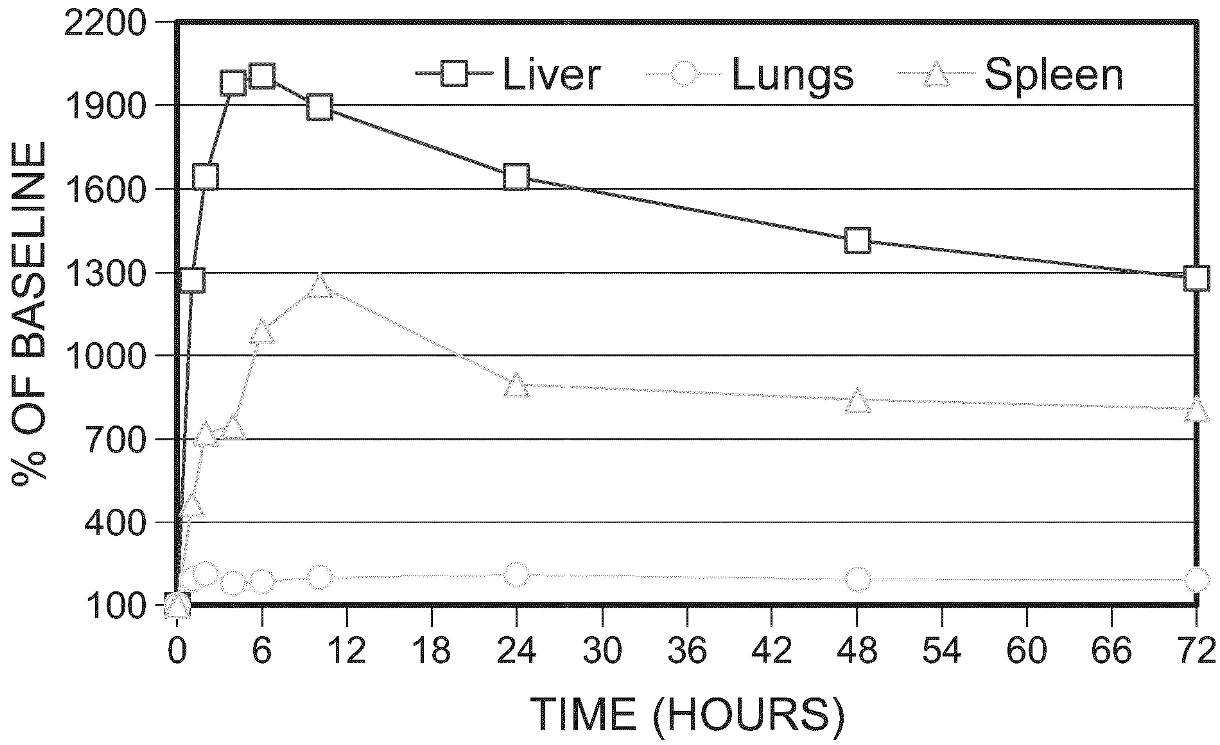
2200
1900
1600
1300
1000
700
400
100
% OF BASELINE
Liver
Lungs
Spleen
0
6
12
18
24
30
36
42
48
54
60
66
72
TIME (HOURS)

**FIG. 10**

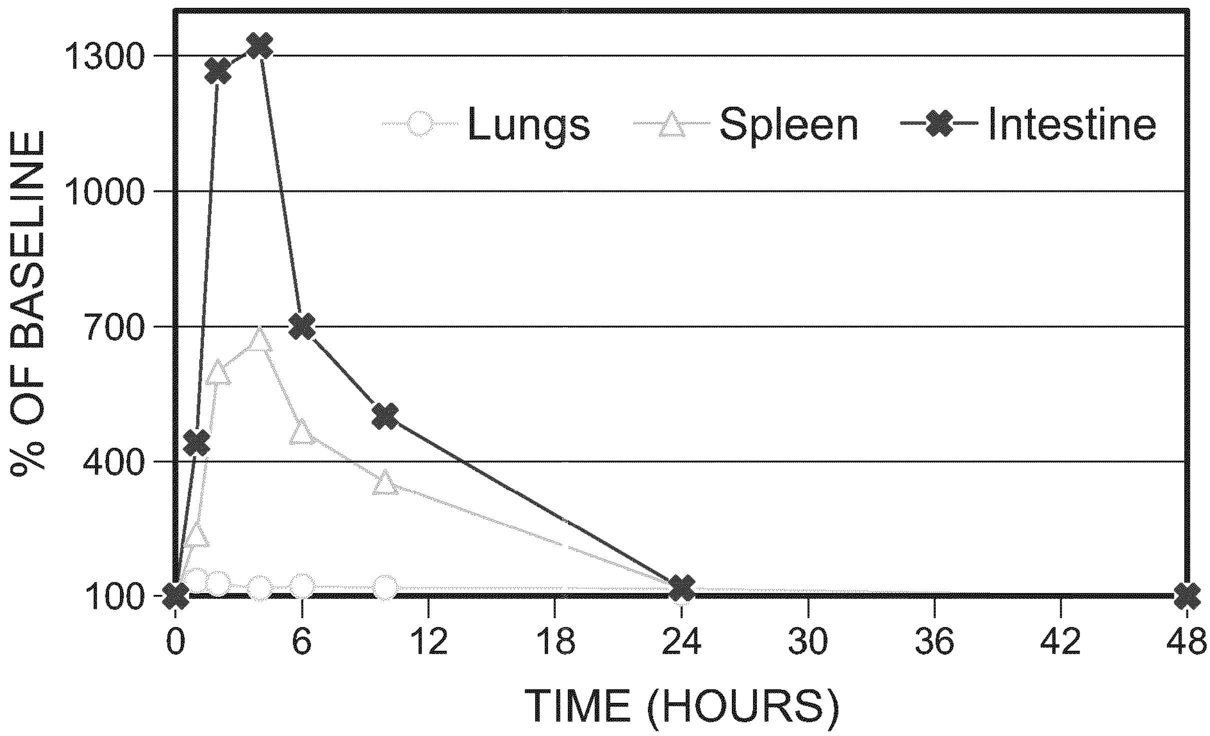
% OF BASELINE
1300
1000
700
400
100
Lungs
Spleen
Intestine
0
6
12
18
24
30
36
42
48
TIME (HOURS)

**FIG. 11**

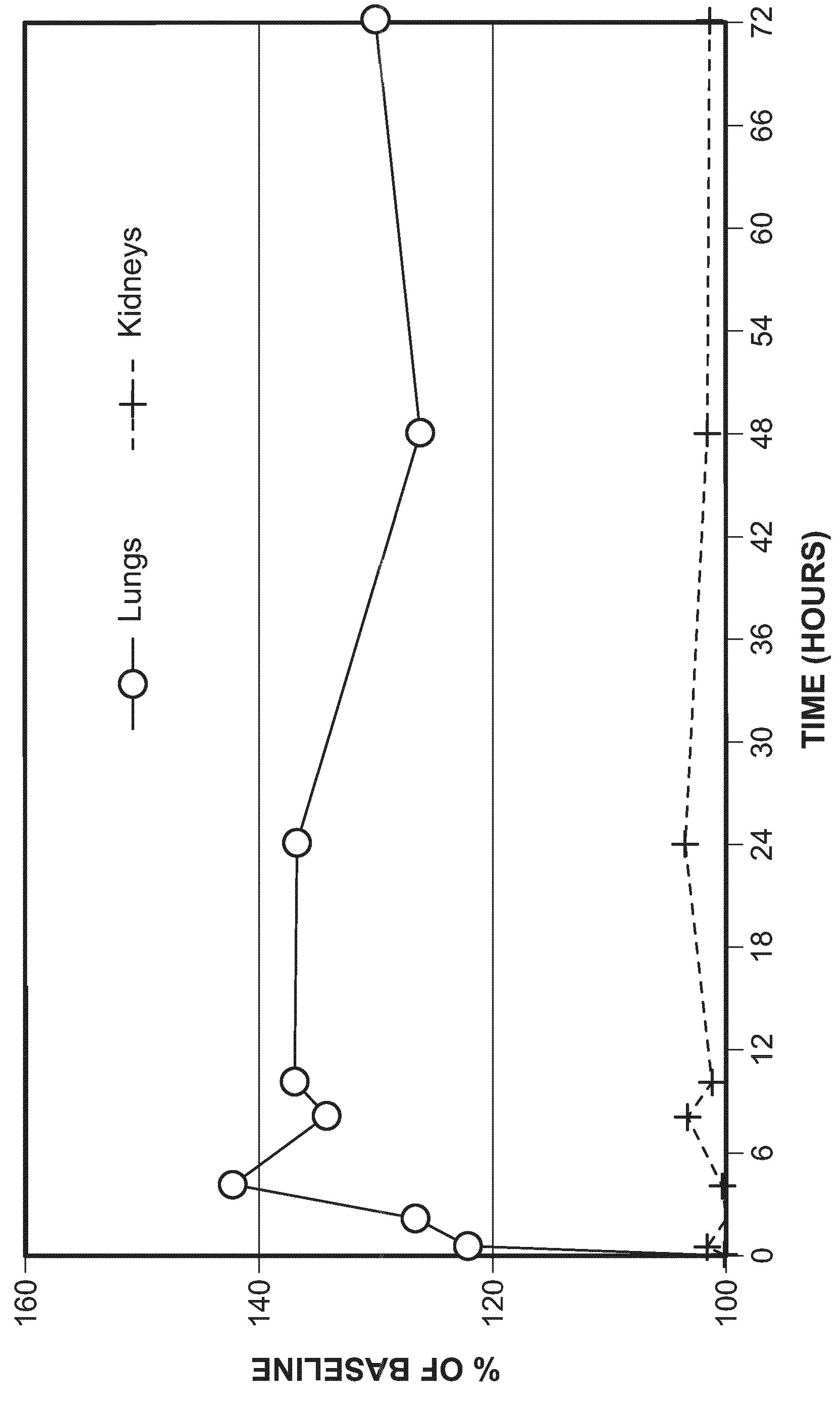
Lungs
Kidneys
% OF BASELINE
160
140
120
100
TIME (HOURS)
0
6
12
18
24
30
36
42
48
54
60
66
72

FIG. 12

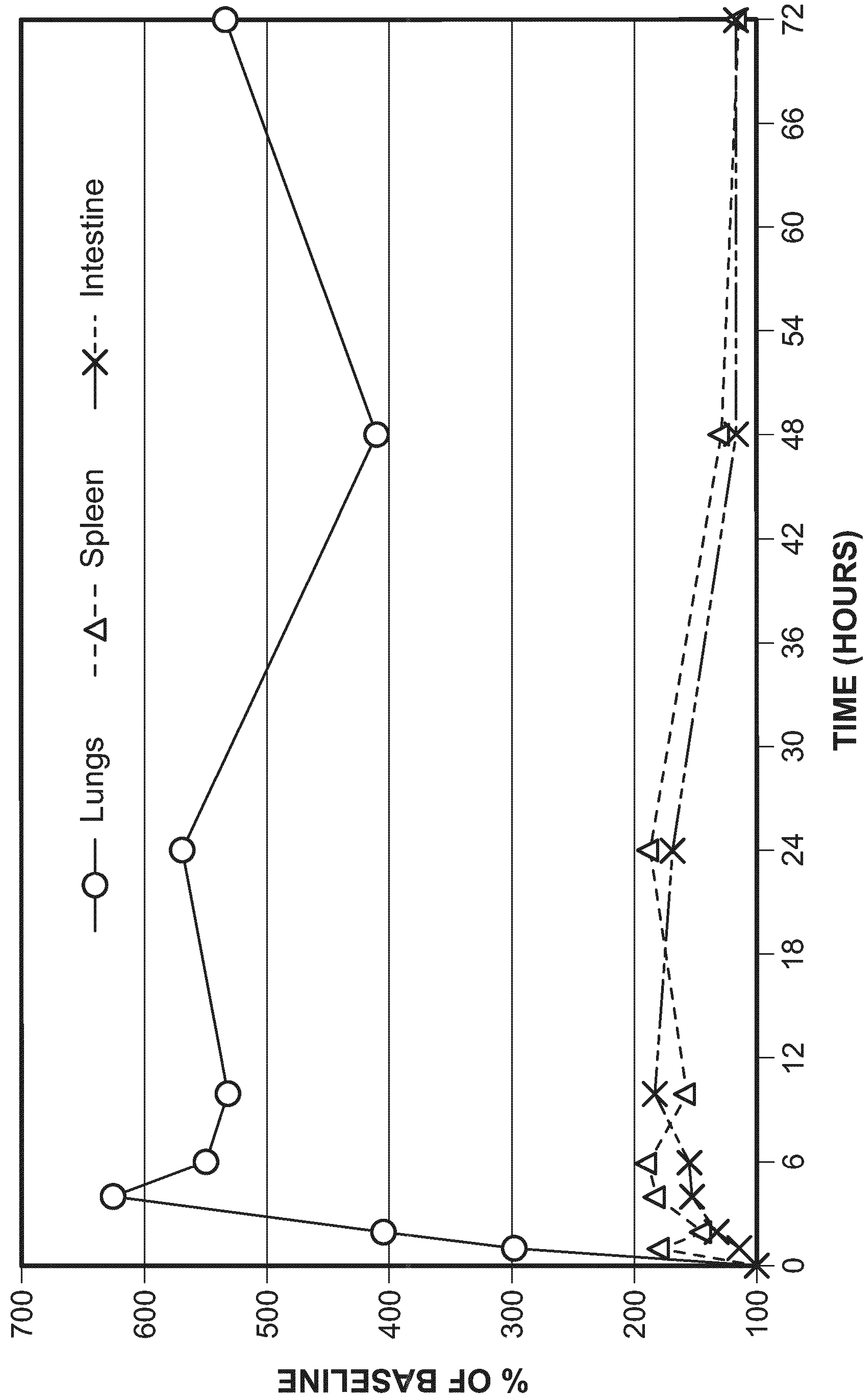
Lungs
Spleen
Intestine
% OF BASELINE
700
600
500
400
300
200
100
0
6
12
18
24
30
36
42
48
54
60
66
72
TIME (HOURS)

FIG. 13

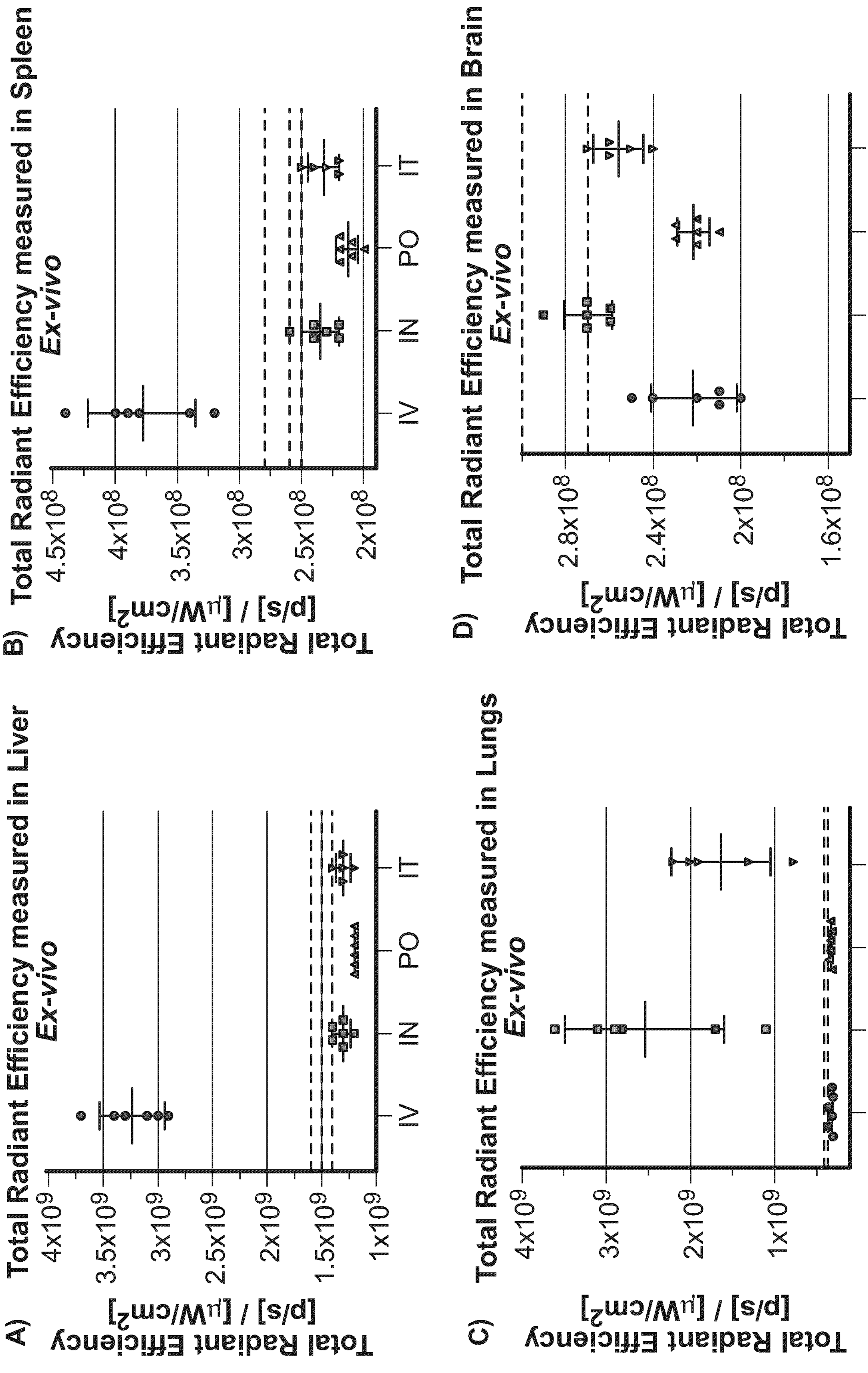
A) Total Radiant Efficiency measured in Liver
Ex-vivo
Total Radiant Efficiency [p/s] / [µW/cm2]
4x10^9
3.5x10^9
3x10^9
2.5x10^9
2x10^9
1.5x10^9
1x10^9
IV
IN
PO
IT
B) Total Radiant Efficiency measured in Spleen
Ex-vivo
Total Radiant Efficiency [p/s] / [µW/cm2]
4.5x10^8
4x10^8
3.5x10^8
3x10^8
2.5x10^8
2x10^8
IV
IN
PO
IT
C) Total Radiant Efficiency measured in Lungs
Ex-vivo
Total Radiant Efficiency [p/s] / [µW/cm2]
4x10^9
3x10^9
2x10^9
1x10^9
IV
IN
PO
IT
D) Total Radiant Efficiency measured in Brain
Ex-vivo
Total Radiant Efficiency [p/s] / [µW/cm2]
2.8x10^8
2.4x10^8
2x10^8
1.6x10^8
IV
IN
PO
IT

FIG. 14

A)

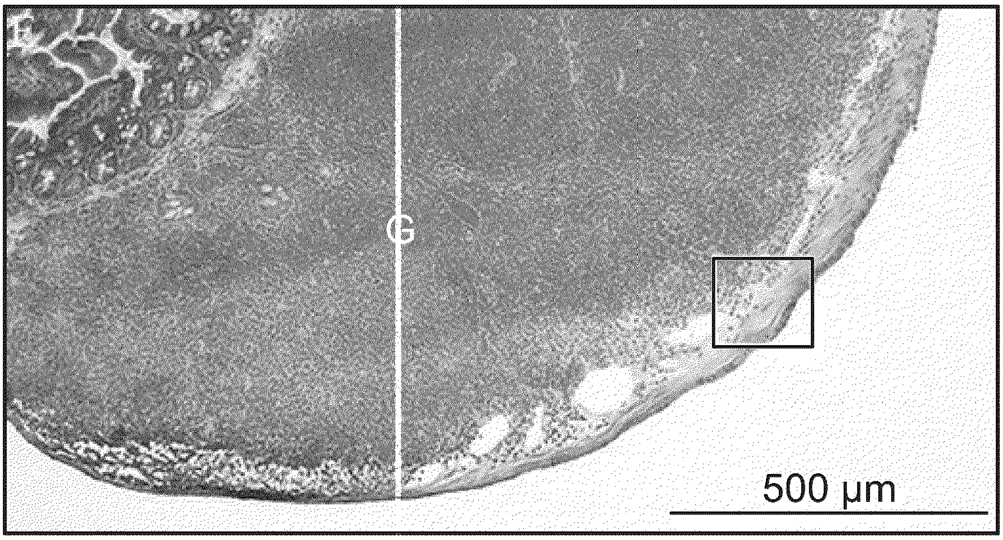


B)

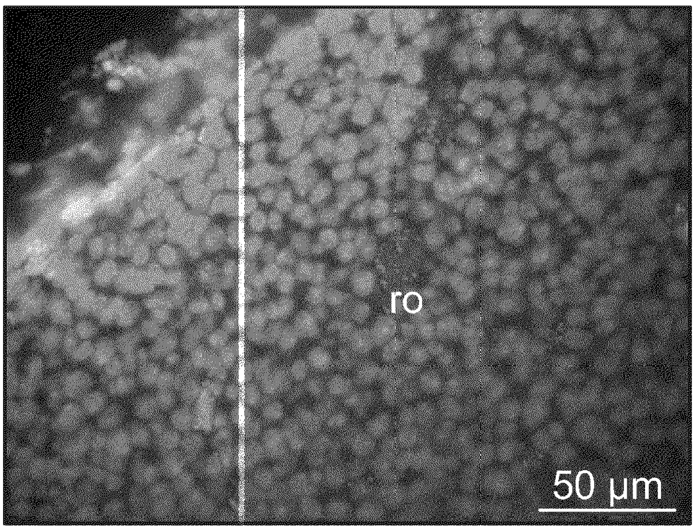


FIG. 15

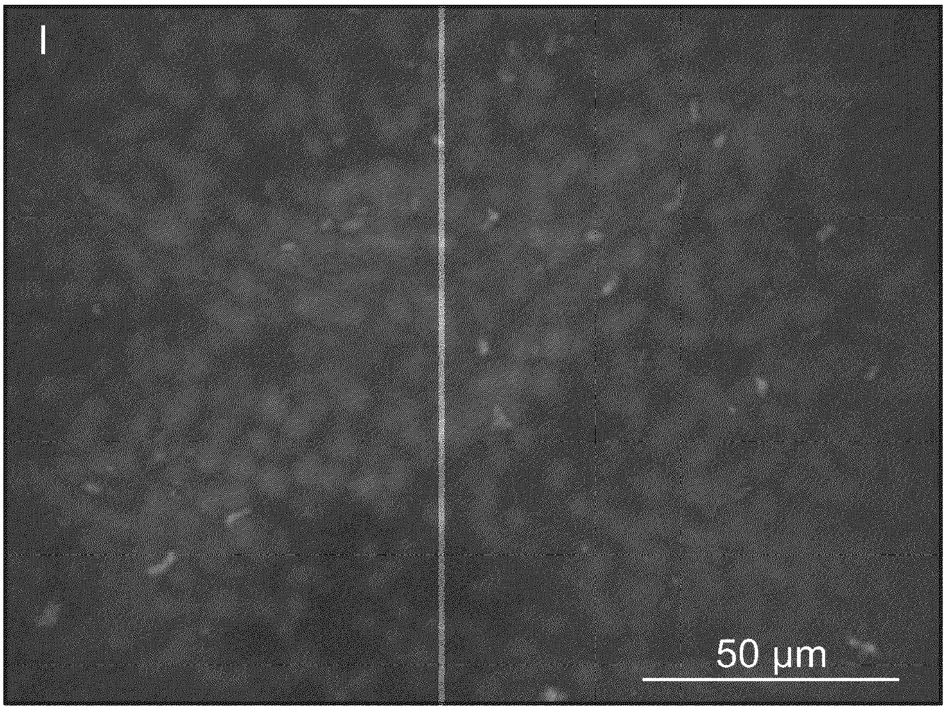


FIG. 16

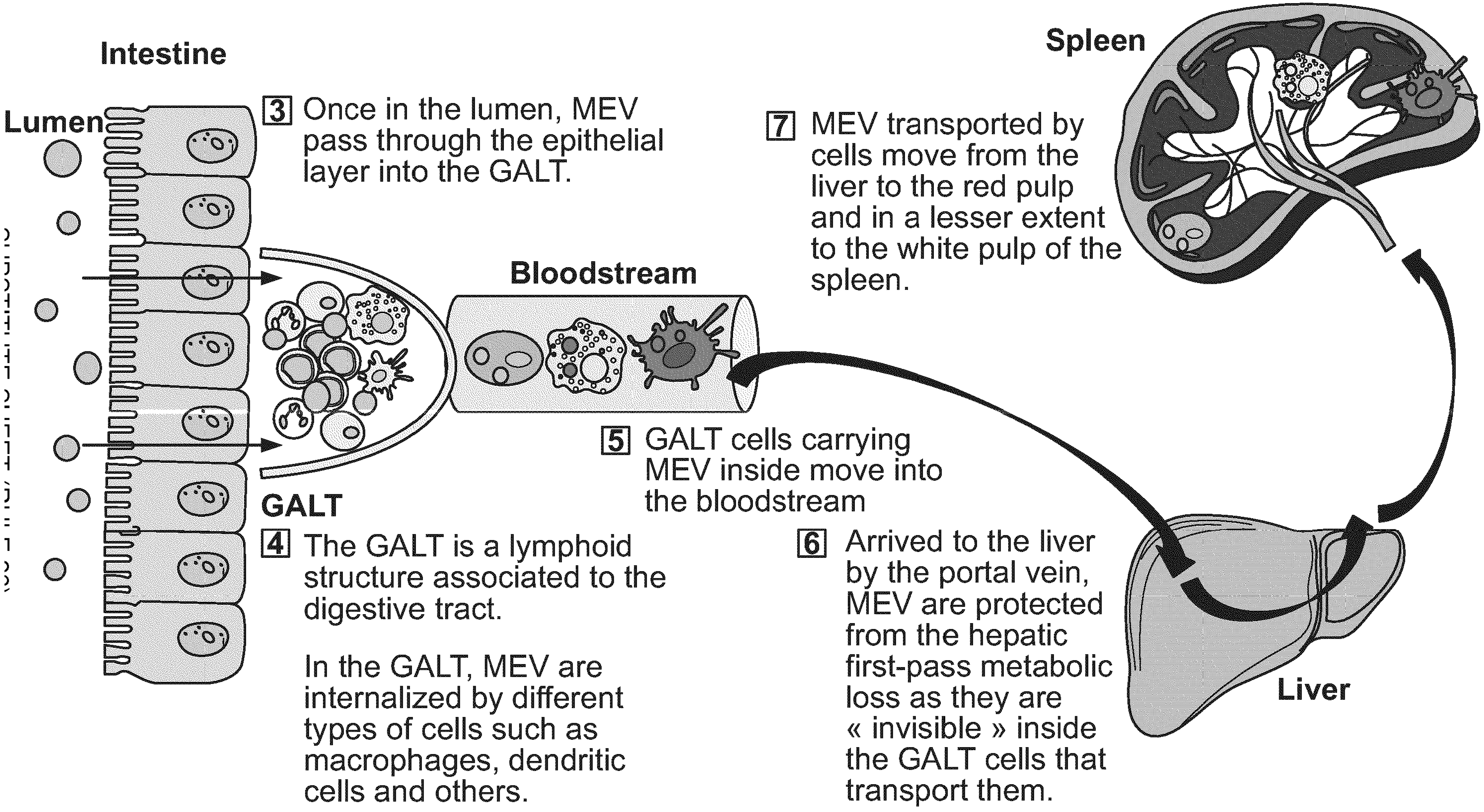
Intestine
Lumen
3 Once in the lumen, MEV pass through the epithelial layer into the GALT.
GALT
4 The GALT is a lymphoid structure associated to the digestive tract.
In the GALT, MEV are internalized by different types of cells such as macrophages, dendritic cells and others.
Bloodstream
5 GALT cells carrying MEV inside move into the bloodstream
6 Arrived to the liver by the portal vein, MEV are protected from the hepatic first-pass metabolic loss as they are « invisible » inside the GALT cells that transport them.
Liver
7 MEV transported by cells move from the liver to the red pulp and in a lesser extent to the white pulp of the spleen.
Spleen

FIG. 17

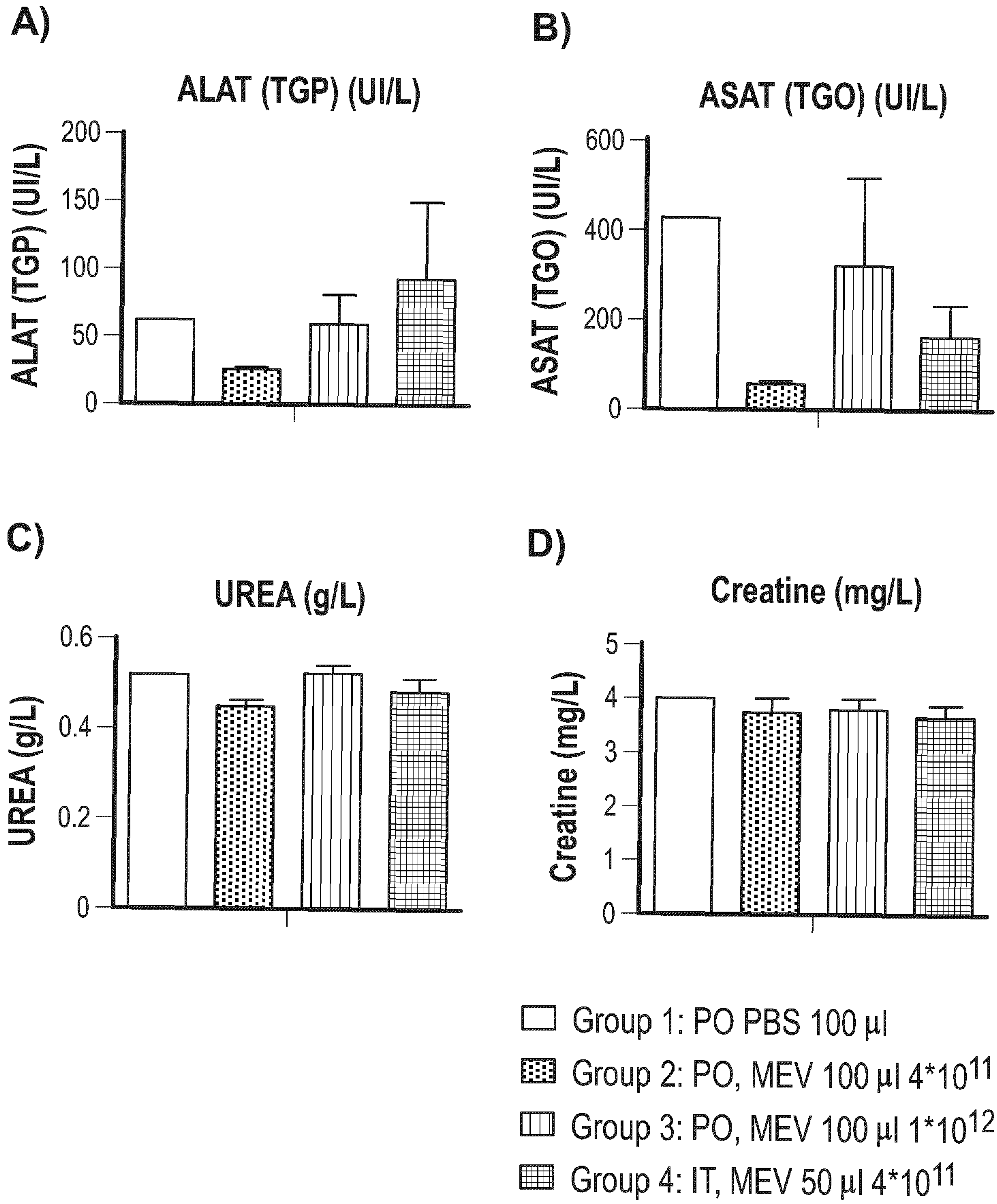
A)
ALAT (TGP) (UI/L)
ALAT (TGP) (UI/L)
200
150
100
50
0
B)
ASAT (TGO) (UI/L)
ASAT (TGO) (UI/L)
600
400
200
0
C)
UREA (g/L)
UREA (g/L)
0.6
0.4
0.2
0
D)
Creatine (mg/L)
Creatine (mg/L)
5
4
3
2
1
0
Group 1: PO PBS 100 µl
Group 2: PO, MEV 100 µl 4*10^11
Group 3: PO, MEV 100 µl 1*10^12
Group 4: IT, MEV 50 µl 4*10^11

FIG. 18

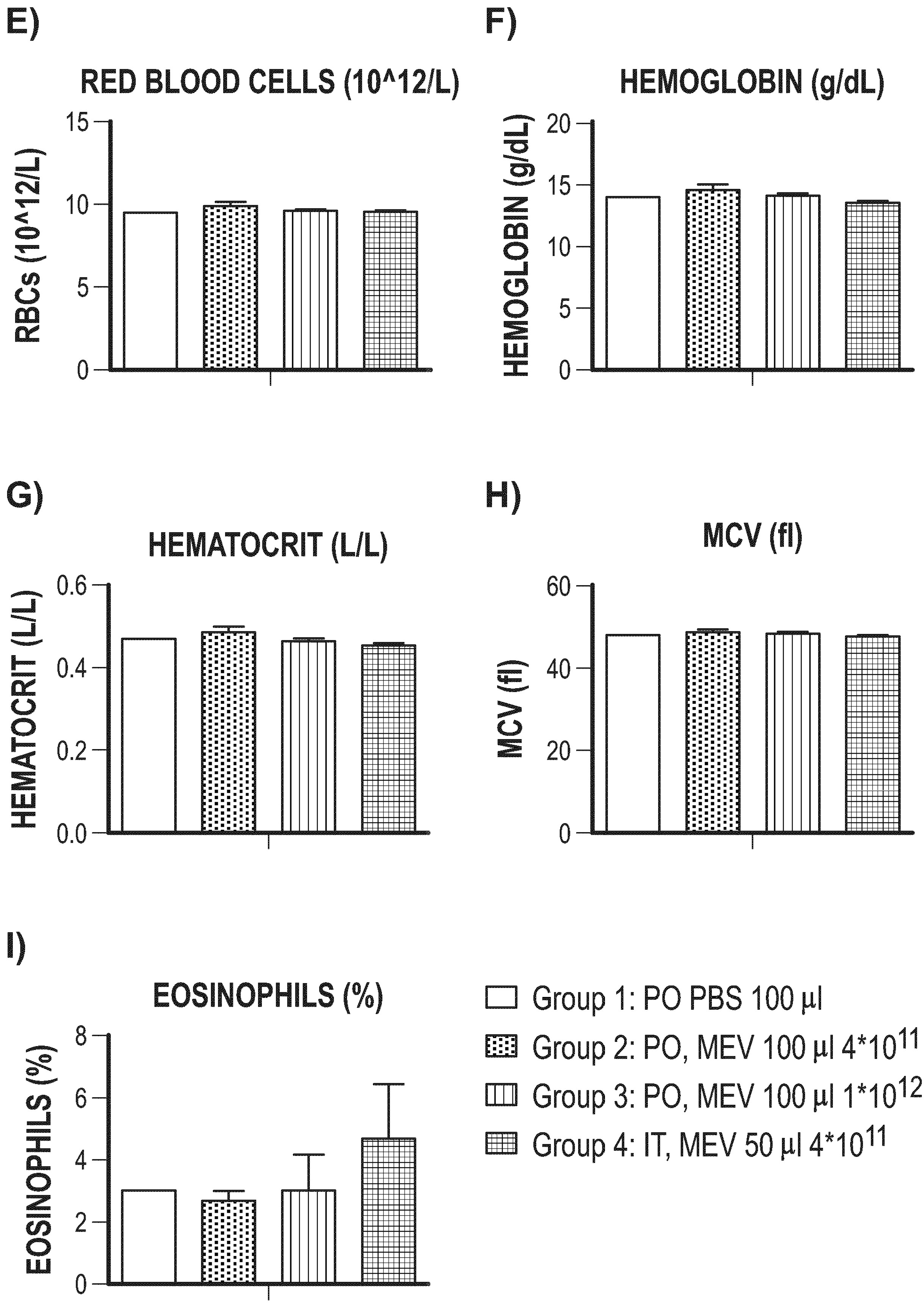
E)
RED BLOOD CELLS (10^12/L)
RBCs (10^12/L)
15
10
5
0
F)
HEMOGLOBIN (g/dL)
HEMOGLOBIN (g/dL)
20
15
10
5
0
G)
HEMATOCRIT (L/L)
HEMATOCRIT (L/L)
0.6
0.4
0.2
0.0
H)
MCV (fl)
MCV (fl)
60
40
20
0
I)
EOSINOPHILS (%)
EOSINOPHILS (%)
8
6
4
2
0
Group 1: PO PBS 100 µl
Group 2: PO, MEV 100 µl 4*10^11
Group 3: PO, MEV 100 µl 1*10^12
Group 4: IT, MEV 50 µl 4*10^11

FIG. 18 (Cont.)

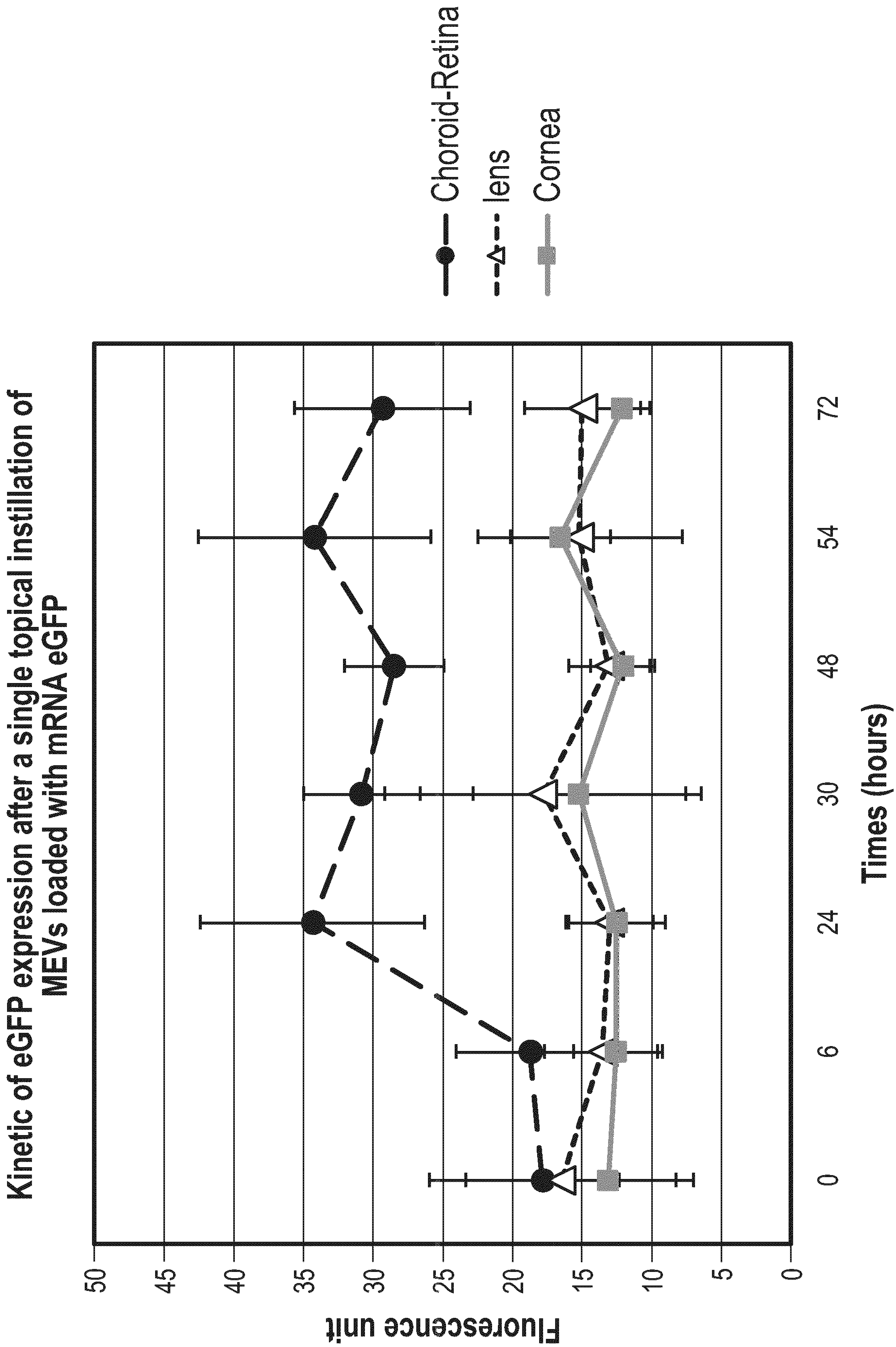
Kinetic of eGFP expression after a single topical instillation of MEVs loaded with mRNA eGFP
Fluorescence unit
0
5
10
15
20
25
30
35
40
45
50
0
6
24
30
48
54
72
Times (hours)
Choroid-Retina
lens
Cornea

FIG. 19

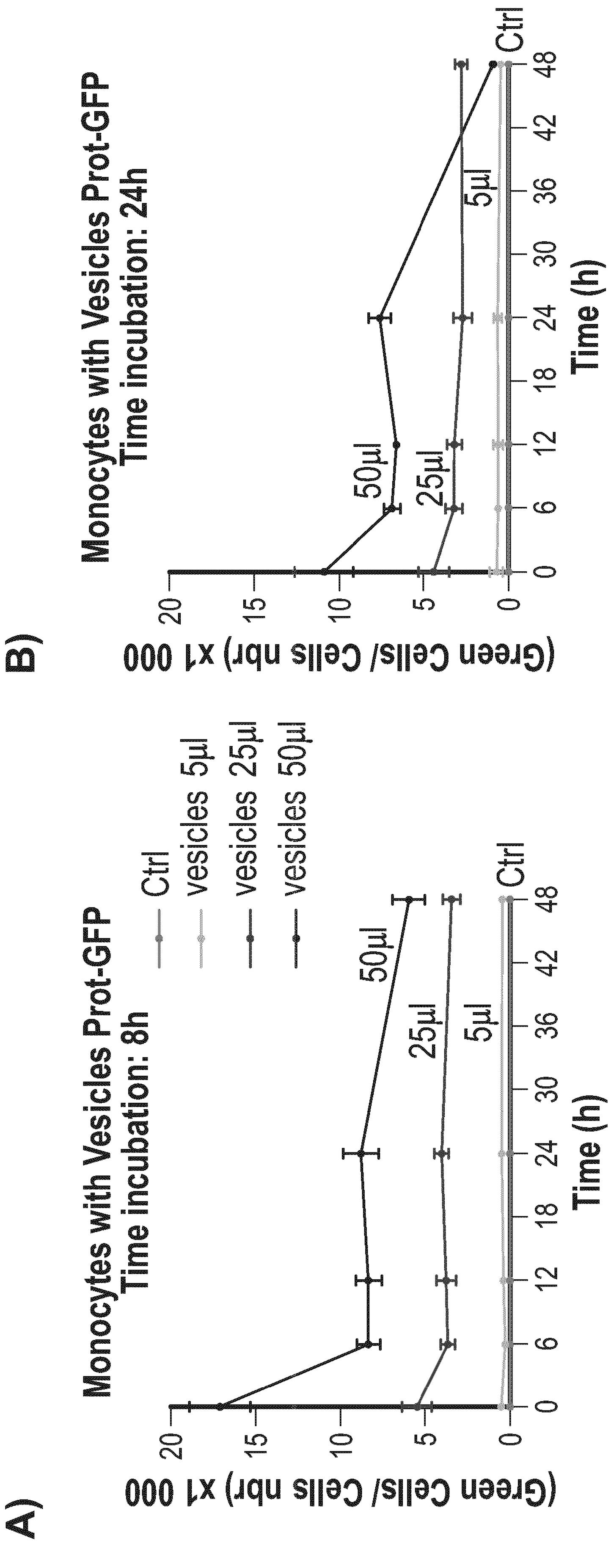
A)
Monocytes with Vesicles Prot-GFP
Time incubation: 8h
(Green Cells/ Cells nbr) x1 000
20
15
10
5
0
0
6
12
18
24
30
36
42
48
Time (h)
Ctrl
vesicles 5µl
vesicles 25µl
vesicles 50µl
50µl
25µl
5µl
Ctrl
B)
Monocytes with Vesicles Prot-GFP
Time incubation: 24h
(Green Cells/ Cells nbr) x1 000
20
15
10
5
0
0
6
12
18
24
30
36
42
48
Time (h)
50µl
25µl
5µl
Ctrl

FIG. 20

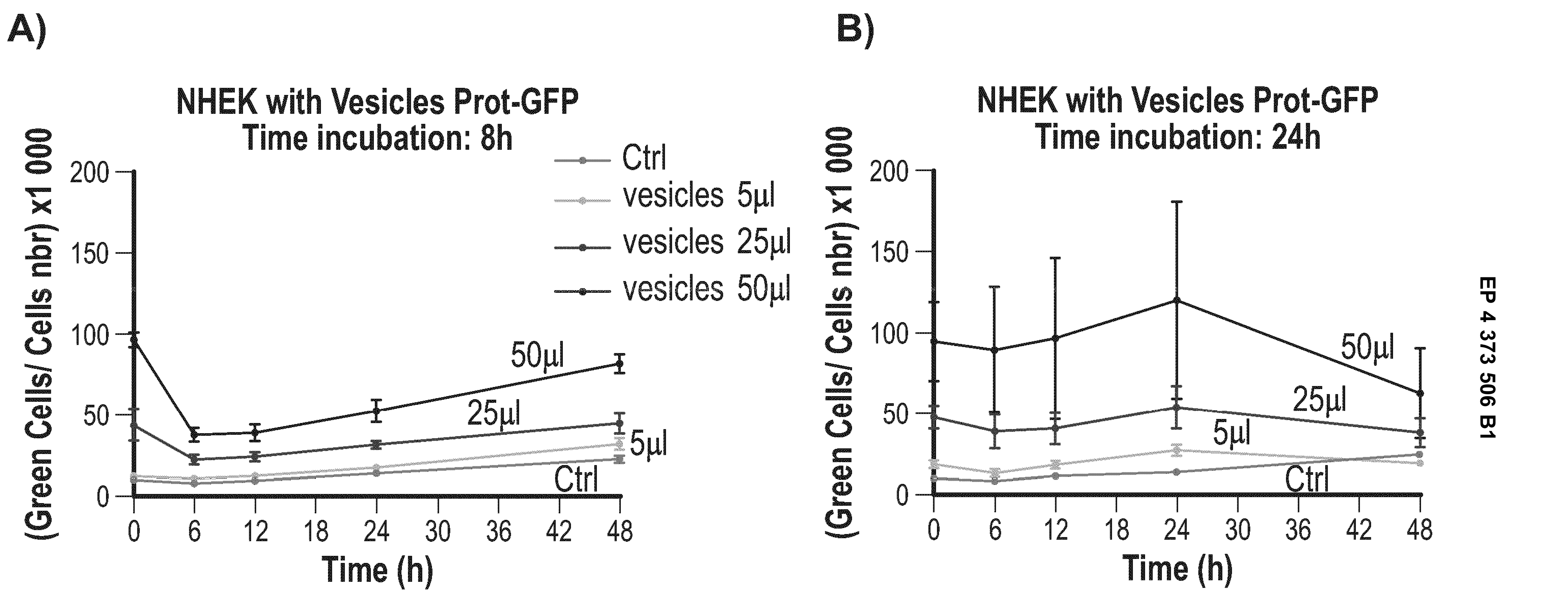
A)
NHEK with Vesicles Prot-GFP
Time incubation: 8h
(Green Cells/ Cells nbr) x1 000
200
150
100
50
0
0
6
12
18
24
30
36
42
48
Time (h)
Ctrl
vesicles 5µl
vesicles 25µl
vesicles 50µl
50µl
25µl
5µl
Ctrl
B)
NHEK with Vesicles Prot-GFP
Time incubation: 24h
(Green Cells/ Cells nbr) x1 000
200
150
100
50
0
0
6
12
18
24
30
36
42
48
Time (h)
50µl
25µl
5µl
Ctrl

FIG. 21

Hep-G2 cell line-24h

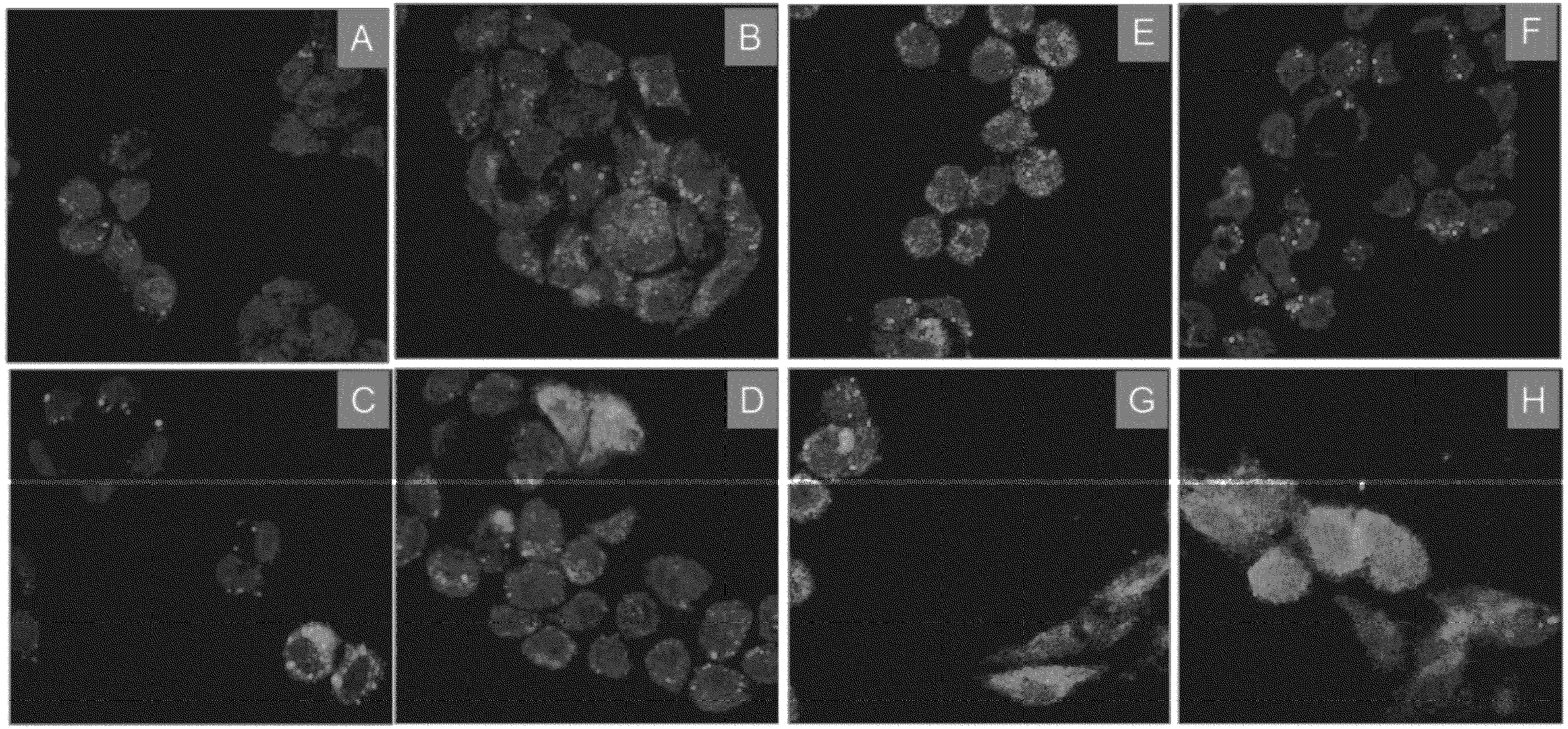


A- CTRL non loaded MEV

B- MEV-mRNA-eGFP
(200 loaded MEV per cell)

C- and D-MEV-mRNA-eGFP
(400 loaded MEV per cell)

E- MEV-GFP protein
(100 loaded MEV per cell)

F- MEV-GFP protein
(200 loaded MEV per cell)

G- and H-MEV-GFP protein
(400 loaded MEV per cell)

FIG. 22

Huh7 cell line-24h

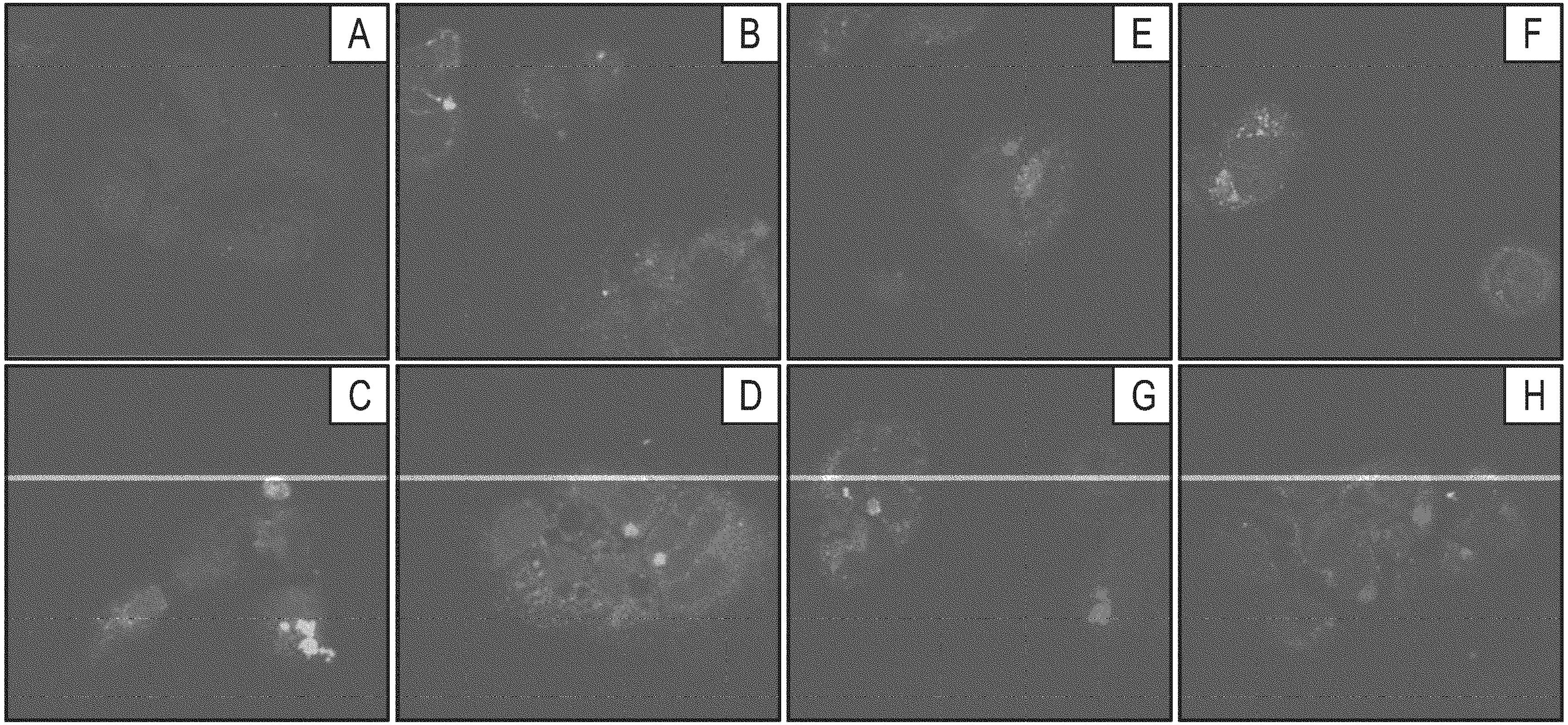


A- CTRL non loaded MEV

B- MEV-mRNA-eGFP
(200 loaded MEV per cell)

C- and D-MEV-mRNA-eGFP
(400 loaded MEV per cell)

E- MEV-GFP protein
(100 loaded MEV per cell)

F- MEV-GFP protein
(200 loaded MEV per cell)

G- and H-MEV-GFP protein
(400 loaded MEV per cell)

FIG. 23

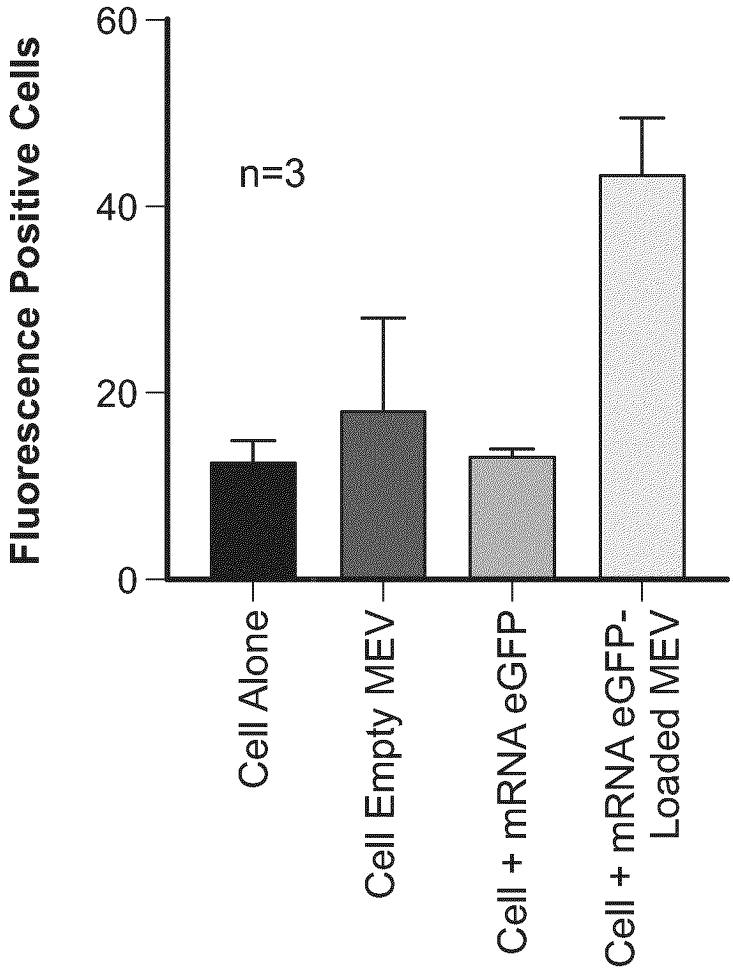
Fluorescence Positive Cells
60
40
20
0
n=3
Cell Alone
Cell Empty MEV
Cell + mRNA eGFP
Cell + mRNA eGFP-
Loaded MEV

FIG. 24

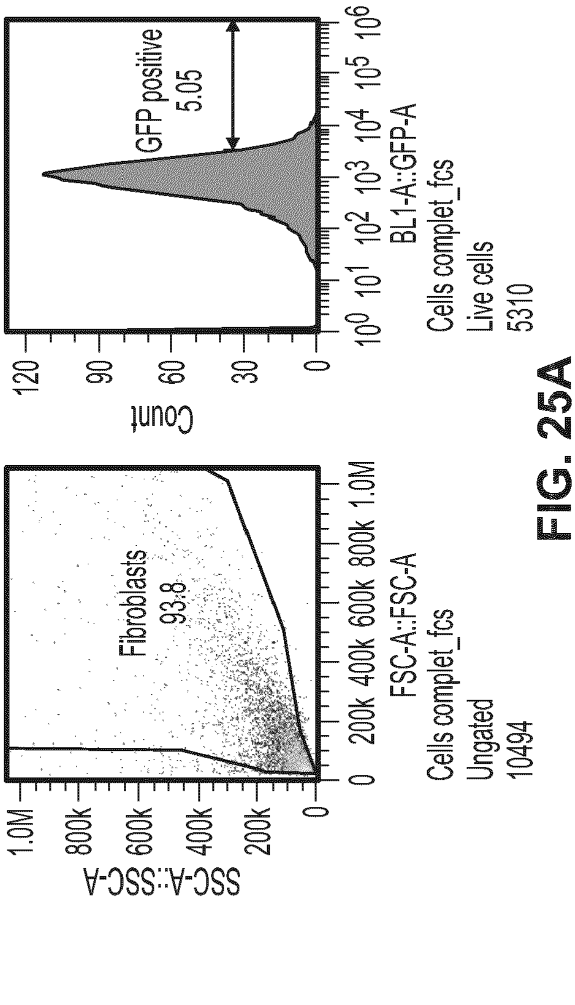


FIG. 25A

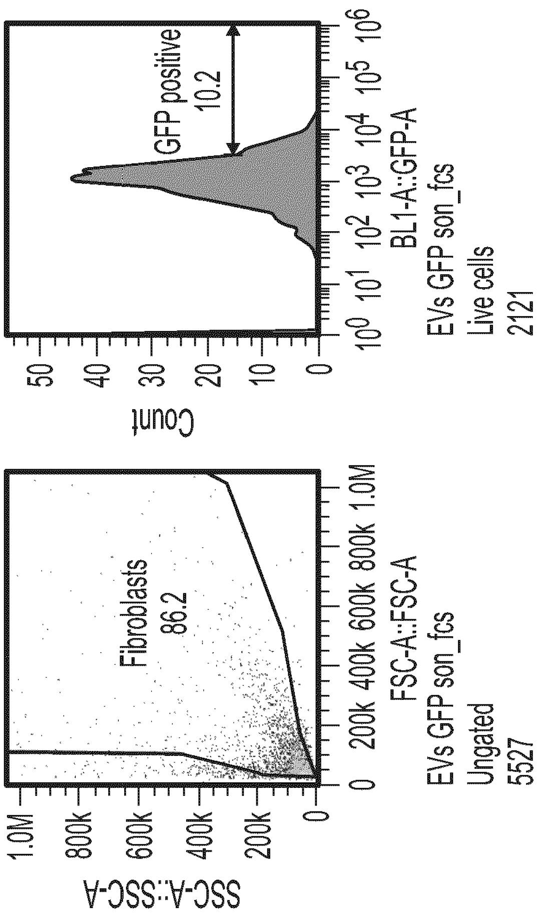


FIG. 25B

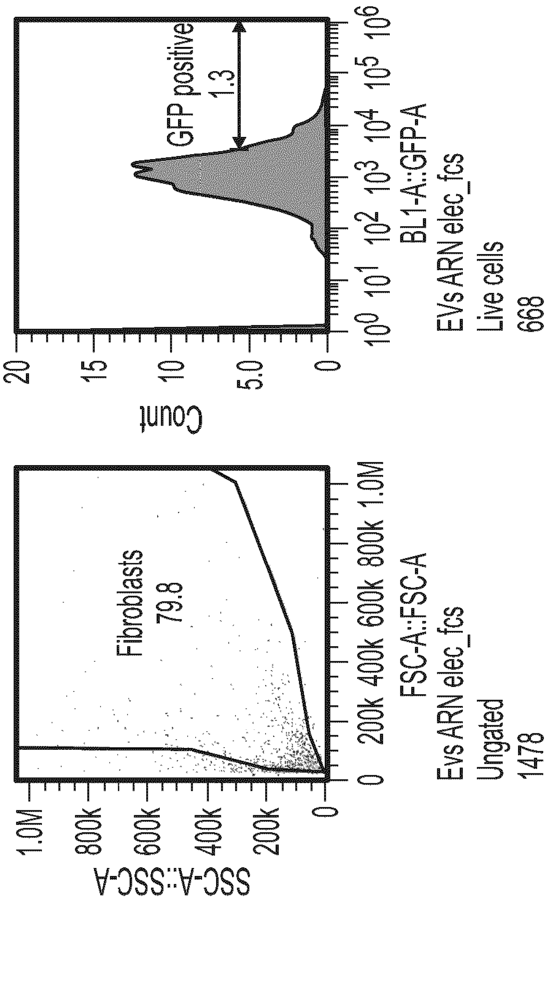


FIG. 25C

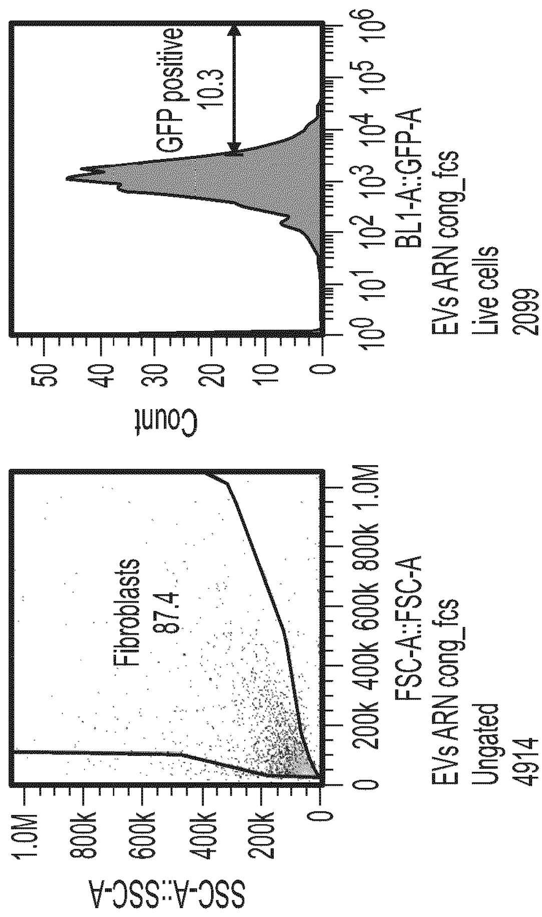


FIG. 25D

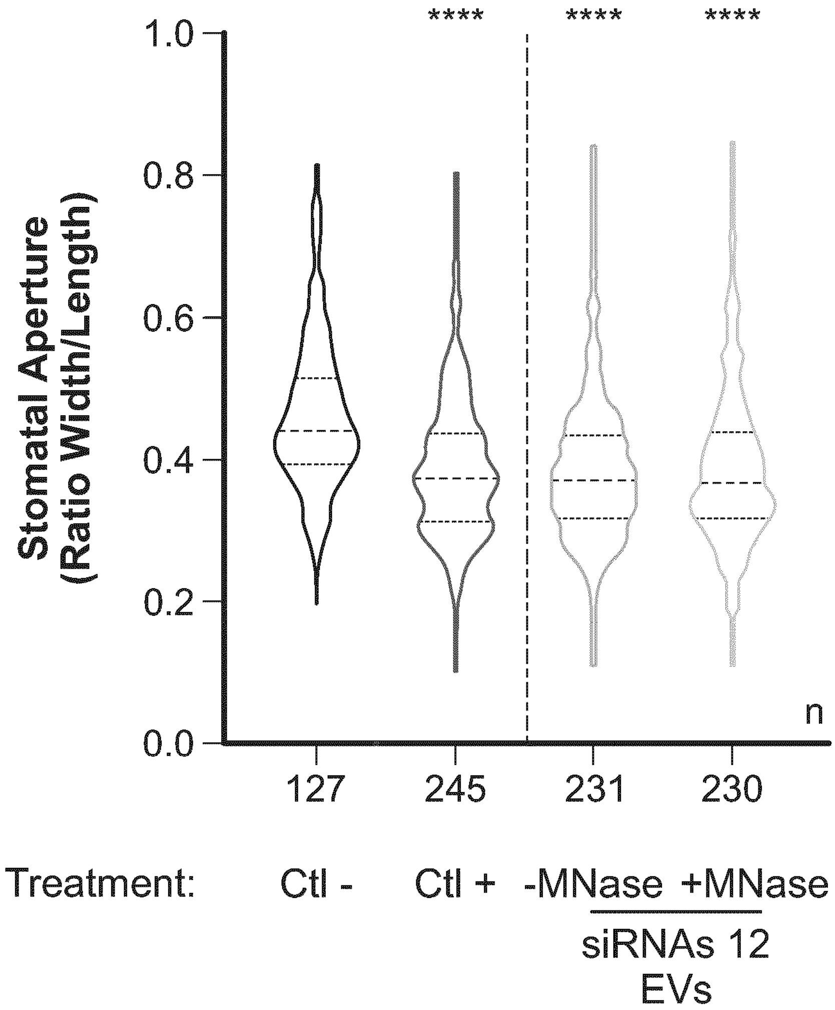
Stomatal Aperture
(Ratio Width/Length)
1.0
0.8
0.6
0.4
0.2
0.0
****
****
****
n
127
245
231
230
Treatment:
Ctl -
Ctl +
-MNase
+MNase
siRNAs 12
EVs

FIG. 26

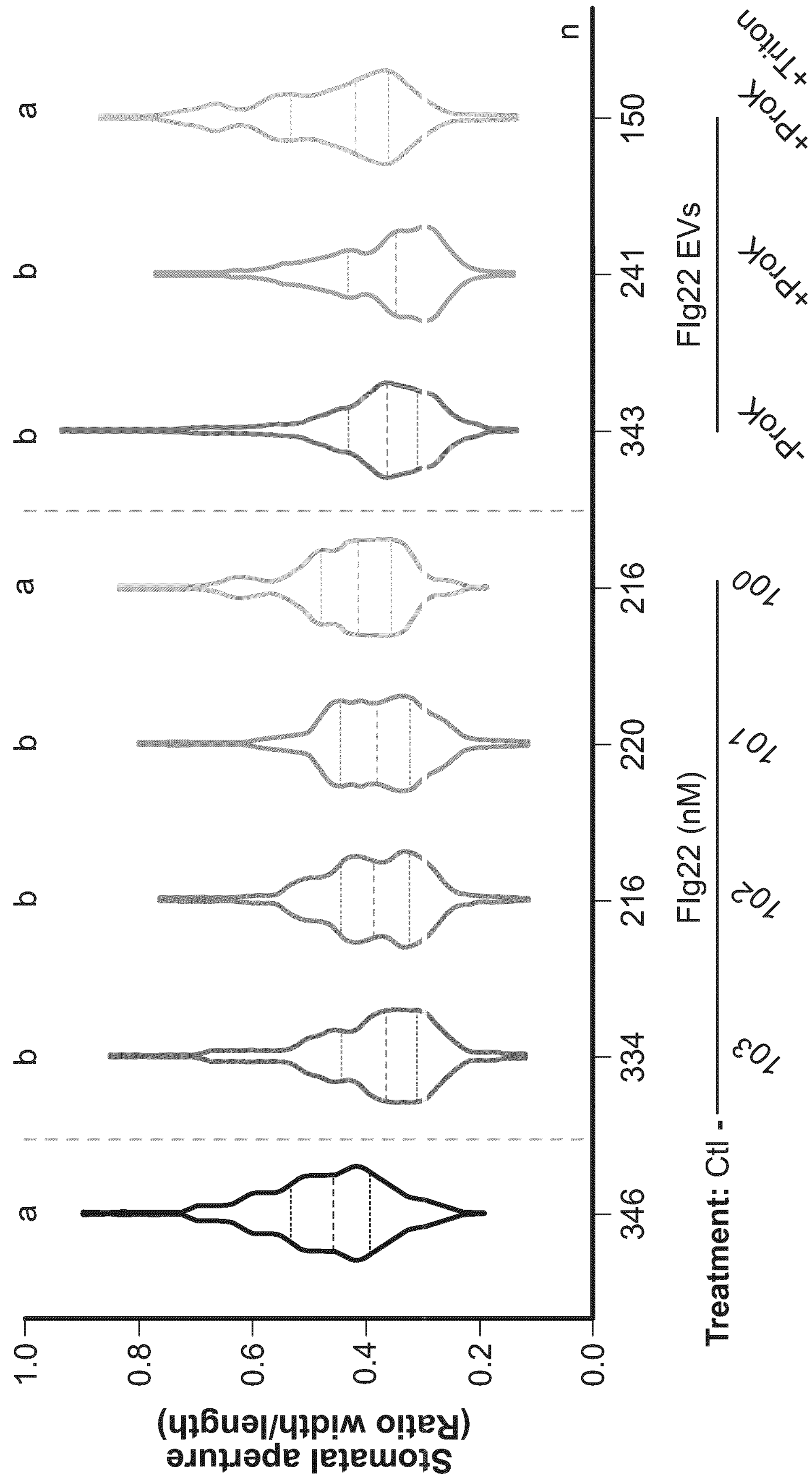
Stomatal aperture
(Ratio width/length)
1.0
0.8
0.6
0.4
0.2
0.0
a
b
b
b
a
b
b
a
n
346
334
216
220
216
343
241
150
Treatment: Ctl -
Flg22 (nM)
$10^3$
$10^2$
$10^1$
$10^0$
Flg22 EVs
-ProK
+ProK
+ProK +Triton

FIG. 27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 9464124 B
- WO 2009082606 A
- JP 2014240428 A
- WO 2011072292 A2
- WO 2010141724 A
- WO 2020097540 A
- CN 105821081 A
- CN 110699382 A
- US 10195290 B
- AU 2018365299
- US 5804396 A
- WO 9909016 A
- WO 9843960 A
- WO 9738983 A, Warner-Lambert
- WO 9906378 A, Warner-Lambert
- WO 9906396 A, Warner-Lambert
- WO 9630347 A
- WO 9633978 A
- WO 9633979 A, AstraZeneca
- WO 9633980 A
- EP 2019072169 W, Singla, Navarro
- EP 2019072170 W, Singla, Navarro.
- WO 2013048734 A
- WO 2006029161 A
- WO 2007022470 A
- WO 2007130604 A
- WO 2008021157 A
- WO 2009104051 A
- WO 2009142822 A
- WO 2019217459 A
- WO 2020123083 A
- EP 2504435 A
- US 20110223665 A
- US 20120116360 A
- US 20120071540 A
- US 20160257956 A
- US 20150196648 A
- US 20170304459 A
- WO 2011071860 A
- WO 2011072292 A
- WO 2013126803 A
- WO 2020035620 A
- AU 2004257373 A1
- AU 2013203219 B2
- AU 2016225873 A1
- EP 2395012 A
- EP 2888240 A
- US 20140256785 A
- US 20190032051 A
- JP 2018197239 A
- TW 201204351 A
- WO 2011068810 A
- WO 2019243574 A
- WO 2019092287 A
- WO 2020099682 A
- WO 2017161010 A
- WO 2019238626 A
- WO 2015110957 A2
- WO 2019018349 A1
- US 20120315324 A
- US 20190388347 A
- US 20190175506 A
- WO 2017203260 A
- WO 2018102397 A
- WO 2019081474 A
- WO 2019155060 A
- WO 2020041720 A
- AU 2018365299 A1
- SG 11201811149 T
- US 20190202892 A
- WO 2019155060 A1
- WO 2008087562 A
- US 5679377 A
- US 5916597 A
- US 5912015 A
- US 5989463 A
- US 5128326 A
- WO 9915154 A
- WO 9920253 A
- US 5323907 A
- US 5052558 A
- US 5033252 A
- WO 2020035619 A

### Non-patent literature cited in the description

- Computational Molecular Biology. Oxford University Press, 1988
- Biocomputing: Informatics and Genome Projects. Academic Press, 1993

- Computer Analysis of Sequence Data. Humana Press, 1994
- **HEINJE, G.** Sequence Analysis in Molecular Biology. Academic Press, 1987
- Sequence Analysis Primer. M Stockton Press, 1991
- **CARRILLO et al.** *SIAM J Applied Math*, 1988, vol. 48, 1073
- *J. Biol. Chem.*, 1968, vol. 243, 3557-59
- **WATSON et al.** Molecular Biology of the Gene. The Benjamin/Cummings Pub. Co, 1987, 224
- **NEEDLEMAN et al.** *J. Mol. Biol.*, 1970, vol. 48, 443
- *Adv. Appl. Math*, 1981, vol. 2, 482
- **GRIBSKOV et al.** *Nucl. Acids Res.*, 1986, vol. 14, 6745
- Atlas of Protein Sequence and Structure. National Biomedical Research Foundation, 1979, 353-358
- *Adv. Appl. Math.*, 1991, vol. 12, 337-357
- *Biochem.*, 1972, vol. 11 (9), 1726-1732
- **ADAMO et al.** *Journal of Extracellular Vesicles*, 2021, vol. 10, e12081
- **BARKIA et al.** *Mar. Drugs*, 2019, vol. 17 (5), 304
- **KURUVINASHETTI et al.** *20th International Conference on Nanotechnology*, 2020, 354-357
- **PICCIOTTO et al.** *Biomater.*, 2021
- **DOYLE** ; **WANG**. *Cells*, 2019, vol. 8 (7), 727
- **BREAKEFIELD**. *Mol. Neurobiol.*, 2016, vol. 36 (3), 301-312
- **ABELS** ; **BREAKEFIELD**. *Mol. Neurobiol.*, 2016, vol. 36 (3), 301-312
- **ADAMO et al.** *J. Extracell. Vesicles*, 2021, vol. 10, e12081
- **ABELS** ; **BREAKEFIELD**. *Cell Mol. Neurobiol.*, 2016, vol. 36 (3), 301-312
- **NAKASE** ; **FUTAKI**. *Sci. Rep*, 2015, vol. 5, 10112
- **MAAS et al.** *Trends Cell Biol.*, 2017, vol. 27 (3), 172-188
- **KANWAR et al.** *Lab Chip.*, 2014, vol. 14 (11), 1891-1900
- **ZHAO et al.** *Lab Chip.*, 2016, vol. 16 (3), 489-496
- **WANG** ; **BARR**. *Essays Biochem.*, 2018, vol. 62 (2), 205-213
- **WANG** ; **BARR**. *Cell Mol. Neurobiol.*, 2016, vol. 36 (3), 449-457
- **BLANC et al.** *Plant Cell*, 2010, vol. 22 (9), 2943-2955
- **RIOBOO et al.** *Aquat Toxicol*, 2009, vol. 94 (3), 229-37
- **CECCHIN et al.** *Plant J.*, 2019, vol. 100 (6), 1289-1305
- **HOVDE et al.** *Algal Research*, 2018, vol. 35, 449-461
- **GAO et al.** *BMC Genomics*, 2014, vol. 15 (1), 582
- **ARRIOLA et al.** *Plant J.*, 2018, vol. 93 (3), 566-586
- **GUARNIERI et al.** *Front. Bioeng. Biotechnol*, 2018, vol. 6, 37
- **TEH et al.** *Data Brief*, 2019, vol. 27, 104680
- **WANG et al.** *Mol. Biol. Evol.*, 2020, vol. 37 (3), 849-863
- **BLANC et al.** *Plant Cell*, 2010, vol. 22 (9), 2943
- **ZUÑIGA et al.** *Plant Physiol.*, 2016, vol. 172 (1), 589-602
- **CHA et al.** *World J. Microbiol. Biotechnol.*, 2012, vol. 28, 1771-1779
- **NARA et al.** *Int. J. Oncol.*, 2007, vol. 30 (5), 1189-1196
- **MAESHIMA et al.** *Nucleic Acid Ther.*, 2020, vol. 30 (4), 237-248
- **VEAS-PEREZ DE TUDELA et al.** *J. Neurochem.*, 2010, vol. 113 (4), 819-825
- **WATSON et al.** *Cancer Res.*, 1991, vol. 51 (15), 3996-4000
- **YOSHIKAWA et al.** *Mol. Ther. Methods Clin. Dev.*, 2019, vol. 13, 290-302
- **TSUJINO et al.** *Cancer Sci.*, 2019, vol. 110 (7), 2189-2199
- **TIRELLA et al.** *Int. J. Pharm.*, 2019, vol. 561, 114-123
- **NAKADA et al.** *Pancreatology*, 2001, vol. 1 (4), 314-319
- **ADAMS et al.** *Expert Opin. Ther. Targets*, 2015, vol. 20 (6), 737-753
- **POECK et al.** *Nat. Med.*, 2008, vol. 14 (11), 1256-1263
- **SZEGEDI et al.** *Pathol. Oncol. Res.*, 2008, vol. 14 (3), 275-279
- **RIPOLL et al.** *RSC Adv.*, 2018, vol. 8, 20758-20763
- **LIU et al.** *BMC Cancer*, 2012, vol. 12, 519
- **SPÄNKUCH et al.** *Neoplasia*, 2008, vol. 10 (3), 223-234
- **CHAMBRAUD et al.** *FASEB J.*, 2007, vol. 21 (11), 2787-97
- **CHABRAUD et al.** *Proc. Natl. Acad. Sci. U.S.A.*, 2010, vol. 107 (6), 2658-63
- **CHAMBRAUD et al.** *Proc. Natl. Acad. Sci. U.S.A.*
- **FIRE et al.** *Nature*, 1998, vol. 391 (6669), 806-11
- **FIRE** ; **MELLO**. *Nobel Prize in Medicine awarded to Andrew Fire and Craig Mello*, 2006
- **SINGLA et al.** *bioRxiv*, 2019

- **BAULCOMBE**. *Current Opinion in Plant Biology*, 2015, vol. 26, 141-146
- **TENG et al.** *Cell Host & Microbes*, 2018, vol. 24, 637-652
- **SUNDARAM et al.** *iScience*, 2019, vol. 21, 308-327
- **GYÖRGY et al.** *Annu. Rev. Pharmacol. Toxicol.*, 2015, vol. 55, 439-464
- **SANDER** ; **JOUNG**. *Nat. Biotechnol.*, 2014, vol. 32 (4), 347-355
- **FONSECA FERREIRA, A.** ; **GOMES, D**. *Bioengineering (Basel)*, 2019, vol. 6 (1), 4
- **CHAMBRAUD et al.** *Proc. Natl. Acad. Sci. U.S.A.*, 2010, vol. 107 (6), 2658-63
- **LU et al.** RNA silencing in plants by the expression of siRNA duplexes.. *Nucleic Acids Research*, 2004, vol. 32 (21), e171
- **LU et al.** *Nucleic Acids Research*, 2004, vol. 32 (21), e171
- Ansel Introduction to Pharmaceutical Dosage Forms,. 1985, 126
- Goodman and Gilman's: The Pharmacological Bases of Therapeutics,. Pergamon Press, 1990
- Remington's Pharmaceutical Sciences. Mack Publishing Co, 1990
- Pharmaceutical Dosage Forms: Parenteral Medications. Dekker, 1993
- Pharmaceutical Dosage Forms: Tablets. Dekker, 1990
- Pharmaceutical Dosage Forms: Disperse Systems. Dekker, 1990
- Remington: The Science and Practice of Pharmacy (Formerly Remington's Pharmaceutical Sciences. Mack Publishing Company, 1995
- **ALFONSO R. GENNARO**. Remington: The Science and Practice of Pharmacy. Lippincott Williams & Wilkins, 2000
- Remington's Pharmaceutical Sciences. Mack Publishing Co
- Medical Applications of Controlled Release. CRC Pres., Boca Raton, 1974
- Controlled Drug Bioavailability, Drug Product Design and Performance. Wiley, 1984
- **LANGER** ; **PEPPAS**. *J. Macromol. Sci.*, 1983, vol. 23, 61
- **LEVY et al.** *Science*, 1985, vol. 228, 190
- **DURING et al.** *Ann. Neurol.*, 1989, vol. 25, 351
- **HOWARD et al.** *J. Neurosurg.*, 1989, vol. 71, 105
- **WIKLANDER et al.** *J. Extracellular Vesicles*, 2015, vol. 4, 26316
- **VAN DER MEEL et al.** *J. Control. Release*, 2014, vol. 195, 72-85
- **LAI C.P. et al.** *ACS Nano*, 2014, vol. 8, 483-494
- **ZHUANG et al.** *Mol. Ther*, 2011, vol. 19, 1769-1779
- **CHENG et al.** *Protein Cell*, 2019, vol. 10, 295-299
- **SAMUEL et al.** *Nat Commun*, 2021, vol. 12, 3950
- **ZHONG et al.** *Biomaterials*, 2021, vol. 277, 121126
- **CESTA**. *Toxicologic Pathology*, 2006, vol. 34, 455-465
- Targeting Immunomodulatory Agents to the Gut-Associated Lymphoid Tissue. **ZGAIR** ; **SPRINGER et al.** Neuro-Immuno-Gastroenterology. 2016
- **LUONGO et al.** *Current perspectives. International Reviews of Immunology*, 2009, vol. 28 (6), 446-464
- **MACKAY et al.** *J Exp Med*, 1990, vol. 171, 801-17
- **CECCHIN et al.** *The Plant Journal*, 2019, vol. 100, 1289-1305
- **KANG et al.** Biodistribution of extracellular vesicles following administration into animals: A systematic review.. *J Extracell Vesicles.*, 2021, vol. 10, e12085
- **PFAFFL**. *Nucleic Acids Res.*, 2001, vol. 29 (9), e45
- **KOCH A et al.** *J Phytopathol*, 2012, vol. 160 (10), 606-610
- **BROOKS et al.** 17. *Mol Plant Microbe Interact.*, 2004 (2), 162-174
- **FOUTS et al.** *Proc. Natl. Acad. Sci. U.S.A.*, 2004, vol. 99, 2275-2280
- **SREEDHARAN et al.** *Molecular plant-microbe interactions MPMI*, 2006, vol. 19, 768-779
- **KOCH et al.** *Proc. Natl. Acad. Sci. U.S.A.*, 2013, vol. 110, 19324-19329
- **KOCH et al.** *PLOS Pathogens*, 2016, vol. 12 (10), e1005901